(19) Europäisches Patentamt
European Patent Office
Office européen des brevets

(11) EP 3 786 177 A1

(12) # EUROPEAN PATENT APPLICATION
published in accordance with Art. 153(4) EPC

(43) Date of publication:
**03.03.2021 Bulletin 2021/09**

(21) Application number: **19791949.1**

(22) Date of filing: **25.04.2019**

(51) Int Cl.:
***C07K 14/705*** *(2006.01)*
***C07K 14/395*** *(2006.01)*
***C07K 14/47*** *(2006.01)*
***C07K 16/28*** *(2006.01)*
***C12N 5/00*** *(2006.01)*
***A61K 35/12*** *(2015.01)*

(86) International application number:
**PCT/KR2019/005020**

(87) International publication number:
**WO 2019/209051 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2018 KR 20180048486**

(71) Applicant: **Paean Biotechnology Inc.**
**Daejeon 34028 (KR)**

(72) Inventors:
- **HAN, Kyuboem**
  **Daejeon 34028 (KR)**
- **KIM, Chun-Hyung**
  **Daejeon 34028 (KR)**
- **KIM, Yu Jin**
  **Daejeon 34028 (KR)**
- **YU, Shin-Hye**
  **Daejeon 34028 (KR)**
- **KIM, Nayoung**
  **Daejeon 34028 (KR)**
- **KIM, Mi Jin**
  **Daejeon 34028 (KR)**
- **CHOI, Yong-Soo**
  **Daejeon 34028 (KR)**
- **LEE, Seo Eun**
  **Daejeon 34028 (KR)**

(74) Representative: **HGF**
**1 City Walk**
**Leeds LS11 9DX (GB)**

(54) **MODIFIED MITOCHONDRIA AND USE THEREOF**

(57) Mitochondria modified by a targeting protein, according to one embodiment of the present invention, can be effectively delivered to a target. In addition, when a protein of interest bound to the modified mitochondria is delivered into a cell, various activities can be exhibited. The modified mitochondria can effectively cause cancer tissue death, and thus can also be used as an anticancer agent. Furthermore, various activities are exhibited according to a protein of interest loaded on modified mitochondria, and thus the modified mitochondria can be applied in the treatment of various diseases. Additionally, a fusion protein comprising a protein of interest and a fusion protein comprising a targeting protein, according to one embodiment of the present invention, can be used in order to modify mitochondria. Moreover, mitochondria modified with the fusion proteins exhibits various effects in a target cell.

FIG. 6

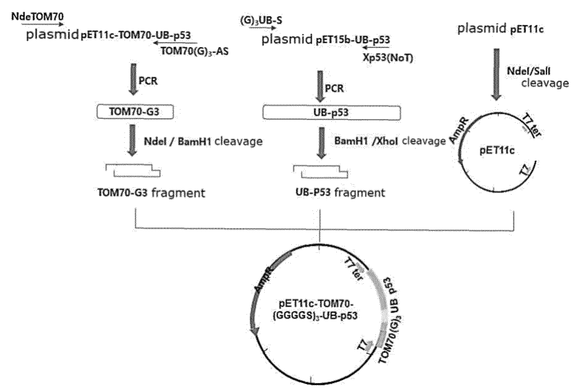


EP 3 786 177 A1

<110> Paean Biotechnology Inc.

<120> MODIFIED MITOCHONDRIA AND USE THEREOF

<130> PCB903024PBT/PCT

<150> KR 10-2018-0048486
<151> 2018-04-26

<160> 92

<170> KoPatentIn 3.0

<210> 1
<211> 39
<212> DNA
<213> Artificial Sequence

<220>
<223> T2p53 primer

<400> 1
aaaaaaccgc ggtggtgagg agccgcagtc agatcctag 39

<210> 2
<211> 35
<212> DNA
<213> Artificial Sequence

<220>
<223> Xp53 primer

<400> 2
aaaaaactcg agtgagtctg agtcaggccc ttctg 35

<210> 3
<211> 1186
<212> DNA
<213> Artificial Sequence

<220>
<223> nucleotides sequence coding p53

<400> 3
ccgcggtggt gaggagccgc agtcagatcc tagcgtcgag ccccctctga gtcaggaaac 60

attttcagac ctgtggaaac tacttcctga aaacaacgtt ctgtccccct tgccgtccca 120

agcaatggat gatttgatgc tgtccccgga cgatattgaa caatggttca ctgaagaccc 180

aggtccagat gaagctccca gaatgccaga ggctgctccc cgcgtggccc ctgcaccagc 240

agctcctaca ccggcggccc ctgcaccagc cccctcctgg cccctgtcat cttctgtccc 300

ttcccagaaa acctaccagg gcagctacgg tttccgtctg ggcttcttgc attctgggac 360

agccaagtct gtgacttgca cgtactcccc tgccctcaac aagatgtttt gccaactggc 420

caagacctgc cctgtgcagc tgtgggttga ttccacaccc ccgcccggca cccgcgtccg 480

cgccatggcc atctacaagc agtcacagca catgacggag gttgtgaggc gctgccccca 540

ccatgagcgc tgctcagata gcgatggtct ggcccctcct cagcatctta tccgagtgga 600

aggaaatttg cgtgtggagt atttggatga cagaaacact tttcgacata gtgtggtggt 660

gccctatgag ccgcctgagg ttggctctga ctgtaccacc atccactaca actacatgtg 720

taacagttcc tgcatgggcg gcatgaaccg gaggcccatc ctcaccatca tcacactgga 780

agactccagt ggtaatctac tgggacggaa cagctttgag gtgcgtgttt gtgcctgtcc 840

tgggagagac cggcgcacag aggaagagaa tctccgcaag aaaggggagc ctcaccacga 900

gctgccccca gggagcacta agcgagcact gcccaacaac accagctcct ctccccagcc 960

aaagaagaaa ccactggatg gagaatattt cacccttcag atccgtgggc gtgagcgctt 1020

cgagatgttc cgagagctga atgaggcctt ggaactcaag gatgcccagg ctgggaagga 1080

gccagggggg agcagggctc actccagcca cctgaagtcc aaaaagggtc agtctacctc 1140

ccgccataaa aaactcatgt tcaagacaga agggcctgac tcagac 1186

<210> 4
<211> 35
<212> DNA
<213> Artificial Sequence

<220>
<223> NdeUB primer

<400> 4
ggattccata tgcaactttt cgtcaaaact ctaac 35

<210> 5
<211> 27
<212> DNA
<213> Artificial Sequence

<220>
<223> T2UB primer

<400> 5
atgaccaccg cggagtctca acaccaa 27

<210> 6
<211> 1460
<212> DNA
<213> Artificial Sequence

<220>
<223> nucleotides sequence coding UB-p53

<400> 6
atgggcagca gccatcatca tcatcatcac agcagcggcc tggtgccgcg cggcagccat 60

atgcaaatct tcgtcaaaac tctaacaggg aagactataa ccctagaggt tgaaccatcc 120

gacactattg aaaacgtcaa agctaaaatt caagataaag aaggtatccc tccggatcag 180

cagagattga tttttgctgg taagcaacta gaagatggta gaaccttgtc tgactacaac 240

atccaaaagg aatctactct tcacttggtg ttgagactcc gcggtggtga ggagccgcag 300

tcagatccta gcgtcgagcc ccctctgagt caggaaacat tttcagacct gtggaaacta 360
cttcctgaaa acaacgttct gtcccccttg ccgtcccaag caatggatga tttgatgctg 420
tccccggacg atattgaaca atggttcact gaagacccag gtccagatga agctcccaga 480
atgccagagg ctgctccccg cgtggcccct gcaccagcag ctcctacacc ggcggcccct 540
gcaccagccc cctcctggcc cctgtcatct tctgtccctt cccagaaaac ctaccagggc 600
agctacggtt tccgtctggg cttcttgcat tctgggacag ccaagtctgt gacttgcacg 660
tactcccctg ccctcaacaa gatgttttgc caactggcca agacctgccc tgtgcagctg 720
tgggttgatt ccacaccccc gcccggcacc cgcgtccgcg ccatggccat ctacaagcag 780
tcacagcaca tgacggaggt tgtgaggcgc tgcccccacc atgagcgctg ctcagatagc 840
gatggtctgg cccctcctca gcatcttatc cgagtggaag gaaatttgcg tgtggagtat 900
ttggatgaca gaaacacttt tcgacatagt gtggtggtgc cctatgagcc gcctgaggtt 960
ggctctgact gtaccaccat ccactacaac tacatgtgta acagttcctg catgggcggc 1020
atgaaccgga ggcccatcct caccatcatc acactggaag actccagtgg taatctactg 1080
ggacggaaca gctttgaggt gcgtgtttgt gcctgtcctg ggagagaccg gcgcacagag 1140
gaagagaatc tccgcaagaa aggggagcct caccacgagc tgcccccagg gagcactaag 1200
cgagcactgc ccaacaacac cagctcctct ccccagccaa agaagaaacc actggatgga 1260
gaatatttca cccttcagat ccgtgggcgt gagcgcttcg agatgttccg agagctgaat 1320
gaggccttgg aactcaagga tgcccaggct gggaaggagc caggggggag cagggctcac 1380
tccagccacc tgaagtccaa aaagggtcag tctacctccc gccataaaaa actcatgttc 1440
aagacagaag ggcctgatag 1460

<210> 7
<211> 59
<212> DNA
<213> Artificial Sequence

<220>
<223> NdeTOM70 primer

<400> 7
gaattccata tgaaaagttt tataactcgg aataaaactg caattttcgc aactgttgc 59

<210> 8
<211> 38
<212> DNA
<213> Artificial Sequence

<220>
<223> TOM70-AS primer

<400> 8
ggtgcatact actattatca aacttttcgt caaaactc 38

<210> 9

<211> 36
<212> DNA
<213> Artificial Sequence

<220>
<223> TOM70UB-S primer

<400> 9
ggctacgtat ttatttccaa cttttcgtca aaactc 36

<210> 10
<211> 24
<212> DNA
<213> Artificial Sequence

<220>
<223> T2UB-AS primer

<400> 10
ggcaccaccg cggagtctca acac 24

<210> 11
<211> 1515
<212> DNA
<213> Artificial Sequence

<220>
<223> nucleotides sequence coding TOM70-UB-p53

<400> 11
atgaaaagtt ttataactcg gaataaaact gcaattttcg caactgttgc tgctacggga 60

accgctattg gtgcatacta ctattatcaa atcttcgtca aaactctaac agggaagact 120

ataaccctag aggttgaacc atccgacact attgaaaacg tcaaagctaa aattcaagat 180

aaagaaggta tccctccgga tcagcagaga ttgatttttg ctggtaagca actagaagat 240

ggtagaacct tgtctgacta caacatccaa aaggaatcta ctcttcactt ggtgttgaga 300

ctccgcggtg gtgaggagcc gcagtcagat cctagcgtcg agccccctct gagtcaggaa 360

acattttcag acctgtggaa actacttcct gaaaacaacg ttctgtcccc cttgccgtcc 420

caagcaatgg atgatttgat gctgtccccg gacgatattg aacaatggtt cactgaagac 480

ccaggtccag atgaagctcc cagaatgcca gaggctgctc cccgcgtggc ccctgcacca 540

gcagctccta caccggcggc ccctgcacca gccccctcct ggcccctgtc atcttctgtc 600

ccttcccaga aaacctacca gggcagctac ggtttccgtc tgggcttctt gcattctggg 660

acagccaagt ctgtgacttg cacgtactcc cctgccctca acaagatgtt ttgccaactg 720

gccaagacct gccctgtgca gctgtgggtt gattccacac cccgcccggg cacccgcgtc 780

cgcgccatgg ccatctacaa gcagtcacag cacatgacgg aggttgtgag gcgctgcccc 840

caccatgagc gctgctcaga tagcgatggt ctggcccctc ctcagcatct tatccgagtg 900

gaaggaaatt tgcgtgtgga gtatttggat gacagaaaca cttttcgaca tagtgtggtg 960

gtgccctatg agccgcctga ggttggctct gactgtacca ccatccacta caactacatg 1020

tgtaacagtt cctgcatggg cggcatgaac cggaggccca tcctcaccat catcacactg 1080

gaagactcca gtggtaatct actgggacgg aacagctttg aggtgcgtgt ttgtgcctgt 1140

cctgggagag accggcgcac agaggaagag aatctccgca agaaagggga gcctcaccac 1200

gagctgcccc cagggagcac taagcgagca ctgcccaaca acaccagctc ctctccccag 1260

ccaaagaaga aaccactgga tggagaatat ttcacccttc agatccgtgg gcgtgagcgc 1320

ttcgagatgt tccgagagct gaatgaggcc ttggaactca aggatgccca ggctgggaag 1380

gagccagggg ggagcagggc tcactccagc cacctgaagt ccaaaaaggg tcagtctacc 1440

tcccgccata aaaaactcat gttcaagaca gaagggcctg actcagacct cgagcaccac 1500

caccaccacc actag 1515

<210> 12
<211> 59
<212> DNA
<213> Artificial Sequence

<220>
<223> TOM70(G)3-AS primer

<400> 12
gcccccggat cctccacccc cgcttccgcc acctccataa tagtagtatg caccaatag 59

<210> 13
<211> 32
<212> DNA
<213> Artificial Sequence

<220>
<223> (G)3UB-S primer

<400> 13
ggtggaggat ccgggggcgc ggaagccaaa tc 32

<210> 14
<211> 32
<212> DNA
<213> Artificial Sequence

<220>
<223> Xp53(noT) primer

<400> 14
aaaaaactcg aggtctgagt caggcccttc tg 32

<210> 15
<211> 1560
<212> DNA
<213> Artificial Sequence

<220>
<223> nucleotides sequence coding TOM70-(GGGGS)3-UB-p53

<400> 15
atgaaaagtt ttataactcg gaataaaact gcaattttcg caactgttgc tgctacggga 60
accgctattg gtgcatacta ctattatgga ggtggcggaa gcgggggtgg aggatccggg 120
ggcggcggaa gccaaatctt cgtcaaaact ctaacaggga agactataac cctagaggtt 180
gaaccatccg acactattga aaacgtcaaa gctaaaattc aagataaaga aggtatccct 240
ccggatcagc agagattgat tttgctggt aagcaactag aagatggtag aaccttgtct 300
gactacaaca tccaaaagga atctactctt cacttggtgt tgagactccg cggtggtgag 360
gagccgcagt cagatcctag cgtcgagccc cctctgagtc aggaaacatt ttcagacctg 420
tggaaactac ttcctgaaaa caacgttctg tccccttgc cgtcccaagc aatggatgat 480
ttgatgctgt ccccggacga tattgaacaa tggttcactg aagacccagg tccagatgaa 540
gctcccagaa tgccagaggc tgctccccgc gtggcccctg caccagcagc tcctacaccg 600
gcggcccctg caccagcccc ctcctggccc ctgtcatctt ctgtcccttc ccagaaaacc 660
taccagggca gctacggttt ccgtctgggc ttcttgcatt ctgggacagc caagtctgtg 720
acttgcacgt actcccctgc cctcaacaag atgttttgcc aactggccaa gacctgccct 780
gtgcagctgt gggttgattc cacacccccg cccggcaccc gcgtccgcgc catggccatc 840
tacaagcagt cacagcacat gacggaggtt gtgaggcgct gcccccacca tgagcgctgc 900
tcagatagcg atggtctggc ccctcctcag catcttatcc gagtggaagg aaatttgcgt 960
gtggagtatt tggatgacag aaacactttt cgacatagtg tggtggtgcc ctatgagccg 1020
cctgaggttg gctctgactg taccaccatc cactacaact acatgtgtaa cagttcctgc 1080
atgggcggca tgaaccggag gcccatcctc accatcatca cactggaaga ctccagtggt 1140
aatctactgg gacggaacag ctttgaggtg cgtgtttgtg cctgtcctgg gagagaccgg 1200
cgcacagagg aagagaatct ccgcaagaaa ggggagcctc accacgagct gcccccaggg 1260
agcactaagc gagcactgcc caacaacacc agctcctctc cccagccaaa gaagaaacca 1320
ctggatggag aatatttcac ccttcagatc cgtgggcgtg agcgcttcga gatgttccga 1380
gagctgaatg aggccttgga actcaaggat gcccaggctg ggaaggagcc aggggggagc 1440
agggctcact ccagccacct gaagtccaaa aagggtcagt ctacctcccg ccataaaaaa 1500
ctcatgttca agacagaagg gcctgactca gacctcgagc accaccacca ccaccactag 1560
1560

<210> 16
<211> 51
<212> DNA
<213> Artificial Sequence

<220>
<223> B(G)3p53

<400> 16
ggtggaggat ccgggggcgg cggaagcgag gagccgcagt cagatcctag c 51

<210> 17
<211> 1335
<212> DNA
<213> Artificial Sequence

<220>
<223> nucleotides sequence coding TOM70-(GGGGS)3-p53

<400> 17
atgaaaagtt ttataactcg gaataaaact gcaattttcg caactgttgc tgctacggga 60

accgctattg gtgcatacta ctattatgga ggtggcggaa gcgggggtgg aggatccggg 120

ggcggcggaa gcgaggagcc gcagtcagat cctagcgtcg agccccctct gagtcaggaa 180

acattttcag acctgtggaa actacttcct gaaaacaacg ttctgtcccc cttgccgtcc 240

caagcaatgg atgatttgat gctgtccccg gacgatattg aacaatggtt cactgaagac 300

ccaggtccag atgaagctcc cagaatgcca gaggctgctc cccgcgtggc ccctgcacca 360

gcagctccta caccggcggc ccctgcacca gcccctcct ggcccctgtc atcttctgtc 420

ccttcccaga aaacctacca gggcagctac ggtttccgtc tgggcttctt gcattctggg 480

acagccaagt ctgtgacttg cacgtactcc cctgccctca acaagatgtt ttgccaactg 540

gccaagacct gccctgtgca gctgtgggtt gattccacac ccccgcccgg cacccgcgtc 600

cgcgccatgg ccatctacaa gcagtcacag cacatgacgg aggttgtgag gcgctgcccc 660

caccatgagc gctgctcaga tagcgatggt ctggcccctc ctcagcatct tatccgagtg 720

gaaggaaatt tgcgtgtgga gtatttggat gacagaaaca cttttcgaca tagtgtggtg 780

gtgccctatg agccgcctga ggttggctct gactgtacca ccatccacta caactacatg 840

tgtaacagtt cctgcatggg cggcatgaac cggaggccca tcctcaccat catcacactg 900

gaagactcca gtggtaatct actgggacgg aacagctttg aggtgcgtgt ttgtgcctgt 960

cctgggagag accggcgcac agaggaagag aatctccgca agaaagggga gcctcaccac 1020

gagctgcccc cagggagcac taagcgagca ctgcccaaca acaccagctc ctctccccag 1080

ccaaagaaga aaccactgga tggagaatat ttcacccttc agatccgtgg gcgtgagcgc 1140

ttcgagatgt tccgagagct gaatgaggcc ttggaactca aggatgccca ggctgggaag 1200

gagccagggg ggagcagggc tcactccagc cacctgaagt ccaaaaaggg tcagtctacc 1260

tcccgccata aaaaactcat gttcaagaca gaagggcctg actcagacct cgagcaccac 1320

caccaccacc actag 1335

<210> 18
<211> 32
<212> DNA
<213> Artificial Sequence

<220>
<223> Xp53(noT) primer

<400> 18

aaaaaactcg aggtctgagt caggcccttc tg 32

<210> 19
<211> 36
<212> DNA
<213> Artificial Sequence

<220>
<223> XTOM7 primer

<400> 19
aaaaaactcg agtttgccat tcgctggggc tttatc 36

<210> 20
<211> 38
<212> DNA
<213> Artificial Sequence

<220>
<223> LTOM7 primer

<400> 20
aaaaaagtcg acttatcccc aaagtaggct caaaacag 38

<210> 21
<211> 1578
<212> DNA
<213> Artificial Sequence

<220>
<223> nucleotides sequence coding UB-p53-TOM7

<400> 21
atgggcagca gccatcatca tcatcatcac agcagcggcc tggtgccgcg cggcagccat 60

atgcaaatct tcgtcaaaac tctaacaggg aagactataa ccctagaggt tgaaccatcc 120

gacactattg aaaacgtcaa agctaaaatt caagataaag aaggtatccc tccggatcag 180

cagagattga tttttgctgg taagcaacta gaagatggta gaaccttgtc tgactacaac 240

atccaaaagg aatctactct tcacttggtg ttgagactcc gcggtggtga ggagccgcag 300

tcagatccta gcgtcgagcc ccctctgagt caggaaacat tttcagacct atggaaacta 360

cttcctgaaa acaacgttct gtcccccttg ccgtcccaag caatggatga tttgatgctg 420

tccccggacg atattgaaca atggttcact gaagacccag gtccagatga agctcccaga 480

atgccagagg ctgctccccg cgtggcccct gcaccagcag ctcctacacc ggcggcccct 540

gcaccagccc cctcctggcc cctgtcatct tctgtccctt cccagaaaac ctaccagggc 600

agctacggtt tccgtctggg cttcttgcat tctgggacag ccaagtctgt gacttgcacg 660

tactcccctg ccctcaacaa gatgttttgc caactggcca agacctgccc tgtgcagctg 720

tgggttgatt ccacaccccc gcccggcacc cgcgtccgcg ccatggccat ctacaagcag 780

tcacagcaca tgacggaggt tgtgaggcgc tgcccccacc atgagcgctg ctcagatagc 840

gatggtctgg cccctcctca gcatcttatc cgagtggaag gaaatttgcg tgtggagtat 900

ttggatgaca gaaacacttt tcgacatagt gtggtggtgc cctatgagcc gcctgaggtt 960

ggctctgact gtaccaccat ccactacaac tacatgtgta acagttcctg catgggcggc 1020

atgaaccgga ggcccatcct caccatcatc acactggaag actccagtgg taatctactg 1080

ggacggaaca gctttgaggt gcatgtttgt gcctgtcctg ggagagaccg gcgcacagag 1140

gaagagaatc tccgcaagaa aggggagcct caccacgagc tgcccccagg gagcactaag 1200

cgagcactgt ccaacaacac cagctcctct ccccagccaa agaagaaacc actggatgga 1260

gaatatttca cccttcagat ccgtgggcgt gagcgcttcg agatgttccg agagctgaat 1320

gaggccttgg aactcaagga tgcccaggct gggaaggagc caggggggag cagggctcac 1380

tccagccacc tgaagtccaa aaagggtcag tctacctccc gccataaaaa actcatgttc 1440

aagacagaag ggcctgactc agacctcgag tttgccattc gctggggctt tatccctctt 1500

gtgatttacc tgggatttaa gaggggtgca gatcccggaa tgcctgaacc aactgttttg 1560

agcctacttt ggggatag 1578

<210> 22
<211> 35
<212> DNA
<213> Artificial Sequence

<220>
<223> Rp53 primer

<400> 22
aaaaaagaat tcatggtctg agtcaggccc ttctg 35

<210> 23
<211> 1266
<212> DNA
<213> Artificial Sequence

<220>
<223> nucleotides sequence coding p53-myc/His

<400> 23
atggaggagc cgcagtcaga tcctagcgtc gagccccctc tgagtcagga aacattttca 60

gacctgtgga aactacttcc tgaaaacaac gttctgtccc ccttgccgtc ccaagcaatg 120

gatgatttga tgctgtcccc ggacgatatt gaacaatggt tcactgaaga cccaggtcca 180

gatgaagctc ccagaatgcc agaggctgct ccccgcgtgg cccctgcacc agcagctcct 240

acaccggcgg cccctgcacc agccccctcc tggcccctgt catcttctgt cccttcccag 300

aaaacctacc agggcagcta cggtttccgt ctgggcttct tgcattctgg gacagccaag 360

tctgtgactt gcacgtactc ccctgccctc aacaagatgt tttgccaact ggccaagacc 420

tgccctgtgc agctgtgggt tgattccaca cccccgcccg gcacccgcgt ccgcgccatg 480

gccatctaca agcagtcaca gcacatgacg gaggttgtga ggcgctgccc ccaccatgag 540

cgctgctcag atagcgatgg tctggcccct cctcagcatc ttatccgagt ggaaggaaat 600

ttgcgtgtgg agtatttgga tgacagaaac acttttcgac atagtgtggt ggtgccctat 660

gagccgcctg aggttggctc tgactgtacc accatccact acaactacat gtgtaacagt 720

tcctgcatgg gcggcatgaa ccggaggccc atcctcacca tcatcacact ggaagactcc 780

agtggtaatc tactgggacg gaacagcttt gaggtgcgtg tttgtgcctg tcctgggaga 840

gaccggcgca cagaggaaga gaatctccgc aagaaagggg agcctcacca cgagctgccc 900

ccagggagca ctaagcgagc actgcccaac aacaccagct cctctcccca gccaaagaag 960

aaaccactgg atggagaata tttcaccctt cagatccgtg ggcgtgagcg cttcgagatg 1020

ttccgagagc tgaatgaggc cttggaactc aaggatgccc aggctgggaa ggagccaggg 1080

gggagcaggg ctcactccag ccacctgaag tccaaaaagg gtcagtctac ctcccgccat 1140

aaaaaactca tgttcaagac agaagggcct gactcagacc tcgagtctag agggcccttc 1200

gaacaaaaac tcatctcaga agaggatctg aatatgcata ccggtcatca tcaccatcac 1260

cattga 1266

<210> 24
<211> 51
<212> DNA
<213> Artificial Sequence

<220>
<223> T2GZMB primer

<400> 24
aaaaaaccgc ggtggtatca tcgggggaca tgaggcacat gaggccaagc c 51

<210> 25
<211> 38
<212> DNA
<213> Artificial Sequence

<220>
<223> XGZMB(noT) primer

<400> 25
aaaaaactcg aggtagcgtt tcatggtttt ctttatcc 38

<210> 26
<211> 691
<212> DNA
<213> Artificial Sequence

<220>
<223> nucleotides sequence coding GranzymeB

<400> 26
ccgcggtggt atcatcgggg gacatgaggc caagccccac tcccgcccct acatggctta 60

tcttatgatc tgggatcaga agtctctgaa gaggtgcggt ggcttcctga tacaagacga 120

cttcgtgctg acagctgctc actgttgggg aagctccata aatgtcacct tgggggccca 180

caatatcaaa gaacaggagc cgacccagca gtttatccct gtgaaaagac ccatccccca 240

tccagcctat aatcctaaga acttctccaa cgacatcatg ctactgcagc tggagagaaa 300

ggccaagcgg accagagctg tgcagcccct caggctacct agcaacaagg cccaggtgaa 360

gccagggcag acatgcagtg tggccggctg gggcagacgg gccccccctgg gaaaacactc 420

acacacacta caagaggtga agatgacagt gcaggaagat cgaaagtgcg aatctgactt 480

acgccattat tacgacagta ccattgagtt gtgcgtgggg gacccagaga ttaaaaagac 540

ttcctttaag gggactctg gaggccctct tgtgtgtaac aaggtggccc agggcattgt 600

ctcctatgga cgaaacaatg gcatgcctcc acgagcctgc accaaagtct caagctttgt 660

acactggata aagaaaacca tgaaacgcta c 691

<210> 27
<211> 1065
<212> DNA
<213> Artificial Sequence

<220>
<223> nucleotides sequence coding TOM70-(GGGGS)3-UB-Granzyme B

<400> 27
atgaaaagtt ttataactcg gaataaaact gcaattttcg caactgttgc tgctacggga 60

accgctattg gtgcatacta ctattatgga ggtggcggaa gcgggggtgg aggatccggg 120

ggcggcggaa gccaaatctt cgtcaaaact ctaacaggga agactataac cctagaggtt 180

gaaccatccg acactattga aaacgtcaaa gctaaaattc aagataaaga aggtatccct 240

ccggatcagc agagattgat ttttgctggt aagcaactag aagatggtag aaccttgtct 300

gactacaaca tccaaaagga atctactctt cacttggtgt tgagactccg cggtggtatc 360

atcgggggac atgaggccaa gccccactcc cgcccctaca tggcttatct tatgatctgg 420

gatcagaagt ctctgaagag gtgcggtggc ttcctgatac gagacgactt cgtgctgaca 480

gctgctcact gttggggaag ctccataaat gtcaccttgg gggcccacaa tatcaaagaa 540

caggagccga cccagcagtt tatccctgtg aaaagaccca tcccccatcc agcctataat 600

cctaagaact tctccaacga catcatgcta ctgcagctgg agagaaaggc caagcggacc 660

agagctgtgc agcccctcag gctacctagc aacaaggccc aggtgaagcc agggcagaca 720

tgcagtgtgg ccggctgggg gcagacggcc cccctgggaa aacactcaca cacactacaa 780

gaggtgaaga tgacagtgca ggaagatcga aagtgcgaat ctgacttacg ccattattac 840

gacagtacca ttgagttgtg cgtgggggac ccagagatta aaaagacttc ctttaagggg 900

gactctggag gccctcttgt gtgtaacaag gtggcccagg gcattgtctc ctatggacga 960

aacaatggca tgcctccacg agcctgcacc aaagtctcaa gctttgtaca ctggataaag 1020

aaaaccatga aacgctacct cgagcaccac caccaccacc actag 1065

<210> 28
<211> 1083

<212> DNA
<213> Artificial Sequence

<220>
<223> nucleotides sequence coding UB-Granzyme B-TOM7

<400> 28
atgggcagca gccatcatca tcatcatcac agcagcggcc tggtgccgcg cggcagccat 60

atgcaaatct tcgtcaaaac tctaacaggg aagactataa ccctagaggt tgaaccatcc 120

gacactattg aaaacgtcaa agctaaaatt caagataaag aaggtatccc tccggatcag 180

cagagattga tttttgctgg taagcaacta gaagatggta gaaccttgtc tgactacaac 240

atccaaaagg aatctactct tcacttggtg ttgagactcc gcggtggtat catcggggga 300

catgaggcca agccccactc ccgcccctac atggcttatc ttatgatctg ggatcagaag 360

tctctgaaga ggtgcggtgg cttcctgata cgagacgact tcgtgctgac agctgctcac 420

tgttggggaa gctccataaa tgtcaccttg gggcccacaa atatcaaaga acaggagccg 480

acccagcagt ttatccctgt gaaaagaccc atcccccatc cagcctataa tcctaagaac 540

ttctccaacg acatcatgct actgcagctg gagagaaagg ccaagcggac cagagctgtg 600

cagcccctca ggctacctag caacaaggcc caggtgaagc cagggcagac atgcagtgtg 660

gccggctggg ggcagacggc ccccctggga aaacactcac acacactaca agaggtgaag 720

atgacagtgc aggaagatcg aaagtgcgaa tctgacttac gccattatta cgacagtacc 780

attgagttgt gcgtggggga cccagagatt aaaaagactt cctttaaggg ggactctgga 840

ggccctcttg tgtgtaacaa ggtggcccag ggcattgtct cctatggacg aaacaatggc 900

atgcctccac gagcctgcac caaagtctca agctttgtac actggataaa gaaaaccatg 960

aaacgctacc tcgagtttgc cattcgctgg ggctttatcc ctcttgtgat ttacctggga 1020

tttaagaggg gtgcagatcc cggaatgcct gaaccaactg ttttgagcct actttgggga 1080

tag 1083

<210> 29
<211> 39
<212> DNA
<213> Artificial Sequence

<220>
<223> T2RKIP primer

<400> 29
aaaaaaccgc ggtggtccgg tggacctcag caagtggtc 39

<210> 30
<211> 41
<212> DNA
<213> Artificial Sequence

<220>
<223> XRKIP(noT) primer

<400> 30
aaaaaactcg agcttcccag acagctgctc gtacagtttg g 41

<210> 31
<211> 568
<212> DNA
<213> Artificial Sequence

<220>
<223> nucleotides sequence coding RKIP

<400> 31
ccgcggtggt ccggtggacc tcagcaagtg gtccgggccc ttgagcctgc aagaagtgga 60

cgagcagccg cagcacccac tgcatgtcac ctacgccggg gcggcggtgg acgagctggg 120

caaagtgctg acgcccaccc aggttaagaa tagacccacc agcatttcgt gggatggtct 180

tgattcaggg aagctctaca ccttggtcct gacagacccg gatgctccca gcaggaagga 240

tcccaaatac agagaatggc atcatttcct ggtggtcaac atgaagggca atgacatcag 300

cagtggcaca gtcctctccg attatgtggg ctcggggcct cccaagggca caggcctcca 360

ccgctatgtc tggctggttt acgagcagga caggccgcta aagtgtgacg agcccatcct 420

cagcaaccga tctggagacc accgtggcaa attcaaggtg gcgtccttcc gtaaaaagta 480

tgagctcagg gccccggtgg ctggcacgtg ttaccaggcc gagtgggatg actatgtgcc 540

caaactgtac gagcagctgt ctgggaag 568

<210> 32
<211> 942
<212> DNA
<213> Artificial Sequence

<220>
<223> nucleotides sequence coding TOM70-(GGGGS)3-UB-RKIP

<400> 32
atgaaaagtt ttataactcg gaataaaact gcaattttcg caactgttgc tgctacggga 60

accgctattg gtgcatacta ctattatgga ggtggcggaa gcgggggtgg aggatccggg 120

ggcggcggaa gccaaatctt cgtcaaaact ctaacaggga agactataac cctagaggtt 180

gaaccatccg acactattga aaacgtcaaa gctaaaattc aagataaaga aggtatccct 240

ccggatcagc agagattgat tttgctggt aagcaactag aagatggtag aaccttgtct 300

gactacaaca tccaaaagga atctactctt cacttggtgt tgagactccg cggtggtccg 360

gtggacctca gcaagtggtc cgggcccttg agcctgcaag aagtggacga gcagccgcag 420

cacccactgc atgtcaccta cgccggggcg gcggtggacg agctgggcaa agtgctgacg 480

cccacccagg ttaagaatag acccaccagc atttcgtggg atggtcttga ttcagggaag 540

ctctacacct tggtcctgac agacccggat gctcccagca ggaaggatcc caaatacaga 600

gaatggcatc atttcctggt ggtcaacatg aagggcaatg acatcagcag tggcacagtc 660

ctctccgatt atgtgggctc ggggcctccc aagggcacag gcctccaccg ctatgtctgg 720

ctggtttacg agcaggacag gccgctaaag tgtgacgagc ccatcctcag caaccgatct 780

ggagaccacc gtggcaaatt caaggtggcg tccttccgta aaaagtatga gctcagggcc 840

ccggtggctg gcacgtgtta ccaggccgag tgggatgact atgtgcccaa actgtacgag 900

cagctgtctg ggaagctcga gcaccaccac caccaccact ag 942

<210> 33
<211> 42
<212> DNA
<213> Artificial Sequence

<220>
<223> T2PTEN primer

<400> 33
aaaaaaccgc ggtggtacag ccatcatcaa agagatcgtt ag 42

<210> 34
<211> 36
<212> DNA
<213> Artificial Sequence

<220>
<223> XPTEN(noT) primer

<400> 34
aaaaaactcg aggacttttg taatttgtgt atgctg 36

<210> 35
<211> 1216
<212> DNA
<213> Artificial Sequence

<220>
<223> nucleotides sequence coding PTEN

<400> 35
ccgcggtggt acagccatca tcaaagagat cgttagcaga aacaaaagga gatatcaaga 60

ggatggattc gacttagact tgacctatat ttatccaaac attattgcta tgggatttcc 120

tgcagaaaga cttgaaggcg tatacaggaa caatattgat gatgtagtaa ggtttttgga 180

ttcaaagcat aaaaaccatt acaagatata caatctttgt gctgaaagac attatgacac 240

cgccaaattt aattgcagag ttgcacaata tcctttgaa gaccataacc caccacagct 300

agaacttatc aaacccttt gtgaagatct tgaccaatgg ctaagtgaag atgacaatca 360

tgttgcagca attcactgta aagctggaaa gggacgaact ggtgtaatga tatgtgcata 420

tttattacat cggggcaaat ttttaaaggc acaagaggcc ctagatttct atggggaagt 480

aaggaccaga gacaaaaagg gagtaactat tcccagtcag aggcgctatg tgtattatta 540

tagctacctg ttaaagaatc atctggatta tagaccagtg gcactgttgt ttcacaagat 600

gatgtttgaa actattccaa tgttcagtgg cggaacttgc aatcctcagt ttgtggtctg 660

ccagctaaag gtgaagatat attcctccaa ttcaggaccc acacgacggg aagacaagtt 720

catgtacttt gagttccctc agccgttacc tgtgtgtggt gatatcaaag tagagttctt 780

ccacaaacag aacaagatgc taaaaaagga caaaatgttt cacttttggg taaatacatt 840

cttcatacca ggaccagagg aaacctcaga aaaagtagaa aatggaagtc tatgtgatca 900

agaaatcgat agcatttgca gtatagagcg tgcagataat gacaaggaat atctagtact 960

tactttaaca aaaaatgatc ttgacaaagc aaataaagac aaagccaacc gatacttttc 1020

tccaaatttt aaggtgaagc tgtacttcac aaaaacagta gaggagccgt caaatccaga 1080

ggctagcagt tcaacttctg taacaccaga tgttagtgac aatgaacctg atcattatag 1140

atattctgac accactgact ctgatccaga gaatgaacct tttgatgaag atcagcatac 1200

acaaattaca aaagtc 1216

<210> 36
<211> 1590
<212> DNA
<213> Artificial Sequence

<220>
<223> nucleotides sequence coding TOM70-(GGGGS)3-UB-PTEN

<400> 36
atgaaaagtt ttataactcg gaataaaact gcaattttcg caactgttgc tgctacggga 60

accgctattg gtgcatacta ctattatgga ggtggcggaa gcgggggtgg aggatccggg 120

ggcggcggaa gccaaatctt cgtcaaaact ctaacaggga agactataac cctagaggtt 180

gaaccatccg acactattga aaacgtcaaa gctaaaattc aagataaaga aggtatccct 240

ccggatcagc agagattgat tttgctggt aagcaactag aagatggtag aaccttgtct 300

gactacaaca tccaaaagga atctactctt cacttggtgt tgagactccg cggtggtaca 360

gccatcatca aagagatcgt tagcagaaac aaaaggagat atcaagagga tggattcgac 420

ttagacttga cctatattta tccaaacatt attgctatgg gatttcctgc agaaagactt 480

gaaggcgtat acaggaacaa tattgatgat gtagtaaggt tttggattc aaagcataaa 540

aaccattaca agatatacaa tctttgtgct gaaagacatt atgacaccgc caaatttaat 600

tgcagagttg cacaatatcc ttttgaagac cataacccac cacagctaga acttatcaaa 660

cccttttgtg aagatcttga ccaatggcta agtgaagatg acaatcatgt tgcagcaatt 720

cactgtaaag ctggaaaggg acgaactggt gtaatgatat gtgcatattt attacatcgg 780

ggcaaatttt taaaggcaca agaggcccta gatttctatg gggaagtaag gaccagagac 840

aaaaagggag taactattcc cagtcagagg cgctatgtgt attattatag ctacctgtta 900

aagaatcatc tggattatag accagtggca ctgttgtttc acaagatgat gtttgaaact 960

attccaatgt tcagtggcgg aacttgcaat cctcagtttg tggtctgcca gctaaaggtg 1020

aagatatatt cctccaattc aggacccaca cgacgggaag acaagttcat gtactttgag 1080

ttccctcagc cgttacctgt gtgtggtgat atcaaagtag agttcttcca caaacagaac 1140

aagatgctaa aaaaggacaa aatgtttcac ttttgggtaa atacattctt cataccagga 1200

ccagaggaaa cctcagaaaa agtagaaaat ggaagtctat gtgatcaaga aatcgatagc 1260

atttgcagta tagagcgtgc agataatgac aaggaatatc tagtacttac tttaacaaaa 1320

aatgatcttg acaaagcaaa taaagacaaa gccaaccgat acttttctcc aaattttaag 1380

gtgaagctgt acttcacaaa aacagtagag gagccgtcaa atccagaggc tagcagttca 1440

acttctgtaa caccagatgt tagtgacaat gaacctgatc attatagata ttctgacacc 1500

actgactctg atccagagaa tgaacctttt gatgaagatc agcatacaca aattacaaaa 1560

gtcctcgagc accaccacca ccaccactag 1590

<210> 37
<211> 739
<212> DNA
<213> Artificial Sequence

<220>
<223> nucleotides sequence coding scFvHER2

<400> 37
ccgcggtggt gaagtgcaac ttgttgagag tggcggagga ctggtccaac cgggcggttc 60

acttaggctc tcatgtgcag cttcagggtt cacgttcacg gactatacaa tggactgggt 120

gaggcaagcc cctggtaagg gactggagtg ggttgctgac gttaacccta attccggtgg 180

gtccatctac aaccagcgat tcaagggacg atttactctt tcagtcgaca gaagcaaaaa 240

caccctctac ctccagatga actccttgcg ggcagaggat acagcggtct actattgcgc 300

gagaaacttg ggaccaagct tctacttcga ctactggggg caaggaacgc ttgttacggt 360

ttcaagcgga ggtggaggaa gtggaggtgg cggttccggc ggtggcggtt cagatataca 420

gatgacccaa tcacccagtt ctcttagcgc gtctgtaggc gacagggtaa ccataacctg 480

caaggcgtcc caggacgtgt caattggagt tgcctggtat cagcaaaaac ctgggaaagc 540

tccgaagctc ctgatttaca gcgcatctta ccgatatact ggtgtccctt caaggttcag 600

tggcagtgga tctgggacag actttacgct tactatcagc agtctgcaac ctgaggattt 660

cgcgacctac tactgtcagc agtattacat ctatccgtac acgttcggtc aaggtacaaa 720

ggtagaaata aaacgcact 739

<210> 38
<211> 1125
<212> DNA
<213> Artificial Sequence

<220>
<223> nucleotides sequence coding UB-scFvHER2-TOM7

<400> 38
atgggcagca gccatcatca tcatcatcac agcagcggcc tggtgccgcg cggcagccat 60

atgcaaatct tcgtcaaaac tctaacaggg aagactataa ccctagaggt tgaaccatcc 120
gacactattg aaaacgtcaa agctaaaatt caagataaag aaggtatccc tccggatcag 180
cagagattga tttttgctgg taagcaacta gaagatggta gaaccttgtc tgactacaac 240
atccaaaagg aatctactct tcacttggtg ttgagactcc gcggtggtga agtgcaactt 300
gttgagagtg gcggaggact ggtccaaccg ggcggttcac ttaggctctc atgtgcagct 360
tcagggttca cgttcacgga ctatacaatg gactgggtga ggcaagcccc tggtaaggga 420
ctggagtggg ttgctgacgt taaccctaat tccggtgggt ccatctacaa ccagcgattc 480
aagggacgat ttactctttc agtcgacaga agcaaaaaca ccctctacct ccagatgaac 540
tccttgcggg cagaggatac agcggtctac tattgcgcga gaaacttggg accaagcttc 600
tacttcgact actgggggca aggaacgctt gttacggttt caagcggagg tggaggaagt 660
ggaggtggcg gttccggcgg tggcggttca gatatacaga tgacccaatc acccagttct 720
cttagcgcgt ctgtaggcga cagggtaacc ataacctgca aggcgtccca ggacgtgtca 780
attggagttg cctggtatca gcaaaaacct gggaaagctc cgaagctcct gatttacagc 840
gcatcttacc gatatactgg tgtcccttca aggttcagtg gcagtggatc tgggacagac 900
tttacgctta ctatcagcag tctgcaacct gaggatttcg cgacctacta ctgtcagcag 960
tattacatct atccgtacac gttcggtcaa ggtacaaagg tagaaataaa acgcactttt 1020
gccattcgct ggggctttat ccctcttgtg atttacctgg gatttaagag ggtgcagat 1080
cccggaatgc ctgaaccaac tgttttgagc ctactttggg gatag 1125

<210> 39
<211> 38
<212> DNA
<213> Artificial Sequence

<220>
<223> RscFvHER2 primer

<400> 39
aaaaaagaat tcatggaagt gcaacttgtt gagagtgg 38

<210> 40
<211> 35
<212> DNA
<213> Artificial Sequence

<220>
<223> XTOM7(noT) primer

<400> 40
aaaaaactcg agtccccaaa gtaggctcaa aacag 35

<210> 41
<211> 924
<212> DNA
<213> Artificial Sequence

<220>
<223> nucleotides sequence coding scFvHER2-TOM7-myc/His

<400> 41
atggaagtgc aacttgttga gagtggcgga ggactggtcc aaccgggcgg ttcacttagg 60

ctctcatgtg cagcttcagg gttcacgttc acggactata caatggactg ggtgaggcaa 120

gcccctggta agggactgga gtgggttgct gacgttaacc ctaattccgg tgggtccatc 180

tacaaccagc gattcaaggg acgatttact ctttcagtcg acagaagcaa aaacaccctc 240

tacctccaga tgaactcctt gcgggcagag gatacagcgg tctactattg cgcgagaaac 300

ttgggaccaa gcttctactt cgactactgg gggcaaggaa cgcttgttac ggtttcaagc 360

ggaggtggag gaagtggagg tggcggttcc ggcggtggcg gttcagatat acagatgacc 420

caatcaccca gttctcttag cgcgtctgta ggcgacaggg taaccataac ctgcaaggcg 480

tcccaggacg tgtcaattgg agttgcctgg tatcagcaaa aacctgggaa agctccgaag 540

ctcctgattt acagcgcatc ttaccgatat actggtgtcc cttcaaggtt cagtggcagt 600

ggatctggga cagactttac gcttactatc agcagtctgc aacctgagga tttcgcgacc 660

tactactgtc agcagtatta catctatccg tacacgttcg gtcaaggtac aaaggtagaa 720

ataaaacgca cttttgccat tcgctggggc tttatccctc ttgtgattta cctgggattt 780

aagaggggtg cagatcccgg aatgcctgaa ccaactgttt tgagcctact ttggggactc 840

gagtctagag ggcccttcga acaaaaactc atctcagaag aggatctgaa tatgcatacc 900

ggtcatcatc accatcacca ttga 924

<210> 42
<211> 760
<212> DNA
<213> Artificial Sequence

<220>
<223> nucleotides sequence coding scFvMEL

<400> 42
ccgcggtggt acggacattg tgatgaccca gtctcaaaaa ttcatgtcca catcagtagg 60

agacagggtc agcgtcacct gcaaggccag tcagaatgtg gatactaatg tagcctggta 120

tcaacaaaaa ccagggcaat ctcctgaacc actgcttttc tcggcatcct accgttacac 180

tggagtccct gatcgcttca caggcagtgg atctgggaca gatttcactc tcaccatcag 240

caatgtgcag tctgaagact tggcagagta tttctgtcag caatataaca gctatcctct 300

gacgttcggt ggaggcacca agctggagat caaaggctcc accagcggca gcggtaagcc 360

aggctccggc gaaggcagca ccaaaggcga agtgaaggtt gaggagtctg gaggaggctt 420

ggtgcaacct ggaggatcca tgaaactctc ctgtgttgtc tctggattca ctttcggtaa 480

ttactggatg aactgggtcc gccagtctcc agagaagggg cttgagtgga ttgcagaaat 540

tagattgaaa tccaataatt ttgcaagata ttatgcggag tctgtgaaag ggaggttcac 600

catctcaaga gatgattcca aaagtagtgt ctacctgcaa atgatcaacc taagagctga 660

agatactggc atttattact gtaccagtta tggtaactac gttgggcact attttgacca 720

ctggggccaa ggcaccactc tcaccgtctc cctttgggga 760

<210> 43
<211> 1137
<212> DNA
<213> Artificial Sequence

<220>
<223> nucleotides sequence coding UB-scFvMEL-TOM7

<400> 43
atgggcagca gccatcatca tcatcatcac agcagcggcc tggtgccgcg cggcagccat 60

atgcaaatct tcgtcaaaac tctaacaggg aagactataa ccctagaggt tgaaccatcc 120

gacactattg aaaacgtcaa agctaaaatt caagataaag aaggtatccc tccggatcag 180

cagagattga tttttgctgg taagcaacta gaagatggta gaaccttgtc tgactacaac 240

atccaaaagg aatctactct tcacttggtg ttgagactcc gcggtggtac ggacattgtg 300

atgacccagt ctcaaaaatt catgtccaca tcagtaggag acagggtcag cgtcacctgc 360

aaggccagtc agaatgtgga tactaatgta gcctggtatc aacaaaaacc agggcaatct 420

cctgaaccac tgcttttctc ggcatcctac cgttacactg gagtccctga tcgcttcaca 480

ggcagtggat ctgggacaga tttcactctc accatcagca atgtgcagtc tgaagacttg 540

gcagagtatt tctgtcagca atataacagc tatcctctga cgttcggtgg aggcaccaag 600

ctggagatca aaggctccac cagcggcagc ggtaagccag gctccggcga aggcagcacc 660

aaaggcgaag tgaaggttga ggagtctgga ggaggcttgg tgcaacctgg aggatccatg 720

aaactctcct gtgttgtctc tggattcact ttcggtaatt actggatgaa ctgggtccgc 780

cagtctccag agaaggggct tgagtggatt gcagaaatta gattgaaatc caataatttt 840

gcaagatatt atgcggagtc tgtgaaaggg aggttcacca tctcaagaga tgattccaaa 900

agtagtgtct acctgcaaat gatcaaccta agagctgaag atactggcat ttattactgt 960

accagttatg gtaactacgt tgggcactat tttgaccact ggggccaagg caccactctc 1020

accgtctcct catttgccat tcgctggggc tttatccctc ttgtgattta cctgggattt 1080

aagaggggtg cagatcccgg aatgcctgaa ccaactgttt tgagcctact ttgggga 1137

<210> 44
<211> 36
<212> DNA
<213> Artificial Sequence

<220>
<223> RscFvMEL primer

<400> 44
aaaaaagaat tcatgaaaac aagtaaccca ggagtg 36

<210> 45
<211> 939
<212> DNA
<213> Artificial Sequence

<220>
<223> nucleotides sequence coding scFvMEL-TOM7-myc/His

<400> 45
atgacggaca ttgtgatgac ccagtctcaa aaattcatgt ccacatcagt aggagacagg 60

gtcagcgtca cctgcaaggc cagtcagaat gtggatacta atgtagcctg gtatcaacaa 120

aaaccagggc aatctcctga accactgctt ttctcggcat cctaccgtta cactggagtc 180

cctgatcgct tcacaggcag tggatctggg acagatttca ctctcaccat cagcaatgtg 240

cagtctgaag acttggcaga gtatttctgt cagcaatata acagctatcc tctgacgttc 300

ggtggaggca ccaagctgga gatcaaaggc tccaccagcg gcagcggtaa gccaggctcc 360

ggcgaaggca gcaccaaagg cgaagtgaag gttgaggagt ctggaggagg cttggtgcaa 420

cctggaggat ccatgaaact ctcctgtgtt gtctctggat tcactttcgg taattactgg 480

atgaactggg tccgccagtc tccagagaag ggcttgagt ggattgcaga aattagattg 540

aaatccaata attttgcaag atattatgcg gagtctgtga aagggaggtt caccatctca 600

agagatgatt ccaaaagtag tgtctacctg caaatgatca acctaagagc tgaagatact 660

ggcatttatt actgtaccag ttatggtaac tacgttgggc actattttga ccactggggc 720

caaggcacca ctctcaccgt ctcctcattt gccattcgct ggggctttat ccctcttgtg 780

atttacctgg gatttaagag gggtgcagat cccggaatgc ctgaaccaac tgttttgagc 840

ctactttggg gactcgagtc tagagggccc ttcgaacaaa aactcatctc agaagaggat 900

ctgaatatgc ataccggtca tcatcaccat caccattga 939

<210> 46
<211> 750
<212> DNA
<213> Artificial Sequence

<220>
<223> nucleotides sequence coding scFvPD-L1

<400> 46
atggacatcg tgatgagcca gtctcccagc agcctggctg tgtctgctgg ggagaaggtg 60

accatgtcct gcaagagctc ccagtccctg ctgaacagcc gcaccaggaa gaactacctg 120

gcctggtacc agcagaagcc aggccagagc cccaagctcc tcatctactg ggccagcacc 180

cgggagagcg gggtgcctga ccgcttcact ggaagtggca gcggcacaga cttcaccctg 240

accatcagct ctgtgcaggc cgaggacctg gcagtgtact actgccagca aagctatgat 300

gtggtgacat ttggagctgg caccaagctg gagctgaagg gaggtggcgg aagcgggggt 360

ggaggatccg gggcggcgg aagccaggtc caggtgcagc agagcggggc tgagctggcc 420

gagcccgggg cctctgtgaa gatgagctgc aaggcttctg gctacatctt caccagctac 480

tggatgcact ggctcaagca gaggcctggg caggggctgg agtggatcgg ctatatcaac 540

cccagcagtg actacaatga atattctgag aagttcatgg acaaagccac cctgactgct 600

gacaaggcca gcaccaccgc ctacatgcag ctgatcagcc tgacctcaga ggacagcgct 660

gtgtactact gtgcccggag cggctggctg gtgcacgggg actattattt tgattattgg 720

ggccagggca ccacactgac agtgagcagc 750

<210> 47
<211> 948
<212> DNA
<213> Artificial Sequence

<220>
<223> nucleotides sequence coding scFvPD-L1-TOM7-myc/His

<400> 47
atggacatcg tgatgagcca gtctcccagc agcctggctg tgtctgctgg ggagaaggtg 60

accatgtcct gcaagagctc ccagtccctg ctgaacagcc gcaccaggaa gaactacctg 120

gcctggtacc agcagaagcc aggccagagc cccaagctcc tcatctactg ggccagcacc 180

cgggagagcg gggtgcctga ccgcttcact ggaagtggca gcggcacaga cttcaccctg 240

accatcagct ctgtgcaggc cgaggacctg gcagtgtact actgccagca aagctatgat 300

gtggtgacat ttggagctgg caccaagctg gagctgaagg gaggtggcgg aagcgggggt 360

ggaggatccg gggcggcgg aagccaggtc caggtgcagc agagcggggc tgagctggcc 420

gagcccgggg cctctgtgaa gatgagctgc aaggcttctg gctacatctt caccagctac 480

tggatgcact ggctcaagca gaggcctggg caggggctgg agtggatcgg ctatatcaac 540

cccagcagtg actacaatga atattctgag aagttcatgg acaaagccac cctgactgct 600

gacaaggcca gcaccaccgc ctacatgcag ctgatcagcc tgacctcaga ggacagcgct 660

gtgtactact gtgcccggag cggctggctg gtgcacgggg actattattt tgattattgg 720

ggccagggca ccacactgac agtgagcagc ctcgagtttg ccattcgctg ggctttatc 780

cctcttgtga tttacctggg atttaagagg ggtgcagatc ccggaatgcc tgaaccaact 840

gttttgagcc tactttgggg actcgagtct agagggccct tcgaacaaaa actcatctca 900

gaagaggatc tgaatatgca taccggtcat catcaccatc accattga 948

<210> 48
<211> 395
<212> PRT
<213> Artificial Sequence

<220>
<223> amino acid sequence of p53

<400> 48
Arg Gly Gly Glu Glu Pro Gln Ser Asp Pro Ser Val Glu Pro Pro Leu
1 5 10 15

Ser Gln Glu Thr Phe Ser Asp Leu Trp Lys Leu Leu Pro Glu Asn Asn
20 25 30

Val Leu Ser Pro Leu Pro Ser Gln Ala Met Asp Asp Leu Met Leu Ser
35 40 45

Pro Asp Asp Ile Glu Gln Trp Phe Thr Glu Asp Pro Gly Pro Asp Glu
50 55 60

Ala Pro Arg Met Pro Glu Ala Ala Pro Arg Val Ala Pro Ala Pro Ala
65 70 75 80

Ala Pro Thr Pro Ala Ala Pro Ala Pro Ala Pro Ser Trp Pro Leu Ser
85 90 95

Ser Ser Val Pro Ser Gln Lys Thr Tyr Gln Gly Ser Tyr Gly Phe Arg
100 105 110

Leu Gly Phe Leu His Ser Gly Thr Ala Lys Ser Val Thr Cys Thr Tyr
115 120 125

Ser Pro Ala Leu Asn Lys Met Phe Cys Gln Leu Ala Lys Thr Cys Pro
130 135 140

Val Gln Leu Trp Val Asp Ser Thr Pro Pro Pro Gly Thr Arg Val Arg
145 150 155 160

Ala Met Ala Ile Tyr Lys Gln Ser Gln His Met Thr Glu Val Val Arg
165 170 175

Arg Cys Pro His His Glu Arg Cys Ser Asp Ser Asp Gly Leu Ala Pro
180 185 190

Pro Gln His Leu Ile Arg Val Glu Gly Asn Leu Arg Val Glu Tyr Leu
195 200 205

Asp Asp Arg Asn Thr Phe Arg His Ser Val Val Val Pro Tyr Glu Pro
210 215 220

Pro Glu Val Gly Ser Asp Cys Thr Thr Ile His Tyr Asn Tyr Met Cys
225 230 235 240

Asn Ser Ser Cys Met Gly Gly Met Asn Arg Arg Pro Ile Leu Thr Ile
245 250 255

Ile Thr Leu Glu Asp Ser Ser Gly Asn Leu Leu Gly Arg Asn Ser Phe
260 265 270

Glu Val Arg Val Cys Ala Cys Pro Gly Arg Asp Arg Arg Thr Glu Glu
275 280 285

Glu Asn Leu Arg Lys Lys Gly Glu Pro His His Glu Leu Pro Pro Gly
290 295 300

Ser Thr Lys Arg Ala Leu Pro Asn Asn Thr Ser Ser Ser Pro Gln Pro
305 310 315 320

Lys Lys Lys Pro Leu Asp Gly Glu Tyr Phe Thr Leu Gln Ile Arg Gly
325 330 335

Arg Glu Arg Phe Glu Met Phe Arg Glu Leu Asn Glu Ala Leu Glu Leu
340 345 350

Lys Asp Ala Gln Ala Gly Lys Glu Pro Gly Gly Ser Arg Ala His Ser
355 360 365

Ser His Leu Lys Ser Lys Lys Gly Gln Ser Thr Ser Arg His Lys Lys

370 375 380

Leu Met Phe Lys Thr Glu Gly Pro Asp Ser Asp
385 390 395

<210> 49
<211> 488
<212> PRT
<213> Artificial Sequence

<220>
<223> amino acid sequence of UB-p53

<400> 49
Met Gly Ser Ser His His His His His His Ser Ser Gly Leu Val Pro
1 5 10 15

Arg Gly Ser His Met Gln Ile Phe Val Lys Thr Leu Thr Gly Lys Thr
20 25 30

Ile Thr Leu Glu Val Glu Pro Ser Asp Thr Ile Glu Asn Val Lys Ala
35 40 45

Lys Ile Gln Asp Lys Glu Gly Ile Pro Pro Asp Gln Gln Arg Leu Ile
50 55 60

Phe Ala Gly Lys Gln Leu Glu Asp Gly Arg Thr Leu Ser Asp Tyr Asn
65 70 75 80

Ile Gln Lys Glu Ser Thr Leu His Leu Val Leu Arg Leu Arg Gly Gly
85 90 95

Glu Glu Pro Gln Ser Asp Pro Ser Val Glu Pro Pro Leu Ser Gln Glu
100 105 110

Thr Phe Ser Asp Leu Trp Lys Leu Leu Pro Glu Asn Asn Val Leu Ser
115 120 125

Pro Leu Pro Ser Gln Ala Met Asp Asp Leu Met Leu Ser Pro Asp Asp
130 135 140

Ile Glu Gln Trp Phe Thr Glu Asp Pro Gly Pro Asp Glu Ala Pro Arg
145 150 155 160

Met Pro Glu Ala Ala Pro Arg Val Ala Pro Ala Pro Ala Ala Pro Thr
165 170 175

Pro Ala Ala Pro Ala Pro Ala Pro Ser Trp Pro Leu Ser Ser Ser Val
180 185 190

Pro Ser Gln Lys Thr Tyr Gln Gly Ser Tyr Gly Phe Arg Leu Gly Phe
195 200 205

Leu His Ser Gly Thr Ala Lys Ser Val Thr Cys Thr Tyr Ser Pro Ala
210 215 220

Leu Asn Lys Met Phe Cys Gln Leu Ala Lys Thr Cys Pro Val Gln Leu
225 230 235 240

Trp Val Asp Ser Thr Pro Pro Pro Gly Thr Arg Val Arg Ala Met Ala
245 250 255

Ile Tyr Lys Gln Ser Gln His Met Thr Glu Val Val Arg Arg Cys Pro
260 265 270

His His Glu Arg Cys Ser Asp Ser Asp Gly Leu Ala Pro Pro Gln His

275 280 285

Leu Ile Arg Val Glu Gly Asn Leu Arg Val Glu Tyr Leu Asp Asp Arg
290 295 300

Asn Thr Phe Arg His Ser Val Val Val Pro Tyr Glu Pro Pro Glu Val
305 310 315 320

Gly Ser Asp Cys Thr Thr Ile His Tyr Asn Tyr Met Cys Asn Ser Ser
325 330 335

Cys Met Gly Gly Met Asn Arg Arg Pro Ile Leu Thr Ile Ile Thr Leu
340 345 350

Glu Asp Ser Ser Gly Asn Leu Leu Gly Arg Asn Ser Phe Glu Val Arg
355 360 365

Val Cys Ala Cys Pro Gly Arg Asp Arg Arg Thr Glu Glu Glu Asn Leu
370 375 380

Arg Lys Lys Gly Glu Pro His His Glu Leu Pro Pro Gly Ser Thr Lys
385 390 395 400

Arg Ala Leu Pro Asn Asn Thr Ser Ser Ser Pro Gln Pro Lys Lys Lys
405 410 415

Pro Leu Asp Gly Glu Tyr Phe Thr Leu Gln Ile Arg Gly Arg Glu Arg
420 425 430

Phe Glu Met Phe Arg Glu Leu Asn Glu Ala Leu Glu Leu Lys Asp Ala
435 440 445

Gln Ala Gly Lys Glu Pro Gly Gly Ser Arg Ala His Ser Ser His Leu
450 455 460

Lys Ser Lys Lys Gly Gln Ser Thr Ser Arg His Lys Lys Leu Met Phe
465 470 475 480

Lys Thr Glu Gly Pro Asp Ser Asp
485

<210> 50
<211> 504
<212> PRT
<213> Artificial Sequence

<220>
<223> amino acid sequence of TOM70-UB-p53

<400> 50
Met Lys Ser Phe Ile Thr Arg Asn Lys Thr Ala Ile Phe Ala Thr Val
1 5 10 15

Ala Ala Thr Gly Thr Ala Ile Gly Ala Tyr Tyr Tyr Tyr Gln Ile Phe
20 25 30

Val Lys Thr Leu Thr Gly Lys Thr Ile Thr Leu Glu Val Glu Pro Ser
35 40 45

Asp Thr Ile Glu Asn Val Lys Ala Lys Ile Gln Asp Lys Glu Gly Ile
50 55 60

Pro Pro Asp Gln Gln Arg Leu Ile Phe Ala Gly Lys Gln Leu Glu Asp
65 70 75 80

Gly Arg Thr Leu Ser Asp Tyr Asn Ile Gln Lys Glu Ser Thr Leu His

85 90 95

Leu Val Leu Arg Leu Arg Gly Gly Glu Glu Pro Gln Ser Asp Pro Ser
100 105 110

Val Glu Pro Pro Leu Ser Gln Glu Thr Phe Ser Asp Leu Trp Lys Leu
115 120 125

Leu Pro Glu Asn Asn Val Leu Ser Pro Leu Pro Ser Gln Ala Met Asp
130 135 140

Asp Leu Met Leu Ser Pro Asp Asp Ile Glu Gln Trp Phe Thr Glu Asp
145 150 155 160

Pro Gly Pro Asp Glu Ala Pro Arg Met Pro Glu Ala Ala Pro Arg Val
165 170 175

Ala Pro Ala Pro Ala Ala Pro Thr Pro Ala Ala Pro Ala Pro Ala Pro
180 185 190

Ser Trp Pro Leu Ser Ser Ser Val Pro Ser Gln Lys Thr Tyr Gln Gly
195 200 205

Ser Tyr Gly Phe Arg Leu Gly Phe Leu His Ser Gly Thr Ala Lys Ser
210 215 220

Val Thr Cys Thr Tyr Ser Pro Ala Leu Asn Lys Met Phe Cys Gln Leu
225 230 235 240

Ala Lys Thr Cys Pro Val Gln Leu Trp Val Asp Ser Thr Pro Pro Pro
245 250 255

Gly Thr Arg Val Arg Ala Met Ala Ile Tyr Lys Gln Ser Gln His Met
260 265 270

Thr Glu Val Val Arg Arg Cys Pro His His Glu Arg Cys Ser Asp Ser
275 280 285

Asp Gly Leu Ala Pro Pro Gln His Leu Ile Arg Val Glu Gly Asn Leu
290 295 300

Arg Val Glu Tyr Leu Asp Asp Arg Asn Thr Phe Arg His Ser Val Val
305 310 315 320

Val Pro Tyr Glu Pro Pro Glu Val Gly Ser Asp Cys Thr Thr Ile His
325 330 335

Tyr Asn Tyr Met Cys Asn Ser Ser Cys Met Gly Gly Met Asn Arg Arg
340 345 350

Pro Ile Leu Thr Ile Ile Thr Leu Glu Asp Ser Ser Gly Asn Leu Leu
355 360 365

Gly Arg Asn Ser Phe Glu Val Arg Val Cys Ala Cys Pro Gly Arg Asp
370 375 380

Arg Arg Thr Glu Glu Glu Asn Leu Arg Lys Lys Gly Glu Pro His His
385 390 395 400

Glu Leu Pro Pro Gly Ser Thr Lys Arg Ala Leu Pro Asn Asn Thr Ser
405 410 415

Ser Ser Pro Gln Pro Lys Lys Lys Pro Leu Asp Gly Glu Tyr Phe Thr
420 425 430

Leu Gln Ile Arg Gly Arg Glu Arg Phe Glu Met Phe Arg Glu Leu Asn
435 440 445

Glu Ala Leu Glu Leu Lys Asp Ala Gln Ala Gly Lys Glu Pro Gly Gly
    450                 455                 460

Ser Arg Ala His Ser Ser His Leu Lys Ser Lys Lys Gly Gln Ser Thr
465                 470                 475                 480

Ser Arg His Lys Lys Leu Met Phe Lys Thr Glu Gly Pro Asp Ser Asp
                485                 490                 495

Leu Glu His His His His His His
            500

<210> 51
<211> 519
<212> PRT
<213> Artificial Sequence

<220>
<223> amino acid sequence of TOM70-(GGGGS)3-UB-p53

<400> 51
Met Lys Ser Phe Ile Thr Arg Asn Lys Thr Ala Ile Phe Ala Thr Val
1               5                   10                  15

Ala Ala Thr Gly Thr Ala Ile Gly Ala Tyr Tyr Tyr Tyr Gly Gly Gly
            20                  25                  30

Gly Ser Gly Gly Gly Gly Ser Gly Gly Gly Gly Ser Gln Ile Phe Val
        35                  40                  45

Lys Thr Leu Thr Gly Lys Thr Ile Thr Leu Glu Val Glu Pro Ser Asp
    50                  55                  60

Thr Ile Glu Asn Val Lys Ala Lys Ile Gln Asp Lys Glu Gly Ile Pro
65                  70                  75                  80

Pro Asp Gln Gln Arg Leu Ile Phe Ala Gly Lys Gln Leu Glu Asp Gly
                85                  90                  95

Arg Thr Leu Ser Asp Tyr Asn Ile Gln Lys Glu Ser Thr Leu His Leu
            100                 105                 110

Val Leu Arg Leu Arg Gly Gly Glu Glu Pro Gln Ser Asp Pro Ser Val
        115                 120                 125

Glu Pro Pro Leu Ser Gln Glu Thr Phe Ser Asp Leu Trp Lys Leu Leu
    130                 135                 140

Pro Glu Asn Asn Val Leu Ser Pro Leu Pro Ser Gln Ala Met Asp Asp
145                 150                 155                 160

Leu Met Leu Ser Pro Asp Asp Ile Glu Gln Trp Phe Thr Glu Asp Pro
                165                 170                 175

Gly Pro Asp Glu Ala Pro Arg Met Pro Glu Ala Ala Pro Arg Val Ala
            180                 185                 190

Pro Ala Pro Ala Ala Pro Thr Pro Ala Ala Pro Ala Pro Ala Pro Ser
        195                 200                 205

Trp Pro Leu Ser Ser Ser Val Pro Ser Gln Lys Thr Tyr Gln Gly Ser
    210                 215                 220

Tyr Gly Phe Arg Leu Gly Phe Leu His Ser Gly Thr Ala Lys Ser Val
225                 230                 235                 240

Thr Cys Thr Tyr Ser Pro Ala Leu Asn Lys Met Phe Cys Gln Leu Ala
245 250 255

Lys Thr Cys Pro Val Gln Leu Trp Val Asp Ser Thr Pro Pro Pro Gly
260 265 270

Thr Arg Val Arg Ala Met Ala Ile Tyr Lys Gln Ser Gln His Met Thr
275 280 285

Glu Val Val Arg Arg Cys Pro His His Glu Arg Cys Ser Asp Ser Asp
290 295 300

Gly Leu Ala Pro Pro Gln His Leu Ile Arg Val Glu Gly Asn Leu Arg
305 310 315 320

Val Glu Tyr Leu Asp Asp Arg Asn Thr Phe Arg His Ser Val Val Val
325 330 335

Pro Tyr Glu Pro Pro Glu Val Gly Ser Asp Cys Thr Thr Ile His Tyr
340 345 350

Asn Tyr Met Cys Asn Ser Ser Cys Met Gly Gly Met Asn Arg Arg Pro
355 360 365

Ile Leu Thr Ile Ile Thr Leu Glu Asp Ser Ser Gly Asn Leu Leu Gly
370 375 380

Arg Asn Ser Phe Glu Val Arg Val Cys Ala Cys Pro Gly Arg Asp Arg
385 390 395 400

Arg Thr Glu Glu Glu Asn Leu Arg Lys Lys Gly Glu Pro His His Glu
405 410 415

Leu Pro Pro Gly Ser Thr Lys Arg Ala Leu Pro Asn Asn Thr Ser Ser
420 425 430

Ser Pro Gln Pro Lys Lys Lys Pro Leu Asp Gly Glu Tyr Phe Thr Leu
435 440 445

Gln Ile Arg Gly Arg Glu Arg Phe Glu Met Phe Arg Glu Leu Asn Glu
450 455 460

Ala Leu Glu Leu Lys Asp Ala Gln Ala Gly Lys Glu Pro Gly Gly Ser
465 470 475 480

Arg Ala His Ser Ser His Leu Lys Ser Lys Lys Gly Gln Ser Thr Ser
485 490 495

Arg His Lys Lys Leu Met Phe Lys Thr Glu Gly Pro Asp Ser Asp Leu
500 505 510

Glu His His His His His His
515

<210> 52
<211> 444
<212> PRT
<213> Artificial Sequence

<220>
<223> amino acid sequence of TOM70-(GGGGS)3-p53

<400> 52
Met Lys Ser Phe Ile Thr Arg Asn Lys Thr Ala Ile Phe Ala Thr Val
1 5 10 15

Ala Ala Thr Gly Thr Ala Ile Gly Ala Tyr Tyr Tyr Tyr Gly Gly Gly
20 25 30

Gly Ser Gly Gly Gly Gly Ser Gly Gly Gly Gly Ser Glu Glu Pro Gln
35 40 45

Ser Asp Pro Ser Val Glu Pro Pro Leu Ser Gln Glu Thr Phe Ser Asp
50 55 60

Leu Trp Lys Leu Leu Pro Glu Asn Asn Val Leu Ser Pro Leu Pro Ser
65 70 75 80

Gln Ala Met Asp Asp Leu Met Leu Ser Pro Asp Asp Ile Glu Gln Trp
85 90 95

Phe Thr Glu Asp Pro Gly Pro Asp Glu Ala Pro Arg Met Pro Glu Ala
100 105 110

Ala Pro Arg Val Ala Pro Ala Pro Ala Ala Pro Thr Pro Ala Ala Pro
115 120 125

Ala Pro Ala Pro Ser Trp Pro Leu Ser Ser Ser Val Pro Ser Gln Lys
130 135 140

Thr Tyr Gln Gly Ser Tyr Gly Phe Arg Leu Gly Phe Leu His Ser Gly
145 150 155 160

Thr Ala Lys Ser Val Thr Cys Thr Tyr Ser Pro Ala Leu Asn Lys Met
165 170 175

Phe Cys Gln Leu Ala Lys Thr Cys Pro Val Gln Leu Trp Val Asp Ser
180 185 190

Thr Pro Pro Pro Gly Thr Arg Val Arg Ala Met Ala Ile Tyr Lys Gln
195 200 205

Ser Gln His Met Thr Glu Val Val Arg Arg Cys Pro His His Glu Arg
210 215 220

Cys Ser Asp Ser Asp Gly Leu Ala Pro Pro Gln His Leu Ile Arg Val
225 230 235 240

Glu Gly Asn Leu Arg Val Glu Tyr Leu Asp Asp Arg Asn Thr Phe Arg
245 250 255

His Ser Val Val Val Pro Tyr Glu Pro Pro Glu Val Gly Ser Asp Cys
260 265 270

Thr Thr Ile His Tyr Asn Tyr Met Cys Asn Ser Ser Cys Met Gly Gly
275 280 285

Met Asn Arg Arg Pro Ile Leu Thr Ile Ile Thr Leu Glu Asp Ser Ser
290 295 300

Gly Asn Leu Leu Gly Arg Asn Ser Phe Glu Val Arg Val Cys Ala Cys
305 310 315 320

Pro Gly Arg Asp Arg Arg Thr Glu Glu Glu Asn Leu Arg Lys Lys Gly
325 330 335

Glu Pro His His Glu Leu Pro Pro Gly Ser Thr Lys Arg Ala Leu Pro
340 345 350

Asn Asn Thr Ser Ser Ser Pro Gln Pro Lys Lys Lys Pro Leu Asp Gly
355 360 365

Glu Tyr Phe Thr Leu Gln Ile Arg Gly Arg Glu Arg Phe Glu Met Phe
370 375 380

Arg Glu Leu Asn Glu Ala Leu Glu Leu Lys Asp Ala Gln Ala Gly Lys
385 390 395 400

Glu Pro Gly Gly Ser Arg Ala His Ser Ser His Leu Lys Ser Lys Lys
405 410 415

Gly Gln Ser Thr Ser Arg His Lys Lys Leu Met Phe Lys Thr Glu Gly
420 425 430

Pro Asp Ser Asp Leu Glu His His His His His His
435 440

<210> 53
<211> 525
<212> PRT
<213> Artificial Sequence

<220>
<223> amino acid sequence of UB-p53-TOM7

<400> 53
Met Gly Ser Ser His His His His His His Ser Ser Gly Leu Val Pro
1 5 10 15

Arg Gly Ser His Met Gln Ile Phe Val Lys Thr Leu Thr Gly Lys Thr
20 25 30

Ile Thr Leu Glu Val Glu Pro Ser Asp Thr Ile Glu Asn Val Lys Ala
35 40 45

Lys Ile Gln Asp Lys Glu Gly Ile Pro Pro Asp Gln Gln Arg Leu Ile
50 55 60

Phe Ala Gly Lys Gln Leu Glu Asp Gly Arg Thr Leu Ser Asp Tyr Asn
65 70 75 80

Ile Gln Lys Glu Ser Thr Leu His Leu Val Leu Arg Leu Arg Gly Gly
85 90 95

Glu Glu Pro Gln Ser Asp Pro Ser Val Glu Pro Pro Leu Ser Gln Glu
100 105 110

Thr Phe Ser Asp Leu Trp Lys Leu Leu Pro Glu Asn Asn Val Leu Ser
115 120 125

Pro Leu Pro Ser Gln Ala Met Asp Asp Leu Met Leu Ser Pro Asp Asp
130 135 140

Ile Glu Gln Trp Phe Thr Glu Asp Pro Gly Pro Asp Glu Ala Pro Arg
145 150 155 160

Met Pro Glu Ala Ala Pro Arg Val Ala Pro Ala Pro Ala Ala Pro Thr
165 170 175

Pro Ala Ala Pro Ala Pro Ala Pro Ser Trp Pro Leu Ser Ser Ser Val
180 185 190

Pro Ser Gln Lys Thr Tyr Gln Gly Ser Tyr Gly Phe Arg Leu Gly Phe
195 200 205

Leu His Ser Gly Thr Ala Lys Ser Val Thr Cys Thr Tyr Ser Pro Ala
210 215 220

Leu Asn Lys Met Phe Cys Gln Leu Ala Lys Thr Cys Pro Val Gln Leu
225 230 235 240

Trp Val Asp Ser Thr Pro Pro Pro Gly Thr Arg Val Arg Ala Met Ala
245 250 255

Ile Tyr Lys Gln Ser Gln His Met Thr Glu Val Val Arg Arg Cys Pro
260 265 270

His His Glu Arg Cys Ser Asp Ser Asp Gly Leu Ala Pro Pro Gln His
275 280 285

Leu Ile Arg Val Glu Gly Asn Leu Arg Val Glu Tyr Leu Asp Asp Arg
290 295 300

Asn Thr Phe Arg His Ser Val Val Val Pro Tyr Glu Pro Pro Glu Val
305 310 315 320

Gly Ser Asp Cys Thr Thr Ile His Tyr Asn Tyr Met Cys Asn Ser Ser
325 330 335

Cys Met Gly Gly Met Asn Arg Arg Pro Ile Leu Thr Ile Ile Thr Leu
340 345 350

Glu Asp Ser Ser Gly Asn Leu Leu Gly Arg Asn Ser Phe Glu Val His
355 360 365

Val Cys Ala Cys Pro Gly Arg Asp Arg Arg Thr Glu Glu Glu Asn Leu
370 375 380

Arg Lys Lys Gly Glu Pro His His Glu Leu Pro Pro Gly Ser Thr Lys
385 390 395 400

Arg Ala Leu Ser Asn Asn Thr Ser Ser Ser Pro Gln Pro Lys Lys Lys
405 410 415

Pro Leu Asp Gly Glu Tyr Phe Thr Leu Gln Ile Arg Gly Arg Glu Arg
420 425 430

Phe Glu Met Phe Arg Glu Leu Asn Glu Ala Leu Glu Leu Lys Asp Ala
435 440 445

Gln Ala Gly Lys Glu Pro Gly Gly Ser Arg Ala His Ser Ser His Leu
450 455 460

Lys Ser Lys Lys Gly Gln Ser Thr Ser Arg His Lys Lys Leu Met Phe
465 470 475 480

Lys Thr Glu Gly Pro Asp Ser Asp Leu Glu Phe Ala Ile Arg Trp Gly
485 490 495

Phe Ile Pro Leu Val Ile Tyr Leu Gly Phe Lys Arg Gly Ala Asp Pro
500 505 510

Gly Met Pro Glu Pro Thr Val Leu Ser Leu Leu Trp Gly
515 520 525

<210> 54
<211> 421
<212> PRT
<213> Artificial Sequence

<220>
<223> amino acid sequence of p53-myc/His

<400> 54
Met Glu Glu Pro Gln Ser Asp Pro Ser Val Glu Pro Pro Leu Ser Gln
1 5 10 15

Glu Thr Phe Ser Asp Leu Trp Lys Leu Leu Pro Glu Asn Asn Val Leu
20 25 30

Ser Pro Leu Pro Ser Gln Ala Met Asp Asp Leu Met Leu Ser Pro Asp
35 40 45

Asp Ile Glu Gln Trp Phe Thr Glu Asp Pro Gly Pro Asp Glu Ala Pro
50 55 60

Arg Met Pro Glu Ala Ala Pro Arg Val Ala Pro Ala Pro Ala Ala Pro
65 70 75 80

Thr Pro Ala Ala Pro Ala Pro Ala Pro Ser Trp Pro Leu Ser Ser Ser
85 90 95

Val Pro Ser Gln Lys Thr Tyr Gln Gly Ser Tyr Gly Phe Arg Leu Gly
100 105 110

Phe Leu His Ser Gly Thr Ala Lys Ser Val Thr Cys Thr Tyr Ser Pro
115 120 125

Ala Leu Asn Lys Met Phe Cys Gln Leu Ala Lys Thr Cys Pro Val Gln
130 135 140

Leu Trp Val Asp Ser Thr Pro Pro Pro Gly Thr Arg Val Arg Ala Met
145 150 155 160

Ala Ile Tyr Lys Gln Ser Gln His Met Thr Glu Val Val Arg Arg Cys
165 170 175

Pro His His Glu Arg Cys Ser Asp Ser Asp Gly Leu Ala Pro Pro Gln
180 185 190

His Leu Ile Arg Val Glu Gly Asn Leu Arg Val Glu Tyr Leu Asp Asp
195 200 205

Arg Asn Thr Phe Arg His Ser Val Val Val Pro Tyr Glu Pro Pro Glu
210 215 220

Val Gly Ser Asp Cys Thr Thr Ile His Tyr Asn Tyr Met Cys Asn Ser
225 230 235 240

Ser Cys Met Gly Gly Met Asn Arg Arg Pro Ile Leu Thr Ile Ile Thr
245 250 255

Leu Glu Asp Ser Ser Gly Asn Leu Leu Gly Arg Asn Ser Phe Glu Val
260 265 270

Arg Val Cys Ala Cys Pro Gly Arg Asp Arg Arg Thr Glu Glu Glu Asn
275 280 285

Leu Arg Lys Lys Gly Glu Pro His His Glu Leu Pro Pro Gly Ser Thr
290 295 300

Lys Arg Ala Leu Pro Asn Asn Thr Ser Ser Ser Pro Gln Pro Lys Lys
305 310 315 320

Lys Pro Leu Asp Gly Glu Tyr Phe Thr Leu Gln Ile Arg Gly Arg Glu
325 330 335

Arg Phe Glu Met Phe Arg Glu Leu Asn Glu Ala Leu Glu Leu Lys Asp
340 345 350

Ala Gln Ala Gly Lys Glu Pro Gly Gly Ser Arg Ala His Ser Ser His
355 360 365

Leu Lys Ser Lys Lys Gly Gln Ser Thr Ser Arg His Lys Lys Leu Met

370 375 380

Phe Lys Thr Glu Gly Pro Asp Ser Asp Leu Glu Ser Arg Gly Pro Phe
385 390 395 400

Glu Gln Lys Leu Ile Ser Glu Glu Asp Leu Asn Met His Thr Gly His
405 410 415

His His His His His
420

<210> 55
<211> 230
<212> PRT
<213> Artificial Sequence

<220>
<223> amino acid sequence of GranzymeB

<400> 55
Arg Gly Gly Ile Ile Gly Gly His Glu Ala Lys Pro His Ser Arg Pro
1 5 10 15

Tyr Met Ala Tyr Leu Met Ile Trp Asp Gln Lys Ser Leu Lys Arg Cys
20 25 30

Gly Gly Phe Leu Ile Gln Asp Asp Phe Val Leu Thr Ala Ala His Cys
35 40 45

Trp Gly Ser Ser Ile Asn Val Thr Leu Gly Ala His Asn Ile Lys Glu
50 55 60

Gln Glu Pro Thr Gln Gln Phe Ile Pro Val Lys Arg Pro Ile Pro His
65 70 75 80

Pro Ala Tyr Asn Pro Lys Asn Phe Ser Asn Asp Ile Met Leu Leu Gln
85 90 95

Leu Glu Arg Lys Ala Lys Arg Thr Arg Ala Val Gln Pro Leu Arg Leu
100 105 110

Pro Ser Asn Lys Ala Gln Val Lys Pro Gly Gln Thr Cys Ser Val Ala
115 120 125

Gly Trp Gly Gln Thr Ala Pro Leu Gly Lys His Ser His Thr Leu Gln
130 135 140

Glu Val Lys Met Thr Val Gln Glu Asp Arg Lys Cys Glu Ser Asp Leu
145 150 155 160

Arg His Tyr Tyr Asp Ser Thr Ile Glu Leu Cys Val Gly Asp Pro Glu
165 170 175

Ile Lys Lys Thr Ser Phe Lys Gly Asp Ser Gly Gly Pro Leu Val Cys
180 185 190

Asn Lys Val Ala Gln Gly Ile Val Ser Tyr Gly Arg Asn Asn Gly Met
195 200 205

Pro Pro Arg Ala Cys Thr Lys Val Ser Ser Phe Val His Trp Ile Lys
210 215 220

Lys Thr Met Lys Arg Tyr
225 230

<210> 56
<211> 354
<212> PRT
<213> Artificial Sequence

<220>
<223> amino acid sequence of TOM70-(GGGGS)3-UB-Granzyme B

<400> 56
Met Lys Ser Phe Ile Thr Arg Asn Lys Thr Ala Ile Phe Ala Thr Val
1               5                   10                  15

Ala Ala Thr Gly Thr Ala Ile Gly Ala Tyr Tyr Tyr Tyr Gly Gly Gly
            20                  25                  30

Gly Ser Gly Gly Gly Gly Ser Gly Gly Gly Gly Ser Gln Ile Phe Val
        35                  40                  45

Lys Thr Leu Thr Gly Lys Thr Ile Thr Leu Glu Val Glu Pro Ser Asp
    50                  55                  60

Thr Ile Glu Asn Val Lys Ala Lys Ile Gln Asp Lys Glu Gly Ile Pro
65                  70                  75                  80

Pro Asp Gln Gln Arg Leu Ile Phe Ala Gly Lys Gln Leu Glu Asp Gly
                85                  90                  95

Arg Thr Leu Ser Asp Tyr Asn Ile Gln Lys Glu Ser Thr Leu His Leu
            100                 105                 110

Val Leu Arg Leu Arg Gly Gly Ile Ile Gly Gly His Glu Ala Lys Pro
        115                 120                 125

His Ser Arg Pro Tyr Met Ala Tyr Leu Met Ile Trp Asp Gln Lys Ser
    130                 135                 140

Leu Lys Arg Cys Gly Gly Phe Leu Ile Arg Asp Asp Phe Val Leu Thr
145                 150                 155                 160

Ala Ala His Cys Trp Gly Ser Ser Ile Asn Val Thr Leu Gly Ala His
                165                 170                 175

Asn Ile Lys Glu Gln Glu Pro Thr Gln Gln Phe Ile Pro Val Lys Arg
            180                 185                 190

Pro Ile Pro His Pro Ala Tyr Asn Pro Lys Asn Phe Ser Asn Asp Ile
        195                 200                 205

Met Leu Leu Gln Leu Glu Arg Lys Ala Lys Arg Thr Arg Ala Val Gln
    210                 215                 220

Pro Leu Arg Leu Pro Ser Asn Lys Ala Gln Val Lys Pro Gly Gln Thr
225                 230                 235                 240

Cys Ser Val Ala Gly Trp Gly Gln Thr Ala Pro Leu Gly Lys His Ser
                245                 250                 255

His Thr Leu Gln Glu Val Lys Met Thr Val Gln Glu Asp Arg Lys Cys
            260                 265                 270

Glu Ser Asp Leu Arg His Tyr Tyr Asp Ser Thr Ile Glu Leu Cys Val
        275                 280                 285

Gly Asp Pro Glu Ile Lys Lys Thr Ser Phe Lys Gly Asp Ser Gly Gly
    290                 295                 300

Pro Leu Val Cys Asn Lys Val Ala Gln Gly Ile Val Ser Tyr Gly Arg

305 310 315 320

Asn Asn Gly Met Pro Pro Arg Ala Cys Thr Lys Val Ser Ser Phe Val
325 330 335

His Trp Ile Lys Lys Thr Met Lys Arg Tyr Leu Glu His His His His
340 345 350

His His

<210> 57
<211> 360
<212> PRT
<213> Artificial Sequence

<220>
<223> amino acid sequence of UB-Granzyme B-TOM7

<400> 57
Met Gly Ser Ser His His His His His His Ser Ser Gly Leu Val Pro
1 5 10 15

Arg Gly Ser His Met Gln Ile Phe Val Lys Thr Leu Thr Gly Lys Thr
20 25 30

Ile Thr Leu Glu Val Glu Pro Ser Asp Thr Ile Glu Asn Val Lys Ala
35 40 45

Lys Ile Gln Asp Lys Glu Gly Ile Pro Pro Asp Gln Gln Arg Leu Ile
50 55 60

Phe Ala Gly Lys Gln Leu Glu Asp Gly Arg Thr Leu Ser Asp Tyr Asn
65 70 75 80

Ile Gln Lys Glu Ser Thr Leu His Leu Val Leu Arg Leu Arg Gly Gly
85 90 95

Ile Ile Gly Gly His Glu Ala Lys Pro His Ser Arg Pro Tyr Met Ala
100 105 110

Tyr Leu Met Ile Trp Asp Gln Lys Ser Leu Lys Arg Cys Gly Gly Phe
115 120 125

Leu Ile Arg Asp Asp Phe Val Leu Thr Ala Ala His Cys Trp Gly Ser
130 135 140

Ser Ile Asn Val Thr Leu Gly Ala His Asn Ile Lys Glu Gln Glu Pro
145 150 155 160

Thr Gln Gln Phe Ile Pro Val Lys Arg Pro Ile Pro His Pro Ala Tyr
165 170 175

Asn Pro Lys Asn Phe Ser Asn Asp Ile Met Leu Leu Gln Leu Glu Arg
180 185 190

Lys Ala Lys Arg Thr Arg Ala Val Gln Pro Leu Arg Leu Pro Ser Asn
195 200 205

Lys Ala Gln Val Lys Pro Gly Gln Thr Cys Ser Val Ala Gly Trp Gly
210 215 220

Gln Thr Ala Pro Leu Gly Lys His Ser His Thr Leu Gln Glu Val Lys
225 230 235 240

Met Thr Val Gln Glu Asp Arg Lys Cys Glu Ser Asp Leu Arg His Tyr

245 250 255

Tyr Asp Ser Thr Ile Glu Leu Cys Val Gly Asp Pro Glu Ile Lys Lys
260 265 270

Thr Ser Phe Lys Gly Asp Ser Gly Gly Pro Leu Val Cys Asn Lys Val
275 280 285

Ala Gln Gly Ile Val Ser Tyr Gly Arg Asn Asn Gly Met Pro Pro Arg
290 295 300

Ala Cys Thr Lys Val Ser Ser Phe Val His Trp Ile Lys Lys Thr Met
305 310 315 320

Lys Arg Tyr Leu Glu Phe Ala Ile Arg Trp Gly Phe Ile Pro Leu Val
325 330 335

Ile Tyr Leu Gly Phe Lys Arg Gly Ala Asp Pro Gly Met Pro Glu Pro
340 345 350

Thr Val Leu Ser Leu Leu Trp Gly
355 360

<210> 58
<211> 189
<212> PRT
<213> Artificial Sequence

<220>
<223> amino acid sequence of RKIP

<400> 58
Arg Gly Gly Pro Val Asp Leu Ser Lys Trp Ser Gly Pro Leu Ser Leu
1 5 10 15

Gln Glu Val Asp Glu Gln Pro Gln His Pro Leu His Val Thr Tyr Ala
20 25 30

Gly Ala Ala Val Asp Glu Leu Gly Lys Val Leu Thr Pro Thr Gln Val
35 40 45

Lys Asn Arg Pro Thr Ser Ile Ser Trp Asp Gly Leu Asp Ser Gly Lys
50 55 60

Leu Tyr Thr Leu Val Leu Thr Asp Pro Asp Ala Pro Ser Arg Lys Asp
65 70 75 80

Pro Lys Tyr Arg Glu Trp His His Phe Leu Val Val Asn Met Lys Gly
85 90 95

Asn Asp Ile Ser Ser Gly Thr Val Leu Ser Asp Tyr Val Gly Ser Gly
100 105 110

Pro Pro Lys Gly Thr Gly Leu His Arg Tyr Val Trp Leu Val Tyr Glu
115 120 125

Gln Asp Arg Pro Leu Lys Cys Asp Glu Pro Ile Leu Ser Asn Arg Ser
130 135 140

Gly Asp His Arg Gly Lys Phe Lys Val Ala Ser Phe Arg Lys Lys Tyr
145 150 155 160

Glu Leu Arg Ala Pro Val Ala Gly Thr Cys Tyr Gln Ala Glu Trp Asp
165 170 175

Asp Tyr Val Pro Lys Leu Tyr Glu Gln Leu Ser Gly Lys

180 185

<210> 59
<211> 313
<212> PRT
<213> Artificial Sequence

<220>
<223> amino acid sequence of TOM70-(GGGGS)3-UB-RKIP

<400> 59
Met Lys Ser Phe Ile Thr Arg Asn Lys Thr Ala Ile Phe Ala Thr Val
1 5 10 15

Ala Ala Thr Gly Thr Ala Ile Gly Ala Tyr Tyr Tyr Tyr Gly Gly Gly
20 25 30

Gly Ser Gly Gly Gly Gly Ser Gly Gly Gly Gly Ser Gln Ile Phe Val
35 40 45

Lys Thr Leu Thr Gly Lys Thr Ile Thr Leu Glu Val Glu Pro Ser Asp
50 55 60

Thr Ile Glu Asn Val Lys Ala Lys Ile Gln Asp Lys Glu Gly Ile Pro
65 70 75 80

Pro Asp Gln Gln Arg Leu Ile Phe Ala Gly Lys Gln Leu Glu Asp Gly
85 90 95

Arg Thr Leu Ser Asp Tyr Asn Ile Gln Lys Glu Ser Thr Leu His Leu
100 105 110

Val Leu Arg Leu Arg Gly Gly Pro Val Asp Leu Ser Lys Trp Ser Gly
115 120 125

Pro Leu Ser Leu Gln Glu Val Asp Glu Gln Pro Gln His Pro Leu His
130 135 140

Val Thr Tyr Ala Gly Ala Ala Val Asp Glu Leu Gly Lys Val Leu Thr
145 150 155 160

Pro Thr Gln Val Lys Asn Arg Pro Thr Ser Ile Ser Trp Asp Gly Leu
165 170 175

Asp Ser Gly Lys Leu Tyr Thr Leu Val Leu Thr Asp Pro Asp Ala Pro
180 185 190

Ser Arg Lys Asp Pro Lys Tyr Arg Glu Trp His His Phe Leu Val Val
195 200 205

Asn Met Lys Gly Asn Asp Ile Ser Ser Gly Thr Val Leu Ser Asp Tyr
210 215 220

Val Gly Ser Gly Pro Pro Lys Gly Thr Gly Leu His Arg Tyr Val Trp
225 230 235 240

Leu Val Tyr Glu Gln Asp Arg Pro Leu Lys Cys Asp Glu Pro Ile Leu
245 250 255

Ser Asn Arg Ser Gly Asp His Arg Gly Lys Phe Lys Val Ala Ser Phe
260 265 270

Arg Lys Lys Tyr Glu Leu Arg Ala Pro Val Ala Gly Thr Cys Tyr Gln
275 280 285

Ala Glu Trp Asp Asp Tyr Val Pro Lys Leu Tyr Glu Gln Leu Ser Gly

290 295 300

Lys Leu Glu His His His His His His
305 310

<210> 60
<211> 405
<212> PRT
<213> Artificial Sequence

<220>
<223> amino acid sequence of PTEN

<400> 60
Arg Gly Gly Thr Ala Ile Ile Lys Glu Ile Val Ser Arg Asn Lys Arg
1 5 10 15

Arg Tyr Gln Glu Asp Gly Phe Asp Leu Asp Leu Thr Tyr Ile Tyr Pro
20 25 30

Asn Ile Ile Ala Met Gly Phe Pro Ala Glu Arg Leu Glu Gly Val Tyr
35 40 45

Arg Asn Asn Ile Asp Asp Val Val Arg Phe Leu Asp Ser Lys His Lys
50 55 60

Asn His Tyr Lys Ile Tyr Asn Leu Cys Ala Glu Arg His Tyr Asp Thr
65 70 75 80

Ala Lys Phe Asn Cys Arg Val Ala Gln Tyr Pro Phe Glu Asp His Asn
85 90 95

Pro Pro Gln Leu Glu Leu Ile Lys Pro Phe Cys Glu Asp Leu Asp Gln
100 105 110

Trp Leu Ser Glu Asp Asp Asn His Val Ala Ala Ile His Cys Lys Ala
115 120 125

Gly Lys Gly Arg Thr Gly Val Met Ile Cys Ala Tyr Leu Leu His Arg
130 135 140

Gly Lys Phe Leu Lys Ala Gln Glu Ala Leu Asp Phe Tyr Gly Glu Val
145 150 155 160

Arg Thr Arg Asp Lys Lys Gly Val Thr Ile Pro Ser Gln Arg Arg Tyr
165 170 175

Val Tyr Tyr Tyr Ser Tyr Leu Leu Lys Asn His Leu Asp Tyr Arg Pro
180 185 190

Val Ala Leu Leu Phe His Lys Met Met Phe Glu Thr Ile Pro Met Phe
195 200 205

Ser Gly Gly Thr Cys Asn Pro Gln Phe Val Val Cys Gln Leu Lys Val
210 215 220

Lys Ile Tyr Ser Ser Asn Ser Gly Pro Thr Arg Arg Glu Asp Lys Phe
225 230 235 240

Met Tyr Phe Glu Phe Pro Gln Pro Leu Pro Val Cys Gly Asp Ile Lys
245 250 255

Val Glu Phe Phe His Lys Gln Asn Lys Met Leu Lys Lys Asp Lys Met
260 265 270

Phe His Phe Trp Val Asn Thr Phe Phe Ile Pro Gly Pro Glu Glu Thr

            275                 280                 285

Ser Glu Lys Val Glu Asn Gly Ser Leu Cys Asp Gln Glu Ile Asp Ser
    290                 295                 300

Ile Cys Ser Ile Glu Arg Ala Asp Asn Asp Lys Glu Tyr Leu Val Leu
305                 310                 315                 320

Thr Leu Thr Lys Asn Asp Leu Asp Lys Ala Asn Lys Asp Lys Ala Asn
                325                 330                 335

Arg Tyr Phe Ser Pro Asn Phe Lys Val Lys Leu Tyr Phe Thr Lys Thr
            340                 345                 350

Val Glu Glu Pro Ser Asn Pro Glu Ala Ser Ser Ser Thr Ser Val Thr
        355                 360                 365

Pro Asp Val Ser Asp Asn Glu Pro Asp His Tyr Arg Tyr Ser Asp Thr
    370                 375                 380

Thr Asp Ser Asp Pro Glu Asn Glu Pro Phe Asp Glu Asp Gln His Thr
385                 390                 395                 400

Gln Ile Thr Lys Val
                405

<210>  61
<211>  529
<212>  PRT
<213>  Artificial Sequence

<220>
<223>  amino acid sequence of TOM70-(GGGGS)3-UB-PTEN

<400>  61
Met Lys Ser Phe Ile Thr Arg Asn Lys Thr Ala Ile Phe Ala Thr Val
1               5                   10                  15

Ala Ala Thr Gly Thr Ala Ile Gly Ala Tyr Tyr Tyr Tyr Gly Gly Gly
            20                  25                  30

Gly Ser Gly Gly Gly Gly Ser Gly Gly Gly Gly Ser Gln Ile Phe Val
        35                  40                  45

Lys Thr Leu Thr Gly Lys Thr Ile Thr Leu Glu Val Glu Pro Ser Asp
    50                  55                  60

Thr Ile Glu Asn Val Lys Ala Lys Ile Gln Asp Lys Glu Gly Ile Pro
65                  70                  75                  80

Pro Asp Gln Gln Arg Leu Ile Phe Ala Gly Lys Gln Leu Glu Asp Gly
                85                  90                  95

Arg Thr Leu Ser Asp Tyr Asn Ile Gln Lys Glu Ser Thr Leu His Leu
            100                 105                 110

Val Leu Arg Leu Arg Gly Gly Thr Ala Ile Ile Lys Glu Ile Val Ser
        115                 120                 125

Arg Asn Lys Arg Arg Tyr Gln Glu Asp Gly Phe Asp Leu Asp Leu Thr
    130                 135                 140

Tyr Ile Tyr Pro Asn Ile Ile Ala Met Gly Phe Pro Ala Glu Arg Leu
145                 150                 155                 160

Glu Gly Val Tyr Arg Asn Asn Ile Asp Asp Val Val Arg Phe Leu Asp

165 170 175

Ser Lys His Lys Asn His Tyr Lys Ile Tyr Asn Leu Cys Ala Glu Arg
180 185 190

His Tyr Asp Thr Ala Lys Phe Asn Cys Arg Val Ala Gln Tyr Pro Phe
195 200 205

Glu Asp His Asn Pro Pro Gln Leu Glu Leu Ile Lys Pro Phe Cys Glu
210 215 220

Asp Leu Asp Gln Trp Leu Ser Glu Asp Asp Asn His Val Ala Ala Ile
225 230 235 240

His Cys Lys Ala Gly Lys Gly Arg Thr Gly Val Met Ile Cys Ala Tyr
245 250 255

Leu Leu His Arg Gly Lys Phe Leu Lys Ala Gln Glu Ala Leu Asp Phe
260 265 270

Tyr Gly Glu Val Arg Thr Arg Asp Lys Lys Gly Val Thr Ile Pro Ser
275 280 285

Gln Arg Arg Tyr Val Tyr Tyr Tyr Ser Tyr Leu Leu Lys Asn His Leu
290 295 300

Asp Tyr Arg Pro Val Ala Leu Leu Phe His Lys Met Met Phe Glu Thr
305 310 315 320

Ile Pro Met Phe Ser Gly Gly Thr Cys Asn Pro Gln Phe Val Val Cys
325 330 335

Gln Leu Lys Val Lys Ile Tyr Ser Ser Asn Ser Gly Pro Thr Arg Arg
340 345 350

Glu Asp Lys Phe Met Tyr Phe Glu Phe Pro Gln Pro Leu Pro Val Cys
355 360 365

Gly Asp Ile Lys Val Glu Phe Phe His Lys Gln Asn Lys Met Leu Lys
370 375 380

Lys Asp Lys Met Phe His Phe Trp Val Asn Thr Phe Phe Ile Pro Gly
385 390 395 400

Pro Glu Glu Thr Ser Glu Lys Val Glu Asn Gly Ser Leu Cys Asp Gln
405 410 415

Glu Ile Asp Ser Ile Cys Ser Ile Glu Arg Ala Asp Asn Asp Lys Glu
420 425 430

Tyr Leu Val Leu Thr Leu Thr Lys Asn Asp Leu Asp Lys Ala Asn Lys
435 440 445

Asp Lys Ala Asn Arg Tyr Phe Ser Pro Asn Phe Lys Val Lys Leu Tyr
450 455 460

Phe Thr Lys Thr Val Glu Glu Pro Ser Asn Pro Glu Ala Ser Ser Ser
465 470 475 480

Thr Ser Val Thr Pro Asp Val Ser Asp Asn Glu Pro Asp His Tyr Arg
485 490 495

Tyr Ser Asp Thr Thr Asp Ser Asp Pro Glu Asn Glu Pro Phe Asp Glu
500 505 510

Asp Gln His Thr Gln Ile Thr Lys Val Leu Glu His His His His His
515 520 525

His

<210> 62
<211> 246
<212> PRT
<213> Artificial Sequence

<220>
<223> amino acid sequence of scFvHER2

<400> 62
Arg Gly Gly Glu Val Gln Leu Val Glu Ser Gly Gly Gly Leu Val Gln
1               5                   10                  15

Pro Gly Gly Ser Leu Arg Leu Ser Cys Ala Ala Ser Gly Phe Thr Phe
            20                  25                  30

Thr Asp Tyr Thr Met Asp Trp Val Arg Gln Ala Pro Gly Lys Gly Leu
        35                  40                  45

Glu Trp Val Ala Asp Val Asn Pro Asn Ser Gly Gly Ser Ile Tyr Asn
    50                  55                  60

Gln Arg Phe Lys Gly Arg Phe Thr Leu Ser Val Asp Arg Ser Lys Asn
65                  70                  75                  80

Thr Leu Tyr Leu Gln Met Asn Ser Leu Arg Ala Glu Asp Thr Ala Val
                85                  90                  95

Tyr Tyr Cys Ala Arg Asn Leu Gly Pro Ser Phe Tyr Phe Asp Tyr Trp
            100                 105                 110

Gly Gln Gly Thr Leu Val Thr Val Ser Ser Gly Gly Gly Gly Ser Gly
        115                 120                 125

Gly Gly Gly Ser Gly Gly Gly Gly Ser Asp Ile Gln Met Thr Gln Ser
    130                 135                 140

Pro Ser Ser Leu Ser Ala Ser Val Gly Asp Arg Val Thr Ile Thr Cys
145                 150                 155                 160

Lys Ala Ser Gln Asp Val Ser Ile Gly Val Ala Trp Tyr Gln Gln Lys
                165                 170                 175

Pro Gly Lys Ala Pro Lys Leu Leu Ile Tyr Ser Ala Ser Tyr Arg Tyr
            180                 185                 190

Thr Gly Val Pro Ser Arg Phe Ser Gly Ser Gly Ser Gly Thr Asp Phe
        195                 200                 205

Thr Leu Thr Ile Ser Ser Leu Gln Pro Glu Asp Phe Ala Thr Tyr Tyr
    210                 215                 220

Cys Gln Gln Tyr Tyr Ile Tyr Pro Tyr Thr Phe Gly Gln Gly Thr Lys
225                 230                 235                 240

Val Glu Ile Lys Arg Thr
                245

<210> 63
<211> 374
<212> PRT
<213> Artificial Sequence

<220>
<223> amino acid sequence of UB-scFvHER2-TOM7

<400> 63
Met Gly Ser Ser His His His His His His Ser Ser Gly Leu Val Pro
1 5 10 15

Arg Gly Ser His Met Gln Ile Phe Val Lys Thr Leu Thr Gly Lys Thr
20 25 30

Ile Thr Leu Glu Val Glu Pro Ser Asp Thr Ile Glu Asn Val Lys Ala
35 40 45

Lys Ile Gln Asp Lys Glu Gly Ile Pro Pro Asp Gln Gln Arg Leu Ile
50 55 60

Phe Ala Gly Lys Gln Leu Glu Asp Gly Arg Thr Leu Ser Asp Tyr Asn
65 70 75 80

Ile Gln Lys Glu Ser Thr Leu His Leu Val Leu Arg Leu Arg Gly Gly
85 90 95

Glu Val Gln Leu Val Glu Ser Gly Gly Gly Leu Val Gln Pro Gly Gly
100 105 110

Ser Leu Arg Leu Ser Cys Ala Ala Ser Gly Phe Thr Phe Thr Asp Tyr
115 120 125

Thr Met Asp Trp Val Arg Gln Ala Pro Gly Lys Gly Leu Glu Trp Val
130 135 140

Ala Asp Val Asn Pro Asn Ser Gly Gly Ser Ile Tyr Asn Gln Arg Phe
145 150 155 160

Lys Gly Arg Phe Thr Leu Ser Val Asp Arg Ser Lys Asn Thr Leu Tyr
165 170 175

Leu Gln Met Asn Ser Leu Arg Ala Glu Asp Thr Ala Val Tyr Tyr Cys
180 185 190

Ala Arg Asn Leu Gly Pro Ser Phe Tyr Phe Asp Tyr Trp Gly Gln Gly
195 200 205

Thr Leu Val Thr Val Ser Ser Gly Gly Gly Gly Ser Gly Gly Gly Gly
210 215 220

Ser Gly Gly Gly Gly Ser Asp Ile Gln Met Thr Gln Ser Pro Ser Ser
225 230 235 240

Leu Ser Ala Ser Val Gly Asp Arg Val Thr Ile Thr Cys Lys Ala Ser
245 250 255

Gln Asp Val Ser Ile Gly Val Ala Trp Tyr Gln Gln Lys Pro Gly Lys
260 265 270

Ala Pro Lys Leu Leu Ile Tyr Ser Ala Ser Tyr Arg Tyr Thr Gly Val
275 280 285

Pro Ser Arg Phe Ser Gly Ser Gly Ser Gly Thr Asp Phe Thr Leu Thr
290 295 300

Ile Ser Ser Leu Gln Pro Glu Asp Phe Ala Thr Tyr Tyr Cys Gln Gln
305 310 315 320

Tyr Tyr Ile Tyr Pro Tyr Thr Phe Gly Gln Gly Thr Lys Val Glu Ile
325 330 335

Lys Arg Thr Phe Ala Ile Arg Trp Gly Phe Ile Pro Leu Val Ile Tyr
340 345 350

Leu Gly Phe Lys Arg Gly Ala Asp Pro Gly Met Pro Glu Pro Thr Val
355 360 365

Leu Ser Leu Leu Trp Gly
370

<210> 64
<211> 307
<212> PRT
<213> Artificial Sequence

<220>
<223> amino acid sequence of scFvHER2-TOM7-myc/His

<400> 64
Met Glu Val Gln Leu Val Glu Ser Gly Gly Gly Leu Val Gln Pro Gly
1 5 10 15

Gly Ser Leu Arg Leu Ser Cys Ala Ala Ser Gly Phe Thr Phe Thr Asp
20 25 30

Tyr Thr Met Asp Trp Val Arg Gln Ala Pro Gly Lys Gly Leu Glu Trp
35 40 45

Val Ala Asp Val Asn Pro Asn Ser Gly Gly Ser Ile Tyr Asn Gln Arg
50 55 60

Phe Lys Gly Arg Phe Thr Leu Ser Val Asp Arg Ser Lys Asn Thr Leu
65 70 75 80

Tyr Leu Gln Met Asn Ser Leu Arg Ala Glu Asp Thr Ala Val Tyr Tyr
85 90 95

Cys Ala Arg Asn Leu Gly Pro Ser Phe Tyr Phe Asp Tyr Trp Gly Gln
100 105 110

Gly Thr Leu Val Thr Val Ser Ser Gly Gly Gly Gly Ser Gly Gly Gly
115 120 125

Gly Ser Gly Gly Gly Gly Ser Asp Ile Gln Met Thr Gln Ser Pro Ser
130 135 140

Ser Leu Ser Ala Ser Val Gly Asp Arg Val Thr Ile Thr Cys Lys Ala
145 150 155 160

Ser Gln Asp Val Ser Ile Gly Val Ala Trp Tyr Gln Gln Lys Pro Gly
165 170 175

Lys Ala Pro Lys Leu Leu Ile Tyr Ser Ala Ser Tyr Arg Tyr Thr Gly
180 185 190

Val Pro Ser Arg Phe Ser Gly Ser Gly Ser Gly Thr Asp Phe Thr Leu
195 200 205

Thr Ile Ser Ser Leu Gln Pro Glu Asp Phe Ala Thr Tyr Tyr Cys Gln
210 215 220

Gln Tyr Tyr Ile Tyr Pro Tyr Thr Phe Gly Gln Gly Thr Lys Val Glu
225 230 235 240

Ile Lys Arg Thr Phe Ala Ile Arg Trp Gly Phe Ile Pro Leu Val Ile
245 250 255

Tyr Leu Gly Phe Lys Arg Gly Ala Asp Pro Gly Met Pro Glu Pro Thr
260 265 270

Val Leu Ser Leu Leu Trp Gly Leu Glu Ser Arg Gly Pro Phe Glu Gln
275 280 285

Lys Leu Ile Ser Glu Glu Asp Leu Asn Met His Thr Gly His His His
290 295 300

His His His
305

<210> 65
<211> 251
<212> PRT
<213> Artificial Sequence

<220>
<223> amino acid sequence of scFvMEL

<400> 65
Arg Gly Gly Thr Asp Ile Val Met Thr Gln Ser Gln Lys Phe Met Ser
1 5 10 15

Thr Ser Val Gly Asp Arg Val Ser Val Thr Cys Lys Ala Ser Gln Asn
20 25 30

Val Asp Thr Asn Val Ala Trp Tyr Gln Gln Lys Pro Gly Gln Ser Pro
35 40 45

Glu Pro Leu Leu Phe Ser Ala Ser Tyr Arg Tyr Thr Gly Val Pro Asp
50 55 60

Arg Phe Thr Gly Ser Gly Ser Gly Thr Asp Phe Thr Leu Thr Ile Ser
65 70 75 80

Asn Val Gln Ser Glu Asp Leu Ala Glu Tyr Phe Cys Gln Gln Tyr Asn
85 90 95

Ser Tyr Pro Leu Thr Phe Gly Gly Gly Thr Lys Leu Glu Ile Lys Gly
100 105 110

Ser Thr Ser Gly Ser Gly Lys Pro Gly Ser Gly Glu Gly Ser Thr Lys
115 120 125

Gly Glu Val Lys Val Glu Glu Ser Gly Gly Gly Leu Val Gln Pro Gly
130 135 140

Gly Ser Met Lys Leu Ser Cys Val Val Ser Gly Phe Thr Phe Gly Asn
145 150 155 160

Tyr Trp Met Asn Trp Val Arg Gln Ser Pro Glu Lys Gly Leu Glu Trp
165 170 175

Ile Ala Glu Ile Arg Leu Lys Ser Asn Asn Phe Ala Arg Tyr Tyr Ala
180 185 190

Glu Ser Val Lys Gly Arg Phe Thr Ile Ser Arg Asp Asp Ser Lys Ser
195 200 205

Ser Val Tyr Leu Gln Met Ile Asn Leu Arg Ala Glu Asp Thr Gly Ile
210 215 220

Tyr Tyr Cys Thr Ser Tyr Gly Asn Tyr Val Gly His Tyr Phe Asp His
225 230 235 240

Trp Gly Gln Gly Thr Thr Leu Thr Val Ser Ser
245 250

<210> 66
<211> 378
<212> PRT
<213> Artificial Sequence

<220>
<223> amino acid sequence of UB-scFvMEL-TOM7

<400> 66
Met Gly Ser Ser His His His His His His Ser Ser Gly Leu Val Pro
1 5 10 15

Arg Gly Ser His Met Gln Ile Phe Val Lys Thr Leu Thr Gly Lys Thr
20 25 30

Ile Thr Leu Glu Val Glu Pro Ser Asp Thr Ile Glu Asn Val Lys Ala
35 40 45

Lys Ile Gln Asp Lys Glu Gly Ile Pro Pro Asp Gln Gln Arg Leu Ile
50 55 60

Phe Ala Gly Lys Gln Leu Glu Asp Gly Arg Thr Leu Ser Asp Tyr Asn
65 70 75 80

Ile Gln Lys Glu Ser Thr Leu His Leu Val Leu Arg Leu Arg Gly Gly
85 90 95

Thr Asp Ile Met Thr Gln Ser Gln Lys Phe Met Ser Thr Ser Val Gly
100 105 110

Asp Arg Val Ser Val Thr Cys Lys Ala Ser Gln Asn Val Asp Thr Asn
115 120 125

Val Ala Trp Tyr Gln Gln Lys Pro Gly Gln Ser Pro Glu Pro Leu Leu
130 135 140

Phe Ser Ala Ser Tyr Arg Tyr Thr Gly Val Pro Asp Arg Phe Thr Gly
145 150 155 160

Ser Gly Ser Gly Thr Asp Phe Thr Leu Thr Ile Ser Asn Val Gln Ser
165 170 175

Glu Asp Leu Ala Glu Tyr Phe Cys Gln Gln Tyr Asn Ser Tyr Pro Leu
180 185 190

Thr Phe Gly Gly Gly Thr Lys Leu Glu Ile Lys Gly Ser Thr Ser Gly
195 200 205

Ser Gly Lys Pro Gly Ser Gly Glu Gly Ser Thr Lys Gly Glu Val Lys
210 215 220

Val Glu Glu Ser Gly Gly Gly Leu Val Gln Pro Gly Gly Ser Met Lys
225 230 235 240

Leu Ser Cys Val Val Ser Gly Phe Thr Phe Gly Asn Tyr Trp Met Asn
245 250 255

Trp Val Arg Gln Ser Pro Glu Lys Gly Leu Glu Trp Ile Ala Glu Ile
260 265 270

Arg Leu Lys Ser Asn Asn Phe Ala Arg Tyr Tyr Ala Glu Ser Val Lys
275 280 285

Gly Arg Phe Thr Ile Ser Arg Asp Asp Ser Lys Ser Ser Val Tyr Leu
    290                 295                 300

Gln Met Ile Asn Leu Arg Ala Glu Asp Thr Gly Ile Tyr Tyr Cys Thr
305                 310                 315                 320

Ser Tyr Gly Asn Tyr Val Gly His Tyr Phe Asp His Trp Gly Gln Gly
                325                 330                 335

Thr Thr Leu Thr Val Ser Ser Phe Ala Ile Arg Trp Gly Phe Ile Pro
            340                 345                 350

Leu Val Ile Tyr Leu Gly Phe Lys Arg Gly Ala Asp Pro Gly Met Pro
        355                 360                 365

Glu Pro Thr Val Leu Ser Leu Leu Trp Gly
    370                 375

<210> 67
<211> 312
<212> PRT
<213> Artificial Sequence

<220>
<223> amino acid sequence of scFvMEL-TOM7-myc/His

<400> 67
Met Thr Asp Ile Val Met Thr Gln Ser Gln Lys Phe Met Ser Thr Ser
1               5                   10                  15

Val Gly Asp Arg Val Ser Val Thr Cys Lys Ala Ser Gln Asn Val Asp
            20                  25                  30

Thr Asn Val Ala Trp Tyr Gln Gln Lys Pro Gly Gln Ser Pro Glu Pro
        35                  40                  45

Leu Leu Phe Ser Ala Ser Tyr Arg Tyr Thr Gly Val Pro Asp Arg Phe
    50                  55                  60

Thr Gly Ser Gly Ser Gly Thr Asp Phe Thr Leu Thr Ile Ser Asn Val
65                  70                  75                  80

Gln Ser Glu Asp Leu Ala Glu Tyr Phe Cys Gln Gln Tyr Asn Ser Tyr
                85                  90                  95

Pro Leu Thr Phe Gly Gly Gly Thr Lys Leu Glu Ile Lys Gly Ser Thr
            100                 105                 110

Ser Gly Ser Gly Lys Pro Gly Ser Gly Glu Gly Ser Thr Lys Gly Glu
        115                 120                 125

Val Lys Val Glu Glu Ser Gly Gly Gly Leu Val Gln Pro Gly Gly Ser
    130                 135                 140

Met Lys Leu Ser Cys Val Val Ser Gly Phe Thr Phe Gly Asn Tyr Trp
145                 150                 155                 160

Met Asn Trp Val Arg Gln Ser Pro Glu Lys Gly Leu Glu Trp Ile Ala
                165                 170                 175

Glu Ile Arg Leu Lys Ser Asn Asn Phe Ala Arg Tyr Tyr Ala Glu Ser
            180                 185                 190

Val Lys Gly Arg Phe Thr Ile Ser Arg Asp Asp Ser Lys Ser Ser Val
        195                 200                 205

Tyr Leu Gln Met Ile Asn Leu Arg Ala Glu Asp Thr Gly Ile Tyr Tyr
210 215 220

Cys Thr Ser Tyr Gly Asn Tyr Val Gly His Tyr Phe Asp His Trp Gly
225 230 235 240

Gln Gly Thr Thr Leu Thr Val Ser Ser Phe Ala Ile Arg Trp Gly Phe
245 250 255

Ile Pro Leu Val Ile Tyr Leu Gly Phe Lys Arg Gly Ala Asp Pro Gly
260 265 270

Met Pro Glu Pro Thr Val Leu Ser Leu Leu Trp Gly Leu Glu Ser Arg
275 280 285

Gly Pro Phe Glu Gln Lys Leu Ile Ser Glu Glu Asp Leu Asn Met His
290 295 300

Thr Gly His His His His His His
305 310

<210> 68
<211> 250
<212> PRT
<213> Artificial Sequence

<220>
<223> amino acid sequence of scFvPD-L1

<400> 68
Met Asp Ile Val Met Ser Gln Ser Pro Ser Ser Leu Ala Val Ser Ala
1 5 10 15

Gly Glu Lys Val Thr Met Ser Cys Lys Ser Ser Gln Ser Leu Leu Asn
20 25 30

Ser Arg Thr Arg Lys Asn Tyr Leu Ala Trp Tyr Gln Gln Lys Pro Gly
35 40 45

Gln Ser Pro Lys Leu Leu Ile Tyr Trp Ala Ser Thr Arg Glu Ser Gly
50 55 60

Val Pro Asp Arg Phe Thr Gly Ser Gly Ser Gly Thr Asp Phe Thr Leu
65 70 75 80

Thr Ile Ser Ser Val Gln Ala Glu Asp Leu Ala Val Tyr Tyr Cys Gln
85 90 95

Gln Ser Tyr Asp Val Val Thr Phe Gly Ala Gly Thr Lys Leu Glu Leu
100 105 110

Lys Gly Gly Gly Gly Ser Gly Gly Gly Gly Ser Gly Gly Gly Gly Ser
115 120 125

Gln Val Gln Val Gln Gln Ser Gly Ala Glu Leu Ala Glu Pro Gly Ala
130 135 140

Ser Val Lys Met Ser Cys Lys Ala Ser Gly Tyr Ile Phe Thr Ser Tyr
145 150 155 160

Trp Met His Trp Leu Lys Gln Arg Pro Gly Gln Gly Leu Glu Trp Ile
165 170 175

Gly Tyr Ile Asn Pro Ser Ser Asp Tyr Asn Glu Tyr Ser Glu Lys Phe
180 185 190

Met Asp Lys Ala Thr Leu Thr Ala Asp Lys Ala Ser Thr Thr Ala Tyr
195 200 205

Met Gln Leu Ile Ser Leu Thr Ser Glu Asp Ser Ala Val Tyr Tyr Cys
210 215 220

Ala Arg Ser Gly Trp Leu Val His Gly Asp Tyr Tyr Phe Asp Tyr Trp
225 230 235 240

Gly Gln Gly Thr Thr Leu Thr Val Ser Ser
245 250

<210> 69
<211> 315
<212> PRT
<213> Artificial Sequence

<220>
<223> amino acid sequence of scFvPD-L1-TOM7-myc/His

<400> 69
Met Asp Ile Val Met Ser Gln Ser Pro Ser Ser Leu Ala Val Ser Ala
1 5 10 15

Gly Glu Lys Val Thr Met Ser Cys Lys Ser Ser Gln Ser Leu Leu Asn
20 25 30

Ser Arg Thr Arg Lys Asn Tyr Leu Ala Trp Tyr Gln Gln Lys Pro Gly
35 40 45

Gln Ser Pro Lys Leu Leu Ile Tyr Trp Ala Ser Thr Arg Glu Ser Gly
50 55 60

Val Pro Asp Arg Phe Thr Gly Ser Gly Ser Gly Thr Asp Phe Thr Leu
65 70 75 80

Thr Ile Ser Ser Val Gln Ala Glu Asp Leu Ala Val Tyr Tyr Cys Gln
85 90 95

Gln Ser Tyr Asp Val Val Thr Phe Gly Ala Gly Thr Lys Leu Glu Leu
100 105 110

Lys Gly Gly Gly Gly Ser Gly Gly Gly Gly Ser Gly Gly Gly Gly Ser
115 120 125

Gln Val Gln Val Gln Gln Ser Gly Ala Glu Leu Ala Glu Pro Gly Ala
130 135 140

Ser Val Lys Met Ser Cys Lys Ala Ser Gly Tyr Ile Phe Thr Ser Tyr
145 150 155 160

Trp Met His Trp Leu Lys Gln Arg Pro Gly Gln Gly Leu Glu Trp Ile
165 170 175

Gly Tyr Ile Asn Pro Ser Ser Asp Tyr Asn Glu Tyr Ser Glu Lys Phe
180 185 190

Met Asp Lys Ala Thr Leu Thr Ala Asp Lys Ala Ser Thr Thr Ala Tyr
195 200 205

Met Gln Leu Ile Ser Leu Thr Ser Glu Asp Ser Ala Val Tyr Tyr Cys
210 215 220

Ala Arg Ser Gly Trp Leu Val His Gly Asp Tyr Tyr Phe Asp Tyr Trp
225 230 235 240

Gly Gln Gly Thr Thr Leu Thr Val Ser Ser Leu Glu Phe Ala Ile Arg
245 250 255

Trp Gly Phe Ile Pro Leu Val Ile Tyr Leu Gly Phe Lys Arg Gly Ala
260 265 270

Asp Pro Gly Met Pro Glu Pro Thr Val Leu Ser Leu Leu Trp Gly Leu
275 280 285

Glu Ser Arg Gly Pro Phe Glu Gln Lys Leu Ile Ser Glu Glu Asp Leu
290 295 300

Asn Met His Thr Gly His His His His His His
305 310 315

<210> 70
<211> 15
<212> DNA
<213> Artificial Sequence

<220>
<223> linker

<400> 70
ggggsggggs ggggs

<210> 71
<211> 75
<212> PRT
<213> Artificial Sequence

<220>
<223> amino acid sequence of ubiquitin

<400> 71
Gln Leu Phe Val Lys Thr Leu Thr Gly Lys Thr Val Thr Leu Glu Val
1 5 10 15

Glu Ser Ser Asp Thr Ile Asp Asn Val Lys Ser Lys Ile Gln Asp Lys
20 25 30

Glu Gly Ile Pro Pro Asp Gln Gln Arg Leu Ile Phe Ala Gly Lys Gln
35 40 45

Leu Glu Asp Gly Arg Thr Leu Ser Asp Tyr Asn Ile Gln Lys Glu Ser
50 55 60

Thr Leu His Leu Val Leu Arg Leu Arg Gly Gly
65 70 75

<210> 72
<211> 225
<212> DNA
<213> Artificial Sequence

<220>
<223> nucleotides sequence coding ubiquitin

<400> 72
caacttttcg tcaaaactct aacagggaag actgtaaccc tagaggttga atcttccgac

actattgaca acgtcaaaag taaaattcaa gataaagaag gtatccctcc ggatcagcag

agattgattt ttgctggtaa gcaactagaa gatggtagaa ccttgtctga ctacaacatc

caaaaggaat ctactcttca cttggtgttg agactccgcg gtggt

<210> 73
<211> 400
<212> PRT
<213> Artificial Sequence

<220>
<223> amino acid sequence of p53

<400> 73
Glu Glu Pro Gln Ser Asp Pro Ser Val Glu Pro Pro Leu Ser Gln Glu
1               5                   10                  15

Thr Phe Ser Asp Leu Trp Lys Leu Leu Pro Glu Asn Asn Val Leu Ser
            20                  25                  30

Pro Leu Pro Ser Gln Ala Met Asp Asp Leu Met Leu Ser Pro Asp Asp
        35                  40                  45

Ile Glu Gln Trp Phe Thr Glu Asp Pro Gly Pro Asp Glu Ala Pro Arg
    50                  55                  60

Met Pro Glu Ala Ala Pro Arg Val Ala Pro Ala Pro Ala Ala Pro Thr
65                  70                  75                  80

Pro Ala Ala Pro Ala Pro Ala Pro Ser Trp Pro Leu Ser Ser Ser Val
                85                  90                  95

Pro Ser Gln Lys Thr Tyr Gln Gly Ser Tyr Gly Phe Arg Leu Gly Phe
            100                 105                 110

Leu His Ser Gly Thr Ala Lys Ser Val Thr Cys Thr Tyr Ser Pro Ala
        115                 120                 125

Leu Asn Lys Met Phe Cys Gln Leu Ala Lys Thr Cys Pro Val Gln Leu
    130                 135                 140

Trp Val Asp Ser Thr Pro Pro Pro Gly Thr Arg Val Arg Ala Met Ala
145                 150                 155                 160

Ile Tyr Lys Gln Ser Gln His Met Thr Glu Val Val Arg Arg Cys Pro
                165                 170                 175

His His Glu Arg Cys Ser Asp Ser Asp Gly Leu Ala Pro Pro Gln His
            180                 185                 190

Leu Ile Arg Val Glu Gly Asn Leu Arg Val Glu Tyr Leu Asp Asp Arg
        195                 200                 205

Asn Thr Phe Arg His Ser Val Val Val Pro Tyr Glu Pro Pro Glu Val
    210                 215                 220

Gly Ser Asp Cys Thr Thr Ile His Tyr Asn Tyr Met Cys Asn Ser Ser
225                 230                 235                 240

Cys Met Gly Gly Met Asn Arg Arg Pro Ile Leu Thr Ile Ile Thr Leu
                245                 250                 255

Glu Asp Ser Ser Gly Asn Leu Leu Gly Arg Asn Ser Phe Glu Val Arg
            260                 265                 270

Val Cys Ala Cys Pro Gly Arg Asp Arg Arg Thr Glu Glu Glu Asn Leu

275 280 285

Arg Lys Lys Gly Glu Pro His His Glu Leu Pro Pro Gly Ser Thr Lys
290 295 300

Arg Ala Leu Pro Asn Asn Thr Ser Ser Ser Pro Gln Pro Lys Lys Lys
305 310 315 320

Pro Leu Asp Gly Glu Tyr Phe Thr Leu Gln Ile Arg Gly Arg Glu Arg
325 330 335

Phe Glu Met Phe Arg Glu Leu Asn Glu Ala Leu Glu Leu Lys Asp Ala
340 345 350

Gln Ala Gly Lys Glu Pro Gly Gly Ser Arg Ala His Ser Ser His Leu
355 360 365

Lys Ser Lys Lys Gly Gln Ser Thr Ser Arg His Lys Lys Leu Met Phe
370 375 380

Lys Thr Glu Gly Pro Asp Ser Asp Leu Glu His His His His His His
385 390 395 400

<210> 74
<211> 1203
<212> DNA
<213> Artificial Sequence

<220>
<223> nucleotides sequence coding p53

<400> 74
gaggagccgc agtcagatcc tagcgtcgag ccccctctga gtcaggaaac attttcagac

ctgtggaaac tacttcctga aaacaacgtt ctgtccccct tgccgtccca agcaatggat

gatttgatgc tgtccccgga cgatattgaa caatggttca ctgaagaccc aggtccagat

gaagctccca gaatgccaga ggctgctccc cgcgtggccc ctgcaccagc agctcctaca

ccggcggccc ctgcaccagc cccctcctgg cccctgtcat cttctgtccc ttcccagaaa

acctaccagg gcagctacgg tttccgtctg ggcttcttgc attctgggac agccaagtct

gtgacttgca cgtactcccc tgccctcaac aagatgtttt gccaactggc caagacctgc

cctgtgcagc tgtgggttga ttccacaccc ccgcccggca cccgcgtccg cgccatggcc

atctacaagc agtcacagca catgacggag gttgtgaggc gctgccccca ccatgagcgc

tgctcagata gcgatggtct ggcccctcct cagcatctta tccgagtgga aggaaatttg

cgtgtggagt atttggatga cagaaacact tttcgacata gtgtggtggt gccctatgag

ccgcctgagg ttggctctga ctgtaccacc atccactaca actacatgtg taacagttcc

tgcatgggcg gcatgaaccg gaggcccatc ctcaccatca tcacactgga agactccagt

ggtaatctac tgggacggaa cagctttgag gtgcgtgttt gtgcctgtcc tgggagagac

cggcgcacag aggaagagaa tctccgcaag aaaggggagc ctcaccacga gctgccccca

gggagcacta agcgagcact gcccaacaac accagctcct ctccccagcc aaagaagaaa

ccactggatg gagaatattt caccettcag atccgtgggc gtgagcgctt cgagatgttc

cgagagctga atgaggcctt ggaactcaag gatgcccagg ctgggaagga gccagggggg
agcagggctc actccagcca cctgaagtcc aaaaagggtc agtctacctc ccgccataaa
aaactcatgt tcaagacaga agggcctgac tcagacctcg agcaccacca ccaccaccac
tag

<210> 75
<211> 29
<212> PRT
<213> Artificial Sequence

<220>
<223> TOM70 of S. cerevisiae

<400> 75
Met Lys Ser Phe Ile Thr Arg Asn Lys Thr Ala Ile Phe Ala Thr Val
1 5 10 15

Ala Ala Thr Gly Thr Ala Ile Gly Ala Tyr Tyr Tyr Tyr
20 25

<210> 76
<211> 60
<212> PRT
<213> Artificial Sequence

<220>
<223> TOM70 of Homo sapiens

<400> 76
Met Ala Ala Ser Lys Pro Val Glu Ala Ala Val Val Ala Ala Ala Val
1 5 10 15

Pro Ser Ser Gly Ser Gly Val Gly Gly Gly Gly Thr Ala Gly Pro Gly
20 25 30

Thr Gly Gly Leu Pro Arg Trp Gln Leu Ala Leu Ala Val Gly Ala Pro
35 40 45

Leu Leu Leu Gly Ala Gly Ala Ile Tyr Leu Trp Ser
50 55 60

<210> 77
<211> 29
<212> PRT
<213> Artificial Sequence

<220>
<223> TOM20 of S. cerevisiae

<400> 77
Met Ser Gln Ser Asn Pro Ile Leu Arg Gly Leu Ala Ile Thr Thr Ala
1 5 10 15

Ile Ala Ala Leu Ser Ala Thr Gly Tyr Ala Ile Tyr Phe
20 25

<210> 78
<211> 24

<212> PRT
<213> Artificial Sequence

<220>
<223> TOM20 of Homo sapiens

<400> 78
Met Val Gly Arg Asn Ser Ala Ile Ala Ala Gly Val Cys Gly Ala Leu
1 5 10 15

Phe Ile Gly Tyr Cys Ile Tyr Phe
20

<210> 79
<211> 22
<212> PRT
<213> Artificial Sequence

<220>
<223> OM45 of S. cerevisiae

<400> 79
Met Ser Ser Arg Ile Ile Val Gly Ser Ala Ala Leu Ala Ala Ala Ile
1 5 10 15

Thr Ala Ser Ile Met Val
20

<210> 80
<211> 23
<212> PRT
<213> Artificial Sequence

<220>
<223> TOM5 of S. cerevisiae

<400> 80
Ala Ala Tyr Val Ala Ala Phe Leu Trp Val Ser Pro Met Ile Trp His
1 5 10 15

Leu Val Lys Lys Gln Trp Lys
20

<210> 81
<211> 24
<212> PRT
<213> Artificial Sequence

<220>
<223> TOM5 of Homo sapiens

<400> 81
Ile Arg Asn Phe Leu Ile Tyr Val Ala Leu Leu Arg Val Thr Pro Phe
1 5 10 15

Ile Leu Lys Lys Leu Asp Ser Ile
20

<210> 82
<211> 41

<212> PRT
<213> Artificial Sequence

<220>
<223> TOM7 of S. cerevisiae

<400> 82
Ile Leu Thr Leu Thr His Asn Val Ala His Tyr Gly Trp Ile Pro Phe
1 5 10 15

Val Leu Tyr Leu Gly Trp Ala His Thr Ser Asn Arg Pro Asn Phe Leu
20 25 30

Asn Leu Leu Ser Pro Leu Pro Ser Val
35 40

<210> 83
<211> 35
<212> PRT
<213> Artificial Sequence

<220>
<223> TOM7 of Homo sapiens

<400> 83
Phe Ala Ile Arg Trp Gly Phe Ile Pro Leu Val Ile Tyr Leu Gly Phe
1 5 10 15

Lys Arg Gly Ala Asp Pro Gly Met Pro Glu Pro Thr Val Leu Ser Leu
20 25 30

Leu Trp Gly
35

<210> 84
<211> 55
<212> PRT
<213> Artificial Sequence

<220>
<223> TOM22 of S. cerevisiae

<400> 84
Leu Ala Trp Thr Leu Thr Thr Thr Ala Leu Leu Leu Gly Val Pro Leu
1 5 10 15

Ser Leu Ser Ile Leu Ala Glu Gln Gln Leu Ile Glu Met Glu Lys Thr
20 25 30

Phe Asp Leu Gln Ser Asp Ala Asn Asn Ile Leu Ala Gln Gly Glu Lys
35 40 45

Asp Ala Ala Ala Thr Ala Asn
50 55

<210> 85
<211> 60
<212> PRT
<213> Artificial Sequence

<220>
<223> TOM22 of Homo sapiens

<400> 85
Ala Ala Leu Trp Ile Gly Thr Thr Ser Phe Met Ile Leu Val Leu Pro
1 5 10 15

Val Val Phe Glu Thr Glu Lys Leu Gln Met Glu Gln Gln Gln Gln Leu
20 25 30

Gln Gln Arg Gln Ile Leu Leu Gly Pro Asn Thr Gly Leu Ser Gly Gly
35 40 45

Met Pro Gly Ala Leu Pro Ser Leu Pro Gly Lys Ile
50 55 60

<210> 86
<211> 25
<212> PRT
<213> Artificial Sequence

<220>
<223> Fis1 of S. cerevisiae

<400> 86
Gly Val Val Val Ala Gly Gly Val Leu Ala Gly Ala Val Ala Val Ala
1 5 10 15

Ser Phe Phe Leu Arg Asn Lys Arg Arg
20 25

<210> 87
<211> 31
<212> PRT
<213> Artificial Sequence

<220>
<223> Fis1 of Homo sapiens

<400> 87
Gly Leu Val Gly Met Ala Ile Val Gly Gly Met Ala Leu Gly Val Ala
1 5 10 15

Gly Leu Ala Gly Leu Ile Gly Leu Ala Val Ser Lys Ser Lys Ser
20 25 30

<210> 88
<211> 25
<212> PRT
<213> Artificial Sequence

<220>
<223> Bcl-2 alpha of Homo sapiens

<400> 88
Leu Ser Leu Lys Thr Leu Leu Ser Leu Ala Leu Val Gly Ala Cys Ile
1 5 10 15

Thr Leu Gly Ala Tyr Leu Gly His Lys
20 25

<210> 89
<211> 19
<212> PRT
<213> Artificial Sequence

<220>
<223> VAMP1 of S. cerevisiae

<400> 89
Met Ile Met Leu Gly Ala Ile Cys Ala Ile Ile Val Val Val Ile Val
1 5 10 15

Arg Arg Asp

<210> 90
<211> 22
<212> PRT
<213> Artificial Sequence

<220>
<223> VAMP1 of Homo sapiens

<400> 90
Met Met Ile Met Leu Gly Ala Ile Cys Ala Ile Ile Val Val Val Ile
1 5 10 15

Val Ile Tyr Phe Phe Thr
20

<210> 91
<211> 60
<212> DNA
<213> Artificial Sequence

<220>
<223> P53-promter-S

<400> 91
gggcatgctc gggcatgccc gggcatgctc gggcatgccc gggcatgctc gggcatgccc 60

60

<210> 92
<211> 60
<212> DNA
<213> Artificial Sequence

<220>
<223> P53-promter-AS

<400> 92
gggcatgccc gagcatgccc gggcatgccc gagcatgccc gggcatgccc gagcatgccc 60

60

## Description

### Technical Field

The present invention provides a fusion protein capable of modifying mitochondria, mitochondria modified by the fusion protein, and a pharmaceutical composition comprising the same as an active ingredient.

### Background Art

Mitochondria are cellular organelles of eukaryotic cells involved in the synthesis and regulation of adenosine triphosphate (ATP), an intracellular energy source. Mitochondria are associated with various metabolic pathways in vivo, for example, cell signaling, cell differentiation, cell death, as well as control of cell cycle and cell growth. Mitochondria have their own genomes and are organelles that play a central role in the energy metabolism of cells. Mitochondria produce energy through the electron transport and oxidative phosphorylation process, and play an important role in being involved in apoptosis signaling pathways.

It has been reported that a reduction in energy production due to a decrease in mitochondrial function causes various diseases. When the function of the electron transport chain reaction decreases according to the variation of the mitochondria genome and proteome, a reduction in ATP production, an excessive reactive oxygen production, a decrease in calcium regulation function and the like occur. In this case, a change in the membrane permeability of the mitochondria occurs, and the function of apoptosis may occur abnormally and lead to cancer and incurable diseases.

As such, human diseases that have been reported to result from mitochondrial dysfunction include mitochondria related genetic disease (Wallace DC 1999), diabetes mellitus (Maechler P 2001), heart disease (Sorescu D 2002), senile dementia such as Parkinson's disease or Alzheimer's disease (Lin MT 2006), and the occurrence of various cancers (Petros JA, 2005) and cancer metastasis (Ishikawa K, 2008) and the like have been reported. In addition, features commonly found in more than 200 types of various cancers consisted of impaired apoptosis function, increased inflammatory response, and increased abnormal metabolism. All of these processes are closely related to mitochondrial function, and the correlation between cancer and mitochondria is drawing attention.

On the other hand, it is known that normal cells produce 36 ATP per molecule of glucose through an electron transport system process, but cancer cells, unlike normal cells, produce 2 ATP per molecule of glucose through glycolysis under a sufficient oxygen condition (aerobic glycolysis). As such, it is known that cancer cells, unlike normal cells, use the inefficient glycolysis process in terms of energy in order to produce amino acids, lipids, nucleic acids and the like necessary for rapid cell proliferation. For this reason, it is known that cancer cells require less oxygen and produce a larger amount of lactic acid than normal cells.

Therefore, a change in the composition of the cancer microenvironment due to abnormal metabolism occurring in cancer cells, an inhibition of apoptosis caused by dysfunctional mitochondria, and an increase in inflammatory response, and abnormal metabolic reaction in cancer cells play a very important role in cancer proliferation. Thus, developing metabolism-related anticancer agents using these features may be a good way capable of solving the side effects and economic problems of conventional anticancer agents.

It is known that mitochondria enter into cells when the mitochondria present in the cells are isolated, and the cells are treated therewith in vitro, or the mitochondria are injected into the body. By using this phenomenon, it is possible that normal mitochondria isolated from cells are injected into the body to treat diseases caused by mitochondrial dysfunction, or in particular, to treat diseases by delivering effectively a specific protein into cells by using mitochondria as a carrier, but no reports have been made on this.

### Technical Problem

An object of the present invention is to provide an effective protein delivery system by showing that mitochondria can be used as a means to effectively deliver proteins capable of exhibiting various pharmacological effects into cells. In addition, an object of the present invention is to provide a recombinant protein for effectively delivering a drug, and to provide modified mitochondria that is produced using the same. In addition, an object of the present invention is to provide a pharmaceutical composition comprising the modified mitochondria as an active ingredient.

### Solution to Problem

In order to solve the above problems, there is provided modified mitochondria in which a foreign protein is bound to the outer membrane of the mitochondria. In addition, in order to prepare the modified mitochondria, there is provided a fusion protein comprising a mitochondrial outer membrane anchoring peptide and a desired pharmacological protein. In addition, there is provided a fusion protein comprising an antibody or a fragment thereof and a mitochondrial outer membrane anchoring peptide.

## Effect of the Invention

When the mitochondria to which the foreign protein is bound are administered to the human body, the foreign protein may be effectively delivered into the cell. In addition, the damaged function of the cell can be repaired by a pharmacologically active protein delivered into the cell. In addition, when the mitochondria to which the foreign protein comprising a pharmacologically active protein is bound are delivered into the cell, the pharmacologically active protein is dissociated from the mitochondria in the cell, and a useful role can be expected. In addition, the modified mitochondria comprising an antibody fragment may be effectively delivered to targeted cells. In particular, when a fragment of an antibody targeting a protein present on the surface of cancer tissue is bound to the surface of the mitochondria, the modified mitochondria may be effectively delivered into cancer cells. Therefore, the introduction of the modified mitochondria not only may restore the damaged electron transport system of the cells, but also may prevent or treat various diseases by the pharmacologically active protein bound to the modified mitochondria.

## Brief Description of Drawings

Figure 1 is a schematic diagram of a method for preparing pTA-p53.
Figure 2 is a schematic diagram of a method for preparing a pET15b-UB-p53 vector.
Figure 3 shows the expression of a UB-p53 protein in E. coli.
Figure 4 is a schematic diagram of a method for preparing a pET11C-TOM70-UB-p53 vector.
Figure 5 shows the expression of a TOM70-UB-p53 protein in E. coli.
Figure 6 is a schematic diagram of a method for preparing a pET11C-TOM70-(GGGGS)3-UB-p53 vector.
Figure 7 shows the expression of a TOM70-(GGGGS)3-UB-p53 protein in E. coli.
Figure 8 shows a method of preparing a pET11C-TOM70-(GGGGS)3-p53 vector.
Figure 9 shows the expression of a TOM70-(GGGGS)3-p53 protein in E. coli.
Figure 10 shows a method of preparing a pET15b-UB-p53-TOM7 vector.
Figure 11 shows the expression of a UB-p53-TOM7 protein in E. coli.
Figure 12 shows a method of preparing a pCMV-p53-myc/His vector.
Figure 13 shows the expression of a p53-myc/His protein in transformed CHO.
Figure 14 shows the results of purifying a TOM70-(GGGGS)3-p53 protein and then identifying the same.
Figure 15 is a view showing a purified TOM70-(GGGGS)3-p53 protein.
Figure 16 shows the results of purifying a TOM70-(GGGGS)3-UB-p53 protein and then identifying the same.
Figure 17 is a view showing a purified TOM70-(GGGGS)3-UB-p53 protein.
Figure 18 shows the results of purifying a UB-p53 protein and then identifying the same.
Figure 19 is a view showing a purified UB-p53 protein.
Figure 20 shows the results of purifying a UB-p53-TOM7 protein and then identifying the same.
Figure 21 is a view showing a purified UB-p53-TOM7 protein.
Figure 22 shows a method of preparing a pTA-GranzymeB vector.
Figure 23 shows a method of preparing a pET11C-TOM70-(GGGGS)3-UB-GranzymeB vector.
Figure 24 shows the expression of a TOM70-(GGGGS)3-UB-GranzymeB protein in E. coli.
Figure 25 shows a method of preparing a pET15b-UB-GranzymeB-TOM7 vector.
Figure 26 shows the expression of a UB-GranzymeB-TOM7 protein in E. coli.
Figure 27 shows the results of purifying a TOM70-(GGGGS)3-UB-Granzyme B protein.
Figure 28 is a view showing a purified TOM70-(GGGGS)3-UB-GranzymeB protein.
Figure 29 shows a method of preparing a pTA-RKIP vector.
Figure 30 shows a method of preparing a pET11C-TOM70-(GGGGS)3-UB-RKIP vector.
Figure 31 shows the expression of a TOM70-(GGGGS)3-UB-RKIP protein in E. coli.
Figure 32 shows the results of purifying a TOM70-(GGGGS)3-UB-RKIP protein.
Figure 33 is a view showing a purified TOM70-(GGGGS)3-UB-RKIP protein.
Figure 34 shows a method of preparing a pTA-PTEN vector.
Figure 35 shows a method of preparing a pET11C-TOM70-(GGGGS)3-UB-PTEN vector.
Figure 36 shows the expression of a TOM70-(GGGGS)3-UB-PTE protein in E. coli.
Figure 37 shows the results of purifying a TOM70-(GGGGS)3-UB-PTEN protein.
Figure 38 is a view showing a purified TOM70-(GGGGS)3-UB-PTEN protein.
Figure 39 shows the results of purifying a UB-GFP-TOM7 protein and then identifying the same.

Figure 40 is a view showing a purified UB-GFP-TOM7 protein.

Figure 41 shows the results of purifying a TOM70-(GGGGS)3-UB-GFP protein and then identifying the same

Figure 42 is a view showing a purified TOM70-(GGGGS)3-UB-GFP protein.

Figure 43 shows a method of preparing a pET15b-UB-scFvBER2-TOM7 vector.

Figure 44 shows the expression of a UB-scFvHER2-TOM7 protein in E. coli.

Figure 45 shows a method of preparing a pCMV-scFvHER2-TOM7-myc/His vector.

Figure 46 shows the expression of a scFvHER2-TOM7-myc/His protein in transformed CHO.

Figure 47 shows the results of purifying a UB-ScFvHER2-TOM7 protein.

Figure 48 is a view showing a purified UB-ScFvHER2-TOM7 protein.

Figure 49 shows a method of preparing a pET15b-UB-scFvMEL-TOM7 vector.

Figure 50 shows the expression of a UB-scFvMEL-TOM7 protein in E. coli.

Figure 51 shows a method of preparing a pCMV-scFvMEL-TOM7-myc/His vector.

Figure 52 shows the expression of a scFvMEL-TOM7-myc/His protein in transformed CHO.

Figure 53 shows a method of preparing a pCMV-scFvPD-L1-TOM7-myc/His vector.

Figure 54 shows the expression of a scFvPD-L1-TOM7-myc/His protein in transformed CHO.

Figure 55 is a view confirming whether a fluorescent protein is bound to the outer membrane of the mitochondria. In this case, the mitochondria are stained with MitoTracker CMXRos to show red color, and TOM70-UB-GFP shows green color. The area where the two portions are overlapped shows yellow color. In this case, the magnification of 55a is 200-fold, and the magnification of 55b is 600-fold.

Figure 56 shows the results of identifying the recombinant protein TOM70-(GGGGS)3-UB-p53 and UB-p53-TOM7 bound to the outer membrane of the foreign mitochondria using Western blot analysis.

Figure 57 shows the results of observing the degree of intracellular injection according to the concentration of mitochondria using a fluorescence microscope after isolation of foreign mitochondria, and then injection of the mitochondria into cells.

Figure 58 is a view confirming the influence of normal mitochondria on the proliferation of skin cancer cells.

Figure 59 is a view confirming the influence of normal mitochondria on the inhibition of reactive oxygen species (ROS) production in skin cancer cells.

Figure 60 is a view confirming the influence of normal mitochondria on drug resistance.

Figure 61 is a view confirming the influence of normal mitochondria on the expression of an antioxidant gene in cells.

Figure 62 is a view showing the influence of normal mitochondria on the expression of a gene involved in cancer cell metastasis.

Figure 63 is a schematic diagram of a method for confirming loading of the recombinant protein p53 on the outer membrane of the foreign mitochondria and injection of the recombinant protein p53 into the cell.

Figure 64 is a view confirming that the recombinant protein p53 is loaded on the outer membrane of the foreign mitochondria and that the p53 is injected into the cell. In this case, the magnification is 200-fold.

Figure 65 is a view confirming that the recombinant protein p53 is loaded on the outer membrane of the foreign mitochondria and that the p53 is injected into the cell. In this case, the magnification is 600-fold.

Figure 66 is a schematic diagram of a method for confirming the apoptosis ability of the modified mitochondria on which p53 injected into the cells is loaded, using a gastric cancer cell line.

Figure 67a is a view confirming the apoptosis ability of the modified mitochondria on which p53 injected into gastric cancer cells is loaded, through a TUNEL assay. In this case, the magnification is 600-fold.

Figure 67b is a view confirming the apoptosis ability of the modified mitochondria on which p53 injected into gastric cancer cells is loaded, through a fluorescence measurement.

Figure 68 is a view confirming the effect of inhibiting cancer cell metastasis by the modified mitochondria loaded with RKIP in MDA-MB-231 cells.

Figure 69 is a view confirming that a single chain variable fragment (ScFv) antibody for targeting cancer cells is expressed in cells.

Figure 70 is a view confirming that a single chain variable fragment (ScFv) antibody for targeting cancer cells is expressed and bound to mitochondria present in the cell using an immunocytochemistry (ICC) experimental method. In this case, the magnification is 200-fold.

Figure 71 is a view confirming that a single chain variable fragment (ScFv) antibody for targeting cancer cells is expressed and bound to mitochondria present in the cell using an immunocytochemistry (ICC) experimental method. In this case, the magnification is 600-fold.

Figure 72 is a view comparing the effect of injecting the mitochondria to which a single chain variable fragment antibody for targeting cancer cells is bound into the gastric cancer cell line.

Figure 73 is a schematic diagram of an animal experiment schedule using the modified mitochondria.

Figure 74 is a photograph in which an increase in a tumor tissue is visually observed.

Figure 75 is a view confirming the change in body weight of mice after administration of the mitochondria and the modified mitochondria.

Figure 76 is a view confirming the tumor size after administration of the mitochondria and the modified mitochondria.

Figure 77 is a view confirming that the modified mitochondria loaded with the TOM-UB-p53 protein is effective in inhibiting the proliferation of A431 cells.

Figure 78 is a view confirming the function of the isolated mitochondria by ATP content.

Figure 79 is a view confirming the function of the isolated mitochondria by membrane potential.

Figure 80 is a view confirming the degree of damage of isolated mitochondria by measuring the mitochondrial ROS (mROS production)

Figure 81a is a view showing the structure of the protein present in the outer membrane of the mitochondria and the amino acid sequence of the N terminal region of TOM70, TOM20 or OM45.

Figure 81b is a view showing the amino acid sequence of the C terminal region of TOM5, TOM7, Fis1, VAMP1B, Cytb5, BCL-2 or BCL-X.

Figure 82 is a view confirming whether the desired protein is dissociated according to the presence or absence of a linker between the outer membrane anchoring peptide and ubiquitin.

Figure 83 is a view confirming that the desired protein bound to the modified mitochondria is separated off from the mitochondria in the cell.

## Best Mode for Carrying out the Invention

Hereinafter, the present invention will be described in detail.

One aspect of the present invention provides modified mitochondria in which a foreign protein is bound to the outer membrane of the mitochondria.

The mitochondria may be obtained from mammals, and may be obtained from humans. Specifically, the mitochondria may be isolated from cells or tissues. For example, the mitochondria may be obtained from somatic cells, germ cells, or stem cells. In addition, the mitochondria may be normal mitochondria obtained from cells in which the biological activity of mitochondria is normal. In addition, the mitochondria may be cultured in vitro.

In addition, the mitochondria may be obtained from an autologous, allogenic, or xenogenic subject. Specifically, the autologous mitochondria refer to mitochondria obtained from tissues or cells of the same subject. In addition, the allogenic mitochondria refer to mitochondria obtained from a subject that belongs to the same species as the subject and has different genotypes for alleles. In addition, the xenogenic mitochondria refer to mitochondria obtained from a subject that belongs to the different species from the subject.

Specifically, the somatic cells may be muscle cells, hepatocytes, nerve cells, fibroblasts, epithelial cells, adipocytes, osteocytes, leukocytes, lymphocytes, platelets, or mucosal cells. In addition, the germ cells are cells that undergo meiosis and mitosis, and may be sperms or eggs. In addition, the stem cells may be any one selected from the group consisting of mesenchymal stem cells, adult stem cells, induced pluripotent stem cells, embryonic stem cells, bone marrow stem cells, neural stem cells, limbal stem cells, and tissue-derived stem cells. In this case, the mesenchymal stem cells may be any one selected from the group consisting of umbilical cord, umbilical cord blood, bone marrow, fat, muscle, nerve, skin, amniotic membrane, and placenta.

On the other hand, when the mitochondria are isolated from specific cells, the mitochondria can be isolated through various known methods, for example, using a specific buffer solution or using a potential difference and a magnetic field and the like.

As used herein, the term "foreign protein" refers to a protein that includes a desired protein capable of functioning inside and outside the cell. In this case, the foreign protein is a protein that does not exist in the mitochondria and may be a recombinant protein. Specifically, the foreign protein may comprise a mitochondria anchoring peptide and a desired protein. In addition, the foreign protein may be a recombinant fusion protein comprising a mitochondria anchoring peptide and a desired protein. In this case, the foreign protein may comprise a mitochondria anchoring peptide. Preferably, the mitochondria anchoring peptide may be a peptide that can be located on the mitochondrial outer membrane. Therefore, the foreign protein can be bound to the outer membrane of the mitochondria by a mitochondria anchoring peptide. The mitochondria anchoring peptide may be a peptide comprising an N terminal region or a C terminal region of a protein present in a mitochondrial membrane protein, and the N terminal region or the C terminal region of a protein present in the outer membrane of the mitochondria protein may be located on the outer membrane of the mitochondria. In this case, the anchoring peptide may further comprise a mitochondria signal sequence.

One embodiment of the protein present in a mitochondrial membrane protein may be any one selected from the group consisting of TOM20, TOM70, OM45, TOM5, TOM6, TOM7, TOM22, Fis1, Bcl-2, Bcl-x and VAMP1B. In particular, when the mitochondria anchoring peptide is derived from any one selected from the group consisting of TOM20, TOM70 and OM45, it may comprise the N terminal region of TOM20, TOM70 or OM45. One embodiment of the mitochondria anchoring peptide may be TOM70 derived from yeast represented by SEQ ID NO: 75, or TOM70 derived from human represented by SEQ ID NO: 76. Another embodiment may be TOM20 derived from yeast represented by SEQ ID NO: 77, or TOM20 derived from human represented by SEQ ID NO: 78. Another embodiment may be OM45 derived from yeast represented by SEQ ID NO: 79.

In addition, when the mitochondria anchoring peptide is derived from any one selected from the group consisting of TOM5, TOM6, TOM7, TOM22, Fis1, Bcl-2, Bcl-x and VAMP1B, it may comprise the C terminal region of any one selected from the group consisting of TOM5, TOM6, TOM7, TOM22, Fis1, Bcl-2, Bcl-x and VAMP1B. One embodiment of the mitochondria anchoring peptide may be TOM5 derived from yeast represented by SEQ ID NO: 80 or TOM5 derived from human represented by SEQ ID NO: 81. Another embodiment may be TOM7 derived from yeast represented by SEQ ID NO: 82, or TOM7 derived from human represented by SEQ ID NO: 83. Another embodiment may be TOM22 derived from yeast represented by SEQ ID NO: 84, or TOM22 derived from human represented by SEQ ID NO: 85. Another embodiment may be Fis1 derived from yeast represented by SEQ ID NO: 86, or Fis1 derived from human represented by SEQ ID NO: 87. Another embodiment may be Bcl-2 alpha derived from human represented by SEQ ID NO: 88. Another embodiment may be VAMP1 derived from yeast represented by SEQ ID NO: 89, or VAMP1 derived from human represented by SEQ ID NO: 90.

In this case, a desired protein capable of functioning inside and outside the cell included in the foreign protein may be any one selected from the group consisting of an active protein exhibiting an activity in a cell, a protein present in a cell, and a protein having the ability to bind to a ligand or receptor present in a cell membrane.

An embodiment of the active protein or the protein present in a cell may be any one selected from the group consisting of p53, Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), RAF kinase inhibitory protein(RKIP), p16, KLF-10, LKB1, LHX6, C-RASSF, DKK-3PD1, Oct3/4, Sox2, Klf4, and c-Myc. When the desired protein is selected from the above group, the desired protein may be bound to an anchoring peptide comprising the N terminal region of TOM20, TOM70 or OM45.

Such fusion protein may be bound in the following order:
N terminal-anchoring peptide comprising the N terminal region of TOM20, TOM70 or OM45-desired protein-C terminal.

In addition, the foreign protein may further comprise an amino acid sequence recognized by a proteolytic enzyme in eukaryotic cells, or ubiquitin or a fragment thereof between a mitochondria anchoring peptide and a desired protein. The proteolytic enzyme in eukaryotic cells refers to an enzyme that degrades a protein present in eukaryotic cells. In this case, because a foreign protein comprises an amino acid sequence recognized by the enzyme that degrades the protein, the foreign protein bound to the mitochondrial outer membrane may be isolated into an anchoring peptide and a desired protein in a cell.

In this case, the ubiquitin fragment may comprise the C terminal Gly-Gly of an amino acid sequence of SEQ ID NO: 71, and may comprise 3 to 75 amino acids consecutive from the C terminal. In addition, the foreign protein may further comprise a linker between a desired protein and ubiquitin or a fragment thereof. In this case, the linker may be composed of 1 to 150 amino acids, or be composed of 10 to 100 amino acids, or be composed of 20 to 50 amino acids, but is not limited thereto. The linker may be composed of amino acids that are appropriately selected from 20 amino acids, preferably be composed of glycine and/or serine. One embodiment of the linker may be composed of 5 to 50 amino acids consisting of glycine and serine. One embodiment of the linker may be (G4S)n, in which n is an integer of 1 to 10, and n may be 1, 2, 3, 4, 5, 6, 7, 8, 9, or 10.

In addition, the protein having the ability to bind to a ligand or receptor present in a cell membrane may be a ligand or receptor present on the surface of a tumor cell. In this case, the ligand or receptor present on the surface of a tumor cell may be, but is not limited to, any one selected from the group consisting of CD19, CD20, melanoma antigen E(MAGE), NY-ESO-1, carcinoembryonic antigen(CEA), mucin 1 cell surface associated(MUC-1), prostatic acid phosphatase(PAP), prostate specific antigen(PSA), survivin, tyrosine related protein 1(tyrp1), tyrosine related protein 1(tyrp2), Brachyury, Mesothelin, Epidermal growth factor receptor(EGFR), human epidermal growth factor receptor 2(HER-2), ERBB2, Wilms tumor protein(WT1), FAP, EpCAM, PD-L1, ACPP, CPT1A, IFNG, CD274, FOLR1, EPCAM, ICAM2, NCAM1, LRRC4, UNC5H2 LILRB2, CEACAM, Nectin-3 and a combination thereof.

In addition, the protein having the ability to bind to a ligand or receptor present in a cell membrane may be an antibody or a fragment thereof that binds to any one selected from the above group. In particular, a fragment of an antibody refers to a fragment having the same complementarity determining region (CDR) as that of the antibody. Specifically, it may be Fab, scFv, F (ab')2 or a combination thereof.

In this case, the desired protein may be bound to an anchoring peptide comprising an C terminal region of any one selected from the group consisting of TOM5, TOM6, TOM7, TOM22, Fis1, Bcl-2, Bcl-x and VAMP1B, and the foreign protein may be bound in the following order:
N terminal-desired protein-anchoring peptide comprising a C terminal region of any one selected from the group consisting of TOM5, TOM6, TOM7, TOM22, Fis1, Bcl-2, Bcl-x and VAMP1B-C terminal.

In addition, the foreign protein may further comprise a linker between a desired protein and a C terminal region of any one selected from the group consisting of TOM5, TOM6, TOM7, TOM22, Fis1, Bcl-2, Bcl-x and VAMP1B. In this case, a linker is as described above. In this case, a desired protein, an active protein, a protein present in a cell, and a protein having the ability to bind to a ligand or receptor present in a cell membrane and the like are as described above.

In one embodiment of the desired protein, an antibody or a fragment thereof targeting a specific cell may be in a form bound to the anchoring peptide comprising the C terminal region of any one selected from the group consisting of TOM5, TOM6, TOM7, TOM22, Fis1, Bcl-2, Bcl-x and VAMP1B. The modified mitochondria to which the desired protein is bound can be easily introduced into a specific target, so that the mitochondria can be efficiently entered into a specific cell.

One embodiment of the modified mitochondria may be in a form to which one or more desired proteins are bound. Specifically, it may be in a form to which a desired protein comprising p53 and a desired protein comprising anti-HER-2 antibody or a fragment thereof are bound. Such modified mitochondria may effectively deliver the mitochondria into cancer cells expressing HER-2. In addition, cancer cells may be effectively killed by p53 bound to the modified mitochondria.

Depending on the purpose of the modified mitochondria, a desired protein comprising one or more active proteins may be constructed and be allowed to be bound to the mitochondria. In addition, a desired protein targeting a cell may be constructed in various ways depending on the targeted cell.

In another aspect of the present invention, there is provided a pharmaceutical composition comprising the above described modified mitochondria as an active ingredient. In this case, use of the pharmaceutical composition may be for the prevention or treatment of cancer. In this case, the cancer may be any one selected from the group consisting of gastric cancer, liver cancer, lung cancer, colorectal cancer, breast cancer, prostate cancer, ovarian cancer, pancreatic cancer, cervical cancer, thyroid cancer, larynx cancer, acute myeloid leukemia, brain tumor, neuroblastoma, retinoblastoma, head and neck cancer, salivary gland cancer and lymphoma.

Specifically, when the active protein kills tumor cells, like p53, or when a protein that inhibits the proliferation is bound to the mitochondria, the modified mitochondria to which p53 is bound may be used as an anticancer agent. In addition, when a protein such as RKIP capable of inhibiting metastasis of cancer cells is bound to the mitochondria, the modified mitochondria to which RKIP is bound may be used as an inhibitor of tumor metastasis. When any one selected from the group consisting of Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), p16, KLF-10, LKB1, LHX6, C-RASSF, DKK-3PD1 and a combination thereof, which are proteins that inhibit the proliferation of cancer cells, or control the phosphorylation reaction in cancer cells, or inhibit the metastasis of cancer cells, is bound to the mitochondria, the modified mitochondria to which the active protein is bound may be used as an anticancer agent.

In addition, for the pharmaceutical composition, the mitochondria may be included at a concentration of 0.1 μg/mL to 500 μg/mL, 0.2 μg/mL to 450 μg/mL, or 0.5 μg/mL to 400 μg/mL, but is not limited thereto. The inclusion of the mitochondria in the above range may facilitate the dose adjustment of mitochondria upon administration and may enhance the degree of improvement of the symptoms of a disease of a patient. In this case, the dose of mitochondria may be determined through the quantification of mitochondria by quantifying the membrane protein of the isolated mitochondria. Specifically, the isolated mitochondria may be quantified through the Bradford protein assay (a paper written by James D. McCully (J Vis Exp. 2014; (91): 51682.).

In addition, for the pharmaceutical composition, an active protein binding to mitochondria may be included at a concentration of 0.1 μg/mL to 500 μg/mL, 0.2 μg/mL to 450 μg/mL, or 0.5 μg/mL to 400 μg/mL, but is not limited thereto. The inclusion of the active protein in the above range may facilitate the dose adjustment of an active protein upon administration and may enhance the degree of improvement of the symptoms of a disease of a patient.

In addition, for the pharmaceutical composition, a targeting protein capable of delivering mitochondria to a specific cell may be included at a concentration of 0.1 μg/mL to 500 μg/mL, 0.2 μg/mL to 450 μg/mL or 0.5 μg/mL to 400 μg/mL, but is not limited thereto. The inclusion of the targeting protein in the above range may facilitate the dose adjustment of a targeting protein upon administration and may enhance the degree of improvement of the symptoms of a disease of a patient.

In particular, the pharmaceutical composition according to the present invention may be administered with mitochondria in an amount of, but not limited thereto, 0.01-5 mg/kg, 0.1-4 mg/kg, or 0.25-2.5 mg/kg per one time on the basis of the body weight of an individual to be administered. That is, it is most preferable in terms of the cell activity to administer the pharmaceutical composition such that the amount of the modified mitochondria falls within the above range on the basis of the body weight of an individual having cancer tissues. In addition, the pharmaceutical composition may be administered 1-10 times, 3-8 times, or 5-6 times, and preferably 5 times. In this case, the administration interval may be 1-7 days, or 2-5 days, and preferably 3 days.

In addition, the pharmaceutical composition according to the present invention may be administered to a human or other mammal that is susceptible to cancer or suffering from cancer. In addition, the pharmaceutical composition may be an injectable preparation that may be intravenously administered or an injectable preparation that may be topically administered, and may be preferably a preparation for injections.

Therefore, the pharmaceutical composition according to the present invention may be prepared as a physically or chemically highly stable injectable preparation by adjusting the pH of the composition by means of a buffer solution such as an acid aqueous solution or phosphate which may be used in an injectable preparation, in order to ensure the stability of the product during distribution of injectable preparations.

Specifically, the pharmaceutical composition of the present invention may contain water for injection. The water for injection is distilled water prepared for dissolving a solid injectable preparation or diluting a water-soluble injectable preparation, and may be glucose injection, xylitol injection, D-mannitol injection, fructose injection, saline, dextran 40 injection, dextran 70 injection, amino acid injection, Ringer's solution, lactic acid-Ringer's solution, phosphate buffer solution having a pH of 3.5 to 7.5, sodium dihydrogen phosphate-citrate buffer solution or the like.

In addition, the pharmaceutical composition of the present invention may include a stabilizer or a dissolution aid. For example, the stabilizer may be sodium pyrosulfite or ethylenediaminetetraacetic acid, and the dissolution aid may be hydrochloric acid, acetic acid, sodium hydroxide, sodium hydrogen carbonate, sodium carbonate or potassium hydroxide.

In addition, the present invention may provide a method for preventing or treating cancer including administering the above-mentioned pharmaceutical composition to an individual. Here, the individual may be a mammal, and preferably a human.

One aspect of the present invention provides a method for preparing the modified mitochondria, comprising a step of mixing the isolated mitochondria with a desired protein comprising an active protein and/or a desired protein comprising a target targeting protein.

In this case, the desired protein and the mitochondria may be mixed in an appropriate ratio. For example, the desired protein : mitochondria may be mixed in a ratio of 1 : 100 to 100 : 1 based on a weight ratio. Specifically, they may be mixed in a ratio of 1 : 10, 1 : 5, 1 : 4, 1 : 3, 1 : 2 or 1 : 1. In addition, the ratio may be 10 : 1, 5 : 1, 4 : 1, 3 : 1 or 2 : 1.

In another aspect of the present invention, there is provided a method for preparing the modified mitochondria from transformed cells by injecting a polynucleotide encoding the above described desired protein into eukaryotic cells. Specifically, there is provided a method for preparing the above described fusion protein, comprising a step of transforming the above described polynucleotide into prokaryotic cells or eukaryotic cells without a ubiquitin degrading enzyme or a proteolytic enzyme in eukaryotic cells; and a step of obtaining a fusion protein. This preparation method is suitable when the desired protein does not comprise an amino acid sequence recognized by a proteolytic enzyme in eukaryotic cells or ubiquitin or a fragment thereof.

In another aspect of the present invention, a desired protein may be prepared using a prokaryotic cell or a prokaryotic cell extract. In addition, there is provided a method for preparing the modified mitochondria using eukaryotic cells without a ubiquitin degrading enzyme or a proteolytic enzyme, or a eukaryotic cell extract.

In another aspect of the present invention, there is provided use of the mitochondria as a means of delivery of a foreign protein. Specifically, the modified mitochondria may be used as a means of intracellular and extracellular delivery of a foreign protein comprising a desired protein capable of functioning inside and outside the cell. The mitochondria may be effectively introduced into cells, and in this case, a foreign protein desired to be delivered to cells may be effectively delivered into cells. In this case, the mitochondria may be used as an effective protein delivery system. The desired protein is as described above.

Another aspect of the present invention provides a fusion protein comprising a mitochondrial outer membrane anchoring peptide and a desired protein. In this case, the desired protein is as described above.

As used herein, the term "mitochondrial outer membrane anchoring peptide" may be the N terminal or C terminal of a protein present in the outer membrane of the mitochondria. The mitochondrial outer membrane anchoring peptide may have an amino acid sequence that is specifically located in the outer membrane of the mitochondria. In this case, the mitochondrial outer membrane anchoring peptide allows the fusion protein disclosed in the present invention to be attached to the outer membrane of the mitochondria. In this case, the mitochondrial outer membrane anchoring peptide may be used in the same sense as the mitochondrial outer membrane targeting peptide.

In addition, the mitochondrial outer membrane anchoring peptide prevents the fusion protein disclosed in the present invention from entering the inside of the mitochondria. The TOM (translocase of the outer membrane) complex present in the mitochondrial outer membrane has a mitochondria target sequence and a single outer membrane anchoring domain at the amino terminus, and most of the carboxy terminus may have a structure that is exposed to the cytoplasm (Figure 81a). The TOM (translocase of the outer membrane) complex present in the mitochondrial outer membrane has a mitochondria target sequence and a single outer membrane anchoring domain at the carboxyl terminus, and most of the amino terminus may also have a structure that is exposed to the cytoplasm (Figure 81b). In addition, the protein present in the outer membrane of the mitochondria may be selected from proteins present in the mitochondria that are present in a eukaryotic cell. For example, it may be selected from proteins present in the mitochondrial outer membrane that are present in yeast, animal cells, or human cells.

In this case, an embodiment of the protein present in the mitochondrial outer membrane may be any one protein selected from the group consisting of TOM20, TOM70, OM45, TOM5, TOM6, TOM7, TOM22, Fis1, Bcl-2, Bcl-x and VAMP1B, or a fragment thereof. In this case, the mitochondrial outer membrane anchoring peptide may be a fragment of any one protein selected from the group consisting of TOM20, TOM70, OM45, TOM5, TOM6, TOM7, TOM22, Fis1, Bcl-2, Bcl-x and VAMP1B. In this case, the outer membrane anchoring peptide may be a C terminal or N terminal polypeptide of TOM20, TOM70, OM45, TOM5, TOM6, TOM7, TOM22, Fis1, Bcl-2, Bcl-x and VAMP1B located in the mitochondrial outer membrane.

In particular, when the mitochondrial outer membrane anchoring peptide is fused to the N terminal of the desired protein, the mitochondrial outer membrane anchoring peptide may comprise a terminal sequence of a protein selected from the group consisting of TOM20, TOM70, and OM45. Preferably, it may be an N terminal sequence of a protein selected from the group consisting of TOM20, TOM70, and OM45. An embodiment of the mitochondrial outer membrane anchoring peptide is as described above.

In addition, when the mitochondrial outer membrane anchoring peptide is fused to the C terminal of the desired protein, the outer membrane targeting protein may comprise a terminal sequence of a protein selected from the group consisting of TOM5, TOM6, TOM7, TOM22, Fis1, Bcl-2, Bcl-X, and VAMP1B. Preferably, it may be a C terminal sequence of a protein selected from the group consisting of TOM5, TOM6, TOM7, TOM22, Fis1, Bcl-2, Bcl-X, and VAMP1B. An embodiment of the mitochondrial outer membrane anchoring peptide is as described above.

As used herein, the term "active protein" may be a protein exhibiting physiological activity. One embodiment of such an active protein may be a protein having decreased function or a modified protein present in damaged cancer cells. One embodiment of the active protein may be a protein that enhances the activity of cells. An embodiment of such an active protein is as described above.

The fusion protein may be a protein to which a mitochondrial outer membrane targeting protein and a desired protein are bound from the N terminal to the C terminal. In this case, it may further comprise ubiquitin or a fragment thereof having a ubiquitin protease specific cleavage site (Glycin-Glycin) between the mitochondrial outer membrane targeting protein and the desired protein. In this case, in order to facilitate cleavage by the ubiquitin protease, it may further comprise a linker containing hydrophilic and polar amino acids, serine, glycine and threonine, between the mitochondrial outer membrane targeting protein and the ubiquitin protein.

As used herein, the term "ubiquitin" refers to a protein that participates in the proteolytic process, also referred to as UB. One embodiment of ubiquitin may be ubiquitin present in the human body or ubiquitin present in yeast. Ubiquitin present in the human body is composed of 76 amino acids. In this case, ubiquitin may be used in a mature form. As used herein, the term "mature form" may refer to a protein in a form from which a signal peptide is removed.

In addition, an enzyme referred to as ubiquitin protease or UBP (ubiquitin-specific protease) is naturally present in eukaryotic cells and may induce the natural dissociation of a desired protein by cleaving the C terminal amino acid glycine-glycine site of ubiquitin in a cell.

In this case, the fragment of ubiquitin may comprise the Gly-Gly amino acid of the C terminal of ubiquitin, and may comprise 3 to 75 amino acids consecutive from the C terminal. Specifically, an embodiment of the fragment of ubiquitin may be Arg-Gly-Gly, Leu-Arg-Gly-Gly, Arg-Leu-Arg-Gly-Gly, or Leu-Arg-Leu-Arg-Gly-Gly. In addition, the fragment of ubiquitin may have an amino acid sequence of SEQ ID NO: 71.

The fusion protein comprising the mitochondrial outer membrane targeting protein and the desired protein may be referred to as a fusion protein that modifies the mitochondria activity. Such fusion protein may have any one of the following structures:

<Structural Formula 1>

N terminal-mitochondrial outer membrane anchoring peptide-desired protein-C terminal

<Structural Formula 2>

N terminal-mitochondrial outer membrane anchoring peptide-ubiquitin or fragment thereof-desired protein-C terminal

<Structural Formula 3>

N terminal-mitochondrial outer membrane targeting peptide-linker 1-ubiquitin or fragment thereof-desired protein-C terminal

<Structural Formula 4>

N terminal-mitochondrial outer membrane anchoring peptide-ubiquitin or fragment thereof-linker 2-desired protein-C terminal

<Structural Formula 5>

N terminal-mitochondrial outer membrane anchoring peptide-linker 1-ubiquitin or fragment thereof-linker 2-desired protein-C terminal

In the above Structural Formulae 1 to 5, the outer membrane anchoring peptide may be a terminal sequence of a protein selected from the group consisting of TOM20, TOM70 and OM45, and the desired protein may be any one selected from the group consisting of p53, Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), RAF kinase inhibitory protein(RKIP), p16, KLF-10, LKB1, LHX6, C-RASSF and DKK-3PD1.

In this case, the linker 1 or 2 may be a polypeptide composed of 1 to 100, 1 to 80, 1 to 50, or 1 to 30 amino acids, respectively, and may be preferably a polypeptide composed of 1 to 30 amino acids that consist of serine, glycine or threonine alone or in combination. In addition, the linker 1 or 2 may be a polypeptide composed of 5 to 15 amino acids, respectively, and may be preferably a polypeptide composed of 5 to 15 amino acids that consist of serine, glycine or threonine alone or in combination. One embodiment of the linker may be (GGGGS)3 (SEQ ID NO: 70).

<Structural Formula 6>

N terminal-desired protein-mitochondrial outer membrane anchoring peptide-C terminal

<Structural Formula 7>

N terminal-desired protein-ubiquitin or a fragment thereof-mitochondrial outer membrane anchoring peptide-C terminal

<Structural Formula 8>

N terminal-desired protein-linker 1-ubiquitin or a fragment thereof-mitochondrial outer membrane anchoring peptide-C terminal

<Structural Formula 9>

N terminal-desired protein-ubiquitin or a fragment thereof-linker 2-mitochondrial outer membrane anchoring peptide-C terminal

<Structural Formula 10>

N terminal-desired protein-linker 1-ubiquitin or fragment thereof-linker 2-mitochondrial outer membrane targeting peptide-C terminal

In the above Structural Formulae 6 to 10, the outer membrane anchoring peptide may be a terminal sequence of a protein selected from the group consisting of TOM5, TOM6, TOM7, TOM22, Fis1, Bcl-2, Bcl-X, and VAMP1B, and the desired protein may be any one selected from the group consisting of p53, Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), RAF kinase inhibitory protein(RKIP), p16, KLF-10, LKB1, LHX6, C-RASSF, DKK-3PD1, Oct3/4, Sox2, Klf4, and c-Myc. In this case, the linker 1 or 2 is as described above.

One aspect of the present invention provides a polynucleotide encoding a fusion protein comprising a mitochondrial outer membrane anchoring peptide and a desired protein.

In addition, one aspect of the present invention provides a vector loaded with the polynucleotide encoding a fusion protein comprising a desired protein.

In addition, one aspect of the present invention provides a host cell in which a vector loaded with a polynucleotide encoding a fusion protein comprising the desired protein is introduced.

One aspect of the present invention provides a fusion protein comprising a target targeting protein and a mitochondrial outer membrane targeting protein.

In this case, the target targeting protein and the mitochondrial outer membrane anchoring peptide may be bound from the N terminal to the C terminal. Here, the mitochondrial outer membrane anchoring peptide may be any one selected from the group consisting of TOM20, TOM70, OM45, TOM5, TOM6, TOM7, TOM22, Fis1, Bcl-2, Bcl-x and VAMP1B.

As used herein, the term "target" refers to a place where the modified mitochondria should be delivered. One embodiment of the target may be a cancer cell. Specifically, one embodiment of the target may be a biomarker present on the surface of cancer cells. Specifically, the target may be a tumor-associated antigen (TAA). In this case, the tumor-associated antigen may be any one selected from the group consisting of CD19, CD20, melanoma antigen E(MAGE), NY-ESO-1, carcinoembryonic antigen(CEA), mucin 1 cell surface associated(MUC-1), prostatic acid phosphatase(PAP), prostate specific antigen(PSA), survivin, tyrosine related protein 1(tyrp1), tyrosine related protein 1(tyrp2), Brachyury, Mesothelin, Epidermal growth factor receptor(EGFR), human epidermal growth factor receptor 2(HER-2), ERBB2, Wilms tumor protein(WT1), FAP, EpCAM, PD-L1, ACPP, CPT1A, IFNG, CD274, FOLR1, EPCAM, ICAM2, NCAM1, LRRC4, UNC5H2 LILRB2, CEACAM, Nectin-3 and a combination thereof.

As used herein, the term "target targeting protein" may be a protein sequence capable of binding to the above described target. In this case, one embodiment of the target targeting protein may be a protein that binds to a biomarker present on the surface of cancer cells. In this case, an embodiment of the biomarker present on the surface of cancer cells may be, but is not limited to, ICAM2, NCAM1, LRRC4, UNC5H2 LILRB2, CEACAM, or Nectin-3. In this case, the target targeting protein may be included in the above described foreign protein.

One embodiment of the target targeting protein may be an antibody or a fragment thereof. In particular, it may be an antibody or a fragment thereof that specifically binds to the tumor-associated antigen. In addition, the fragment of the antibody may be any one selected from the group consisting of Fab, Fab', scFv and F(ab)2.

One embodiment of the target targeting protein may be scFvHER capable of binding to an epidermal growth factor receptor. Another embodiment may be scFvMEL capable of targeting melanoma. Another embodiment may be scFvPD-L1 capable of binding to PD-L1 overexpressed on the surface of cancer cells. Another embodiment may be PD-1 capable of binding to PDL-1 overexpressed on the surface of cancer cells.

One aspect of the present invention may further comprise ubiquitin or a fragment thereof between the target targeting protein and the mitochondrial outer membrane targeting protein. The fusion protein comprising the mitochondria target targeting protein and the desired protein may be referred to as a fusion protein that modifies the mitochondria activity. Such fusion protein may have any one of the following structures:

<Structural Formula 11>

N terminal-target targeting protein-mitochondrial outer membrane anchoring peptide-C terminal

<Structural Formula 12>

N terminal-target targeting protein-ubiquitin or a fragment thereof-mitochondrial outer membrane anchoring peptide-C terminal

<Structural Formula 13>

N terminal-target targeting protein-linker 1-ubiquitin or a fragment thereof-mitochondrial outer membrane anchoring peptide-C terminal

<Structural Formula 14>

N terminal-target targeting protein-ubiquitin or fragment thereof-linker 2-mitochondrial outer membrane anchoring peptide-C terminal

<Structural Formula 15>

N terminal-target targeting protein-linker 1-ubiquitin or fragment thereof-linker 2-mitochondrial outer membrane anchoring peptide-C terminal

In the above Structural Formulae 11 to 15, the outer membrane anchoring peptide may be a terminal sequence of a protein selected from the group consisting of TOM5, TOM6, TOM7, TOM22, Fis1, Bcl-2, Bcl-X, and VAMP1B, and the target targeting protein may be any one selected from the group consisting of tumor associated antigens, CD19, CD20, melanoma antigen E(MAGE), NY-ESO-1, carcinoembryonic antigen(CEA), mucin 1 cell surface associated(MUC-1), prostatic acid phosphatase(PAP), prostate specific antigen(PSA), survivin, tyrosine related protein 1(tyrp1), tyrosine related protein 1(tyrp2), Brachyury, Mesothelin, Epidermal growth factor receptor(EGFR), human epidermal growth factor receptor 2(HER-2), ERBB2, Wilms tumor protein(WT1), FAP, EpCAM, PD-L1, ACPP, CPT1A, IFNG, CD274, FOLR1, EPCAM, ICAM2, NCAM1, LRRC4, UNC5H2 LILRB2, CEACAM, Nectin-3 and a combination thererof. In addition, the target targeting protein may be an antibody specifically binding to a tumor associated antigen or a fragment thereof. In this case, linker 1 or 2, and the amino acid sequence recognized by a proteolytic enzyme are as described above.

<Structural Formula 16>

N terminal-mitochondrial outer membrane anchoring peptide-target targeting protein-C terminal

<Structural Formula 17>

N terminal-mitochondrial outer membrane anchoring peptide-ubiquitin or a fragment thereof-target targeting protein-C terminal

<Structural Formula 18>

N terminal-mitochondrial outer membrane anchoring peptide-linker 1-ubiquitin or a fragment thereof-target targeting protein-C terminal

<Structural Formula 19>

N terminal-mitochondrial outer membrane anchoring peptide-ubiquitin or fragment thereof-linker 2-target targeting protein-C terminal

<Structural Formula 20>

N terminal-mitochondrial outer membrane anchoring peptide-linker 1-ubiquitin or fragment thereof-linker 2-target targeting protein-C terminal

In the above Structural Formulae 16 to 20, the outer membrane anchoring peptide may be any one selected from the group consisting of TOM20, TOM70 and OM45. In addition, the target targeting protein, ubiquitin or a fragment thereof, and linker 1 or 2 are as described above.

One aspect of the present invention provides a polynucleotide encoding a fusion protein comprising a target targeting protein.

In addition, one aspect of the present invention provides a vector loaded with the polynucleotide encoding a fusion protein comprising a target targeting protein.

In addition, one aspect of the present invention provides a host cell in which a vector loaded with a polynucleotide encoding a fusion protein comprising the target targeting protein is introduced. The host cell may be a prokaryotic cell or a eukaryotic cell. In this case, preferably, the eukaryotic cell may be a strain from which an enzyme that degrades ubiquitin is removed.

In addition, one aspect of the present invention provides a method of preparing - modified mitochondria from the transformed cells by injecting a polynucleotide encoding the fusion protein into eukaryotic cells.

## MODE FOR THE INVENTION

Hereinafter, a preferred embodiment will be presented to help the understanding of the present invention. However, the following examples are provided only to easily understand the present invention, and the present invention is not limited to the following examples.

### I. Preparation of fusion protein comprising mitochondrial outer membrane anchoring peptide, linker, ubiquitin and desired protein

#### Example 1. Preparation of fusion protein comprising p53

#### Example 1.1. Amplification of p53 gene

In order to express the human p53 into a recombinant protein, total RNA was extracted from human epithelial cells, and cDNA was synthesized therefrom. Specifically, human dermal fibroblast cells were cultured in 10% serum medium under a condition of 5% carbon dioxide and 37°C ($1x10^6$ cells). Thereafter, the culture solution was removed and washed twice by adding a PBS buffer solution to the cells, and 0.5 ml of RNA extract (Trizol reagent, Thermo Fisher Scientific) was added directly. The mixture to which the RNA extract was added was stood at ambient temperature for 10 minutes, and then 0.1 ml of chloroform was added and stirred for 15 seconds, and then centrifuged at about 12,000 xg for 10 minutes. Next, the separated supernatant was taken, and the same volume of isopropyl alcohol was added and centrifuged again at 12,000 xg for 10 minutes. Thereafter, the liquid was removed and washed once with 75% ethanol, and the RNA was dried at ambient temperature.

About 50 ul of purified distilled water without RNAase was added, and the quantity and purity of RNA was measured using a spectrophotometer. In order to synthesize cDNA, 2 ug of purified total RNA was subjected to a binding reaction with oligo dT at 70°C for 5 minutes. Thereafter, 10X reverse transcription reaction buffer solution, 10 mM dNTP, RNAse inhibitor and M-MLV reverse transcriptase (Enzynomics, Korea) were added, and cDNA synthesis reaction was performed at 42°C for 60 minutes. Thereafter, the reverse transcriptase was inactivated by heating at 72°C for 5 minutes, and then RNase H was added to remove single-stranded RNA, which was used as a template for the polymerase chain reaction of the p53 gene.

In order to obtain the gene of p53 in which the signal peptide sequence was removed from human dermal fibroblast cells, a primer (T2p53) encoding from the amino terminus glutamic acid and a primer (Xp53) encoding from the carboxyl terminus were synthesized, and then PCR was performed using the cDNA prepared above as a template. The sequence of each primer is as described in Table 1 below.

[Table 1]

| Primer | Sequence | SEQ ID NO. |
|---|---|---|
| T2p53 | 5'-AAA AAA CCG CGG TGG TGA GGA GCC GCA GTC AGA TCC TAG-3' | SEQ ID NO: 1 |
| Xp53 | 5'- AAA AAA CTC GAG TGA GTC TGA GTC AGG CCC TTC TG-3' | SEQ ID NO: 2 |

0.2 pmol T2p53 primer and 0.2 pmol Xp53 primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 40 cycles. After the reaction, the amplified DNA fragment of about 1.2 kbp was isolated by electrophoresis on 1% agarose gel, and then inserted into a pGEM-T easy (Promega, USA) vector using T4DNA ligase. As a result of sequencing the DNA thus obtained, it was confirmed that the cDNA encoding a human p53 protein was obtained. The obtained p53 gene was designated as pTA-p53, and the base sequence thereof is the same as the base sequence of SEQ ID NO: 3 (Figure 1).

**Example 1.2. Preparation of E. coli expression vector for p53**

**Example 1.2.1. Preparation of a plasmid, pET15b-UB-p53**

In order to prepare a p53 protein in a form to which ubiquitin is fused, the following expression vector was prepared. In order to obtain the ubiquitin gene, NdeUB primer and T2UB primer were prepared. The sequence of each primer is as described in Table 2 below.

[Table 2]

| Primer | Sequence | SEQ ID NO. |
|---|---|---|
| NdeUB | 5'-GGA TTC CAT ATG CAA CTT TTC GTC AAA ACT CTA AC-3' | SEQ ID NO: 4 |
| T2UB | 5'-ATG ACC ACC GCG GAG TCT CAA CAC CAA-3' | SEQ ID NO: 5 |

0.2 pmol NdeUB primer and 0.2 pmol T2UB primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles to obtain the ubiquitin (UB) gene. The amplified ubiquitin gene was cleaved by the restriction enzymes NdeI and SacII, and the plasmid pTA-p53 was cleaved by the restriction enzymes SacII and XhoI. Thereafter, the DNA fragments of about 210 bp and 1,200 bp were obtained by electrophoresis on 2% agarose gel, respectively, and then inserted into a pET15b vector cleaved by the restriction enzymes NdeI and XhoI using a T4DNA ligase to obtain the plasmid pET15b-UB-p53 (Figure 2). In this case, UB-p53 was represented by the base sequence of SEQ ID NO: 6.

E. coli BL21(DE3) strain was transformed using the plasmid pET15b-UB-p53. Thereafter, the transformed strain was cultured in a Luria-Bertani (LB) solid medium to which the antibiotic ampicillin was added, and then the colonies obtained herein were cultured in a LB liquid medium under a condition of 37°C. Thereafter, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 3, it was confirmed that a ubiquitin-fused p53 protein having a size of about 60 kDa was expressed. In this case, lane M in Figure 3 shows a protein molecular weight marker, and lane 1 shows the precipitate centrifuged after E. coli was crushed 4 hours after adding IPTG, and lane 2 shows the supernatant centrifuged after E. coli was crushed.

**Example 1.2.2. Preparation of a plasmid, pET11C-TOM70-UB-p53**

In order to prepare the p53 protein in the form to which TOM70 binding to the mitochondrial outer membrane and ubiquitin were fused, the expression vector capable of expressing p53 in the form to which TOM70 and ubiquitin were fused was prepared. In order to obtain TOM70 and ubiquitin genes, NdeTOM70 primer, TOM70-AS primer, TOM70UB-S primer and T2UB-AS primer were prepared. The sequence of each primer is as described in Table 3 below.

[Table 3]

| Primer | Sequence | SEQ ID NO. |
|---|---|---|
| NdeTOM70 | 5'-GAA TTC CAT ATG AAA AGT TTT ATA ACT CGG AAT AAA ACT GCA ATT TTC GCA ACT GTT GC -3' | SEQ ID NO: 7 |
| TOM70-AS | 5'- GGT GCA TAC TAC TAT TAT CAAA CTT TTC GTC AAA ACT C-3' | SEQ ID NO: 8 |
| TOM70UB-S | 5'- GGC TAC GT ATT TAT TTC CAA CTT TTC GTC AAA ACT C-3' | SEQ ID NO: 9 |
| T2UB-AS | 5'- GGC ACC ACC GCG GAG TCT CAA CAC 3' | SEQ ID NO: 10 |

In order to obtain the TOM70 gene, 0.2 pmol NdeTOM70 primer and 0.2 pmol TOM70-AS primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles to obtain a TOM70 gene. The amplified DNA fragment was referred to as N-TOM70. The plasmid pET15b-UB-p53 obtained in Example 1.2.1. above was used as a template, and 0.2 pmol TOM70UB-S primer and 0.2 pmol T2UB-AS primer were added, and dNTP 0.2 nM, 1x AccuPrime Taq DNA polymerase reaction buffer solution (Invitrogen, USA) and 1 unit of AccuPrime Taq DNA polymerase were mixed. Thereafter, in a polymerase chain reaction apparatus, amplification reactions of 95°C for 40 seconds, 58°C for 30 seconds, 72°C for 1 minute were performed at 25 cycles to obtain a UB gene. The amplified DNA fragment was referred to as C-UB.

The amplified DNA N-TOM70 and C-UB were used as templates, and 0.2 pmol NdeTOM70 primer, 0.2 pmol T2UB-AS primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles to obtain the ubiquitin gene TOM70-UB to which the amplified TOM70 was fused.

The amplified TOM70-UB gene was cleaved by the restriction enzymes NdeI and SacII, and the plasmid pTA-p53 was cleaved by the SacII and XhoI, and the DNA fragments of 330 bp and 1,500 bp were obtained by electrophoresis on 2% agarose gel, respectively. Thereafter, it was inserted into a pET11c vector cleaved by the restriction enzymes NdeI and SalI using a T4DNA ligase to obtain the plasmid pET11c-TOM70-UB-p53 (Figure 4). In this case, TOM70-UB-p53 was represented by the base sequence of SEQ ID NO: 11.

E. coli BL21(DE3) strain was transformed using a plasmid pET11c-TOM70-UB-p53. Thereafter, the transformed strain was cultured in a Luria-Bertani (LB) solid medium to which the antibiotic ampicillin was added, and then the colonies obtained herein were cultured in a LB liquid medium in a 37°C shaking incubator. Thereafter, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 5, it was confirmed that p53 protein having a size of about 62 kDa in the form to which TOM70 and ubiquitin were fused was expressed. In this case, lane M shows a protein molecular weight marker, and lane 1 shows the supernatant centrifuged after E. coli was crushed 4 hours after adding IPTG.

**Example 1.2.3. Preparation of a plasmid, pET11c-TOM70-(GGGGS)3-UB-p53**

In order to prepare a p53 protein in the form to which TOM70 binding to the mitochondrial outer membrane, a linker (GGGGSGGGGSGGGGS (SEQ ID NO: 70)) and ubiquitin were fused, an expression vector capable of expressing a p53 protein in the form to which TOM70, the linker, and ubiquitin were fused was prepared. In order to obtain a linker gene bound to TOM70, TOM70(G)3-AS primer, (G)3UB-S primer and Xp53 (noT) primer were prepared. The sequence of each primer is as described in Table 4 below.

[Table 4]

| Primer | Sequence | SEQ ID NO. |
|---|---|---|
| TOM70(G)$_3$-AS | 5'-GCC CCC GGA TCC TCC ACC CCC GCT TCC GCC ACC TCC ATA ATA GT AGT ATG CAC CAA TAG -3' | SEQ ID NO: 12 |
| (G)$_3$UB-S | 5'-GGT GGA GGA TCC GGG GGC GC GGA AGC CAA ATC-3' | SEQ ID NO: 13 |
| Xp53(noT) | 5'- AAA AAA CTC GAG GTC TGA GTC AGG CCC TTC TG-3' | SEQ ID NO: 14 |

The plasmid pET11c-TOM70-UB-p53 obtained in Example 1.2.2. above was used as a template, and 0.2 pmol NdeTOM70 primer and 0.2 pmol TOM70(G)3-AS primer were added, and dNTP 0.2 nM, 1x AccuPrime Taq DNA polymerase reaction buffer solution (Invitrogen, USA) and 1 unit of AccuPrime Taq DNA polymerase were mixed. Thereafter, in a polymerase chain reaction apparatus, amplification reactions of 95°C for 40 seconds, 58°C for 30 seconds, 72°C for 1 minute were performed at 25 cycles to obtain a gene TOM70-G3 in which a gene TOM70 and a linker were bound. In addition, the plasmid pET15b-UB-p53 obtained in Example 1.2.1. above was used as a template, and 0.2 pmol (G)3UB-S primer and 0.2 pmol Xp53 (noT) primer were mixed with dNTP 0.2 nM, 1x AccuPrime Taq DNA polymerase reaction buffer solution (Invitrogen, USA) and 1 unit of AccuPrime Taq DNA polymerase.

Thereafter, in a polymerase chain reaction apparatus, amplification reactions of 95°C for 40 seconds, 58°C for 30 seconds, 72°C for 1 minute were performed at 25 cycles to obtain UB-p53, p53 fused with the gene ubiquitin. The amplified TOM70-G3 gene was cleaved by the restriction enzymes NdeI and BamHI, and the amplified UB-p53 gene was cleaved by BamHI and XhoI, and the DNA fragments of 100 bp and 1,500 bp were obtained by electrophoresis on 2% agarose gel, respectively. Thereafter, it was inserted into a pET11c vector cleaved by the restriction enzymes NdeI and SalI using a T4DNA ligase to obtain the plasmid pET11c-TOM70-(GGGGS)3-UB-p53 (Figure 6). In this case, TOM70-(GGGGS)3-UB-p53 was represented by the base sequence of SEQ ID NO: 15.

E. coli BL21(DE3) strain was transformed using the plasmid pET11c-TOM70-(GGGGS)3-UB-p53. Thereafter, the transformed strain was cultured in a Luria-Bertani (LB) solid medium to which the antibiotic ampicillin was added, and then the colonies obtained herein were cultured in a LB liquid medium under a condition of 37°C. Thereafter, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 7, it was confirmed that p53 protein having a size of about 62 kDa in the form to which TOM70, the linker and ubiquitin were fused was expressed. In this case, lane M shows a protein molecular weight marker, lane 1 shows the precipitate centrifuged after E. coli was crushed 4 hours after adding IPTG, and lane 2 shows the supernatant centrifuged after crushing E.coli.

**Example 1.2.4. Preparation of a plasmid, pET11c-TOM70-(GGGGS)3-p53**

In order to prepare a p53 protein in the form to which TOM70 binding to the mitochondrial outer membrane and a linker (GGGGSGGGGSGGGGS) were fused, an expression vector capable of expressing a p53 protein in the form to which TOM70 and the linker were fused was prepared. In order to obtain a p53 gene to which TOM70 and the linker were fused, a primer (B(G)3p53) was prepared. The sequence of each primer is as described in Table 5 below.

[Table 5]

| Primer | Sequence | SEQ ID NO. |
|---|---|---|
| B(G)$_3$p53 | 5'-GGT GGA GGA TCC GGG GGC GGC GGA AGC GAG GAG CCG CAG TCA GAT CCT AGC -3' | SEQ ID NO: 16 |

The plasmid pET11c-TOM70-UB-p53 obtained in Example 1.2.2. above was used as a template, and 0.2 pmol NdeTOM70 primer and 0.2 pmol TOM70(G)3-AS primer were added, and dNTP 0.2 nM, 1x AccuPrime Taq DNA polymerase reaction buffer solution (Invitrogen, USA) and 1 unit of AccuPrime Taq DNA polymerase were mixed. Thereafter, in a polymerase chain reaction apparatus, amplification reactions of 95°C for 40 seconds, 58°C for 30 seconds, 72°C for 1 minute were performed at 25 cycles to obtain a gene TOM70. The amplified DNA fragment was referred to as TOM70-G3.

The plasmid pET15b-UB-p53 obtained in Example 1.2.1. above was used as a template, and 0.2 pmol B(G)3p53 primer and 0.2 pmol Xp53 (noT) primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles. The amplified DNA fragment was referred to as G3-p53.

The amplified DNA fragment, TOM70-G3, was cleaved by NdeI and BamHI, and the DNA fragment G3-53 was cleaved by the restriction enzymes BamHI and XhoI. Then, the DNA fragments of about 150 bp and 1,300 bp were obtained by electrophoresis on 2% agarose gel, respectively, and then inserted into a pET11c vector cleaved by the restriction enzymes NdeI and SalI using a T4DNA ligase to obtain the plasmid pET11c-TOM70-(GGGGS)3-p53 (Figure 8). In this case, TOM70-(GGGGS)3-p53 was represented by the base sequence of SEQ ID NO: 17.

E. coli BL21(DE3) strain was transformed using the plasmid pET11c-TOM70-(GGGGS)3-p53. Thereafter, the transformed strain was cultured in a Luria-Bertani (LB) solid medium to which the antibiotic ampicillin was added, and then the colonies obtained herein were cultured in a LB liquid medium in a 37°C shaking incubator. Thereafter, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 9, it was confirmed that a p53 protein having a size of about 60 kDa in the form to which TOM70 was fused was expressed. In this case, lane M shows a protein molecular weight marker, lane 1 shows the precipitate centrifuged after E. coli was crushed 4 hours after adding IPTG, and lane 2 shows the supernatant centrifuged after crushing E. coli.

**Example 1.2.5. pET15b-UB-p53-TOM7**

In order to prepare a p53 protein in the form to which ubiquitin and TOM7 binding to the mitochondrial outer membrane were fused, an expression vector capable of expressing p53 in a form to which ubiquitin, p53 and TOM were fused in the order was prepared. In order to obtain a p53 gene to which TOM7 and ubiquitin were fused, Xp53(noT) primer, XTOM7 primer and LTOM7 primer were prepared. The sequence of each primer is as described in Table 6 below.

[Table 6]

| Primer | Sequence | SEQ ID NO. |
|---|---|---|
| Xp53(noT) | 5'-AAA AAA CTC GAG GTC TGA GTC AGG CCC TTC TG -3' | SEQ ID NO: 18 |
| XTOM7 | 5'-AAA AAA CTC GAG ttt gcc att cgc tgg ggc ttt atc-3' | SEQ ID NO: 19 |
| LTOM7 | 5'-AAA AAA GTC GAC TTA TCC CCA AAG TAG GCT CAA AAC AG-3' | SEQ ID NO: 20 |

The plasmid pET15b-UB-p53 obtained in Example 1.2.1. above was used as a template, and 0.2 pmol NdeUB primer and 0.2 pmol Xp53(noT) primer were added, and dNTP 0.2 nM, 1x AccuPrime Taq DNA polymerase reaction buffer solution (Invitrogen, USA) and 1 unit of AccuPrime Taq DNA polymerase were mixed. Thereafter, in a polymerase chain reaction apparatus, amplification reactions of 95°C for 40 seconds, 58°C for 30 seconds, 72°C for 1 minute were performed at 25 cycles to obtain a gene UB-p53. In addition, cDNA prepared above was used as a template, and 0.2 pmol XTOM7 primer and 0.2 pmol LTOM7 primer were mixed with dNTP 0.2 nM, 1x AccuPrime Taq DNA polymerase reaction buffer solution (Invitrogen, USA) and 1 unit of AccuPrime Taq DNA polymerase.

Thereafter, in a polymerase chain reaction apparatus, amplification reactions of 95°C for 40 seconds, 58°C for 30 seconds, 72°C for 1 minute were performed at 40 cycles to obtain a gene TOM7. The amplified DNA fragment, UB-p53, was cleaved by the restriction enzymes, NdeI and XhoI, and the amplified TOM7 gene was cleaved by the restriction enzymes XhoI and SalI. The DNA fragments of about 1,500 bp and 150 bp were obtained by electrophoresis on 2% agarose gel, respectively, and then inserted into a pET15b vector cleaved by the restriction enzymes NdeI and XhoI using a T4DNA ligase to obtain the plasmid pET15b-UB-p53-TOM7 (Figure 10). In this case, UB-p53-TOM7 was represented by the base sequence of SEQ ID NO: 21.

E. coli BL21(DE3) strain was transformed using the plasmid pET15b-UB-p53-TOM7. Thereafter, the transformed strain was cultured in a Luria-Bertani (LB) solid medium to which the antibiotic ampicillin was added, and then the colonies obtained herein were cultured in a LB liquid medium under a condition of 37°C. Thereafter, when the cell density reached about 0.2 absorbance at OD600, IPTG was added so that a final 0.5 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 11, it was confirmed that a p53 protein having a size of about 60 kDa in the form to which ubiquitin and TOM7 were fused was expressed. In this case, lane M shows a protein molecular weight marker, lane 1 shows the precipitate centrifuged after E. coli was crushed 4 hours after adding IPTG, and lane 2 shows the supernatant centrifuged after crushing E. coli.

### Example 1.2.6. Construction of a mammalian expression vector, pCMV-p53-myc/His

An expression vector for animal cells capable of expressing p53 was prepared. In order to obtain a p53 gene, Rp53 primer was prepared. The sequence of each primer is as described in Table 7 below.

[Table 7]

| Primer | Sequence | SEQ ID NO. |
|---|---|---|
| Rp53 | 5'-AAA AAA GAA TTC ATG GTC TGA GTC AGG CCC TTC TG -3' | SEQ ID NO: 23 |

The plasmid pET-UB-p53 obtained in Example 1.2.1. above was used as a template, and 0.2 pmol Rp53 primer and 0.2 pmol Xp53(noT) primer were mixed with dNTP 0.2 nM, 1x AccuPrime Taq DNA polymerase reaction buffer solution (Invitrogen, USA) and 1 unit of AccuPrime Taq DNA polymerase were mixed. Thereafter, in a polymerase chain reaction apparatus, amplification reactions of 95°C for 40 seconds, 58°C for 30 seconds, 72°C for 1 minute were performed at 25 cycles to obtain a gene p53.

The amplified p53 gene was cleaved by the restriction enzymes EcoRI and XhoI, and the DNA fragment of about 1,300 bp was obtained by electrophoresis on 2% agarose gel, and then it was inserted into a pcDNA3.1-myc/His A vector cleaved by the restriction enzymes EcoRI and XhoI using a T4DNA ligase to obtain the plasmid pCMV-p53-myc/His (Figure 12). In this case, p53-myc/His was represented by the base sequence of SEQ ID NO: 23.

It was transfected into an animal cell CHO using the plasmid pCMV-p53-myc/His, and the cells was crushed, and then SDS-polyacrylamide electrophoresis was performed, and it was shown by Western blot using an anti-c-myc antibody. As shown in Figure 13, it was confirmed that a p53 protein having a size of about 55 kDa was expressed. In this case, lane M shows a protein molecular weight marker, and lane 1 shows that it was transfected into an animal cell CHO, and the cells was crushed, and then SDS-polyacrylamide electrophoresis was performed, and then it was confirmed by Western blot using an anti-c-myc antibody.

### Example 1.3. Isolation and purification of fusion protein comprising p53

### Example 1.3.1. Isolation and purification of recombinant TOM70-(GGGGS)3-p53 protein derived from E. coli

E. coli BL21(DE3) production strain expressing the recombinant TOM70-(GGGGS)3-p53 protein was inoculated into a LB liquid medium, and cultured under a condition of 37°C. Thereafter, when the absorbance reached 0.4 at OD600, 0.5 mM IPTG was added, and the shaking culture was performed further for 4 hours to express the TOM70-(GGGGS)3-p53 protein.

After the culture was completed, the cells were recovered using centrifugation, and the recovered cells were washed once using PBS, and then the cells were suspended using a PBS solution, and the suspended cells were subjected to a crushing process using a sonicator. The crushed cells were centrifuged using a high speed centrifuge, and then insoluble fractions were recovered, and the recovered insoluble fractions were washed three times using 50 mM Tris, 100 mM ethylenediaminetetraacetic acid (EDTA) pH 8.0 solution. Thereafter, it was dissolved in 6 M guanidine, 100 mM sodium phosphate, 10 mM Tris pH 8.0 solution and filtered using a 0.45 μm filter, and then loaded on a pre-packed nickel chromatography column to perform primary purification.

The solution comprising the TOM70-(GGGGS)3-p53 protein was loaded, and then until the impurities unbound were not detected, a washing solution was flowed using 8 M urea, 50 mM sodium phosphate, 500 mM NaCl, 10 mM imidazole, pH 8.0 solution, and the protein was eluted using 8 M urea, 50 mM sodium phosphate, 500 mM NaCl, 500 mM imidazole, pH 8.0 solution while changing the imidazole concentration to 50 mM, 100 mM, 250 mM, 500 mM (Figure 14). In this case, lane M in Figure 14 shows a protein molecular weight marker, and lane 1 shows a nickel affinity chromatography loading sample. Lane 2 shows that it was not bound to a nickel affinity resin. Lanes 3 to 4 show the results of elution with 8 M UREA/50 mM Na-phosphate/500 mM NaCl/50 mM imidazole solution. Lanes 5 to 7 show the results of elution with 8M UREA/50mM Na-phosphate/500mM NaCl/100mM Imidazole solution. Lanes 8 to 9 show the results of elution with 8M UREA/50mM Na-phosphate/500mM NaCl/250mM Imidazole solution. Lanes 10 to 11 show the results of elution with 8M UREA/50mM Na-phosphate/500mM NaCl/500mM Imidazole solution.

The eluted solution recovered from the nickel chromatography was solution-exchanged with PBS using the principle of osmotic pressure. After the solution exchange was completed, the eluted solution was subjected to centrifugation to recover the supernatant, and a protein amount of the recovered eluted solution was measured by protein quantification method and confirmed using SDS-PAGE. As shown in Figure 15, after the confirmation was completed, the TOM70-(GGGGS)3-p53 protein was quenched with the liquid nitrogen and stored in a cryogenic freezer at -80°C. In this case, lane M shows a protein molecular weight marker, and lane 1 shows the TOM70-(GGGGS)3-p53 protein obtained after dialysis in PBS buffer solution.

**Example 1.3.2. Isolation and purification of recombinant TOM70-(GGGGS)3-UB-p53 protein derived from E. coli**

E. coli expressing the TOM70-(GGGGS)3-UB-p53 recombinant protein was used to isolate and purify the TOM70-(GGGGS)3-UB-p53 protein in the same method as in Example 1.3.1. As a result, the TOM70-(GGGGS)3-UB-p53 protein was eluted (Figure 16). In this case, lane M in Figure 16 shows a protein molecular weight marker, and lane 1 shows a nickel affinity chromatography loading sample. Lane 2 shows that it was not bound to a nickel affinity resin. Lane 3 shows the results of elution with 8M UREA/50mM Na-phosphate/500mM NaCl/50mM Imidazole solution. Lanes 4 to 7 show the results of elution with 8M UREA/50mM Na-phosphate/500mM NaCl/100mM Imidazole solution. Lanes 8 to 11 show the results of elution with 8M UREA/50mM Na-phosphate/500mM NaCl/250mM Imidazole solution.

The protein amount of the recovered eluted solution was measured by protein quantification method and confirmed using SDS-PAGE. As shown in Figure 17, after the confirmation was completed, the TOM70-(GGGGS)3-UB-p53 protein was quenched with the liquid nitrogen and stored in a cryogenic freezer at -80°C. In this case, lane M in Figure 17 shows a protein molecular weight marker, and lane 1 shows the TOM70-(GGGGS)3-UB-p53 protein obtained after dialysis in PBS buffer solution.

**Example 1.3.3. Isolation and purification of recombinant UB-p53 protein derived from E. coli**

BL21(DE3) production strain expressing the UB-p53 protein in the mature form to which ubiquitin was fused was innoculated into a LB liquid medium, and cultured in a shaking incubator at 37°C. When the absorbance reached 0.4 at OD600, 0.5 mM IPTG was added, and the shaking culture was performed further for 4 hours to express the UB-p53 protein in the mature form to which ubiquitin was fused.

Then, the UB-p53 protein was isolated and purified in the same method as in Example 1.3.1. As a result, the UB-p53 protein was eluted (Figure 18). In this case, lane M in Figure 18 shows a protein molecular weight marker, and lane 1 shows a nickel affinity chromatography loading sample. Lane 2 shows that it was not bound to a nickel affinity resin. Lane 3 shows the results of elution with 8M UREA/50mM Na-phosphate/500mM NaCl/50mM Imidazole solution. Lanes 4 to 6 show the results of elution with 8M UREA/50mM Na-phosphate/500mM NaCl/100mM Imidazole solution. Lane 7 to 9 show the results of elution with 8M UREA/50mM Na-phosphate/500mM NaCl/250mM Imidazole solution. Lanes 10 to 11 show the results of elution with 8M UREA/50mM Na-phosphate/500mM NaCl/500mM Imidazole solution.

The protein amount of the recovered eluted solution was measured by protein quantification method and confirmed using SDS-PAGE. As shown in Figure 19, after the confirmation was completed, the UB-p53 protein was quenched with the liquid nitrogen and stored in a cryogenic freezer at -80°C. In this case, lane M in Figure 19 shows a protein molecular weight marker, and lane 1 shows the UB-p53 protein obtained after dialysis in PBS buffer solution.

**Example 1.3.4. Isolation and purification of recombinant UB-p53-TOM7 protein derived from E. coli**

E. coli BL21(DE3) production strain expressing the UB-p53-TOM7 protein in the mature form to which ubiquitin was fused was innoculated into a LB liquid medium, and cultured under a condition of 37°C. When the absorbance reached 0.4 at OD600, 0.5 mM IPTG was added, and the shaking culture was performed further for 4 hours to express the UB-p53-TOM7 protein in the mature form to which ubiquitin was fused.

Then, the UB-p53-TOM7 protein was isolated and purified in the same method as in Example 1.3.1. As a result, the UB-p53-TOM7 protein was eluted (Figure 20). In this case, lane M in Figure 20 shows a protein molecular weight marker, and lane 1 shows a nickel affinity chromatography loading sample. Lane 2 shows that it was not bound to a nickel affinity resin. Lane 3 shows the results of elution with 8M UREA/50mM Na-phosphate/500mM NaCl/10mM Imidazole solution. Lane 4 shows the results of elution with 8M UREA/50mM Na-phosphate/500mM NaCl/50mM Imidazole solution. Lanes 5 to 7 show the results of elution with 8M UREA/50mM Na-phosphate/500mM NaCl/100mM Imidazole solution. Lanes 8 to 9 show the results of elution with 8M UREA/50mM Na-phosphate/500mM NaCl/250mM Imidazole solution. Lanes 10 to 11 show the results of elution with 8M UREA/50mM Na-phosphate/500mM NaCl/500mM Imidazole solution.

The protein amount of the recovered eluted solution was measured by protein quantification method and confirmed using SDS-PAGE. As shown in Figure 21, after the confirmation was completed, the UB-p53 protein was quenched with the liquid nitrogen and stored in a cryogenic freezer at -80°C. In this case, lane M in Figure 21 shows a protein molecular weight marker, and lane 1 shows the UB-p53-TOM7 protein obtained after dialysis in PBS buffer solution.

**Example 2. Preparation of fusion protein comprising Granzyme B**

**Example 2.1. Amplification of Granzyme B gene**

In order to express the human Granzyme B into a recombinant protein, total RNA was extracted from human natural killer cells, and cDNA was synthesized therefrom. Specifically, human natural killer cells were cultured in 10% serum medium under a condition of 5% carbon dioxide and 37°C (1x$10^6$ cells). Thereafter, the RNA was obtained in the same method as in Example 1.1., and then it was used as a template for the polymerase chain reaction of the Granzyme B gene.

In order to obtain the gene of Granzyme B in which the signal peptide sequence was removed from human natural killer cells, T2GZMB primer encoding from the amino terminus isoleucine and XGZMB(noT) primer encoding from the carboxyl terminus were synthesized, and then PCR was performed using the cDNA prepared above as a template. The sequence of each primer is as described in Table 8 below.

[Table 8]

| Primer | Sequence | SEQ ID NO. |
|---|---|---|
| T2GZMB | 5'-AAA AAA CCG CGG TGG TAT CAT CGG GGG ACA TGA GGC ACA TGA GGC CAA GCC -3' | SEQ ID NO: 24 |
| XGZMB(noT) | 5'-AAA AAA CTC GAG GTA GCG TTT CAT GGT TTT CTT TAT CC-3' | SEQ ID NO: 25 |

The cDNA prepared above was used as a template, and 0.2 pmol T2GZMB primer and 0.2 pmol XGZMB(noT) primer were mixed with dNTP 0.2nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 40 cycles. After the reaction, the amplified DNA fragment of about 700 bp was isolated by electrophoresis on 1% agarose gel, and then inserted into a pGEM-T easy (Promega, USA) vector using a T4DNA ligase. As a result of sequencing the DNA thus obtained, it was confirmed that the cDNA encoding a human Granzyme B protein was obtained. The obtained Granzyme B gene was designated as pTA-Granzyme B, and the Granzyme B gene was represented by the base sequence of SEQ ID NO: 26 (Figure 22).

**Example 2.2. Preparation of an E.coli expression vector for Granzyme B protein**

**Example 2.2.1. Preparation of a plasmid, pET11c-TOM70-(GGGGS)3-UB-Granzyme B**

In order to prepare a Granzyme B protein in the form to which TOM70 binding to the mitochondrial outer membrane, a linker (GGGGSGGGGSGGGGS) and ubiquitin were fused, the expression vector capable of expressing Granzyme B in the form to which TOM70, the linker and ubiquitin were fused was prepared.

The plasmid pTA-GranzymeB gene obtained in Example 2.1. above was cleaved by the restriction enzymes SacII and XhoI, and and the DNA fragment of about 700 bp was obtained by electrophoresis on 2% agarose gel. Thereafter, it was inserted into a pET11c-TOM70-(GGGGS)3-UB-(p53) vector cleaved by the restriction enzymes SacII and XhoI using a T4DNA ligase to obtain the plasmid pET11c-TOM70-(GGGGS)3-UB-Granzyme B (SEQ ID NO: 27) (Figure 23).

E. coli BL21(DE3) strain was transformed using a plasmid pET11c-TOM70-(GGGGS)3-UB-Granzyme B. Thereafter, the transformed strain was cultured in a Luria-Bertani (LB) solid medium to which the antibiotic ampicillin was added, and then the colonies obtained herein were cultured in a LB liquid medium in a 37°C shaking incubator. Then, when the cell density reached about 0.2 absorbance at OD600, IPTG was added so that a final 0.5 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 24, it was confirmed that a Granzyme B protein having a size of about 35 kDa in the form to which TOM70, a linker and ubiquitin were fused was expressed. In this case, lane M in Figure 24 shows a protein molecular weight marker, lane 1 shows the precipitate centrifuged after E. coli was crushed 4 hours after adding IPTG, and lane 2 shows the supernatant centrifuged after E. coli was crushed.

**Example 2.2.2. Preparation of a plasmid, pET15b-UB-Granzyme B-TOM7**

In order to prepare a Granzyme B protein in the form to which ubiquitin and TOM7 binding to the mitochondrial outer membrane were fused, the expression vector capable of expressing the Granzyme B protein in the form to which ubiquitin, Granzyme B, and TOM7 were fused in the order was prepared.

The plasmid pTA-Granzyme B gene obtained in Example 2.1. above was cleaved by the restriction enzymes SacII and XhoI, and and the DNA fragment of about 700 bp was obtained by electrophoresis on 2% agarose gel. Thereafter, it was inserted into a pET15b-UB-(p53)-TOM7 vector cleaved by the restriction enzymes SacII and XhoI using a T4DNA ligase to obtain the plasmid pET15b-UB-GranzymeB-TOM7(Figure 25). Here, the UB-GranzymeB-TOM7 was represented by the base sequence of SEQ ID NO: 28.

E. coli BL21(DE3) strain was transformed using the plasmid pET15b-UB-Granzyme B-TOM7. Thereafter, the transformed strain was cultured in a Luria-Bertani (LB) solid medium to which the antibiotic ampicillin was added, and then the colonies obtained herein were cultured in a LB liquid medium under a condition of 37°C. Then, when the cell density reached about 0.2 absorbance at OD600, IPTG was added so that a final 0.5 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 26, it was confirmed that a Granzyme B protein having a size of about 35 kDa in the form to which ubiquitin and TOM70 were fused was expressed. In this case, lane M in Figure 26 shows a protein molecular weight marker, lane 1 shows the precipitate centrifuged after E. coli was crushed 4 hours after adding IPTG, and lane 2 shows the supernatant centrifuged after E. coli was crushed.

**Example 2.3. Isolation and purification of recombinant TOM70-(GGGGS)3-UB-Granzyme B protein derived from E. coli**

The TOM70-(GGGGS)3-UB-GranzymeB protein was isolated and purified in the same method as in Example 1.3.1. As a result, the TOM70-(GGGGS)3-UB-GranzymeB protein was eluted (Figure 27). In this case, lane M in Figure 27 shows a protein molecular weight marker, and lane 1 shows a nickel affinity chromatography loading sample. Lane 2 shows that it was not bound to a nickel affinity resin. Lanes 3 and 4 show the results of elution with 8M UREA/50mM Na-phosphate/500mM NaCl/50mM Imidazole solution. Lanes 5 to 7 show the results of elution with 8M UREA/50mM Na-phosphate/500mM NaCl/100mM Imidazole solution. Lanes 8 to 9 show the results of elution with 8M UREA/50mM Na-phosphate/500mM NaCl/250mM Imidazole solution.

The protein amount of the recovered eluted solution was measured by protein quantification method and confirmed using SDS-PAGE. As shown in Figure 28, after the confirmation was completed, the TOM70-(GGGGS)3-UB-Granzyme B protein was quenched with the liquid nitrogen and stored in a cryogenic freezer at -80°C. In this case, lane M in Figure 28 shows a protein molecular weight marker, and lane 1 shows the TOM70-(GGGGS)3-UB-Granzyme B protein obtained after dialysis in PBS buffer solution.

**Example 3. Preparation of fusion protein comprising RKIP**

**Example 3.1. Amplification of RKIP gene**

In order to express the human RKIP (Raf Kinase Inhibitory Protein) gene into a recombinant protein, total RNA was extracted from human epithelial cells, and cDNA was synthesized therefrom. Human dermal fibroblast cells were cultured in 10% serum medium under a condition of 5% carbon dioxide and 37°C ($1x10^6$ cells). Thereafter, the RNA was obtained in the same method as in Example 1.1., and then it was used as a template for the polymerase chain reaction of the RKIP gene.

In order to obtain the gene of RKIP in which the signal peptide sequence was removed from human dermal fibroblast cells, T2RKIP primer encoding from the amino terminus proline and XRKIP(noT) primer encoding from the carboxyl terminus were synthesized, and then PCR was performed using the cDNA prepared above as a template. The sequence of each primer is as described in Table 9 below.

[Table 9]

| Primer | Sequence | SEQ ID NO. |
|---|---|---|
| T2RKIP | 5'-AAA AAA CCG CGG TGG Tcc ggt gga cct cag caa gtg gtc-3' | SEQ ID NO: 29 |
| XRKIP(noT) | 5'- AAA AAA CTC GAG CTT CCC AGA CAG CTG CTC GTA CAG TTT GG-3' | SEQ ID NO: 30 |

The cDNA prepared above was used as a template, and 0.2 pmol T2RKIP primer and 0.2 pmol XRKIP(noT) primer were mixed with dNTP 0.2nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 40 cycles. After the reaction, the amplified DNA fragment of about 560 bp was isolated by electrophoresis on 1% agarose gel, and then inserted into a pGEM-T easy (Promega, USA) vector using a T4DNA ligase. As a result of sequencing the DNA thus obtained, it was confirmed that the cDNA encoding a human RKIP protein was obtained. The obtained RKIP gene was designated as pTA-RKIP (Figure 29), and the base sequence of the RKIP gene was represented by the base sequence of SEQ ID NO: 31.

**Example 3.2. Preparation of an E.coli expression vector for RKIP protein**

**Example 3.2.1. Preparation of a plasmid, pET11c-TOM70-(GGGGS)3-UB-RKIP**

In order to prepare the RKIP protein in the form to which TOM70 binding to the mitochondrial outer membrane, a linker (GGGGSGGGGSGGGGS) and ubiquitin were fused, the expression vector capable of expressing RKIP in the form to which TOM70, a linker, and ubiquitin were fused was prepared.

The plasmid pTA-RKIP gene obtained in Example 3.1. was cleaved by the restriction enzymes SacII and XhoI, and the DNA fragment of about 560 bp was obtained by electrophoresis on 2% agarose gel, and then it was inserted into a pET11c-TOM70-(GGGGS)3-UB-(p53) vector cleaved by the restriction enzymes SacII and XhoI using a T4DNA ligase to obtain the plasmid pET11-TOM70-(GGGGS)3-UB-RKIP (Figure 30). Here, TOM70-(GGGGS)3-UB-RKIP was represented by the base sequence of SEQ ID NO: 32.

E. coli BL21(DE3) strain was transformed using the plasmid pET11c-TOM70-(GGGGS)3-UB-RKIP. Thereafter, the transformed strain was cultured in a Luria-Bertani (LB) solid medium to which the antibiotic ampicillin was added, and then the colonies obtained herein were cultured in a LB liquid medium in a 37°C shaking incubator. Then, when the cell density reached about 0.2 absorbance at OD600, IPTG was added so that a final 0.5 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 31, it was confirmed that a RKIP protein having a size of about 33 kDa in the form to which TOM70, a linker and ubiquitin were fused was expressed. In this case, lane M in Figure 31 shows a protein molecular weight marker, lane 1 shows the precipitate centrifuged after E. coli was crushed 4 hours after adding IPTG, and lane 2 shows the supernatant centrifuged after E. coli was crushed.

**Example 3.3. Isolation and purification of recombinant TOM70-(GGGGS)3-UB-RKIP protein derived from E. coli**

E. coli BL21(DE3) production strain expressing a recombinant TOM70-(GGGGS)3-UB-RKIP was inoculated into a LB liquid medium, and cultured under a condition of 37°C. When the absorbance reached 0.3 at OD600, it was put in a refrigerator to lower the temperature of the culture solution, and the temperature of the incubator was changed to 18°C, and then 0.5 mM IPTG was added, and the shaking culture was performed further for 1 day to express the TOM70-(GGGGS)3-UB-RKIP protein.

Then, the TOM70-(GGGGS)3-UB-RKIP protein was isolated and purified in the same method as in Example 1.3.1. As a result, the TOM70-(GGGGS)3-UB-RKIP protein was eluted (Figure 32). In this case, lane M in Figure 32 shows a protein molecular weight marker, and lane 1 shows a nickel affinity chromatography loading sample. Lane 2 shows that it was not bound to a nickel affinity resin. Lane 3 shows the results of elution with 50mM Na-phosphate/500mM NaCl/10mM Imidazole. Lanes 4 to 6 show the results of elution with 50mM Na-phosphate/500mM NaCl/50mM Imidazole. Lanes 7 to 8 show the results of elution with 50mM Na-phosphate/500mM NaCl/100mM Imidazole. Lanes 9 to 10 show the results of elution with 50mM Na-phosphate/500mM NaCl/175mM Imidazole. Lanes 11 to 13 show the results of elution with 50mM Na-phosphate/500mM NaCl/250mM Imidazole. Lanes 14 to 16 show the results of elution with 50mM Na-phosphate/500mM NaCl/500mM Imidazole.

The protein amount of the recovered eluted solution was measured by protein quantification method and confirmed using SDS-PAGE. As shown in Figure 33, after the confirmation was completed, the TOM70-(GGGGS)3-UB-RKIP protein was quenched with the liquid nitrogen and stored in a cryogenic freezer at -80°C. In this case, lane M in Figure 33 shows a protein molecular weight marker, and lane 1 shows the TOM70-(GGGGS)3-UB-RKIP protein obtained after dialysis in PBS buffer solution.

**Example 4. Preparation of fusion protein comprising PTEN**

**Example 4.1. Amplification of PTEN gene**

In order to express the human PTEN (Phosphatase and Tensin homolog) into a recombinant protein, total RNA was extracted from human epithelial cells, and cDNA was synthesized therefrom. Fibroblast cells (human dermal fibroblast cells) were cultured in 10% serum medium under a condition of 5% carbon dioxide and 37°C ($1x10^6$ cells). Thereafter, the RNA was obtained in the same method as in Example 1.1., and then it was used as a template for the polymerase chain reaction of the PTEN gene.

In order to obtain the gene of PTEN in which the signal peptide sequence was removed from human dermal fibroblast cells, T2PTEN primer encoding from the amino terminus threonine and XPTEN(noT) primer encoding from the carboxyl terminus were synthesized, and then PCR was performed using the cDNA prepared above as a template. The sequence of each primer is as described in Table 10 below.

[Table 10]

| Primer | Sequence | SEQ ID NO. |
|---|---|---|
| T2PTEN | 5'-AAA AAA CCG CGG TGG Tac age cat cat caa aga gat cgt tag -3' | SEQ ID NO: 33 |
| XPTEN(noT) | 5'- AAA AAA CTC GAG GAC TTT TGT AAT TTG TGT ATG CTG -3' | SEQ ID NO: 34 |

The cDNA prepared above was used as a template, and 0.2 pmol T2PTEN primer and 0.2 pmol XPTEN(noT) primer were mixed with dNTP 0.2nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 40 cycles. After the reaction, the amplified DNA fragment of about 1,200 bp was isolated by electrophoresis on 1% agarose gel, and then inserted into a pGEM-T easy (Promega, USA) vector using a T4DNA ligase. As a result of sequencing the DNA thus obtained, it was confirmed that the cDNA encoding a human RKIP protein was obtained. The obtained PTEN gene was designated as pTA-PTEN (Figure 34), and the base sequence of the PTEN was represented by the base sequence of SEQ ID NO: 35.

**Example 4.2. Preparation of an E.coli expression vector for PTEN protein**

**Example 4.2.1. Preparation of a plasmid, pET11c-TOM70-(GGGGS)3-UB-PTEN**

In order to prepare a PTEN protein in the form to which TOM70 binding to the mitochondrial outer membrane, a linker (GGGGSGGGGSGGGGS) and ubiquitin were fused, the expression vector capable of expressing the PTEN gene in the form to which TOM70, the linker and ubiquitin were fused was prepared.

The plasmid pTA-PTEN gene obtained in Example 4.1. above was cleaved by the restriction enzymes SacII and XhoI, and and the DNA fragment of about 1,200 bp was obtained by electrophoresis on 2% agarose gel. Thereafter, it was inserted into a pET11c-TOM70-(GGGGS)3-UB-(p53) vector cleaved by the restriction enzymes SacII and XhoI using a T4DNA ligase to obtain the plasmid pET11c-TOM70-(GGGGS)3-UB-PTEN (Figure 35). Here, TOM70-(GGGGS)3-UB-PTEN was represented by the base sequence of SEQ ID NO: 36.

E. coli BL21(DE3) strain was transformed using the plasmid pET11c-TOM70-(GGGGS)3-UB-PTEN. Thereafter, the transformed strain was cultured in a Luria-Bertani (LB) solid medium to which the antibiotic ampicillin was added, and then the colonies obtained herein were cultured in a LB liquid medium under the condition of 37°C. Then, when the cell density reached about 0.2 absorbance at OD600, IPTG was added so that a final 0.5 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 36, it was confirmed that a PTEN protein having a size of about 73 kDa in the form to which TOM70, a linker and ubiquitin were fused was expressed. In this case, lane M in Figure 36 shows a protein molecular weight marker, lane 1 shows the precipitate centrifuged after E. coli was crushed 4 hours after adding IPTG, and lane 2 shows the supernatant centrifuged after E. coli was crushed.

**Example 4.3 Isolation and purification of recombinant TOM70-(GGGGS)3-UB-PTEN protein derived from E. coli**

The TOM70-(GGGGS)3-UB-PTEN protein was isolated and purified in the same method as in Example 1.3.1. As a result, the TOM70-(GGGGS)3-UB-PTEN protein was eluted (Figure 37). In this case, lane M in Figure 37 shows a protein molecular weight marker, and lane 1 shows a nickel affinity chromatography loading sample. Lane 2 shows that it was not bound to a nickel affinity resin. Lane 3 shows the results of elution with 8M UREA/50mM Na-phosphate/500mM NaCl/10mM Imidazole solution. Lane 4 shows the results of elution with 8M UREA/50mM Na-phosphate/500mM NaCl/50mM Imidazole solution. Lanes 5 to 8 show the results of elution with 8M UREA/50mM Na-phosphate/500mM NaCl/100mM Imidazole solution. Lanes 9 to 10 show the results of elution with 8M UREA/50mM Na-phosphate/500mM NaCl/250mM Imidazole solution. Lane 11 shows the results of elution with 8M UREA/50mM Na-phosphate/500mM NaCl/500mM Imidazole solution.

The protein amount of the recovered eluted solution was measured by protein quantification method and confirmed using SDS-PAGE. As shown in Figure 38, after the confirmation was completed, the TOM70-(GGGGS)3-UB-PTEN protein was quenched with the liquid nitrogen and stored in a cryogenic freezer at -80°C. In this case, lane M in Figure 38 shows a protein molecular weight marker, and lane 1 shows the TOM70-(GGGGS)3-UB-PTEN protein obtained after dialysis in PBS buffer solution.

**Example 5. Preparation of fusion protein comprising mitochondrial outer membrane protein, ubiquitin and GFP**

**Example 5.1. Isolation and purification of recombinant UB-GFP-TOM7 protein derived from E. coli**

E. coli BL21(DE3) production strain expressing a UB-GFP-TOM7 protein in the mature form to which ubiquitin was fused was inoculated into a LB liquid medium, and cultured under a condition of 37°C. When the absorbance reached 0.3 at OD600, it was put in a refrigerator to lower the temperature of the culture solution, and the temperature of the incubator was changed to 18°C, and then 0.5 mM IPTG was added, and the shaking culture was performed further for 1 day to express the GFP-TOM7 protein in the mature form to which ubiquitin was fused.

After the culture was completed, the cells were recovered using centrifugation, and the recovered cells were washed once using PBS, and then the cells were suspended using 50 mM sodium phosphate, 500 mM NaCl, 10 mM imidazole, pH 8.0 solution, and the suspended cells were subjected to a crushing process using a sonicator. The crushed cells were centrifuged using a high speed centrifuge, and then the supernatant was recovered, and the recovered supernatant was filtered using a 0.45 μm filter, and then loaded on a pre-packed nickel chromatography column to perform primary purification.

The crushing solution comprising the UB-GFP-TOM7 protein in the mature form to which ubiquitin was fused was loaded, and then until the impurities unbound were not detected, a washing solution was flowed using 50 mM sodium phosphate, 500 mM NaCl, 20 mM imidazole, pH 8.0 solution, and the protein was eluted according to the concentration gradient using 50 mM sodium phosphate, 500 mM NaCl, 500 mM imidazole, pH 8.0 solution (Figure 39). In this case, lane M in Figure 39 shows a protein molecular weight marker, and lane 1 shows a nickel affinity chromatography loading sample. Lane 2 shows that it was not bound to a nickel affinity resin. Lane 3 shows the results of elution with 50mM Na-phosphate/500mM NaCl/20mM Imidazole. Lane 4 shows the results of elution with 50mM Na-phosphate/500mM NaCl/55mM Imidazole. Lane 5 shows the results of elution with 50mM Na-phosphate/500mM NaCl/60mM Imidazole. Lane 6 shows the results of elution with 50mM Na-phosphate/500mM NaCl/65mM Imidazole. Lane 7 shows the results of elution with 50mM Na-phosphate/500mM NaCl/70mM Imidazole. Lane 8 shows the results of elution with 50mM Na-phosphate/500mM NaCl/75mM Imidazole. Lane 9 shows the results of elution with 50mM Na-phosphate/500mM NaCl/80mM Imidazole. Lane 10 shows the results of elution with 50mM Na-phosphate/500mM NaCl/85mM Imidazole. Lane 11 shows the results of elution with 50mM Na-phosphate/500mM NaCl/90mM Imidazole. Lane 12 shows the results of elution with 50mM Na-phosphate/500mM NaCl/95mM Imidazole. Lane 13 shows the results of elution with 50mM Na-phosphate/500mM NaCl/100mM Imidazole. Lane 14 shows the results of elution with 50mM Na-phosphate/500mM NaCl/105mM Imidazole.

In order to remove imidazole in the eluted solution, dialysis was performed using the principle of osmotic pressure in a 50 mM sodium phosphate, 500 mM NaCl, pH 8.0 solution (Figure 40). The final UB-GFP-TOM7 protein that was identified was quenched with the liquid nitrogen and stored in a cryogenic freezer at -80°C. In this case, lane M in Figure 40 shows a protein molecular weight marker, and lane 1 shows a protein obtained after dialysis was performed in a 50 mM Na-phosphate/500 mM NaCl solution after mixing a fusion protein fraction.

**Example 5.2. Isolation and purification of recombinant TOM70-(GGGGS)3-UB-GFP protein derived from E. coli**

E. coli BL21(DE3) production strain expressing a recombinant protein TOM70-(GGGGS)3-UB-GFP was inoculated into a LB liquid medium, and cultured under a condition of 37°C. When the absorbance reached 0.3 at OD600, it was put in a refrigerator to lower the temperature of the culture solution, and the temperature of the incubator was changed to 18°C, and then 0.5 mM IPTG was added, and the shaking culture was performed further for 1 day to express the recombinant protein TOM70-(GGGGS)3-UB-GFP.

After the culture was completed, the cells were recovered using centrifugation, and the recovered cells were washed once using PBS, and then the cells were suspended using 50 mM sodium phosphate, 500 mM NaCl, 10 mM imidazole, pH 8.0 solution, and the suspended cells were subjected to a crushing process using a sonicator. The crushed cells were centrifuged using a high speed centrifuge, and then the supernatant was recovered, and the recovered supernatant was filtered using a 0.45 μm filter, and then loaded on a pre-packed nickel chromatography column to perform primary purification.

The crushing solution comprising the recombinant protein the TOM70-(GGGGS)3-UB-GFP was loaded on the column containing nikel resins, and then until the impurities unbound were not detected, a washing solution was flowed using 50 mM sodium phosphate, 500 mM NaCl, 20 mM imidazole, pH 8.0 solution. Then, the protein was eluted using 50 mM sodium phosphate, 500 mM NaCl, 500 mM imidazole, pH 8.0 solution, while changing the concentration of imidazole to 50 mM, 100 mM, 250 mM, 500 mM (Figure 41). In this case, lane M in Figure 41 shows a protein molecular weight marker, and lane 1 shows a nickel affinity chromatography loading sample. Lane 2 shows that it was not bound to a nickel affinity resin. Lane 3 shows the results of elution with 50mM Na-phosphate/500mM NaCl/20mM Imidazole. Lane 4 shows the results of elution with 50mM Na-phosphate/500mM NaCl/50mM Imidazole. Lanes 5 to 8 show the results of elution with 50mM Na-phosphate/500mM NaCl/100mM Imidazole. Lanes 9 to 11 show the results of elution with 50mM Na-phosphate/500mM NaCl/250mM Imidazole. Lane 12 shows the results of elution with 50mM Na-phosphate/500mM NaCl/500mM Imidazole.

The eluted solution recovered from the nickel chromatography was solution-exchanged with PBS buffer solution using the principle of osmotic pressure. After the solution exchange was completed, the final protein TOM70-(GGGGS)3-UB-GFP that was recovered was identified using protein quantification and SDS-PAGE. As shown in Figure 42, after the identification was completed, the TOM70-(GGGGS)3-UB-GFP protein was quenched with the liquid nitrogen and stored in a cryogenic freezer at -80°C. In this case, lane M in Figure 42 shows a protein molecular weight marker, and lane 1 shows TOM70-(GGGGS)3-UB-GFP protein obtained after dialysis was performed in a PBS buffer solution.

## II. Preparation of fusion protein comprising mitochondrial outer membrane targeting protein and target targeting protein

### Example 6. Preparation of fusion protein comprising scFvHER2

### Example 6.1. Synthesis of scFvHER2 gene

In order to express the human scFvHER2 into a recombinant protein, the scFvHER2 gene obtained by requesting gene synthesis from Bionics Co., Ltd. was designated as pUC57-scFvHER2, and the base sequence of scFvHER2 was the same as the base sequence of SEQ ID NO: 37.

### Example 6.2. Preparation of scFvHER2 protein expression vector

### Example 6.2.1. pET15b-UB-scFvHER2-TOM7

In order to prepare a scFvHER2 protein in the form to which ubiquitin and TOM7 binding to the mitochondrial outer membrane were fused, the expression vector capable of expressing the scFvHER2 gene in the form to which ubiquitin and TOM7 were fused was prepared.

The plasmid pUC57-scFvHER2 gene obtained in Example 6.1. was cleaved by the restriction enzymes SacII and XhoI, and the DNA fragment of about 750 bp was obtained by electrophoresis on 2% agarose gel, and then it was inserted into a pET15b-UB-(p53)-TOM7 vector cleaved by the restriction enzymes SacII and XhoI using a T4DNA ligase to obtain the plasmid pET15b-UB-scFvHER2-TOM7 (Figure 39). In this case, UB-scFvHER2-TOM7 was represented by the base sequence of SEQ ID NO: 38.

E. coli BL21(DE3) strain was transformed using the plasmid pET15b-UB-scFvHER2-TOM7. Thereafter, the transformed strain was cultured in a Luria-Bertani (LB) solid medium to which the antibiotic ampicillin was added, and then the colonies obtained herein were cultured in a LB liquid medium under the condition of 37°C. Then, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 44, it was confirmed that a scFvHER2 protein having a size of about 35 kDa in the form to which ubiquitin and TOM7 were fused was expressed. In this case, lane M in Figure 44 shows a protein molecular weight marker, lane 1 shows the precipitate centrifuged after E. coli was crushed 4 hours after adding IPTG, and lane 2 shows the supernatant centrifuged after E. coli was crushed.

**Example 6.2.2. Preparation of pCMV-scFvHER2-TOM7-myc/His**

In order to prepare a scFvHER2 protein in the form to which TOM7 binding to the mitochondrial outer membrane was fused, an expression vector for animal cells capable of expressing scFvHER2 in the form to which TOM7 was fused was prepared. In order to obtain the TOM7 and scFvHER2 genes, RscFvHER2 primer and XTOM7(noT) primer were prepared. The sequence of each primer is as described in Table 11 below.

Table 111

| Primer | Sequence | SEQ ID NO. |
|---|---|---|
| RscFvHER2 | 5'-AAA AAA GAA TTC ATG GAA GTG CAA CTT GTT GAG AGT GG -3' | SEQ ID NO: 39 |
| XTOM7(noT) | 5'- AAA AAA CTC GAG TCC CCA AAG TAG GCT CAA AAC AG-3' | SEQ ID NO: 40 |

The plasmid pET15b-UB-scFvHER2-TOM7 obtained in Example 6.2.1. was used as a template, and 0.2 pmol primer (RscFvHER2) and 0.2 pmol primer (XTOM7(noT)) were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles to obtain a gene scFvHER2-TOM7. The amplified scFvHER2-TOM7 gene was cleaved by the restriction enzymes EcoRI and XhoI, and the DNA fragments of about 850 bp, respectively, were obtained by electrophoresis on 1% agarose gel, and then inserted into a pcDNA3.1-myc/His A vector cleaved by the restriction enzymes EcoRI and XhoI using a T4DNA ligase to obtain the plasmid pCMV-scFvHER2-TOM7-myc/His (Figure 45).

In this case, scFvHER2-TOM7-myc/His was represented by the base sequence of SEQ ID NO: 41. It was transfected into an animal cell CHO using the plasmid pCMV-scFvHER2-TOM7-myc/His, and the cells was crushed, and then SDS-polyacrylamide electrophoresis was performed, and it was shown by Western blot using an anti-c-myc antibody. As shown in Figure 46, it was confirmed that a scFvHER2 protein having a size of about 35 kDa in the form to which TOM7 was fused was expressed. In this case, lane M in Figure 46 shows a protein molecular weight marker, and lane 1 shows that it was transfected into an animal cell CHO, and the cells was crushed, and then SDS-polyacrylamide electrophoresis was performed, and then it was confirmed by Western blot using an anti-c-myc antibody.

**Example 6.3. Isolation and purification of recombinant UB-ScFvHER2-TOM7 protein derived from E. coli**

The UB-ScFvHER2-TOM7 protein was isolated and purified in the same method as in Example 1.3.1. As a result, the UB-ScFvHER2-TOM7 protein was eluted (Figure 47). In this case, lane M in Figure 47 shows a protein molecular weight marker, and lane 1 shows a nickel affinity chromatography loading sample. Lane 2 shows that it was not bound to a nickel affinity resin. Lane 3 shows the results of elution with 8M UREA/50mM Na-phosphate/500mM NaCl/10mM Imidazole. Lanes 4 to 5 show the results of elution with 8M UREA/50mM Na-phosphate/500mM NaCl/50mM Imidazole. Lanes 6 to 8 show the results of elution with 8M UREA/50mM Na-phosphate/500mM NaCl/100mM Imidazole. Lanes 9 to 10 show the results of elution with 8M UREA/50mM Na-phosphate/500mM NaCl/250mM Imidazole. Lane 11 shows the results of elution with 8M UREA/50mM Na-phosphate/500mM NaCl/500mM Imidazole.

The protein amount of the recovered eluted solution was measured by protein quantification method and confirmed using SDS-PAGE. As shown in Figure 48, after the confirmation was completed, the UB-ScFvHER2-TOM7 protein was quenched with the liquid nitrogen and stored in a cryogenic freezer at -80°C. In this case, lane M in Figure 48 shows a protein molecular weight marker, and lane 1 shows the UB-ScFvHER2-TOM7 protein obtained after dialysis in PBS buffer solution.

**Example 7. Preparation of fusion protein comprising scFvMEL**

**Example 7.1. Synthesis of scFvMEL gene**

In order to express the human scFvMEL into a recombinant protein as an antibody fragment against melanoma, the scFvMEL gene obtained by requesting gene synthesis from Bionics Co., Ltd. was designated as pUC57-scFvMEL, and the base sequence of scFvMEL was the same as the base sequence of SEQ ID NO: 42.

### Example 7.2. Preparation of scFvMEL protein expression vector

#### Example 7.2.1. Preparation of pET15b-UB-scFvMEL-TOM7

In order to prepare a scFvMEL protein in the form to which ubiquitin and TOM7 binding to the mitochondrial outer membrane were fused, an expression vector capable of expressing scFvMEL in the form to which ubiquitin and TOM7 were fused was prepared.

The plasmid pUC57-scFvMEL gene obtained in Example 7.1. was cleaved by the restriction enzymes SacII and XhoI, and the DNA fragment of about 750 bp was obtained by electrophoresis on 2% agarose gel, and then inserted into a pET15b-UB-(p53)-TOM7 vector cleaved by the restriction enzymes SacII and XhoI using a T4DNA ligase to obtain the plasmid pET15b-UB-scFvMEL-TOM7 (Figure 49). In this case, UB-scFvMEL-TOM7 was represented by the base sequence of SEQ ID NO: 43.

E. coli BL21(DE3) strain was transformed using the plasmid pET15b-UB-scFvMEL-TOM7. Thereafter, the transformed strain was cultured in a Luria-Bertani (LB) solid medium to which the antibiotic ampicillin was added, and then the colonies obtained herein were cultured in a LB liquid medium in a 37°C shaking incubator. Thereafter, when the cell density reached about 0.2 absorbance at OD600, IPTG was added so that a final 1 mM concentration was made, and then the shaking culture was performed further for about 4 hours.

A portion of E. coli cells was obtained by centrifugation, and then the cells were crushed, and then SDS-polyacrylamide electrophoresis was performed. As shown in Figure 50, it was confirmed that a scFvMEL protein having a size of about 35 kDa in the form to which ubiquitin and TOM7 were fused was expressed. In this case, lane M in Figure 50 shows a protein molecular weight marker, lane 1 shows the precipitate centrifuged after E. coli was crushed 4 hours after adding IPTG, and lane 2 shows the supernatant centrifuged after E. coli was crushed.

#### Example 7.2.2. Preparation of pCMV-scFvMEL-TOM7-myc/His

In order to prepare a scFvMEL protein in the form to which TOM7 binding to the mitochondrial outer membrane was fused, an expression vector for animal cells capable of expressing scFvMEL in the form to which TOM7 was fused was prepared. In order to obtain the TOM7 and scFvMEL genes, a primer (RscFvMEL) was prepared. The sequence of each primer is as described in Table 12 below.

[Table 12]

| Primer | Sequence | SEQ ID NO. |
|---|---|---|
| RscFvMEL | 5'- AAA AAA GAA TTC ATG AAA ACA AGT AAC CCA GGA GTG-3' | SEQ ID NO: 44 |

The plasmid pET15b-UB-scFvMEL-TOM7 obtained in Example 6.2.1. was used as a template, and 0.2 pmol RscFvMEL primer and 0.2 pmol XTOM7(noT) primer were mixed with dNTP 0.2 nM, 1x AccuPrime Taq DNA polymerase reaction buffer solution (Invitrogen, USA) and 1 unit of AccuPrime Taq DNA polymerase. Thereafter, in a polymerase chain reaction apparatus, amplification reactions of 95°C for 40 seconds, 58°C for 30 seconds, 72°C for 1 minute were performed at 25 cycles to obtain scFvMEL-TOM7. The amplified scFvMEL-TOM7 gene was cleaved by the restriction enzymes EcoRI and XhoI, and the DNA fragment of about 850 bp was obtained by electrophoresis on 1% agarose gel. Then, it was inserted into a pcDNA3.1-myc/His A vector cleaved by the restriction enzymes EcoRI and XhoI using a T4DNA ligase to obtain the plasmid pCMV-scFvMEL-TOM7-myc/His (Figure 51). Here, scFvMEL-TOM7-myc/His was represented by the base sequence of SEQ ID NO: 45.

It was transfected into an animal cell CHO using the plasmid pCMV-scFvMEL-TOM7-myc/His, and the cells was crushed, and then SDS-polyacrylamide electrophoresis was performed, and it was shown by Western blot using an anti-c-myc antibody. As shown in Figure 52, it was confirmed that a scFvMEL protein having a size of about 35 kDa in the form to which TOM7 was fused was expressed. In this case, lane M in Figure 52 shows a protein molecular weight marker, and lane 1 shows that it was transfected into an animal cell CHO, and the cells was crushed, and then SDS-polyacrylamide electrophoresis was performed, and then it was confirmed by Western blot using an anti-c-myc antibody.

### Example 8. Preparation of fusion protein comprising scFvPD-L1

#### Example 8.1. Synthesis of scFvPD-L1 gene

In order to express the human scFvPD-L1 into a recombinant protein, the scFvPD-L1 gene obtained by requesting gene synthesis from Bionics Co., Ltd. was designated as pUC57-scFvPD-L1, whose base sequence was the same as the base sequence of SEQ ID NO: 46.

**Example 8.2. Preparation of scFvPD-L1 protein expression vector**

**Example 8.2.1. Preparation of pCMV-scFvPD-L1-TOM7-myc/His**

In order to prepare a scFvPD-L1 protein in the form to which TOM7 binding to the mitochondrial outer membrane was fused, an expression vector for animal cells capable of expressing scFvPD-L1 in the form to which ubiquitin and TOM7 was fused was prepared.

The plasmid pUC57-scFvPD-L1 was cleaved by the restriction enzymes EcoRI and XhoI, and the DNA fragment of about 760 bp was obtained by electrophoresis on 1% agarose gel. Then, it was inserted into a pCMV-(scFvMEL)-TOM7-myc/His vector cleaved by the restriction enzymes EcoRI and XhoI using a T4DNA ligase to obtain the plasmid pCMV-scFvPD-L1-TOM7-myc4Es (Figure 53). In this case, scFvPD-L1-TOM7-myc/His was represented by the base sequence of SEQ ID NO: 47.

It was transfected into an animal cell CHO using the plasmid pCMV-scFvPD-L1-TOM7-myc/His, and the cells was crushed, and then SDS-polyacrylamide electrophoresis was performed, and it was shown by Western blot using an anti-c-myc antibody. As shown in Figure 54, it was confirmed that a scFvPD-L1 protein having a size of about 35 kDa in the form to which TOM7 was fused was expressed. In this case, lane M in Figure 54 shows a protein molecular weight marker, and lane 1 shows that it was transfected into an animal cell CHO, and the cells was crushed, and then SDS-polyacrylamide electrophoresis was performed, and then it was confirmed by Western blot using an anti-c-myc antibody.

**III. Preparation of modified mitochondria to which fusion protein** was **bound**

**Example 9. Preparation of modified mitochondria**

The following experiment was conducted to confirm whether the fluorescent protein fused with the mitochondrial outer membrane binding site binds to the outer membrane of the mitochondria. First, the mitochondria were isolated from mesenchymal stem cells derived from umbilical cord (UC-MSCs) by centrifugation method. Thereafter, they were stained with MitoTracker CMXRos Red. They were mixed with the recombinant protein TOM70-(GGGGS)3-UB-GFP purified from E. coli in the above and incubated at ambient temperature for about 30 minutes.

Thereafter, the unreacted protein was removed by centrifugation and washed twice with PBS buffer solution. Thereafter, the fluorescent protein in the form bound to the mitochondria was observed using a fluorescence microscope. As a control group, the purified GFP protein that does not comprise a mitochondrial outer membrane binding site was used. As a result, it was confirmed that the fluorescent protein fused with the mitochondrial outer membrane binding site (TOM70-(GGGGS)3-UB-GFP) was located in the same place as the mitochondria of mesenchymal stem cells derived from umbilical cord (UC-MSC) (Figure 55a, Figure 55b).

**Example 10. Confirmation of ability of recombinant protein p53 to bind to foreign mitochondrial outer membrane**

The mitochondria that had been isolated from mesenchymal stem cells derived from umbilical cord using centrifugation method were mixed with the purified recombinant protein TOM70-(GGGGS)3-UB-p53 or UB-p53-TOM7, and were allowed to be bound at a ratio of 1:1 under a reaction condition of at 4°C for 1 hour. As a control group, the mitochondria that were not mixed with the protein were used. The binding ability between mitochondria and p53 was confirmed through a Western blot experiment method (Figure 56).

First, the mitochondria and the p53 protein were bound, and then centrifugation was performed at 13,000 rpm for 10 minutes to obtain the mitochondria or the mitochondria to which p53 was bound in the form of a precipitate. The protein that was not bound to the mitochondria was removed through a PBS washing process twice, and the washed precipitate was subjected to protein electrophoresis (SDS-PAGE) and then Western Blot. Rabbit anti-p53 antibody was used as a primary antibody, and anti-rabbit IgG HRP was used as a secondary antibody. The band was confirmed at the same position as a size of 60 kDa, which is a molecular weight expected in the experimental group for mitochondria that did bind to TOM70-(GGGGS)3-UB-p53 or UB-p53-TOM7, compared to the control group for mitochondria alone that did not bind to the protein (Figure 56).

## IV. Confirmation of activity of modified mitochondria to which active protein was bound

### Example 11. Isolation and intracellular injection of foreign mitochondria

The mitochondria were isolated from mesenchymal stem cells derived from umbilical cord (UC-MSCs) using centrifugation method. The isolated mitochondria were stained with Mitotracker CMX Ros, and the concentration and total amount of the isolated mitochondria was confirmed by BCA quantification method, and 0 ug, 1 ug, 5 ug, 10 ug, 50 ug, 100 ug of mitochondria were injected into SNU-484 cells, a gastric cancer cell line, using centrifugation method. As a result of the experiment, it was confirmed that the degree of mitochondria injected into the cells was concentration-dependent on the amount of mitochondria by a fluorescence microscope (Figure 57).

### Example 12. Confirmation of influence of normal mitochondria on cancer cells

The following experiment was conducted to investigate how mitochondria derived from normal cells have an influence on the proliferation of cancer cells and ROS production. First, liver cells (WRL-68), fibroblasts, and mesenchymal stem cells derived from umbilical cord (UC-MSCs) were selected as mitochondria donor cells. The mitochondria were isolated from the cells by a centrifugation fractionation method, respectively. The cancer cell used as a mitochondria recipient cell was a skin epidermal cancer cell, A431 cell line. In this case, the mitochondria were delivered into the skin epidermal cancer cells using centrifugal force according to the concentration (see Korean Patent Appln. No. 10-2017-0151526).

After 24, 48, and 72 hours after introduction, the proliferation of skin epidermal cancer cells and the production of reactive oxygen species (ROS) were observed. As a result, it was confirmed that when mitochondria obtained from normal cells from various origins were injected into cancer cells, there was an effect of inhibiting the proliferation of cancer cells depending on the concentration. In addition, it was confirmed that ROS production in cancer cells was inhibited depending on the concentration of normal mitochondria (Figures 58 and 59).

### Example 13. Confirmation of influence of normal mitochondria on drug resistance

It was investigated how to influence on drug resistance, the expression of an antioxidant gene, cancer metastasis (metastasis), which are features of cancer cells, when mitochondria derived from normal cells were injected into cancer cells, by the following methods. First, normal liver cells (WRL-68) were set as mitochondria donor cells, and the mitochondria were isolated from the cells by centrifugation fractionation method, and the mitochondria were used. HepG2 cells, a liver cancer cell line, were used as cancer cells used as mitochondria recipient cells. The mitochondria were delivered into the liver cancer cells using centrifugal force according to the concentration, and then it was confirmed that as a result of observation of the drug resistance to doxorubicin, an anticancer agent, cancer cell lines that received mitochondria showed higher drug sensitivity (Figure 60).

### Example 14. Confirmation of influence of normal mitochondria on antioxidant effect

As the mitochondria isolated from normal cells were injected into HepG2 cells, a liver cancer cell line, according to the concentration, it was confirmed that the expression of enzyme catalase, an antioxidant protein, and SOD-2 (superoxide dismutase-2) genes in cancer cells were increased (Figure 61).

### Example 15. Confirmation of influence of normal mitochondria on cancer cell metastasis

In relation to metastasis, it was confirmed whether there was the expression of $\alpha$-smooth muscle actin (a-SMA) gene, one of the genes involved in EMT (epithelial to mesenchymal transition). In this case, it was found that, in the case of liver cancer cells that received mitochondria, the expression of $\alpha$-SMA protein was significantly reduced depending on the concentration of mitochondria, compared to liver cancer cells that did not receive mitochondria. On the contrary, it was found that the E-cadherin protein, one of the cell adhesion proteins, was increased depending on the concentration of mitochondria (Figure 62). It was confirmed that the changes of proteins known to be involved in the cancer metastasis are made by normal mitochondria injected into the cancer cells, and thus also influence the metastasis of cancer cells.

### Example 16. Confirmation of loading of recombinant protein p53 on foreign mitochondrial outer membrane and injection into cells

The mitochondria were isolated from mesenchymal stem cells derived from umbilical cord using centrifugation method, and then were stained with Mitotracker CMX Ros, and were mixed with the purified recombinant protein TOM70-(GGGGS)3-UB-p53 or UB-p53-TOM7, and were incubated at a ratio of 1:1 under a reaction condition of at 4°C for 1 hour, and then were centrifuged to remove the unreacted proteins, and then were washed twice with buffer solution PBS, and then the mitochondria in the form to which p53 protein was bound were injected into SNU-484 cells, a gastric cancer cell line, by centrifugation method (Figure 63). In this case, a control group was set to a group that did not use mitochondria and a group that used mitochondria alone. After one day of culture, the p53 protein loaded on the foreign mitochondria injected into the cells was observed with a fluorescence microscope using immunocytochemistry (ICC).

Rabbit anti-p53 antibody was used as a primary antibody, and Goat anti-rabbit IgG Alexa Fluor 488 was used as a secondary antibody. As a result, it was confirmed that TOM70-(GGGGS)3-UB-p53 (green stained) or UB-p53-TOM7 (green stained) protein loaded on the foreign mitochondria (red stained) was located in the cytoplasm in the cells that were injected along with the foreign mitochondria during injection into the cells (Figure 64, 200 magnification and Figure 65, 400 magnification). As a result, it was found that the recombinant protein was easily injected into the cell via the mitochondria.

**Example 17. Confirmation of activity of p53 loaded mitochondria in cancer cell line**

**Example 17.1. Confirmation of apoptosis ability of p53 loaded foreign mitochondria injected into cells using gastric cancer cell line**

The mitochondria isolated from mesenchymal stem cells derived from umbilical cord using centrifugation method were mixed with the recombinant protein TOM70-(GGGGS)3-UB-p53 or UB-p53-TOM7 that was purified from E. coli, and were allowed to be bound at a ratio of 1:1 under a reaction condition of at 4°C for 1 hour. As a control group, the UB-p53 protein that does not comprise TOM70 and TOM70-(GGGGS)3-p53 that does not comprise ubiquitin were used. The proteins unbound were removed by centrifugation and PBS washing process, and the mitochondria to which proteins were bound were injected into a gastric cancer cell line SNU-484, which lacks p53 ability due to the variation of p53 gene, by centrifugation (Figure 66). After one day of culture, the fixation was performed with 4% paraformaldehyde for 1 hour, and then the permeabilization of cells was induced using permeabilization solution (0.1% sodium citrate buffer comprising 0.1% Triton-X-100, pH 7.4), and reacted with TUNEL solution (In situ cell death detection kit, TMR RED, Roche) at 37°C for 1 hour.

In the TUNEL analysis method, the portion where the fragmentation of nucleic acid (DNA fragmentation) occurred is stained in red color, indicating that apoptosis occurs. Compared to the control group, in the cells injected with the mitochondria to which TOM70-(GGGGS)3-ub-p53 or p53-TOM7 was bound, a large amount of red stained portion was found, unlike the control group, indicating that apoptosis occurred by the mitochondria to which TOM70-(GGGGS)3-UB-p53 or UB-p53-TOM7 was bound. In particular, it was confirmed that more apoptosis occurred in the mitochondria to which the protein in the form of TOM70-(GGGGS)3-UB-p53 was bound (Figure 67a).

**Example 17.2. confirmation of apoptosis ability of p53 loaded foreign mitochondria to which luciferase was bound**

In order to confirm whether the biological activity of the delivered TOM70-(GGGGS)3-UB-p53 protein in a recipient cell was maintained after the TOM70-(GGGGS)3-UB-p53 protein in the form bound to the mitochondria obtained in Example 5.2. above was delivered into the recipient cells, a cell-based analysis using a reporter gene was performed. Since the p53 protein is a transcription factor, a gene in which the base sequence RRRCWWGYYY (wherein R represents G or A, W represents A or T, and Y represents C or T) to which the p53 transcription factor can bind is repeated 6 times was synthesized with the following sequence. The base sequence of P53-promter-S is as follows (5'-GGG CAT GCT CGG GCA TGC CCG GGC ATG CTC GGG CAT GCC CGG GCA TGC TCG GGC ATG CCC-3')(SEQ ID NO: 91), and the base sequence of P53-promter-AS is as follows (5'-GGG CAT GCC CGA GCA TGC CCG GGC ATG CCC GAG CAT GCC CGG GCA TGC CCG AGC ATG CCC-3')(SEQ ID NO: 92).

5 ug of the synthesized gene P53-promter-S and 5 ug of the synthesized gene P53-promter-AS were incubated at 70°C for 20 minutes to promote the synthesis of double helix gene, and then the phosphorylation reaction was induced using a polynucleotide T4 kinase enzyme. The double helix gene in which the phosphorylation was induced was inserted into a pGL3 vector cleaved by the restriction enzyme Sma I, and a gene in which the base sequence (RRRCWWGYYY) to which the p53 transcription factor can bind is repeated 6 times was allowed to be bound to luciferase, a reporter gene, to prepare the plasmid p6xp53-Luc. The plasmid p6xp53-Luc and the plasmid pRSVb-gal, a beta-galactosidase expression vector, were transformed into HEK293 cells, human renal cells, by lipofectamine method.

Subsequently, after 6 hours, the HEK293 cells were treated with a combination in which 10 ug of the mitochondria and 5 ug, 10 ug, and 20 ug of the TOM70-(GGGGS)3-UB-p53 protein was bound, respectively. In this case, as a control group, the cells were treated with 10 ug of the mitochondria to which PBS or the p53 protein was bound, respectively. The treated cells were cultured for 18 hours, and then the luciferase activity was measured and analyzed. In this case, in order to correct the efficiency of transformation, the luciferase value divided by the value obtained by measuring the activity of beta-galactosidase was determined as a corrected luciferase value.

It was confirmed that the luciferase value was increased in the cells treated with a combination in which 10 ug of the mitochondria and 5 ug, 10 ug, and 20 ug of the TOM70-(GGGGS)3-UB-p53 protein was bound, respectively. Thus, it was confirmed that the p53 protein entered into the cells and exhibited the activity (Figure 67b).

**Example 18. Confirmation of ability of RKIP loaded foreign mitochondria injected into cells to reduce metastasis of cancer cell line**

The mitochondria isolated from mesenchymal stem cells derived from umbilical cord using centrifugation method were mixed with the purified recombinant protein TOM70-(GGGGS)3-UB-RKIP, and were allowed to be bound at a ratio of 1:1 under a reaction condition of at 4°C for 1 hour. The mitochondria to which the protein was bound were injected by centrifugation into the breast cancer cell line MDA-MB-231, which is known to have increased metastasis ability due to a decrease in RKIP protein.

In order to confirm the ability of metastasis of cancer cells, a cell invasion assay using a transwell plate was performed. The transwell upper-chamber having a pore size of 8 μm was coated with matrigel for 30 minutes at 37°C. As a test group, MDA-MB-231 cells injected with mitochondria alone and MDA-MB-231 cells injected with mitochondria to which RKIP protein was bound were used. Each cell at $1x10^5$ cells was placed in a transwell upper chamber containing serum-free medium, and a medium comprising 10% bovine serum was placed in a lower-chamber. After culturing at 37°C for 12 hours, the fixation was performed with 4% paraformaldehyde for 1 hour, and then the cells that passed through matrigel were stained with 1% crystal violet.

As a result of observation under a microscope, the cells stained in purple were observed in the membrane below the upper-chamber, and this can be said to be a process in which metastasis of cells occurred. It was confirmed that the cells stained in purple were reduced in the experimental group treated with mitochondria alone and the experimental group treated with mitochondria to which RKIP was bound, compared to the control group that was treated with nothing. Four parts were randomly selected, and then the number of stained cells was measured and was plotted on the graph (Figure 68).

**IV. Confirmation of delivery rate of modified mitochondria to which target targeting protein was bound**

**Example 19. Confirmation of intracellular expression of single chain variable fragment (ScFv) antibody for targeting cancer cells and confirmation of binding with mitochondria in cells**

In order to express pCMV-ScFv-HER2-TOM7 or pCMV-ScFv-MEL-TOM7 or pCMV-ScFv-PD-L1-TOM7 in animal cells, the DNA was transfected into CHO cells using Lipofectamine LTX and PLUS or Lipofectamine 2000. GFP-TOM7 DNA was used as a control group. In order to confirm that they are expressed in a cell and binds to mitochondria in the same cell, cytosol and mitochondria were isolated from the transfected cells using centrifugation method and adjusted to the same protein amount using a BCA assay, and then PAGE electrophoresis was performed, and then the results were observed by Western blot. Monoclonal c-myc antibody was used as a primary antibody, and Anti-mouse IgG HRP was used as a secondary antibody.

The bands of ScFv-HER2-TOM7 or ScFv-MEL-TOM7 proteins were identified at the expected size of 35 kDa. Based on that all were identified in the mitochondrial layer, it could be expected that the transfected and expressed proteins were bound to mitochondria in cells by TOM7 (Figure 69).

Next, in order to confirm the binding of the target protein expressed in a cell to the mitochondria in the same cell, the ScFv-HER2-TOM7, ScFv-MEL-TOM7 or ScFv-PD-L1-TOM7 protein expressed in the cell was observed with a fluorescence microscope using an immunocytochemistry (ICC) experimental method. Monoclonal c-myc antibody was used as a primary antibody, and Goat anti-mouse IgG Alexa Fluor 488 was used as a secondary antibody. The mitochondria in the cell were stained with Mitotracker CMX Ros. As a result, it was confirmed that the expressed ScFv-HER2-TOM7, ScFv-MEL-TOM7 or ScFv-PD-L1-TOM7 proteins were colocalized with the mitochondria and were bound to the mitochondria in the cell (Figures 70 and 71).

**Example 20. Isolation of mitochondria to which single chain variable fragment antibody for targeting cancer cells was bound and comparison of injection of mitochondria in gastric cancer cell line**

The mitochondria were isolated from CHO cells into which pCMV-ScFv-HER2-TOM7 or pCMV-ScFv-PD-L1-TOM7 was transfected. As a control group, the mitochondria of CHO cells which were not transformed were isolated and used. The mitochondria isolated from each cell were stained with Mitotracker CMX Ros. SNU-484, a gastric cancer cell line, was treated with the same amount of mitochondria, and the next day, the degree of mitochondria injected into the cells were compared and confirmed using a fluorescence microscope. It was confirmed that, compared to the control group, the mitochondria to which ScFv-HER2-TOM7 or ScFv-PD-L1-TOM7 was bound were injected into cancer cells more than the mitochondria obtained from the control group (Figure 72). Therefore, it was found that the mitochondria to which the target protein was bound is more easily injected into cancer cells when using mitochondria alone.

**VI. Confirmation of in vivo activity of modified mitochondria to which active protein was bound**

**Example 21. Construction of Xenograft model (SNU-484) and administration of test substance**

**Example 21.1. Preparation of cancer cells**

On the day of the experiment, SNU-484 cell line, a gastric cancer cell line, was prepared to be 5 x $10^6$ cells per mouse. The medium of the cells was removed, and then PBS was added to wash the cells. The cells were dissociated using a Trypsin-EDTA solution, and then the cells were placed in a 50 mL tube, and washed twice with PBS buffer solution, and then 20 mL of PBS was added, and the number of cells and viability were measured. Based on the measured number of cells, the number of cells was adjusted to be 5 x $10^6$ cells per mouse, and the cells were prepared by dividing them into groups. The volume to be transplanted per mouse was adjusted to the same amount of 100 μL. As a control group, 100 μL of a cancer cell alone group was prepared.

**Example 21.2. Preparation of test substance**

The mitochondria isolated from umbilical cord blood mesenchymal stem cells as described above were prepared for the transplantation at an amount of 50 μg per mouse based on the protein concentration. In the case of a group to which mitochondria alone were administered, the mitochondria were prepared by mixing well with 100 μL of PBS in which cancer cells were mixed. In the case of the modified mitochondria group, the TOM70-(GGGGS)3-UB-p53 protein was mixed together in a concentration ratio of 1:1 with the amount of mitochondria prepared in the Eppendorf tube before mixing with cancer cells, and was stood at ambient temperature for 1 hour. After the reaction time was over, the supernatant was removed after centrifugation at 20,000 xg for 10 minutes, and the pellet of the mitochondria (MT+TOM70-(GGGGS)3-UB-p53) to which a protein was bound was obtained. It was washed twice using PBS buffer solution, and then the mitochondria (MT+TOM70-(GGGGS)3-UB-p53) to which p53 protein was bound were prepared by mixing well with 100 μL of PBS in which cancer cells were mixed.

**Example 21.3. Preparation of experimental animal and transplantation of test substance**

For the transplantation sample prepared by the groups, matrigel (BD) was added at the same amount as that of PBS and lightly mixed with the cells to prepare 200 μL of test substance per mouse. In this case, all operations were performed on ice. For the model construction, Balb/c nude mice (female, 7-week old) were purchased from RAONBIO, and anesthetized by the inhalation of isoflurane for the transplantation of cancer cells, and then the right back area (on the basis of animal) was sterilized with an alcohol swab. Thereafter, 200 μL was administered subcutaneously to the right back area of the experimental animal using a 1 mL syringe containing the injection solution. After administration, the weight of the animal and the size of the tumor were measured twice a week, and the analysis of the results proceeded while observing up to 3 weeks (Figure 73).

**Example 21.4. Confirmation of tumor formation**

The volume of the tumor was calculated by measuring the long axis length and short axis length of the tumor and applying them to the following equation.

**<Mathematical equation 1>**

long axis X short axis X short axis X 0.5 = tumor volume (mm^3)

**Example 21.5. Observation of physiological and morphological change**

In order to observe the physiological and morphological change of mice by administration of anticancer candidates, the changes in body weight and the tumor size were measured twice a week from the time of administration of cancer cells and test substances (Figure 74).

The weight of the mouse was measured using a scale, and the change by group was analyzed using the values measured twice a week (Figure 75). It was confirmed that there was no significant difference in the change in body weight for 3 weeks between the group into which mitochondria were not injected, the group to which mitochondria were administered alone, and the group into which modified mitochondria were injected. The size of the tumor was calculated by measuring the length of the long axis (length) and short axis (width) of the tumor using a caliper, and then applying them to the equation of Mathematical equation 1 above. The change by group was analyzed using the values measured twice a week (Figure 76). It was found that the size of the tumor was significantly increased over time in the group that was not treated with mitochondria, whereas in the case of mice that were administered with mitochondria, the increase in the size of the tumor slowed down over time. In addition, it was confirmed that the increase in the size of the tumor was significantly lowered in the group that was administered with mitochondria on which p53 protein was loaded, compared to the group that was administered with mitochondria alone (Figure 76).

**Example 22. Confirmation of effect of modified mitochondria on inhibiting proliferation of skin cancer cells**

The mitochondria obtained above to which p53 was bound were delivered to A431 cells, which are skin cancer cells, by centrifugation method, and then the proliferation of A431 cells was observed. In this case, physiological saline was used as a control group, and an equivalent amount of mitochondria to which p53 protein was not fused was used as a control test group. It was confirmed that the mitochondria on which p53 protein, a protein inducing apoptosis, was loaded can significantly inhibit the proliferation of A431 cells, compared to the control group and the group in which only mitochondria were used (Figure 76).

**V. Confirmation of activity of isolated mitochondria**

**Example 23. Confirmation of function of isolated mitochondria: ATP content**

In order to isolate the intracellular mitochondria from mesenchymal stem cells derived from umbilical cord (UC-MSCs), homogenization was performed using a syringe to break the cells, and then continuous centrifugation was performed to obtain the mitochondria. In order to confirm the function of the isolated mitochondria, the mitochondria protein concentration of the isolated mitochondria was quantified through a BCA assay to prepare 5 μg of the mitochondria. The amount of ATP in the mitochondria was confirmed using a CellTiter-Glo luminescence kit (Promega, Madison, WI).

The prepared mitochondria were mixed in 100 ul of PBS, and then prepared in a 96 well plate, and compared to 100 ul of PBS that did not contain mitochondria as a control group. 100 μL of the test solution that was included in the kit was added in the same manner, and reacted and mixed well for 2 minutes in a stirrer, and then reacted at ambient temperature for 10 minutes, and then the amount of ATP was measured using a Luminescence microplate reader. It was confirmed that ATP was increased when mitochondria was included compared to the control group, and the function of mitochondria was confirmed (Figure 78).

**Example 24. Confirmation of function of isolated mitochondria: membrane potential**

In order to confirm the membrane potential of the isolated mitochondria, JC-1 dye (molecular probes, cat no. 1743159) dye was used. The prepared mitochondria were mixed in 50 μL of PBS, and then prepared in a 96 well plate. PBS (50 μL) group that did not contain mitochondria as a control group and CCCP (R&D systems, CAS 555-60-2) treatment group were prepared. CCCP, an ionophore of mitochondria, inhibits mitochondrial function by depolarization of the mitochondrial membrane potential. The CCCP group was reacted with the isolated mitochondria at 50 μM for 10 minutes at room temperature.

Thereafter, it was reacted with JC-1 dye (2 μM) in the same manner, and then the absorbance was measured using a property having a different spectrum according to the concentration generated by a change in the membrane potential. At low concentrations, it exists as a monomer and exhibits green fluorescence, and at high concentrations, dye aggregates (J-aggregate) to exhibit red fluorescence. The mitochondrial membrane potential was analyzed by calculating the ratio of green absorbance to red absorbance. After the reaction was completed, the mitochondria membrane potential was measured using a fluorescence microplate reader (Monomer: Ex 485 / Em 530, J-aggregate: Ex 535 / Em 590). The results are shown in Figure 79.

**Example 25. Confirmation of degree of damage of isolated mitochondria through confirmation of mROS production**

In order to confirm whether 5 μg of mitochondria prepared as described above is damaged, a MitoSOX red indicator (Invitrogen, cat no. M36008) dye capable of analyzing mitochondrial reactive oxygen species in the isolated mitochondria was used. The prepared mitochondria were mixed in 50 μL of PBS, and then prepared in a 96 well plate, and compared to 50 μL of PBS that did not contain mitochondria as a control group. The MitoSOX red dye was mixed

in 50 μL of PBS to be a concentration of 10 μM, and placed in a 96-well plate (final concentration 5 μM), and then reacted in a 37°C, $CO_2$ incubator for 20 minutes. After the reaction was completed, the amount of ROS in mitochondria was measured using a microplate reader (Ex 510/ Em 580). The results are shown in Figure 80.

## VI. Confirmation of dissociation of desired protein bound to mitochondrial outer membrane protein outside and inside cells

### Example 26. Confirmation of dissociation of desired protein bound to mitochondrial outer membrane protein outside cells

In order to obtain a desired protein in a free form when the active protein bound with the mitochondria was injected into the cell, a fusion protein (TOM70-UB-p53 or TOM-UB-GFP) in the form in which ubiquitin protein was inserted between the mitochondrial outer membrane protein and the desired protein was prepared from E. coli. In order to confirm whether ubiquitin sequence was cleaved by UBP1, a ubiquitin cleaving enzyme, the recombinant fusion protein TOM70-UB-p53 was reacted with the UBP1 enzyme at 37°C for 1 hour.

Thereafter, as a result of the analysis by SDS-PAGE electrophoresis, it was confirmed that the dissociation of the ubiquitin protein from the fusion protein did not occur at all by UBP1. This was considered to be an interference phenomenon of the mitochondrial outer membrane protein structurally, and thus, a linker protein composed of the amino acid glycine and serine was inserted between the mitochondrial outer membrane protein and the ubiquitin protein, and a new fusion protein (TOM70-(GGGGS)3-UB-p53 or TOM70-(GGGGS)3-UB-GFP) was obtained by purification from E. coli, and then reacted with UBP1 enzyme at 37°C for 1 hour as described above. As a result, it was confirmed through SDS-PAGE electrophoresis that the 3' end of ubiquitin was cleaved by UBP1 enzyme, and only p53 protein was dissociation as expected (Figure 82).

### Example 26. Confirmation of dissociation of desired protein bound to mitochondrial outer membrane protein inside cells

When the fusion protein (TOM70-(GGGGS)3-UB-p53 or TOM70-(GGGGS)3-UB-GFP) obtained in the above example enters the cell in a state of being bound to mitochondria, it was observed whether the active protein was dissociated by the ubiquitin cleaving enzyme present in the cell. First, the mitochondria obtained from umbilical cord blood mesenchymal cells and the fusion protein TOM70-(GGGGS)3-UB-GFP were reacted for 1 hour in a microtube to allow to be bound, and then the unbound fusion protein was removed by centrifugation and then washed twice with a PBS buffer solution. In this case, the fusion protein (TOM70-(GGGGS)3-GFP) from which ubiquitin was removed was used as a control group.

Thereafter, the protein bound to the mitochondria was injected into MDA-MB-231 cells, a breast cancer cell line, by centrifugation method. After one day, MDA-MB-231 cells were crushed and fractionated into a mitochondrial part and a cytosolic part, respectively, using differentiated gravity. As a result of analysis by SDS-PAGE electrophoresis and Western blot analysis, it was found that in the case of the fusion protein in which ubiquitin was included, GFP proteins dissociated from mitochondrial outer membrane protein, a linker protein and ubiquitin were mostly detected in a cytosolic part, and it was found that in the case of the fusion protein from which ubiquitin was removed, GFP proteins in the form to which mitochondrial outer membrane protein and a linker protein were bound were mostly detected in a mitochondrial fractional part (Figure 83).

As a result, it was found that when the mitochondrial outer membrane protein-linker-ubiquitin-active protein bound to the mitochondria was injected into the cells, the ubiquitin and active protein connection site was cleaved, and the dissociated active protein was released to the cytoplasm, and it was found that through this, mitochondria can be used as a delivery vehicle as one of methods for effectively delivering a useful protein into cells.

## Claims

**1.** Modified mitochondria in which a foreign protein is bound to the outer membrane of the mitochondria.

**2.** The modified mitochondria according to claim 1, wherein the mitochondria are isolated from cells or tissues.

**3.** The modified mitochondria according to claim 2, wherein the cell is any one selected from the group consisting of somatic cells, germ cells, stem cells, and a combination thereof.

**4.** The modified mitochondria according to claim 1, wherein the foreign protein comprises a desired protein capable of functioning inside and outside the cell.

**5.** The modified mitochondria according to claim 1, wherein the foreign protein comprises a mitochondria anchoring peptide.

**6.** The modified mitochondria according to claim 5, wherein the foreign protein is bound to the outer membrane of the mitochondria by a mitochondria anchoring peptide.

**7.** The modified mitochondria according to claim 6, wherein the mitochondria anchoring peptide comprises an N terminal region or a C terminal region of a protein present in the mitochondrial membrane protein.

**8.** The modified mitochondria according to claim 7, **characterized in that** the N terminal region or the C terminal region of the protein present in the mitochondrial membrane protein is located on the outer membrane of the mitochondria.

**9.** The modified mitochondria according to claim 7, **characterized in that** the protein present in the mitochondrial membrane protein is any one selected from the group consisting of TOM20, TOM70, OM45, TOM5, TOM6, TOM7, TOM22, Fis1, Bcl-2, Bcl-x and VAMP1B.

**10.** The modified mitochondria according to claim 7, **characterized in that** the anchoring peptide comprises an N terminal region of any one selected from the group consisting of TOM20, TOM70 and OM45.

**11.** The modified mitochondria according to claim 7, **characterized in that** the mitochondria anchoring peptide comprises a C terminal region of any one selected from the group consisting of TOM5, TOM6, TOM7, TOM22, Fis1, Bcl-2, Bcl-x and VAMP1B.

**12.** The modified mitochondria according to claim 1, wherein the foreign protein is a fusion protein comprising a mitochondria anchoring peptide and a desired protein capable of functioning inside and outside the cell.

**13.** The modified mitochondria according to claim 12, wherein the desired protein is any one selected from the group consisting of an active protein exhibiting an activity in a cell, a protein present in a cell, and a protein having the ability to bind to a ligand or receptor present in a cell membrane.

**14.** The modified mitochondria according to claim 13, wherein the desired protein is any one selected from the group consisting of p53, Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), RAF kinase inhibitory protein(RKIP), p16, KLF-10, LKB1, LHX6, C-RASSF, DKK-3PD1, Oct3/4, Sox2, Klf4, and c-Myc.

**15.** The modified mitochondria according to claim 12, wherein the foreign protein is a desired protein bound to the N terminal region of TOM20, TOM70 or OM45.

**16.** The modified mitochondria according to claim 15, wherein the foreign protein is bound in the following order:
N terminal-N terminal region of TOM20, TOM70 or OM45-desired protein-C terminal.

**17.** The modified mitochondria according to claim 16, wherein the foreign protein further comprises an amino acid sequence recognized by a proteolytic enzyme in eukaryotic cells, or ubiquitin or a fragment thereof between the anchoring peptide and the desired protein.

**18.** The modified mitochondria according to claim 17, wherein the ubiquitin fragment comprises the C terminal Gly-Gly of an amino acid sequence of SEQ ID NO: 71, and comprises 3 to 75 amino acids consecutive from the C terminal.

**19.** The modified mitochondria according to claim 17, wherein the foreign protein further comprises a linker between a desired protein and ubiquitin or a fragment thereof.

**20.** The modified mitochondria according to claim 19, wherein the linker is composed of 1 to 150 amino acids.

**21.** The modified mitochondria according to claim 20, wherein the linker is composed of 5 to 50 amino acids consisting of glycine and serine.

**22.** The modified mitochondria according to claim 21, wherein the linker is (G4S)n, in which n is an integer of 1 to 10.

**23.** The modified mitochondria according to claim 13, wherein the protein having the ability to bind to a ligand or receptor present in a cell membrane is a ligand or receptor present on the surface of a tumor cell.

**24.** The modified mitochondria according to claim 23, wherein the ligand or receptor present on the surface of a tumor cell is any one selected from the group consisting of CD19, CD20, melanoma antigen E(MAGE), NY-ESO-1, carcinoembryonic antigen(CEA), mucin 1 cell surface associated(MUC-1), prostatic acid phosphatase(PAP), prostate specific antigen(PSA), survivin, tyrosine related protein 1(tyrp1), tyrosine related protein 1(tyrp2), Brachyury, Mesothelin, Epidermal growth factor receptor(EGFR), human epidermal growth factor receptor 2(HER-2), ERBB2, Wilms tumor protein(WT1), FAP, EpCAM, PD-L1, ACPP, CPT1A, IFNG, CD274, FOLR1, EPCAM, ICAM2, NCAM1, LRRC4, UNC5H2 LILRB2, CEACAM, Nectin-3, or a combination thereof.

**25.** The modified mitochondria according to claim 12, wherein the foreign protein is bound to a C terminal region of any one selected from the group consisting of TOM5, TOM6, TOM7, TOM22, Fis1, Bcl-2, Bcl-x and VAMP1B.

**26.** The modified mitochondria according to claim 25, wherein the foreign protein is bound in the following order:
N terminal-desired protein-C terminal region of any one selected from the group consisting of TOM5, TOM6, TOM7, TOM22, Fis1, Bcl-2, Bcl-x and VAMP1B-C terminal.

**27.** The modified mitochondria according to claim 26, wherein the foreign protein further comprises a linker between the desired protein and the C terminal region of any one selected from the group consisting of TOM5, TOM6, TOM7, TOM22, Fis1, Bcl-2, Bcl-x and VAMP1B.

**28.** The modified mitochondria according to claim 27, wherein the linker is composed of 1 to 150 amino acids.

**29.** The modified mitochondria according to claim 28, wherein the linker is composed of 5 to 50 amino acids consisting of glycine and serine.

**30.** The modified mitochondria according to claim 21, wherein the linker is (G4S)n, in which n is an integer of 1 to 10.

**31.** A pharmaceutical composition comprising a modified mitochondria according to any of claims 1 to 30 as an active ingredient.

**32.** The pharmaceutical composition according to claim 31, wherein the pharmaceutical composition is for the prevention or treatment of cancer.

**33.** The pharmaceutical composition according to claim 32, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, larynx cancer, acute myeloid leukemia, brain tumor, neuroblastoma, retinoblastoma, head and neck cancer, salivary gland cancer and lymphoma.

**34.** Use of a modified mitochondria as a means of intracellular and extracellular delivery of a foreign protein comprising a desired protein capable of functioning inside and outside the cell.

**35.** The use of the modified mitochondria according to claim 34, wherein the foreign protein comprises a mitochondrial outer membrane anchoring peptide, and is bound to the outer membrane of the mitochondria by the outer membrane anchoring peptide, and is delivered inside and outside the cell.

**36.** A fusion protein comprising a mitochondrial outer membrane anchoring peptide and a desired protein capable of functioning inside and outside the cell.

**37.** The fusion protein according to claim 36, wherein the mitochondrial outer membrane anchoring peptide comprises an N terminal or C terminal sequence of a protein present in the outer membrane of mitochondria.

**38.** The fusion protein according to claim 36, wherein the mitochondrial outer membrane anchoring peptide is any one selected from the group consisting of TOM20, TOM70, OM45, TOM5, TOM6, TOM7, TOM22, Fis1, Bcl-2, Bcl-x and VAMP1B.

**39.** The fusion protein according to claim 36, wherein the desired protein is any one selected from the group consisting of p53, Granzyme B, Bax, Bak, PDCD5, E2F, AP-1(Jun/Fos), EGR-1, Retinoblastoma(RB), phosphatase and tensin homolog(PTEN), E-cadherin, Neurofibromin-2(NF-2), poly[ADP-ribose] synthase 1(PARP-1), BRCA-1, BRCA-2, Adenomatous polyposis coli(APC), Tumor necrosis factor receptor-associated factor(TRAF), RAF kinase inhibitory protein(RKIP), p16, KLF-10, LKB1, LHX6, C-RASSF, DKK-3PD1, Oct3/4, Sox2, Klf4, and c-Myc.

**40.** The fusion protein according to claim 36, wherein when the mitochondrial outer membrane anchoring peptide is TOM20, TOM70 or OM45, the mitochondrial outer membrane anchoring peptide and the desired protein are bound from the N terminal to the C terminal.

**41.** The fusion protein according to claim 36, wherein when the mitochondrial outer membrane anchoring peptide is any one selected from the group consisting of TOM5, TOM6, TOM7, TOM22, Fis1, Bcl-2, Bcl-x and VAMP1B, the desired protein and the mitochondrial outer membrane anchoring peptide are bound from the N terminal to the C terminal.

**42.** The fusion protein according to claim 36, wherein the fusion protein further comprises ubiquitin or a fragment thereof between the mitochondrial outer membrane anchoring peptide and the desired protein.

**43.** The fusion protein according to claim 36, wherein the fusion protein further comprises an amino acid sequence recognized by a proteolytic enzyme in eukaryotic cells between the mitochondrial outer membrane anchoring peptide and the desired protein.

**44.** A polynucleotide encoding the fusion protein according to any one of claims 36 to 43.

**45.** A method for preparing the fusion protein according to any one of claims 36 to 43, comprising
a step of transforming the polynucleotide according to claim 44 into prokaryotic cells, or eukaryotic cells without a ubiquitin degrading enzyme or a proteolytic enzyme in eukaryotic cells; and
a step of obtaining a fusion protein.

**46.** A fusion protein comprising the target targeting protein having the ability to bind to a ligand or receptor present in a cell membrane and the mitochondrial outer membrane anchoring peptide.

**47.** The fusion protein according to claim 46, wherein the mitochondrial outer membrane anchoring peptide is any one selected from the group consisting of TOM5, TOM6, TOM7, TOM22, Fis1, Bcl-2, Bcl-x and VAMP1B.

**48.** The fusion protein according to claim 46, wherein the target targeting protein having the ability to bind to a ligand or receptor present in a cell membrane and the mitochondrial outer membrane anchoring peptide are bound from the N terminal to the C terminal.

**49.** The fusion protein according to claim 48, wherein the target targeting protein having the ability to bind to a ligand or receptor present in a cell membrane is an antibody or a fragment thereof.

**50.** The fusion protein according to claim 49, wherein the fragment of the antibody is any one selected from the group consisting of Fab, Fab', scFv and F (ab)2.

**51.** A polynucleotide encoding the fusion protein according to any one of claims 46 to 50.

**52.** A method for preparing modified mitochondria, comprising a step of mixing the isolated mitochondria with the fusion protein according to any one of claims 36 to 43 and/or the fusion protein according to any one of claims 46 to 50.

**53.** A method for preparing modified mitochondria from the transformed cells by injecting a polynucleotide encoding the fusion protein according to any one of claims 36 to 41 and/or the fusion protein according to any one of claims 46 to 50 into eukaryotic cells.

**FIG. 1**

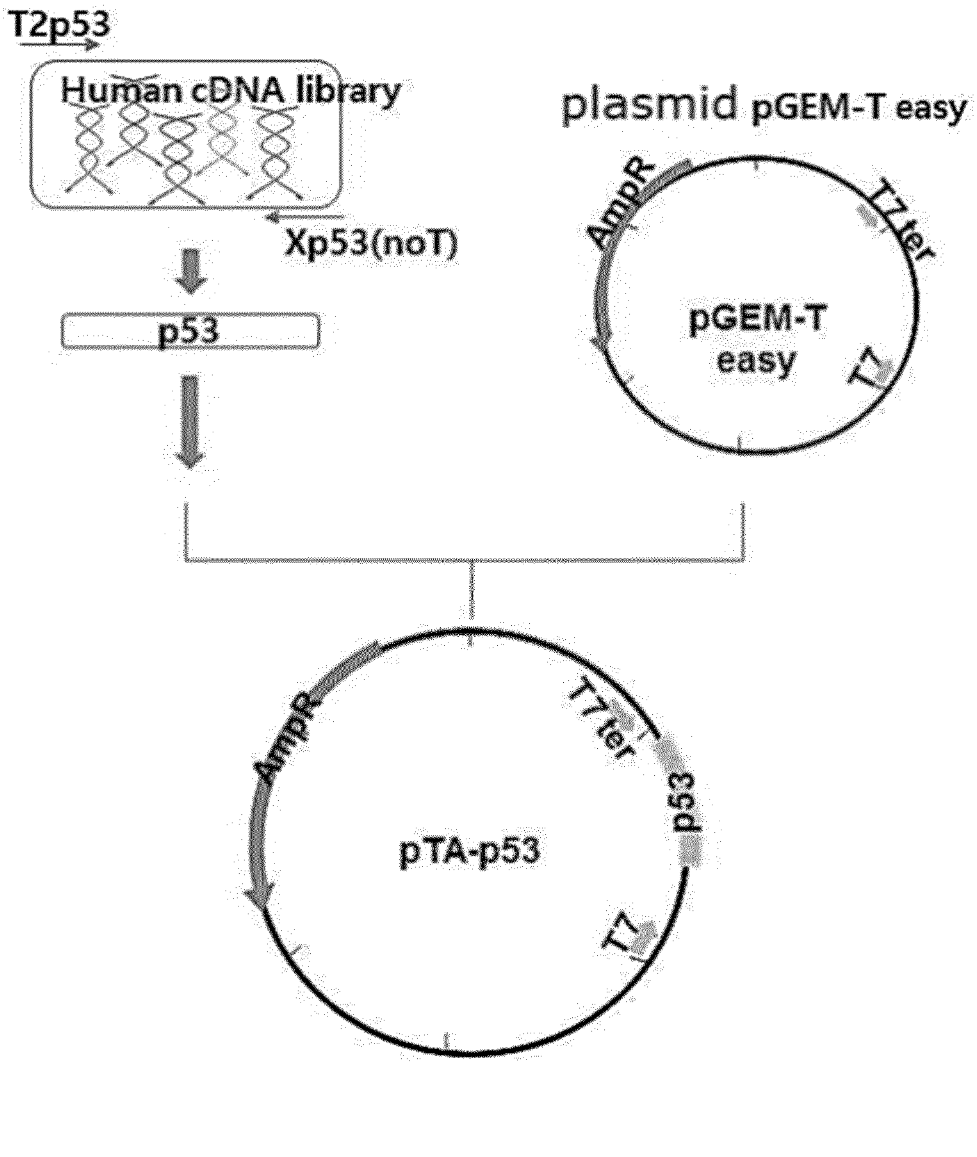
T2p53
Human cDNA library
Xp53(noT)
p53
plasmid pGEM-T easy
AmpR
T7ter
pGEM-T
easy
T7
AmpR
T7 ter
p53
pTA-p53
T7

FIG. 2

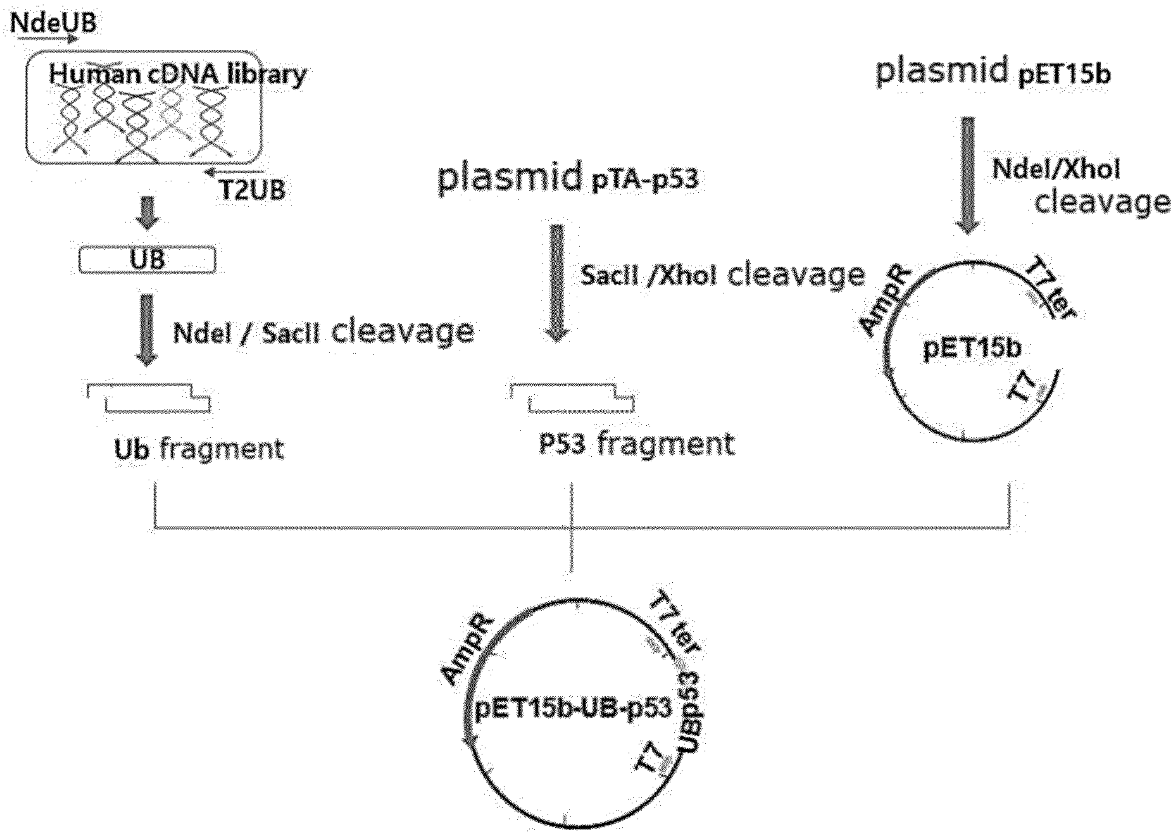
NdeUB
Human cDNA library
T2UB
UB
NdeI / SacII cleavage
Ub fragment
plasmid pTA-p53
SacII /XhoI cleavage
P53 fragment
plasmid pET15b
NdeI/XhoI cleavage
AmpR
T7 ter
pET15b
T7
AmpR
T7 ter
pET15b-UB-p53
UBp53
T7

FIG. 3

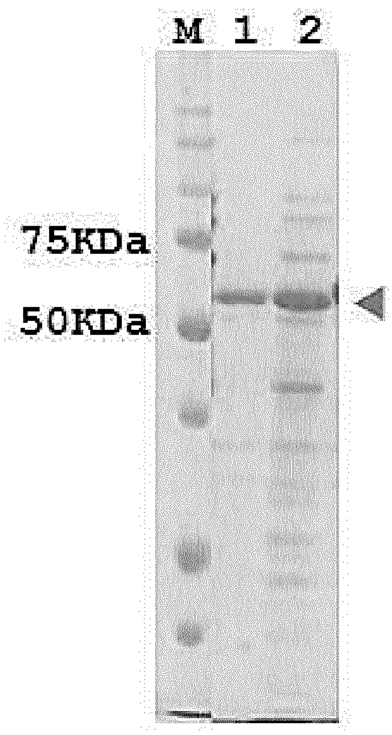
M
1
2
75KDa
50KDa

FIG. 4

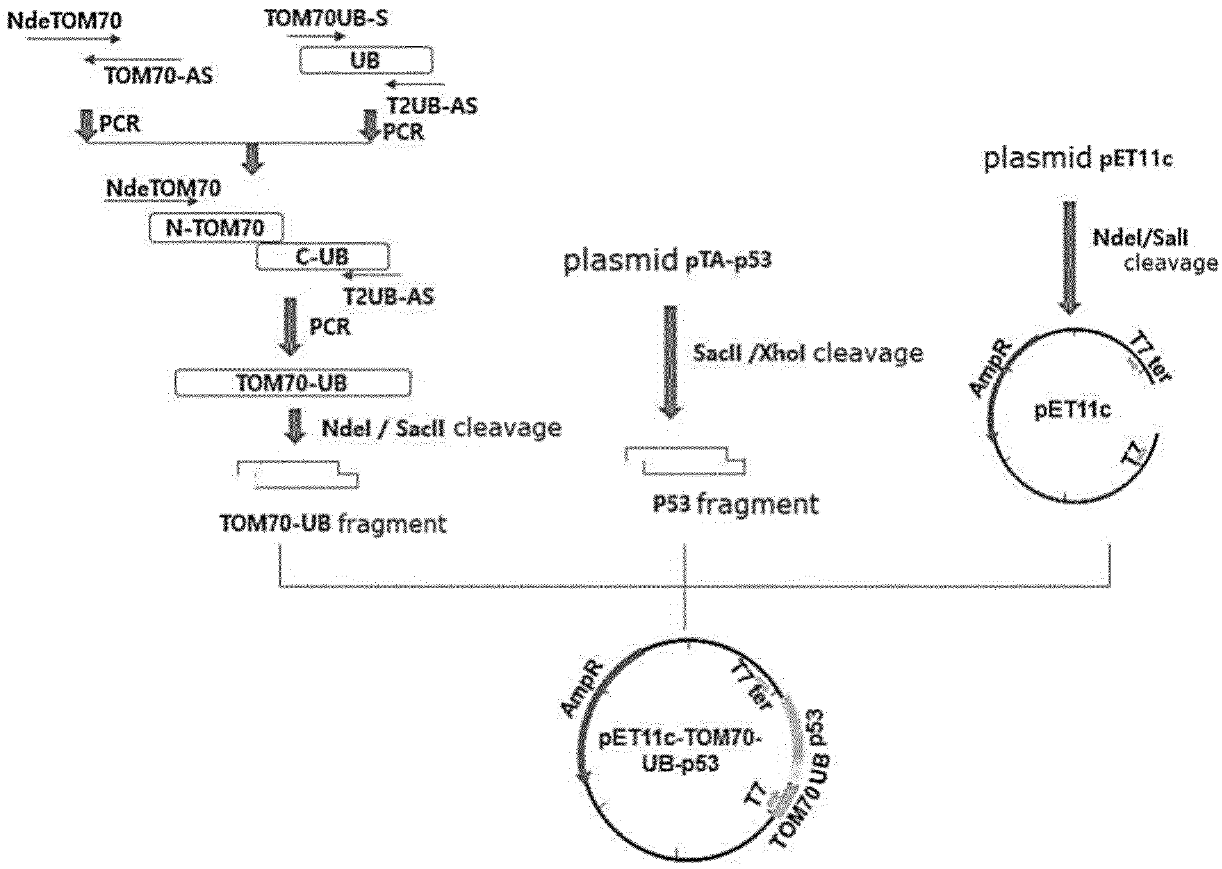
NdeTOM70
TOM70UB-S
UB
TOM70-AS
T2UB-AS
PCR
PCR
NdeTOM70
N-TOM70
C-UB
T2UB-AS
PCR
TOM70-UB
NdeI / SacII cleavage
TOM70-UB fragment
plasmid pTA-p53
SacII /XhoI cleavage
P53 fragment
plasmid pET11c
NdeI/SalI cleavage
AmpR
T7 ter
pET11c
T7
AmpR
T7 ter
pET11c-TOM70-UB-p53
p53
UB
T7
TOM70

FIG. 5

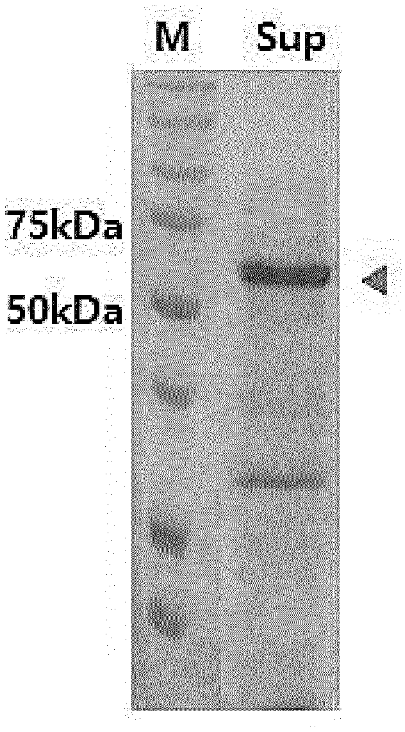
M
Sup
75kDa
50kDa

FIG. 6

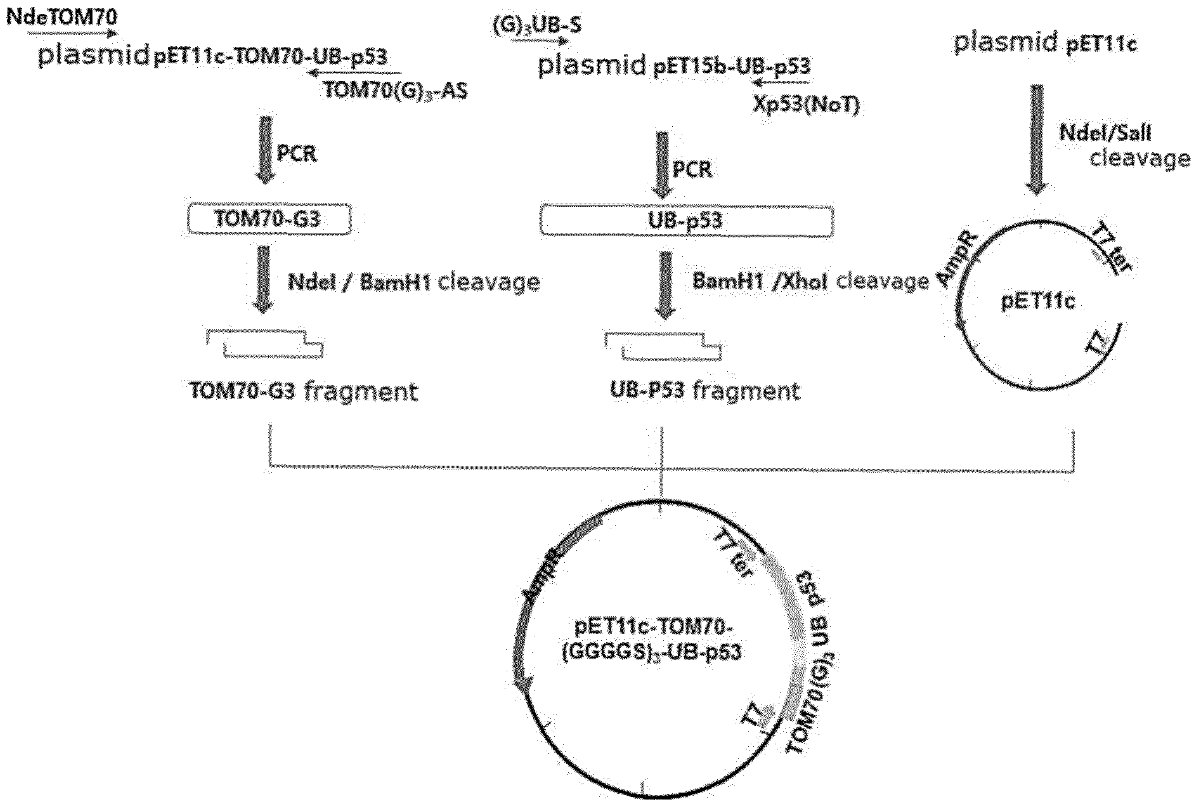
NdeTOM70
plasmid pET11c-TOM70-UB-p53
TOM70(G)3-AS
PCR
TOM70-G3
NdeI / BamH1 cleavage
TOM70-G3 fragment
(G)3UB-S
plasmid pET15b-UB-p53
Xp53(NoT)
PCR
UB-p53
BamH1 /XhoI cleavage
UB-P53 fragment
plasmid pET11c
NdeI/SalI cleavage
AmpR
T7 ter
pET11c
T7
pET11c-TOM70-(GGGGS)3-UB-p53
TOM70(G)3
UB
p53

FIG. 7

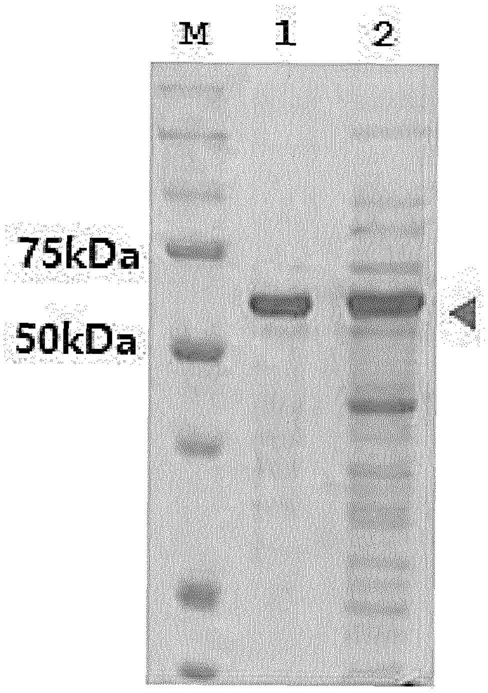
M
1
2
75kDa
50kDa

FIG. 8

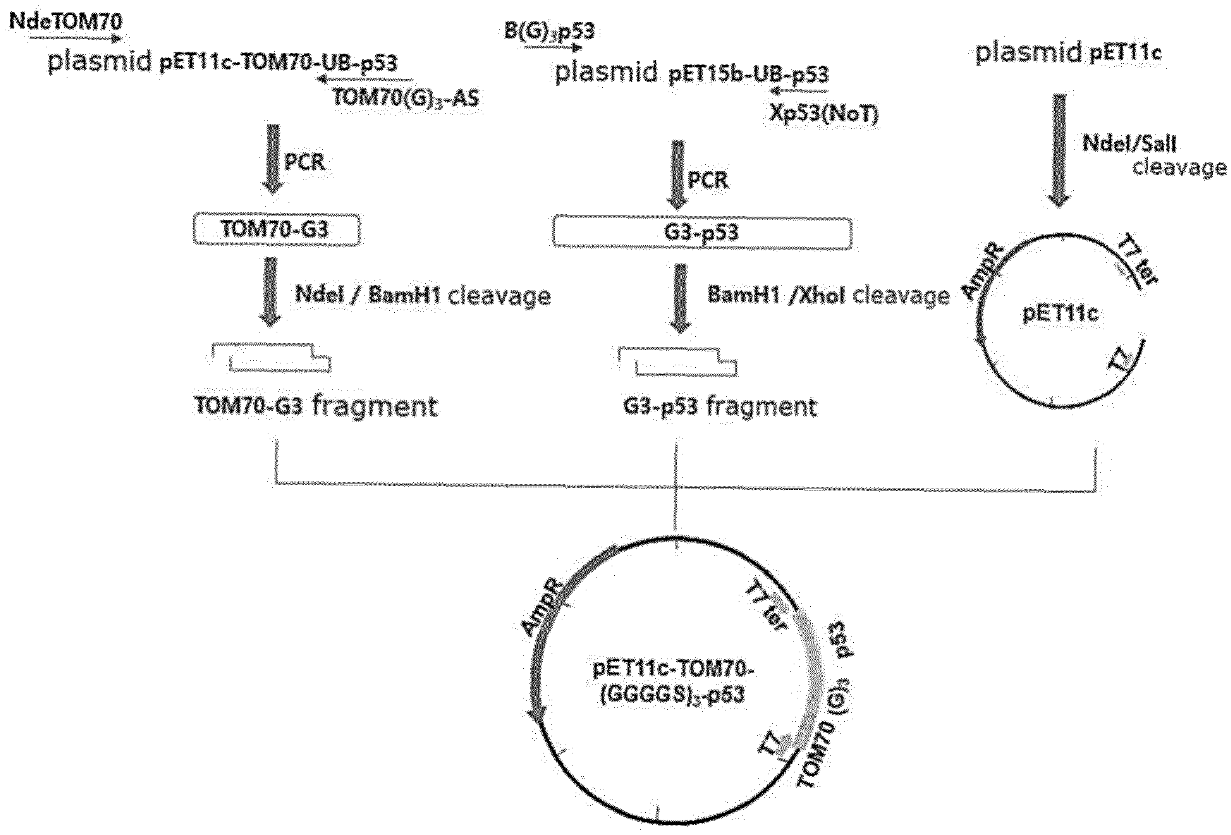
NdeTOM70
plasmid pET11c-TOM70-UB-p53
TOM70(G)3-AS
PCR
TOM70-G3
NdeI / BamH1 cleavage
TOM70-G3 fragment
B(G)3p53
plasmid pET15b-UB-p53
Xp53(NoT)
PCR
G3-p53
BamH1 /XhoI cleavage
G3-p53 fragment
plasmid pET11c
NdeI/SalI cleavage
AmpR
T7 ter
pET11c
T7
pET11c-TOM70-(GGGGS)3-p53
p53
TOM70 (G)3

FIG. 9

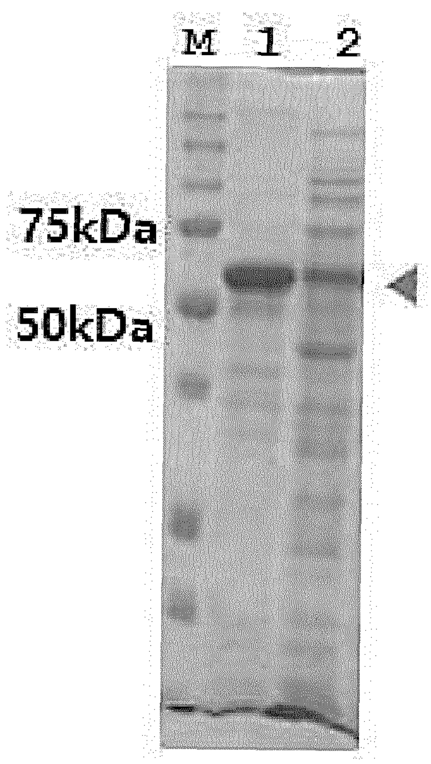
M
1
2
75kDa
50kDa

FIG. 10

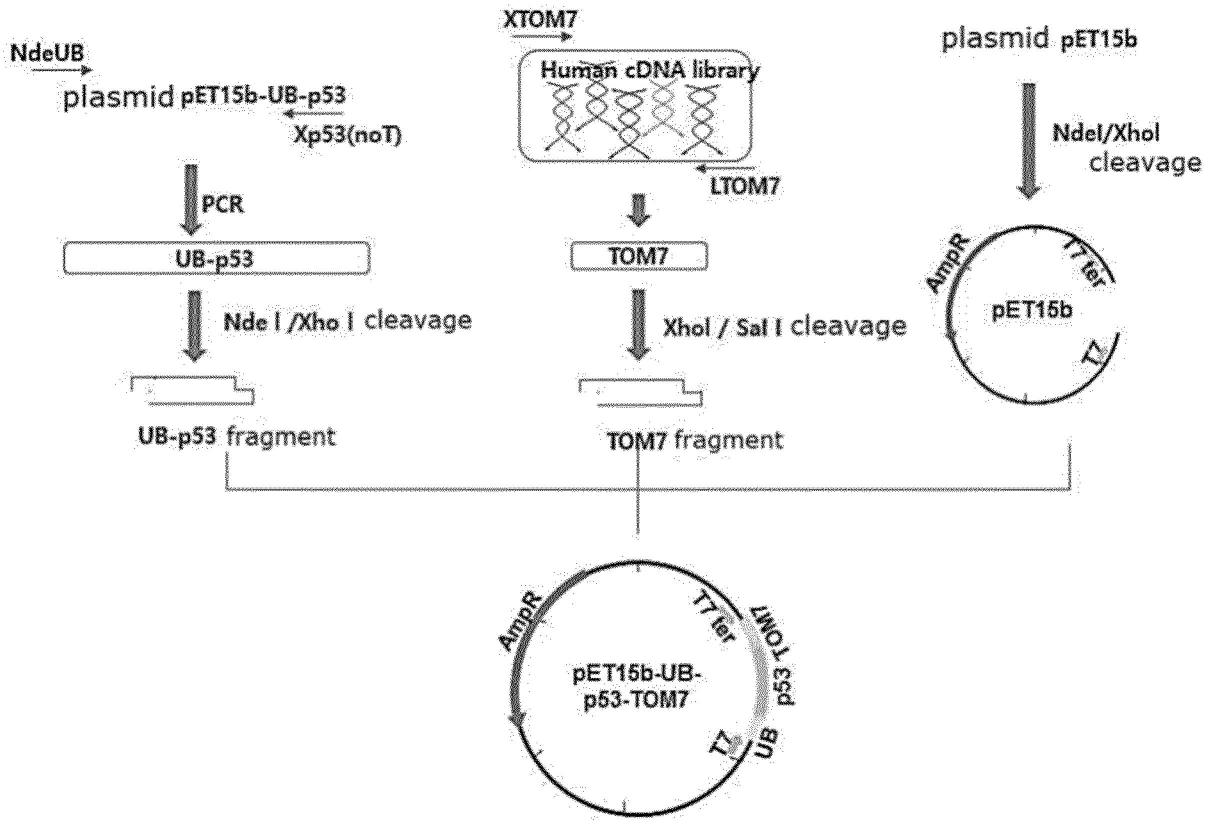
XTOM7
NdeUB
plasmid pET15b-UB-p53
Xp53(noT)
Human cDNA library
LTOM7
plasmid pET15b
NdeI/XhoI
cleavage
PCR
UB-p53
TOM7
AmpR
T7 ter
pET15b
T7
Nde I /Xho I cleavage
XhoI / Sal I cleavage
UB-p53 fragment
TOM7 fragment
AmpR
T7 ter
TOM7
p53
UB
T7
pET15b-UB-p53-TOM7

FIG. 11

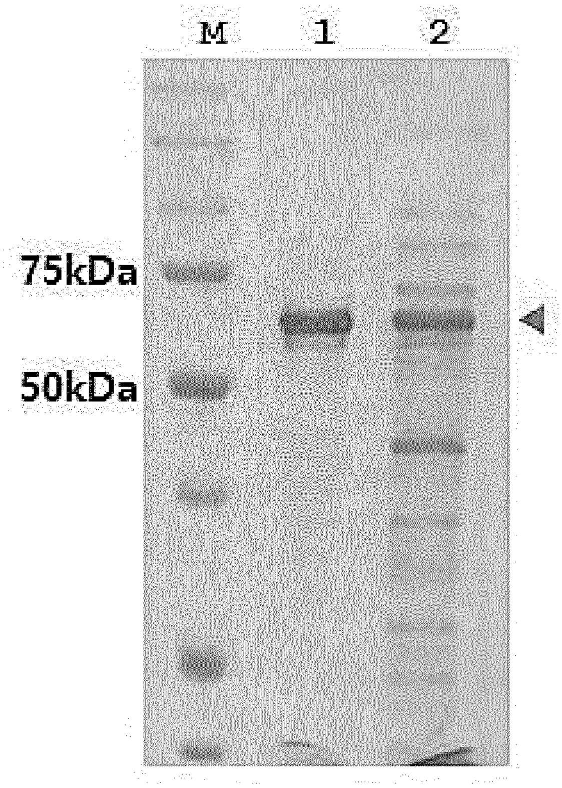
M
1
2
75kDa
50kDa

FIG. 12

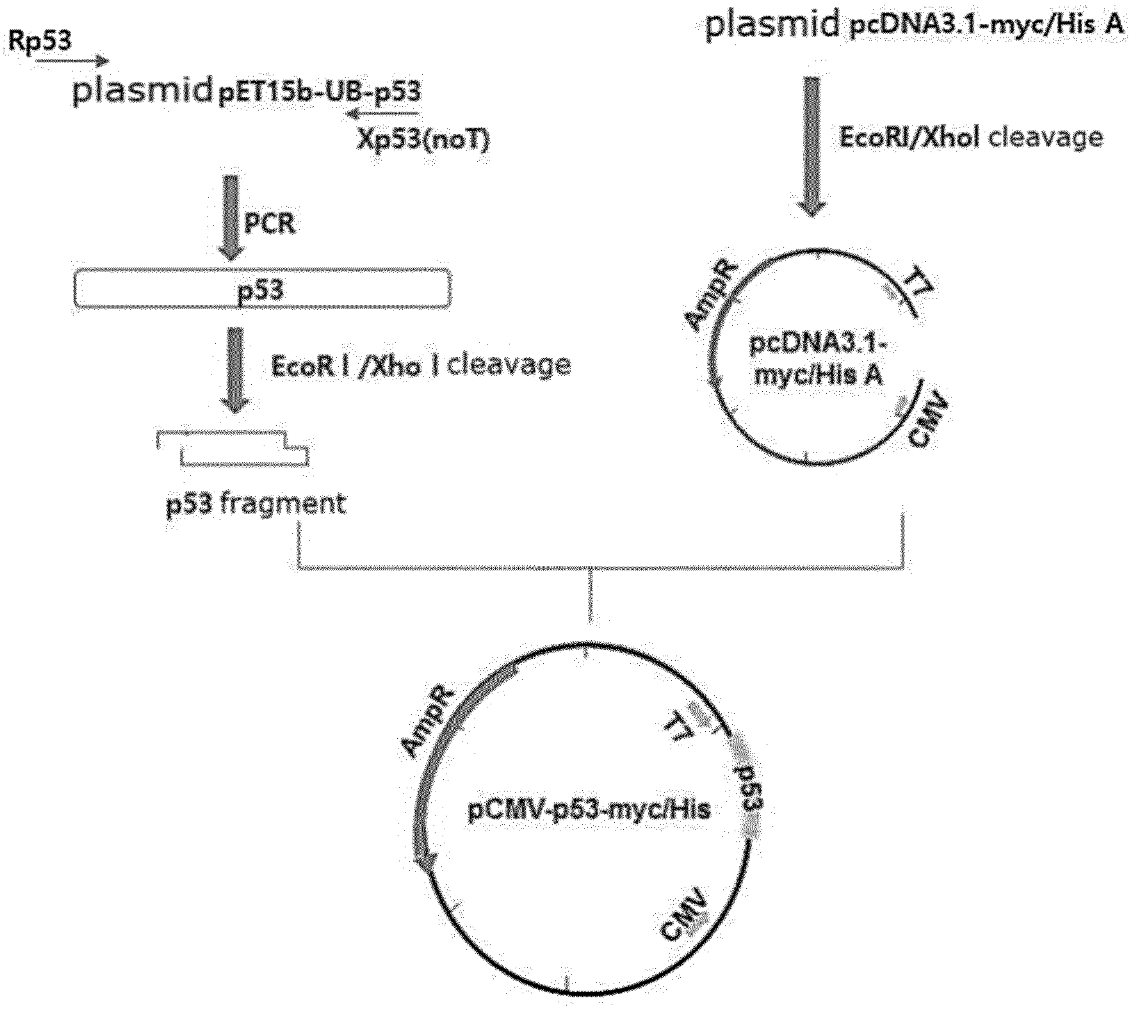
Rp53
plasmid pET15b-UB-p53
Xp53(noT)
PCR
p53
EcoR I /Xho I cleavage
p53 fragment
plasmid pcDNA3.1-myc/His A
EcoRI/XhoI cleavage
AmpR
T7
pcDNA3.1-myc/His A
CMV
AmpR
T7
p53
pCMV-p53-myc/His
CMV

FIG. 13

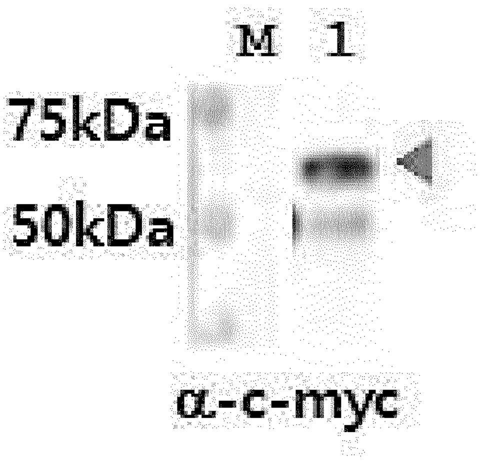
M
1
75kDa
50kDa
α-c-myc

FIG. 14

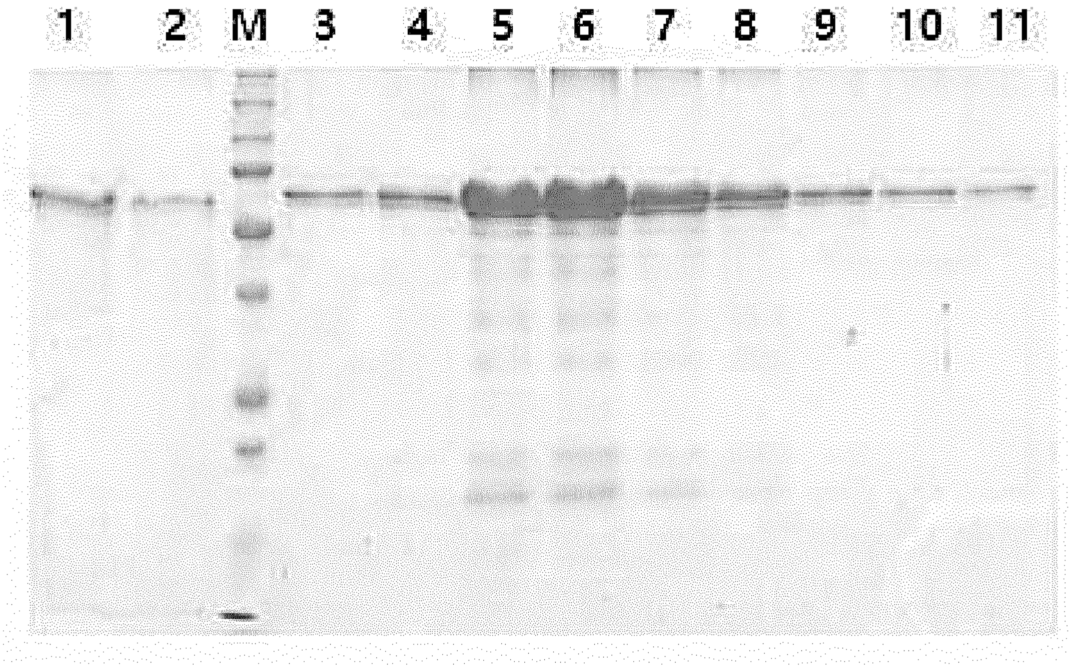


FIG. 15

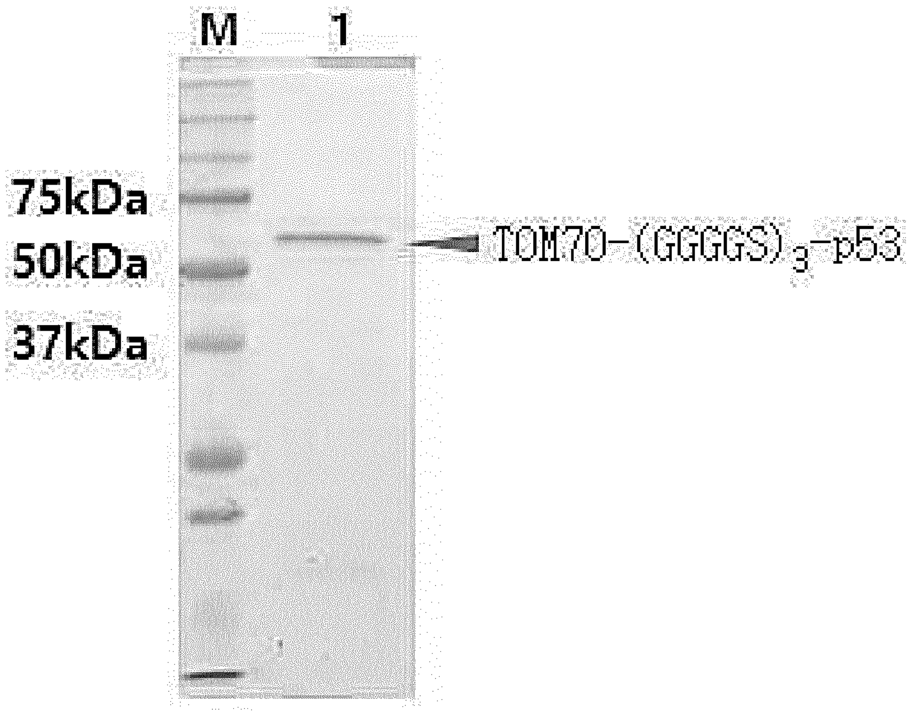

FIG. 16

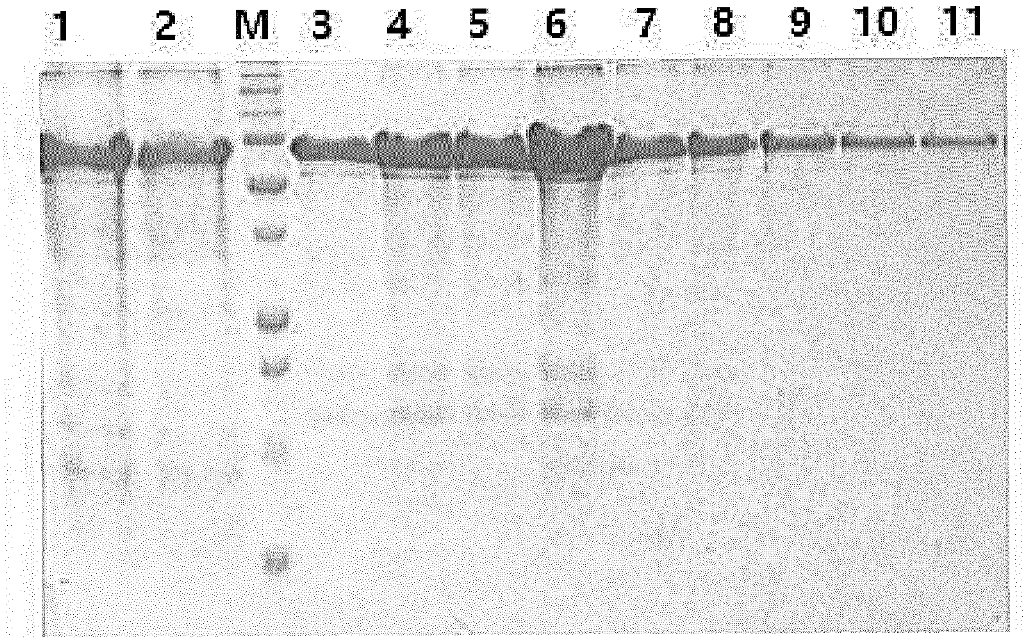


FIG. 17

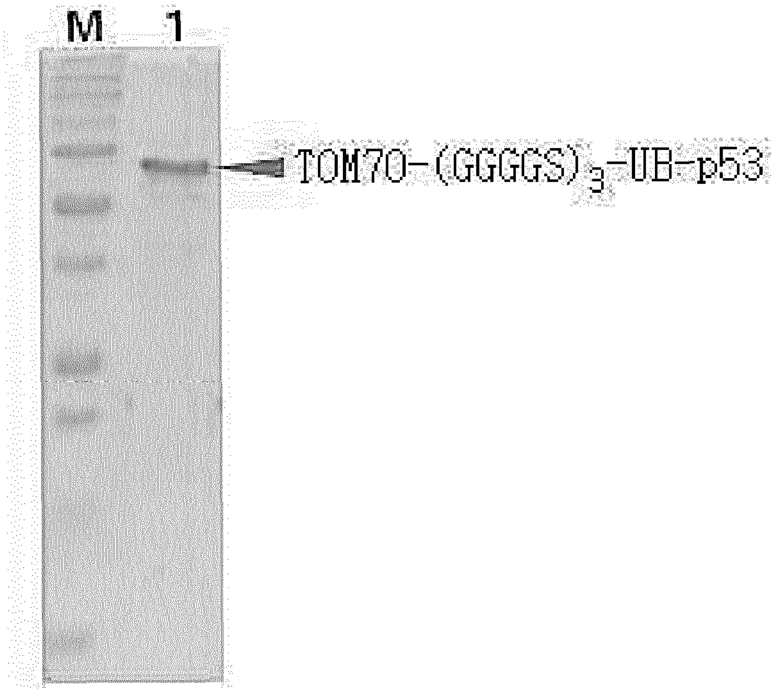

FIG. 18

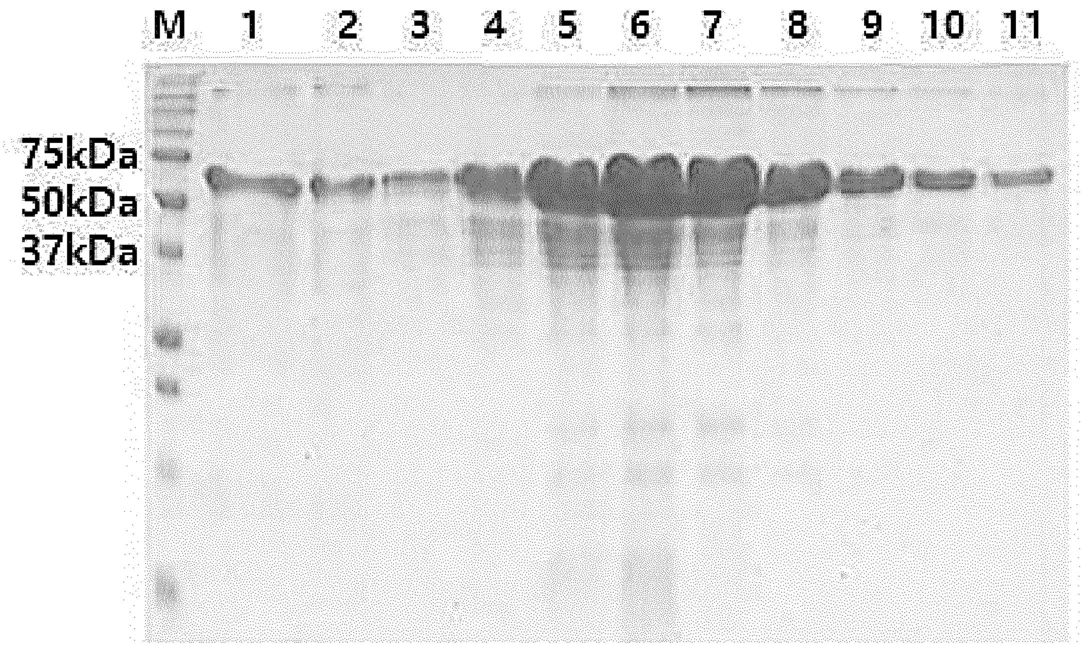
M
1
2
3
4
5
6
7
8
9
10
11
75kDa
50kDa
37kDa

FIG. 19

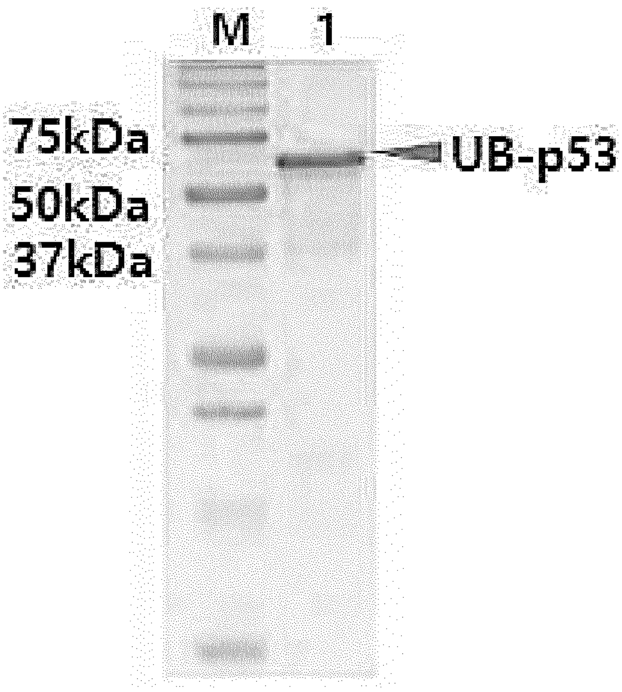
M
1
75kDa
50kDa
37kDa
UB-p53

FIG. 20

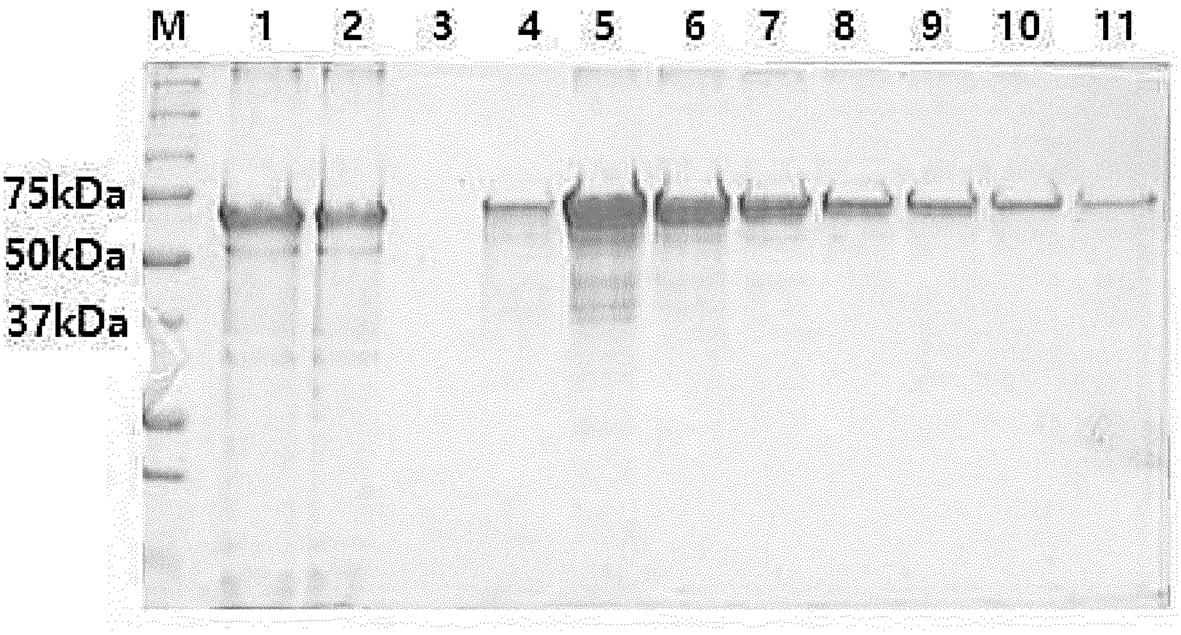
M
1
2
3
4
5
6
7
8
9
10
11
75kDa
50kDa
37kDa

FIG. 21

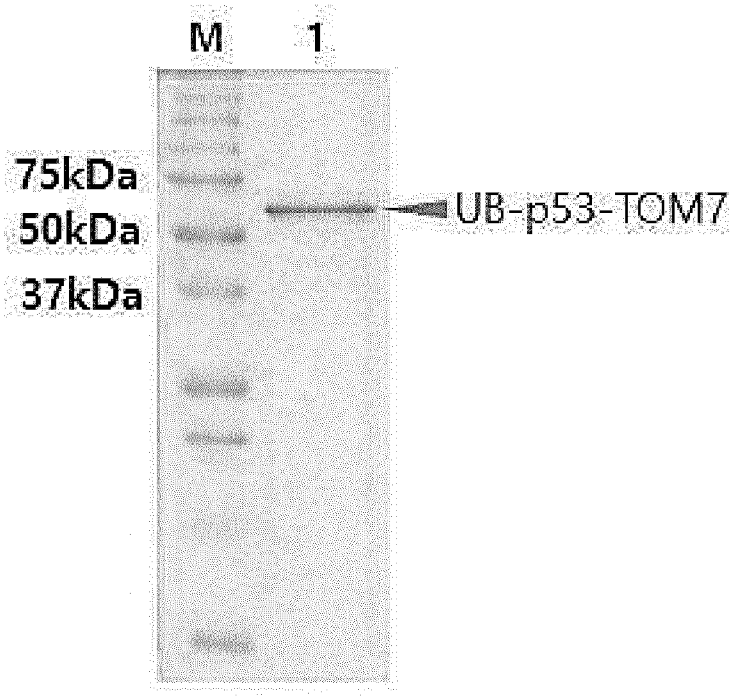
M
1
75kDa
50kDa
37kDa
UB-p53-TOM7

FIG. 22

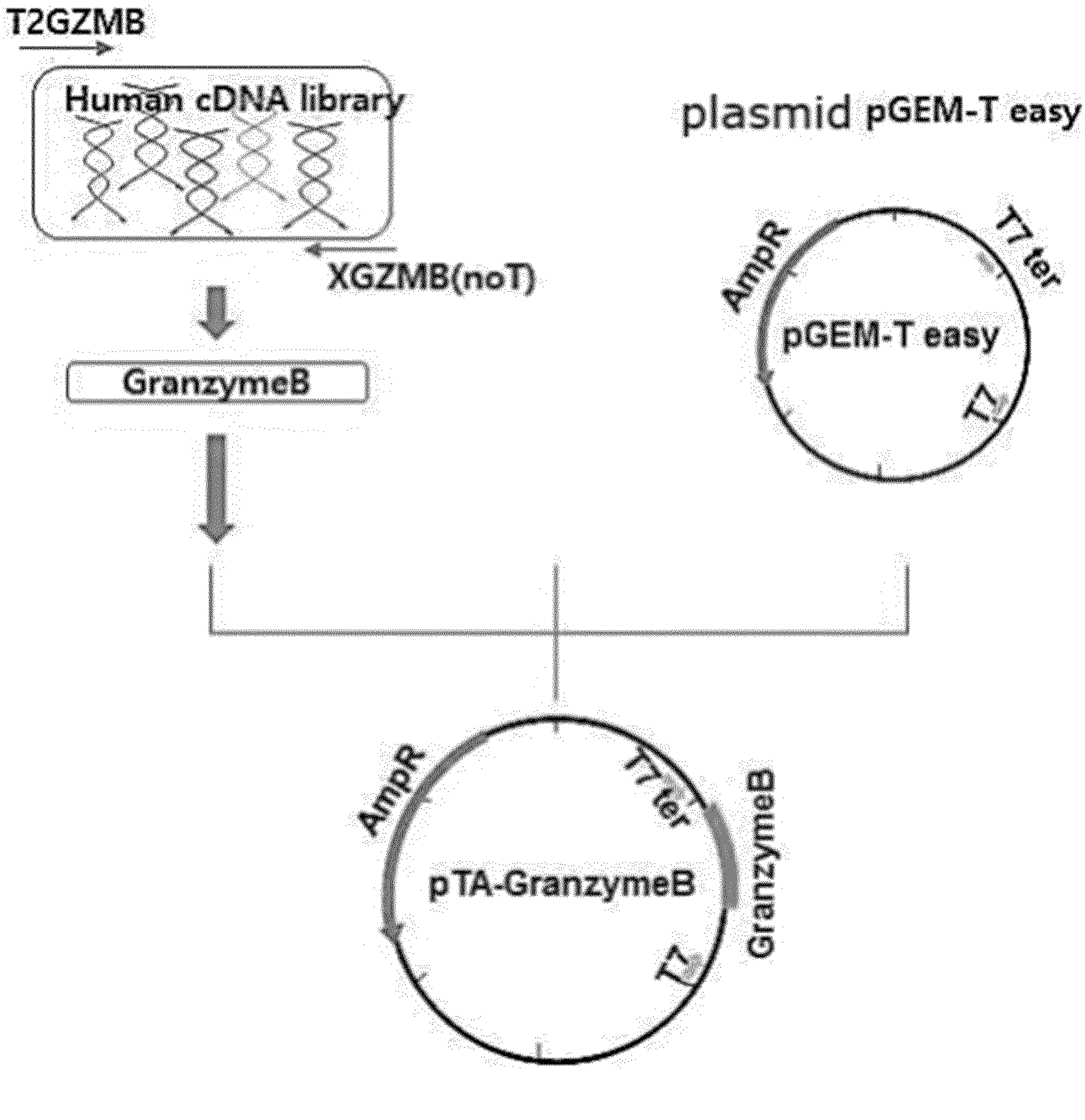
T2GZMB
Human cDNA library
XGZMB(noT)
GranzymeB
plasmid pGEM-T easy
AmpR
T7 ter
pGEM-T easy
T7
AmpR
T7 ter
pTA-GranzymeB
GranzymeB
T7

FIG. 23

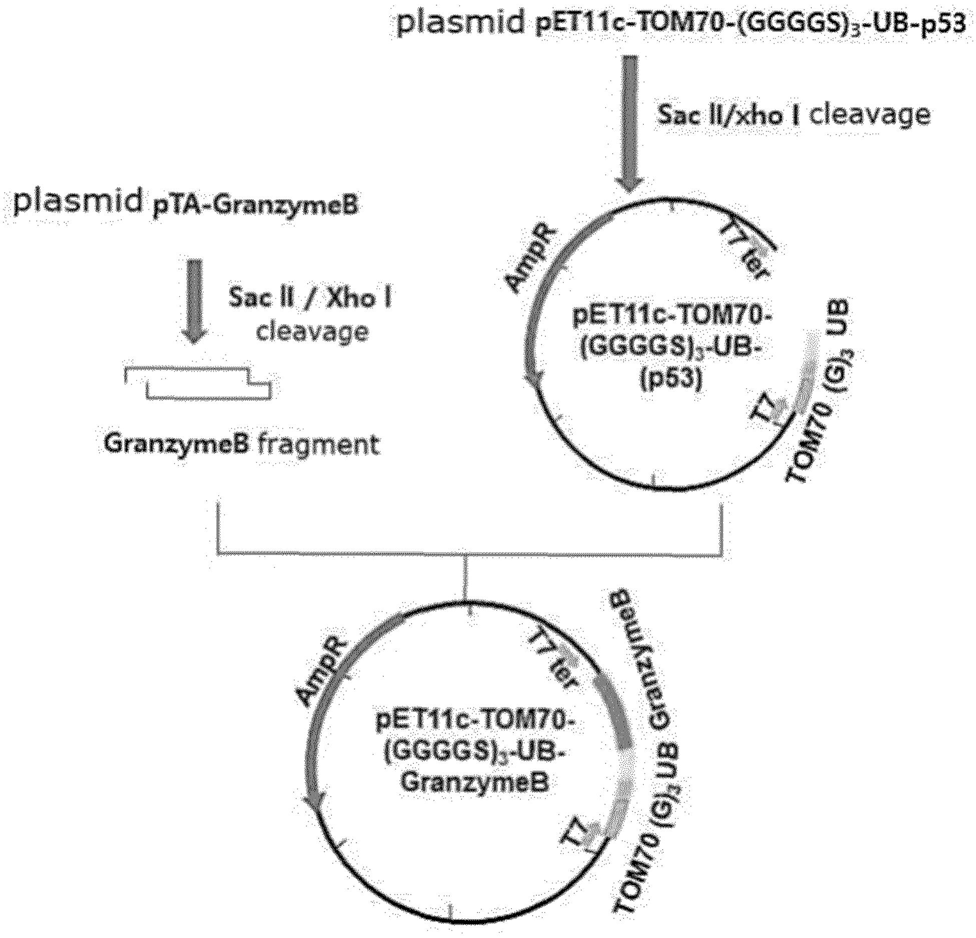
plasmid pET11c-TOM70-(GGGGS)3-UB-p53
Sac II/xho I cleavage
plasmid pTA-GranzymeB
Sac II / Xho I cleavage
GranzymeB fragment
AmpR
T7 ter
pET11c-TOM70-(GGGGS)3-UB-(p53)
UB
(G)3
T7
TOM70
AmpR
T7 ter
GranzymeB
pET11c-TOM70-(GGGGS)3-UB-GranzymeB
UB
(G)3
T7
TOM70

FIG. 24

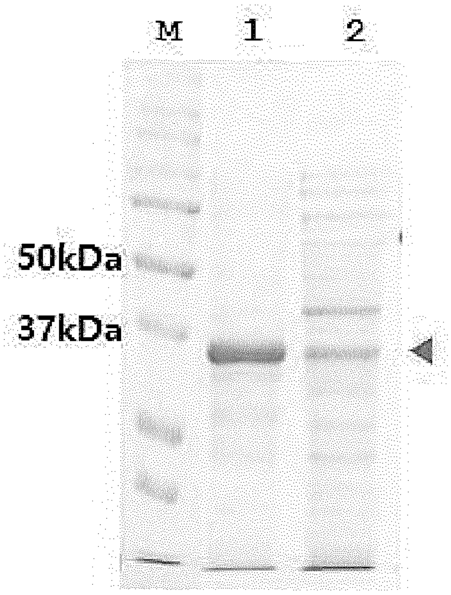
M
1
2
50kDa
37kDa

FIG. 25

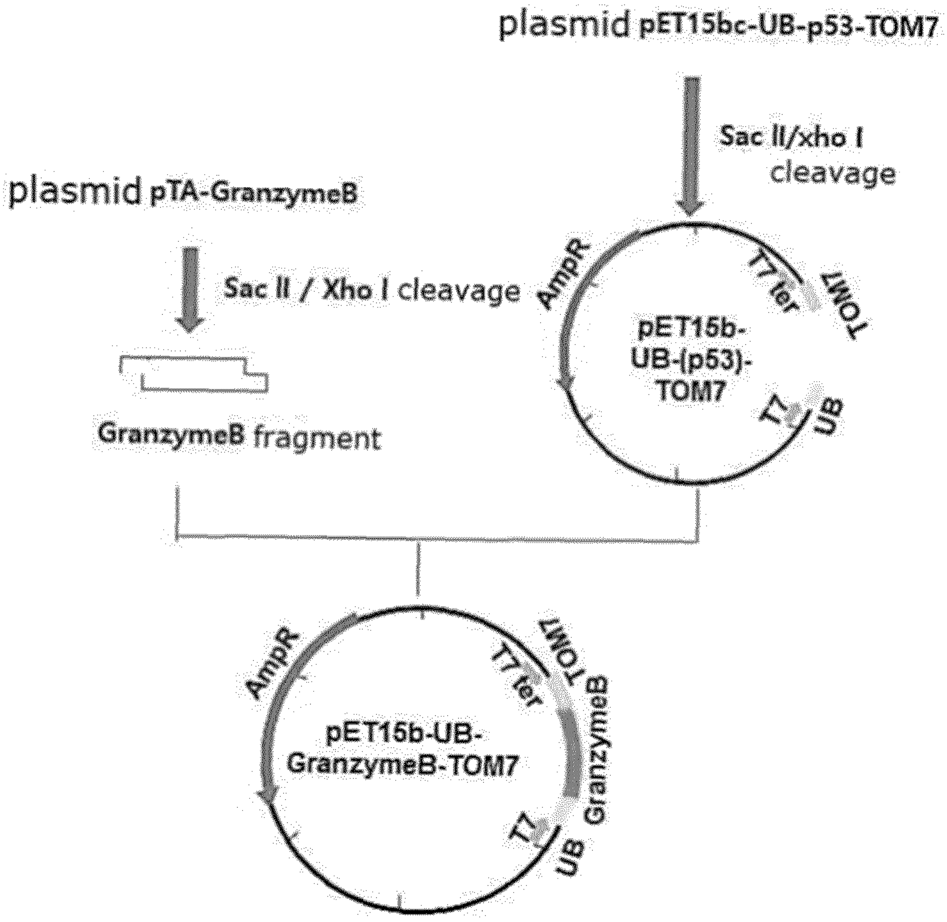
plasmid pET15bc-UB-p53-TOM7
Sac II/xho I
cleavage
plasmid pTA-GranzymeB
Sac II / Xho I cleavage
GranzymeB fragment
AmpR
T7 ter
TOM7
pET15b-
UB-(p53)-
TOM7
T7
UB
AmpR
T7 ter
TOM7
GranzymeB
pET15b-UB-
GranzymeB-TOM7
T7
UB

FIG. 26

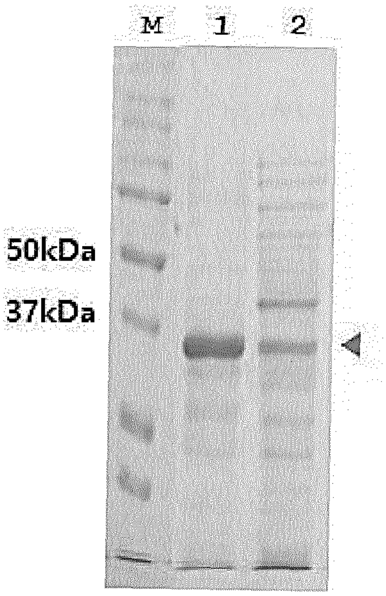
M
1
2
50kDa
37kDa

FIG. 27

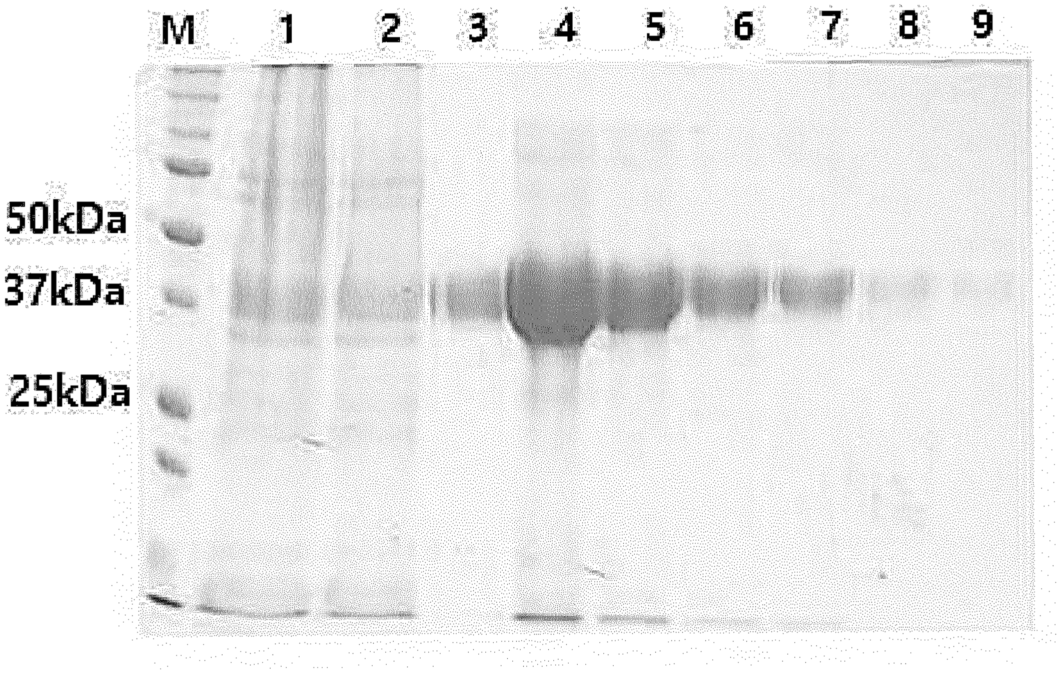
M
1
2
3
4
5
6
7
8
9
50kDa
37kDa
25kDa

FIG. 28

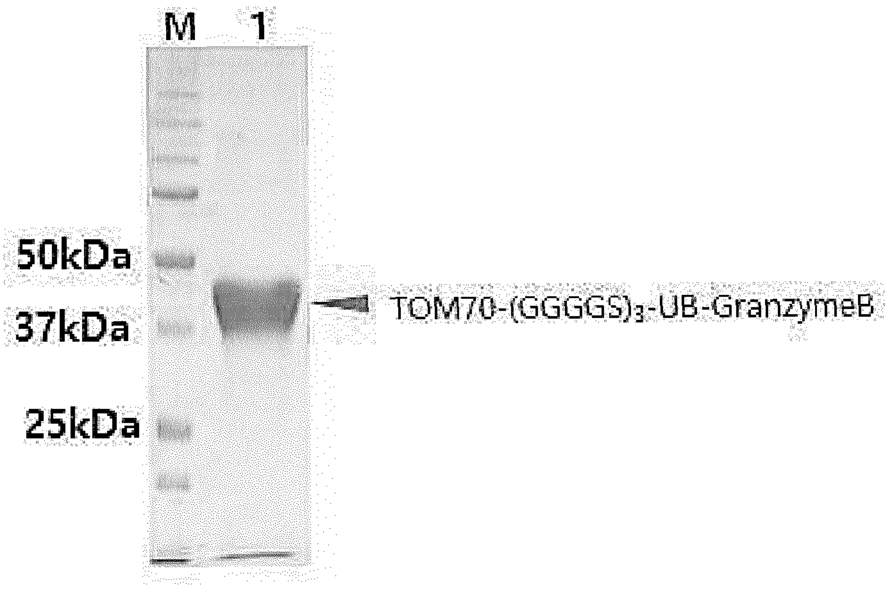
M
1
50kDa
37kDa
25kDa
TOM70-(GGGGS)3-UB-GranzymeB

FIG. 29

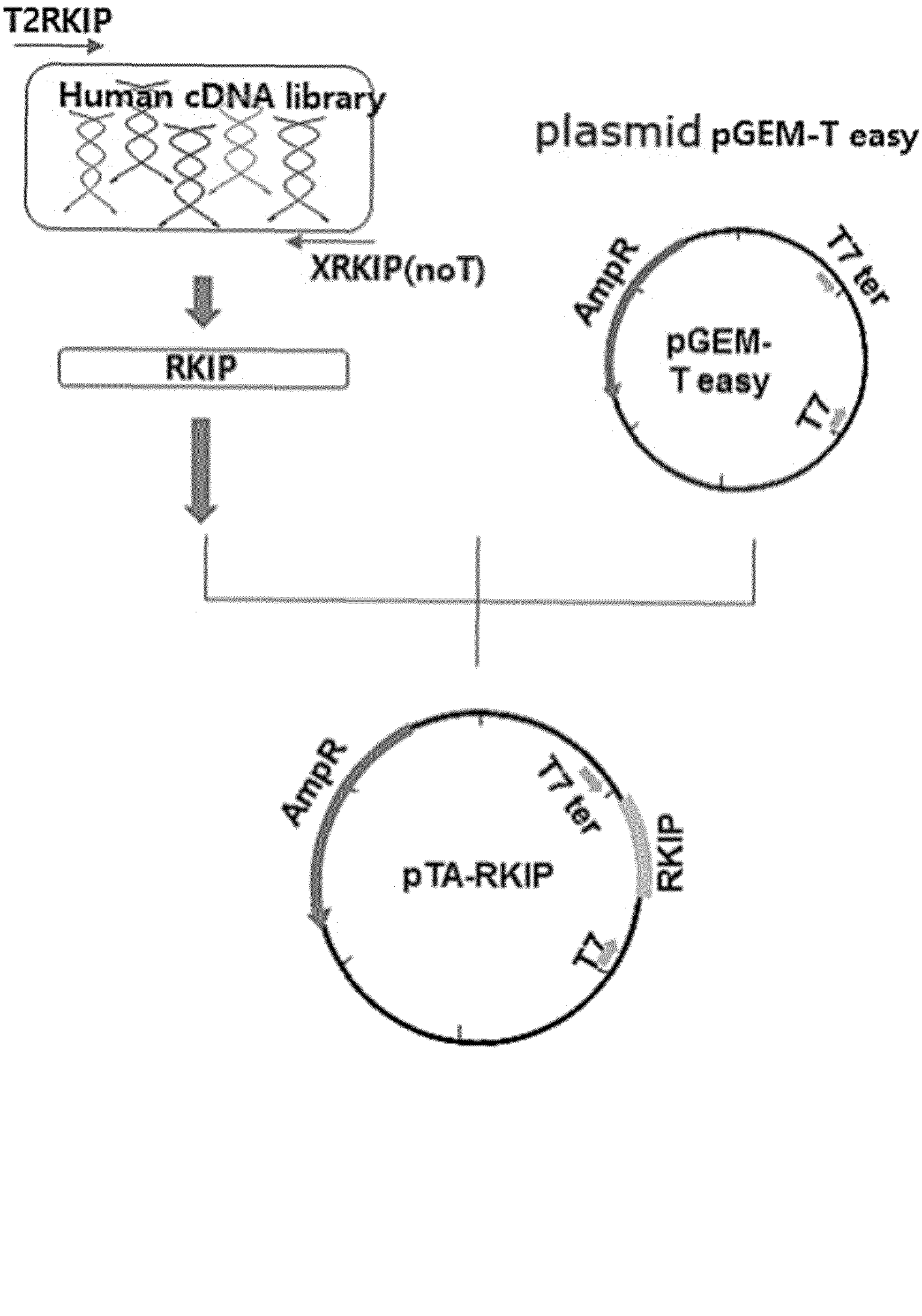
T2RKIP
Human cDNA library
XRKIP(noT)
RKIP
plasmid pGEM-T easy
AmpR
T7 ter
pGEM-
T easy
T7
AmpR
T7 ter
RKIP
pTA-RKIP
T7

FIG. 30

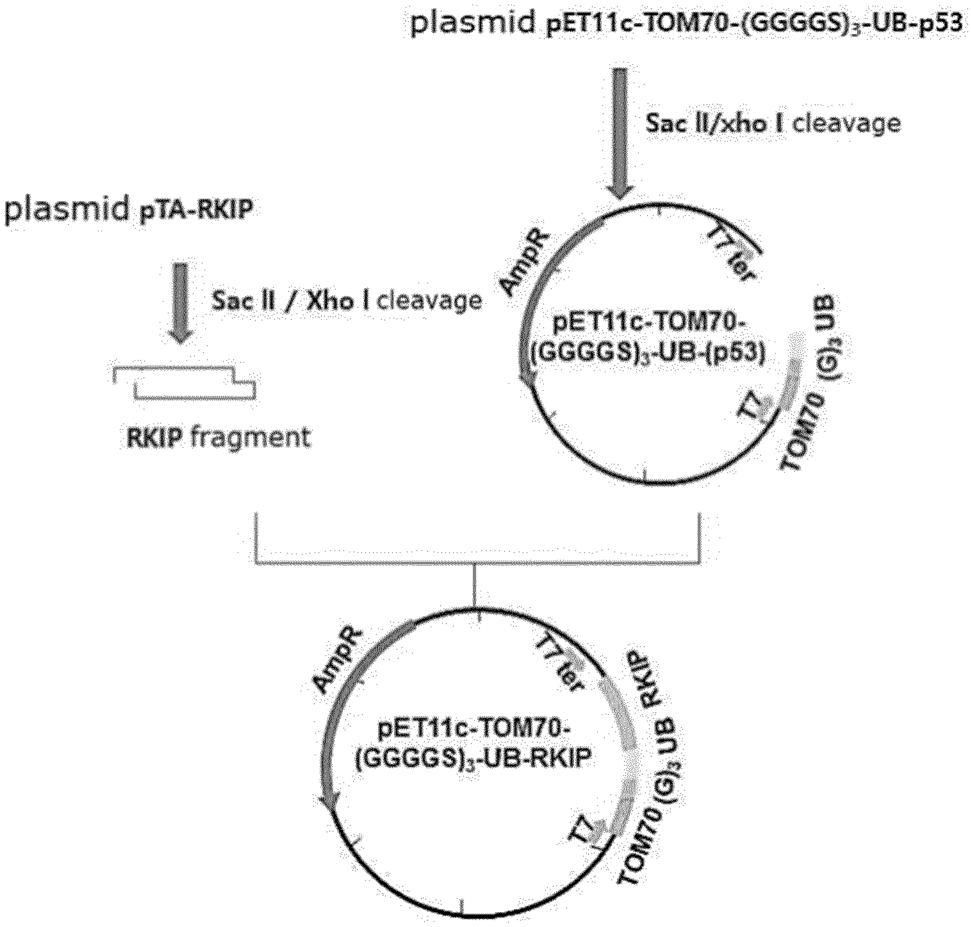
plasmid pET11c-TOM70-(GGGGS)3-UB-p53
Sac II/xho I cleavage
plasmid pTA-RKIP
Sac II / Xho I cleavage
RKIP fragment
AmpR
T7 ter
pET11c-TOM70-(GGGGS)3-UB-(p53)
UB
(G)3
T7
TOM70
AmpR
T7 ter
RKIP
pET11c-TOM70-(GGGGS)3-UB-RKIP
UB
(G)3
T7
TOM70

FIG. 31

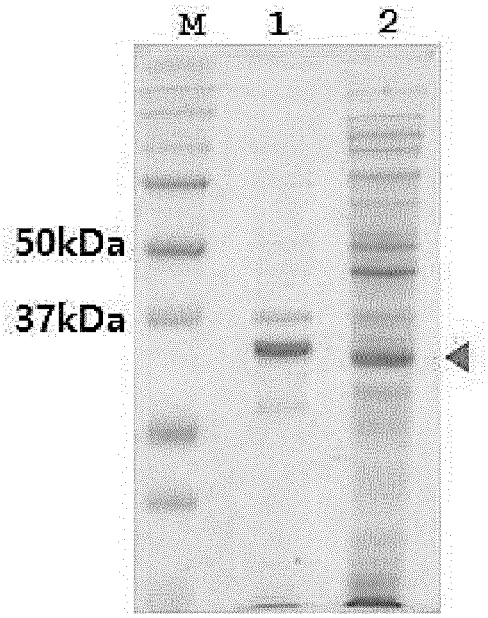
M
1
2
50kDa
37kDa

FIG. 32

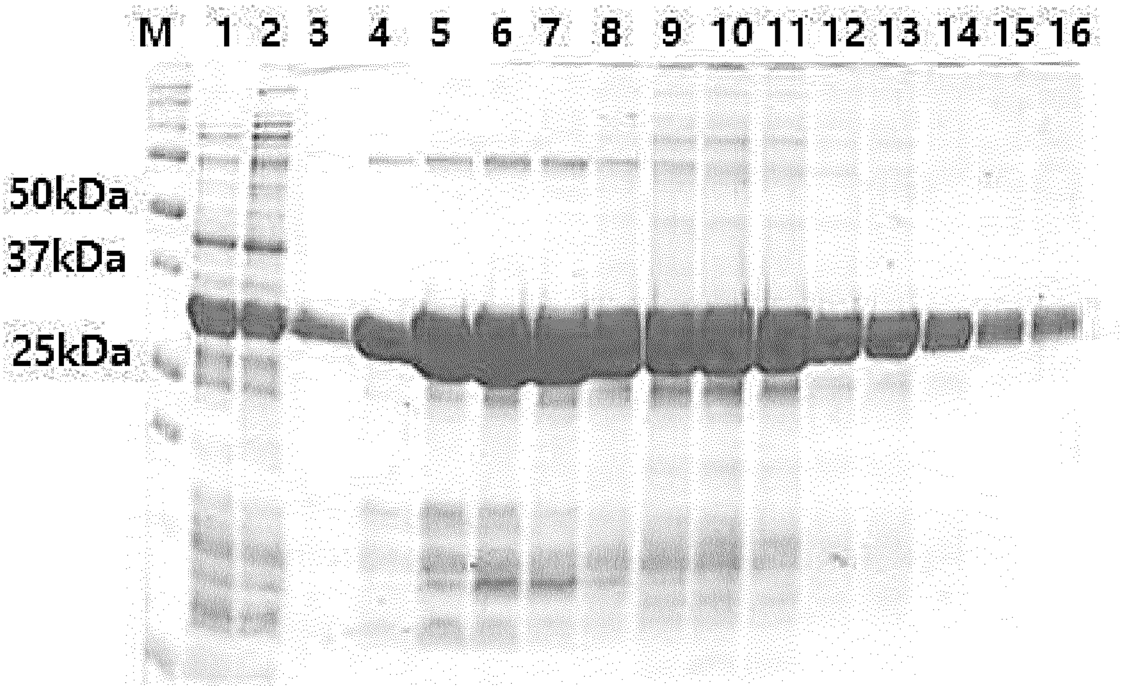
M 1 2 3 4 5 6 7 8 9 10 11 12 13 14 15 16
50kDa
37kDa
25kDa

FIG. 33

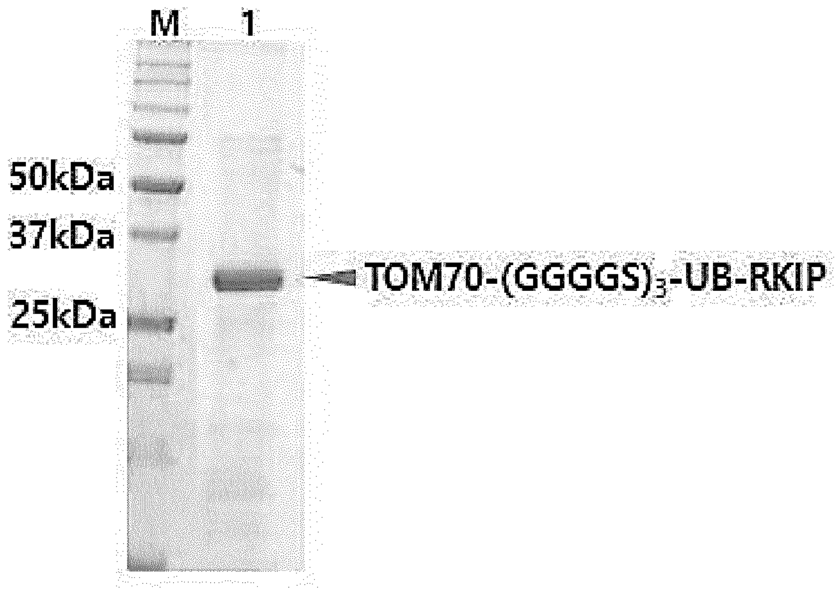
M 1
50kDa
37kDa
25kDa
TOM70-(GGGGS)3-UB-RKIP

**FIG. 34**

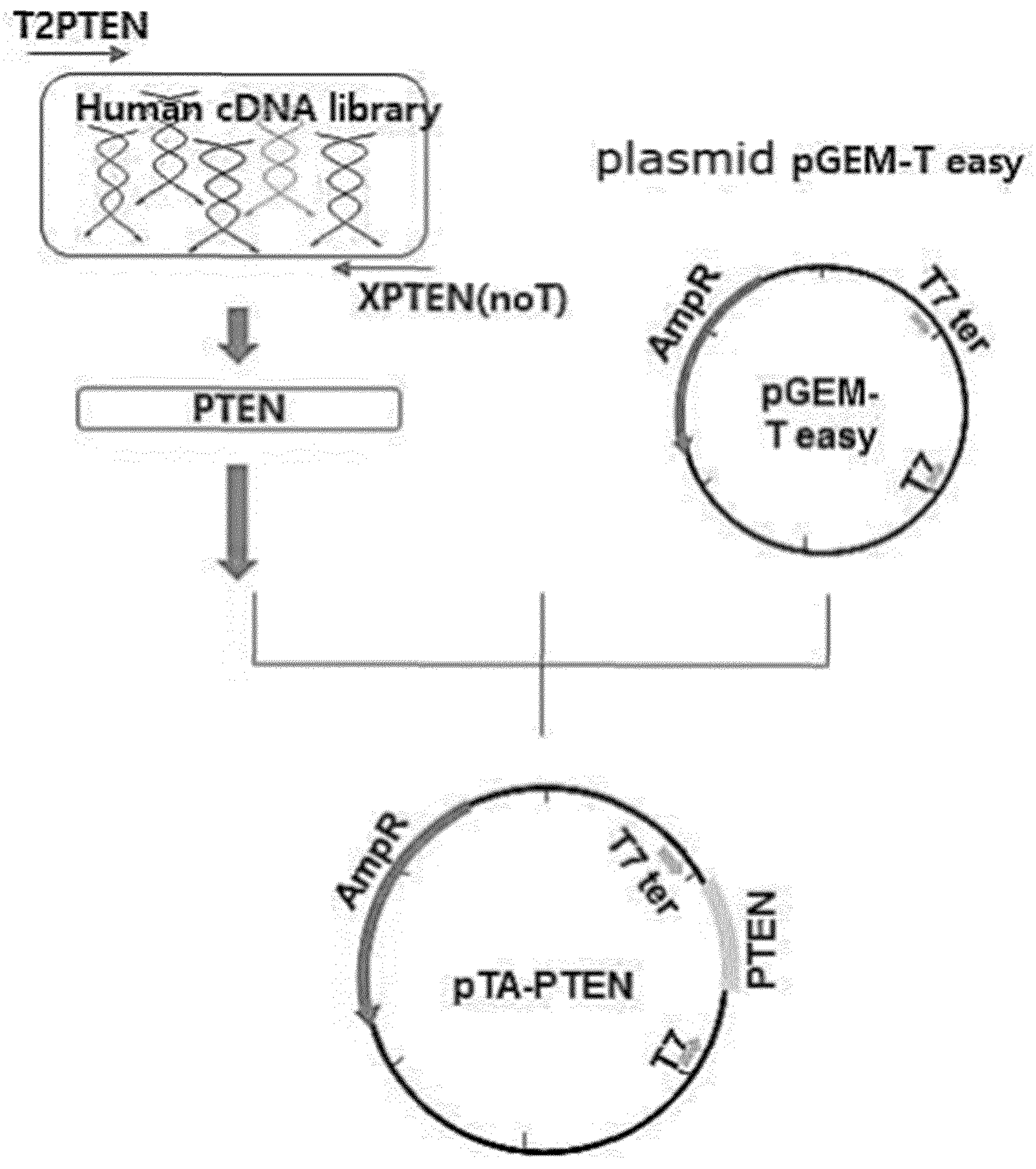
T2PTEN
Human cDNA library
XPTEN(noT)
PTEN
plasmid pGEM-T easy
AmpR
T7 ter
pGEM-
T easy
T7
AmpR
T7 ter
PTEN
pTA-PTEN
T7

FIG. 35

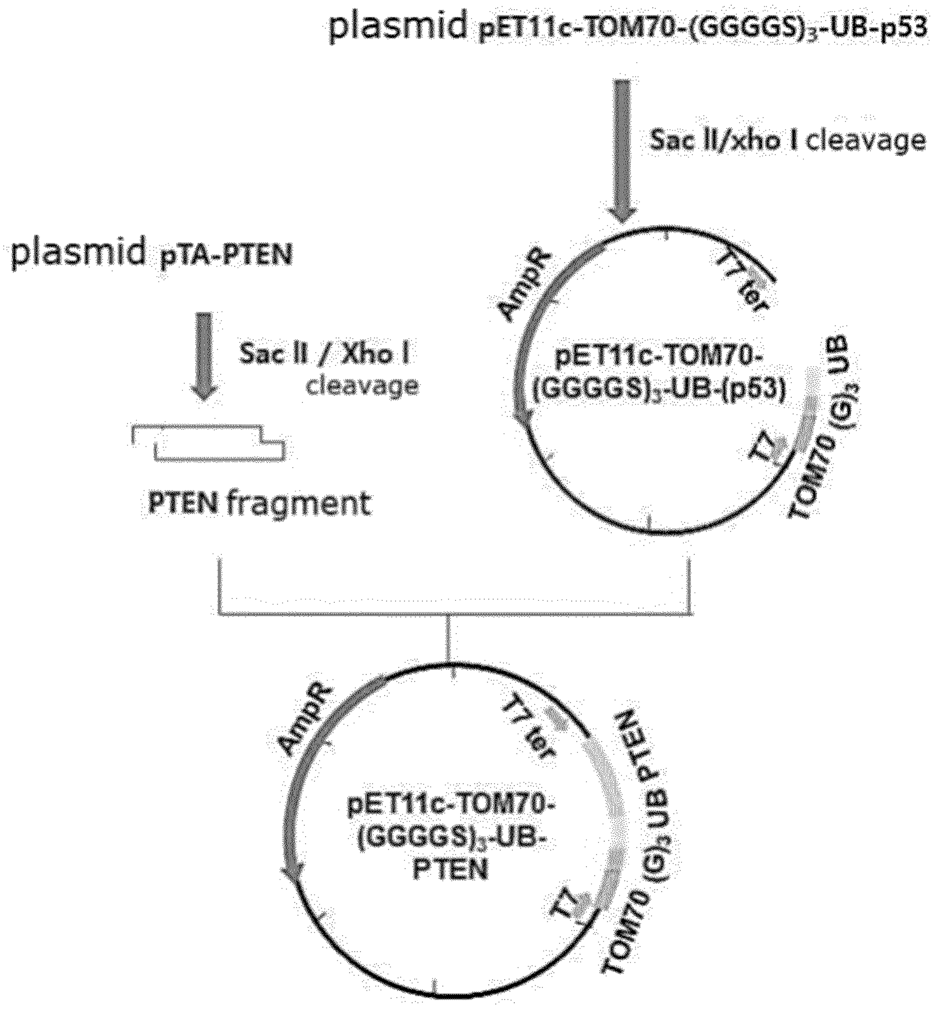
plasmid pET11c-TOM70-(GGGGS)3-UB-p53
Sac II/xho I cleavage
plasmid pTA-PTEN
Sac II / Xho I cleavage
PTEN fragment
AmpR
T7 ter
pET11c-TOM70-(GGGGS)3-UB-(p53)
UB
(G)3
T7
TOM70
pET11c-TOM70-(GGGGS)3-UB-PTEN
PTEN

FIG. 36

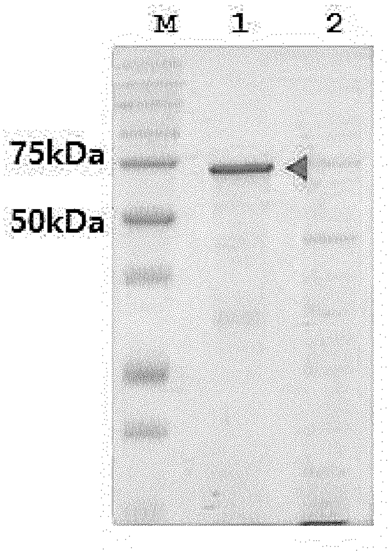
M
1
2
75kDa
50kDa

FIG. 37

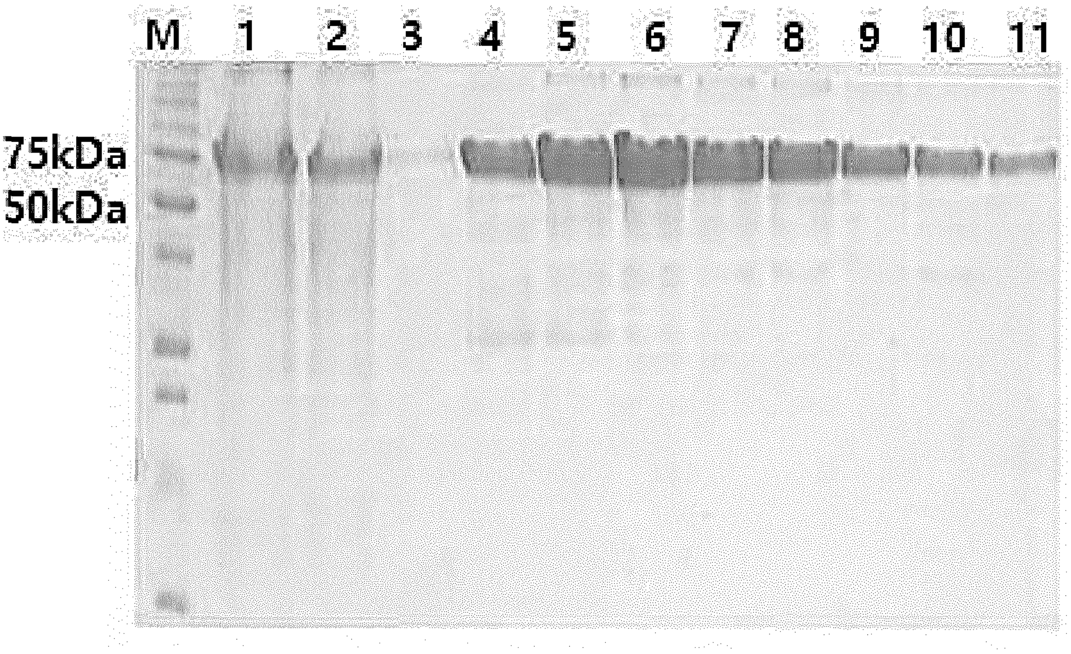
M
1
2
3
4
5
6
7
8
9
10
11
75kDa
50kDa

FIG. 38

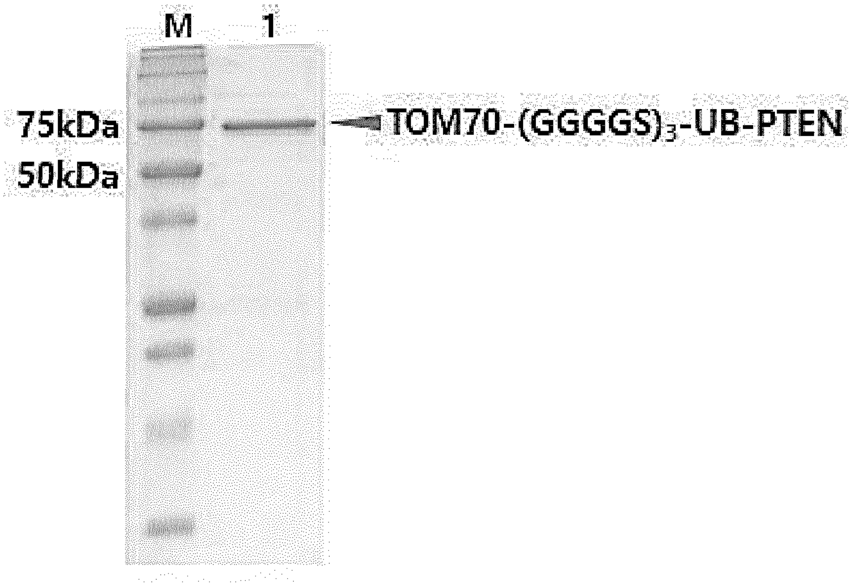
M
1
75kDa
50kDa
TOM70-(GGGGS)$_3$-UB-PTEN

FIG. 39

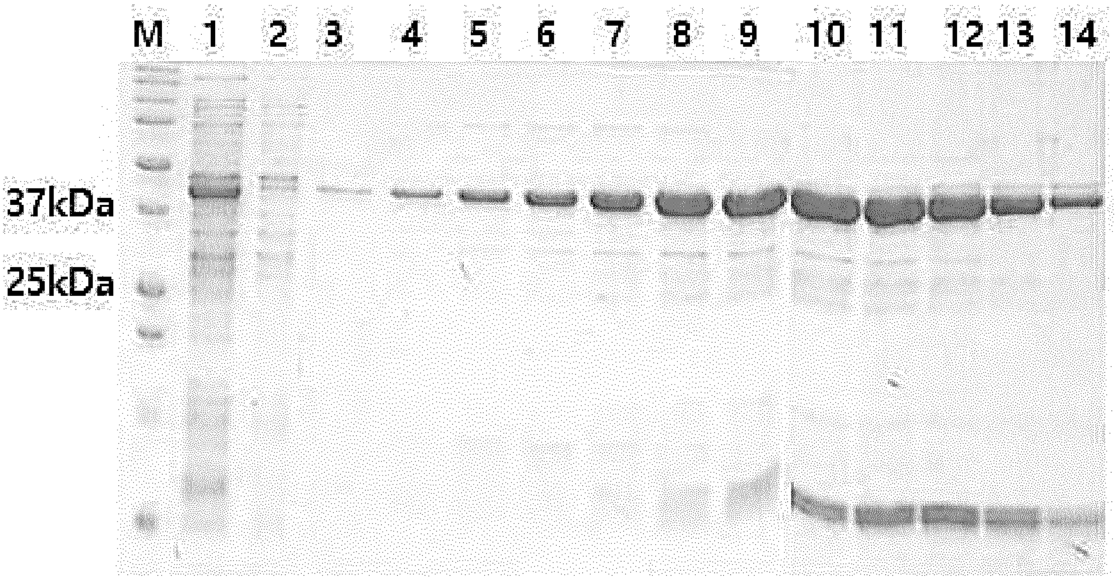
M
1
2
3
4
5
6
7
8
9
10
11
12
13
14
37kDa
25kDa

FIG. 40

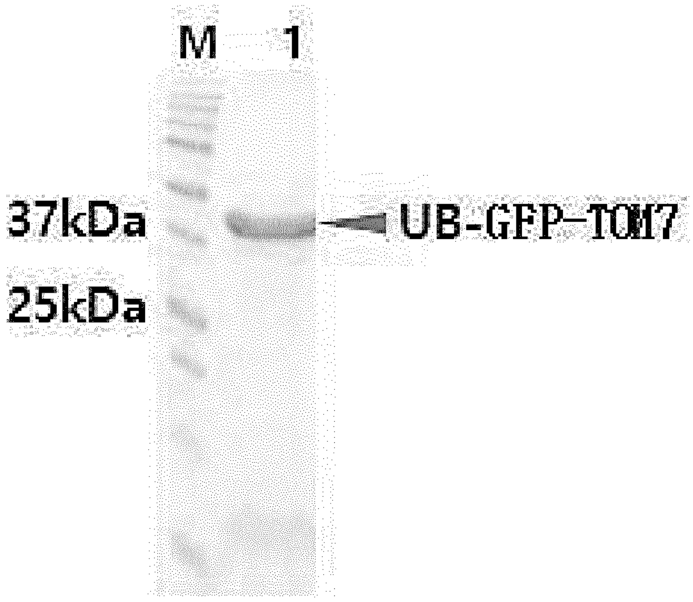
M
1
37kDa
25kDa
UB-GFP-TOM7

FIG. 41

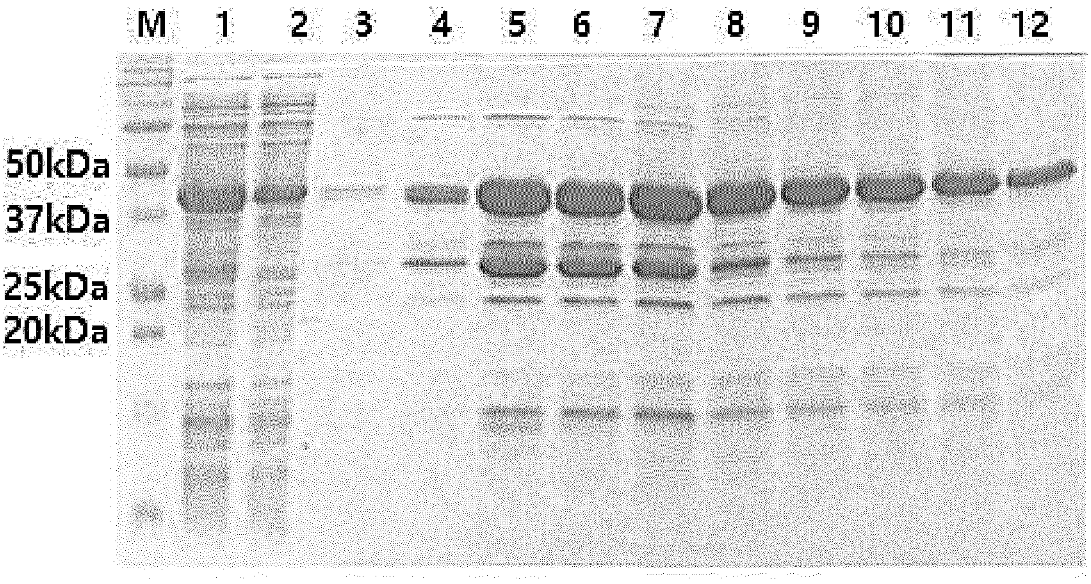
M
1
2
3
4
5
6
7
8
9
10
11
12
50kDa
37kDa
25kDa
20kDa

FIG. 42

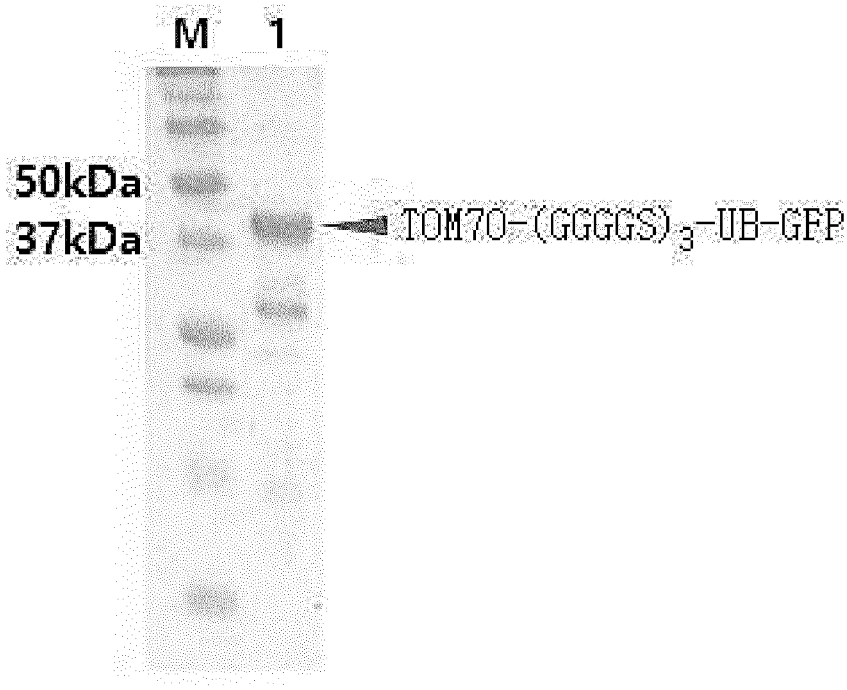
M
1
50kDa
37kDa
TOM70-(GGGGS)3-UB-GFP

FIG. 43

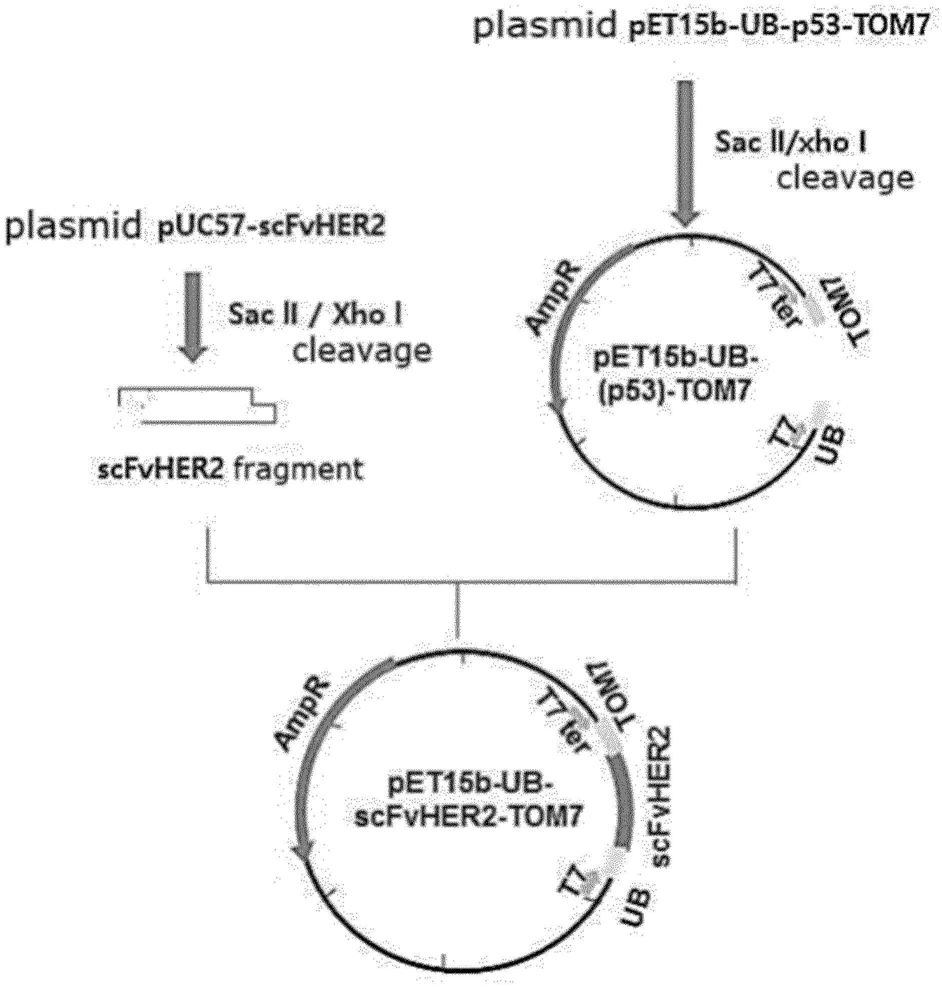
plasmid pET15b-UB-p53-TOM7
Sac II/xho I
cleavage
plasmid pUC57-scFvHER2
Sac II / Xho I
cleavage
scFvHER2 fragment
AmpR
T7 ter
TOM7
pET15b-UB-
(p53)-TOM7
T7
UB
AmpR
T7 ter
TOM7
pET15b-UB-
scFvHER2-TOM7
scFvHER2
T7
UB

FIG. 44

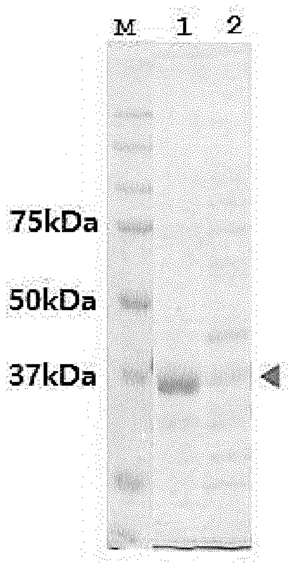
M
1
2
75kDa
50kDa
37kDa

FIG. 45

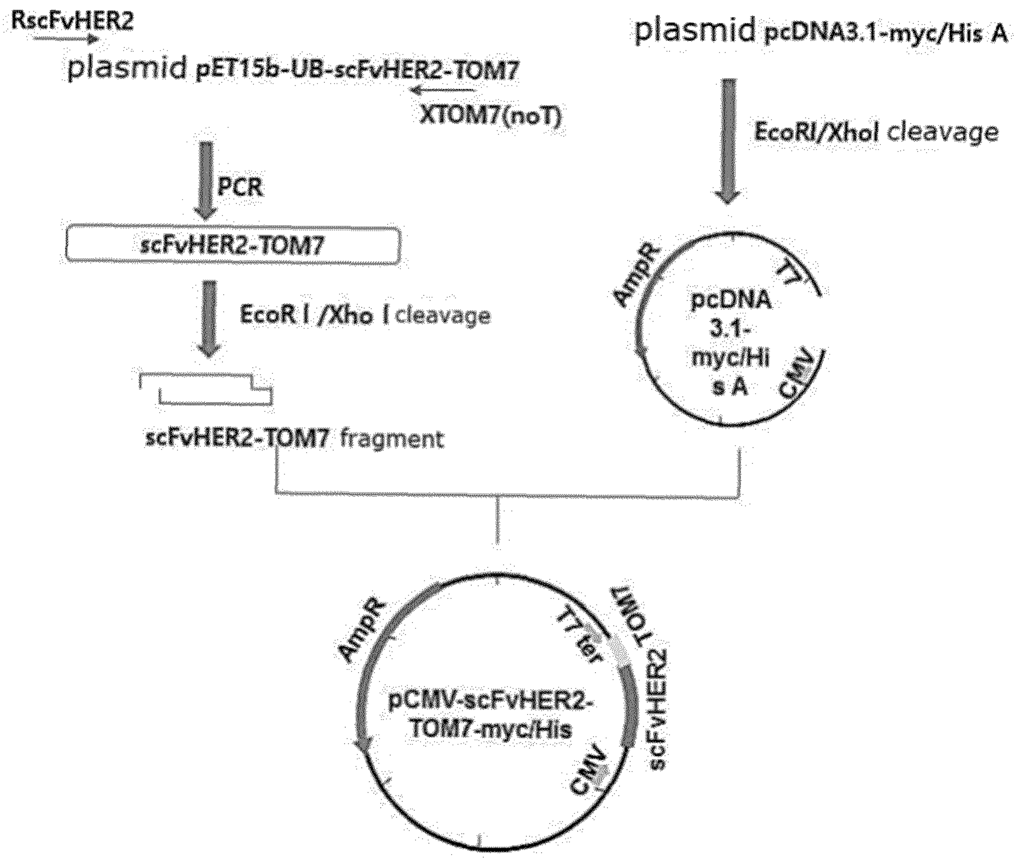
RscFvHER2
plasmid pET15b-UB-scFvHER2-TOM7
XTOM7(noT)
PCR
scFvHER2-TOM7
EcoR I /Xho I cleavage
scFvHER2-TOM7 fragment
plasmid pcDNA3.1-myc/His A
EcoRI/XhoI cleavage
AmpR
T7
pcDNA 3.1-myc/His A
CMV
AmpR
T7 ter
TOM7
scFvHER2
CMV
pCMV-scFvHER2-TOM7-myc/His

FIG. 46

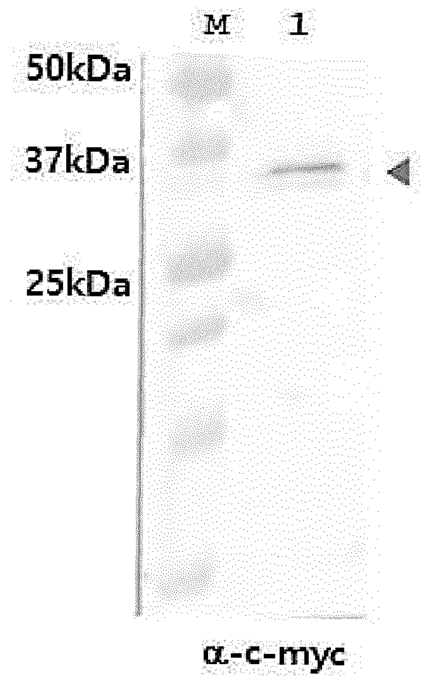
M
1
50kDa
37kDa
25kDa
α-c-myc

FIG. 47

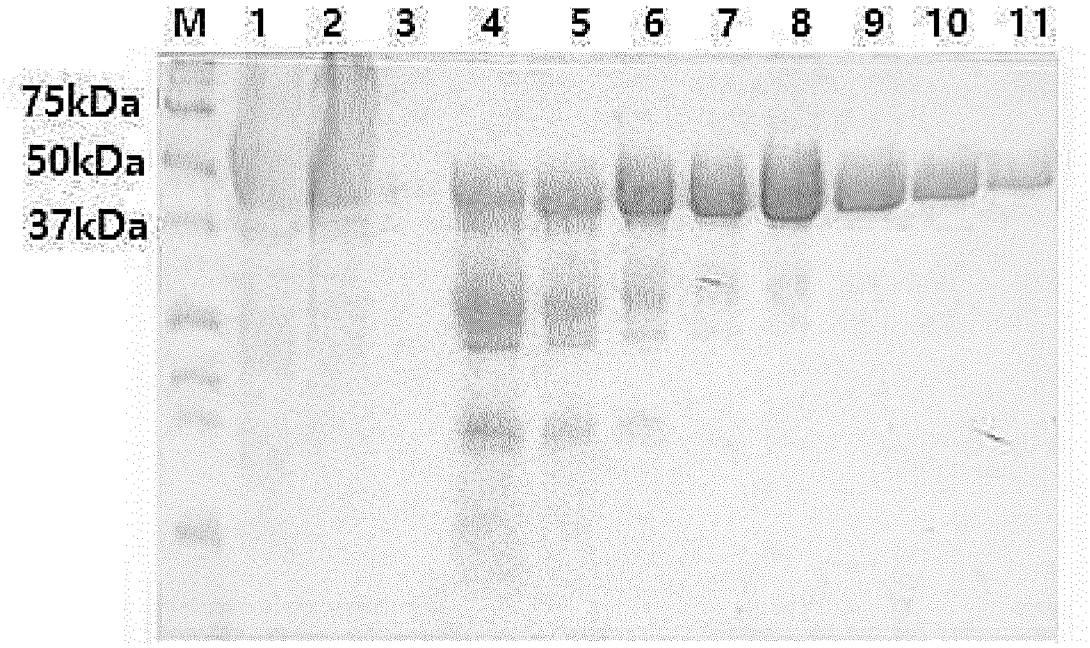
M 1 2 3 4 5 6 7 8 9 10 11
75kDa
50kDa
37kDa

FIG. 48

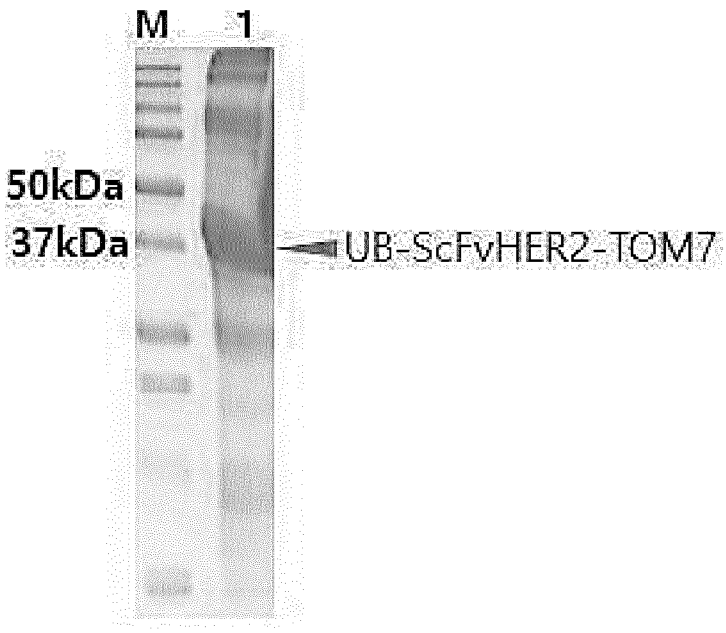
M 1
50kDa
37kDa
UB-ScFvHER2-TOM7

FIG. 49

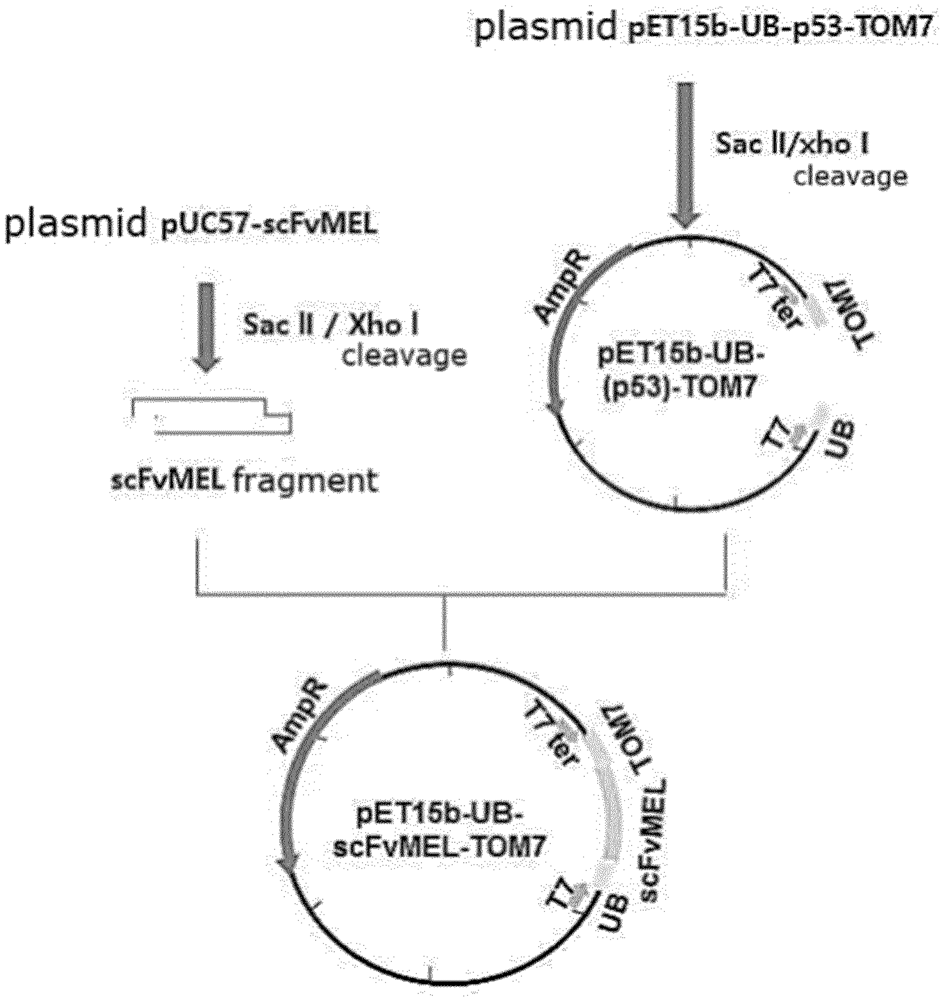
plasmid pET15b-UB-p53-TOM7
Sac II/xho I
cleavage
plasmid pUC57-scFvMEL
Sac II / Xho I
cleavage
scFvMEL fragment
AmpR
T7 ter
TOM7
pET15b-UB-
(p53)-TOM7
T7
UB
AmpR
T7 ter
TOM7
pET15b-UB-
scFvMEL-TOM7
scFvMEL
T7
UB

FIG. 50

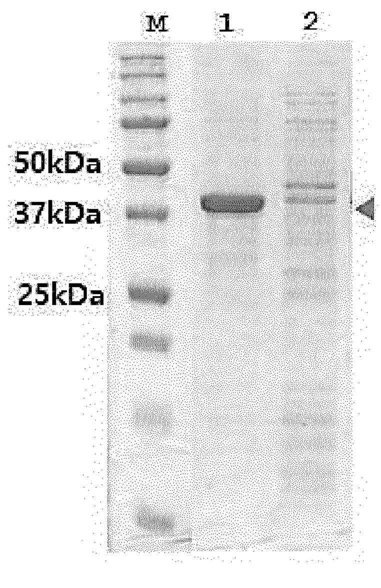
M
1
2
50kDa
37kDa
25kDa

FIG. 51

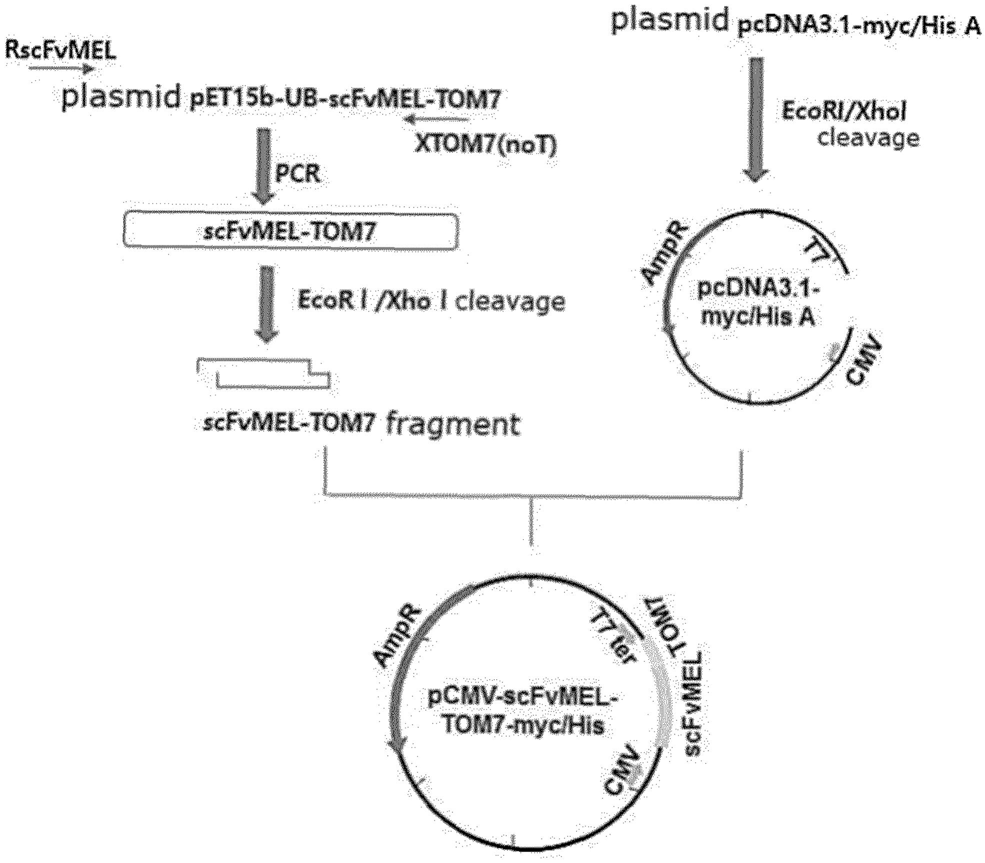
RscFvMEL
plasmid pET15b-UB-scFvMEL-TOM7
XTOM7(noT)
PCR
scFvMEL-TOM7
EcoR I /Xho I cleavage
scFvMEL-TOM7 fragment
plasmid pcDNA3.1-myc/His A
EcoRI/XhoI
cleavage
AmpR
T7
pcDNA3.1-
myc/His A
CMV
AmpR
T7 ter
TOM7
scFvMEL
pCMV-scFvMEL-
TOM7-myc/His
CMV

FIG. 52

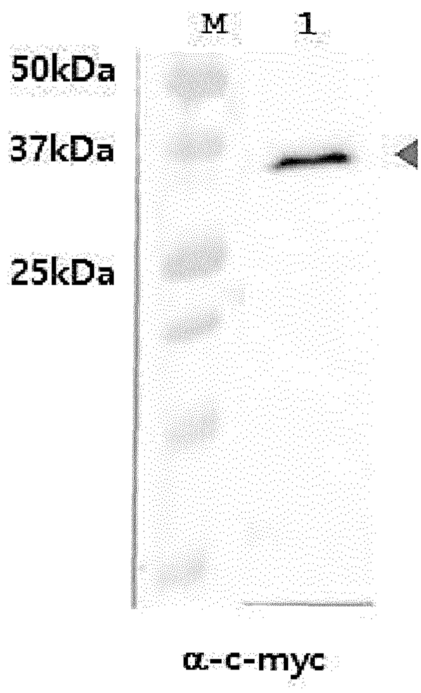
M
1
50kDa
37kDa
25kDa
α-c-myc

FIG. 53

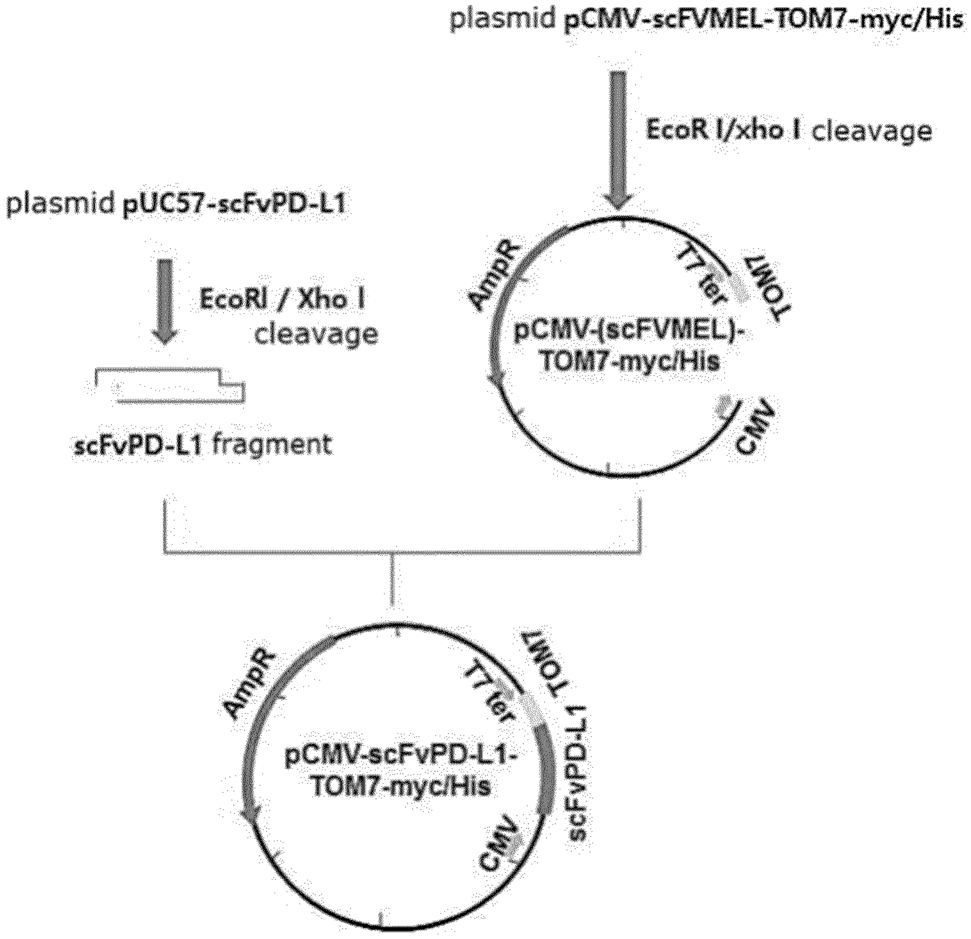
plasmid pCMV-scFVMEL-TOM7-myc/His
EcoR I/xho I cleavage
plasmid pUC57-scFvPD-L1
EcoRI / Xho I
cleavage
scFvPD-L1 fragment
AmpR
T7 ter
TOM7
pCMV-(scFVMEL)-
TOM7-myc/His
CMV
AmpR
T7 ter
TOM7
pCMV-scFvPD-L1-
TOM7-myc/His
scFvPD-L1
CMV

FIG. 54

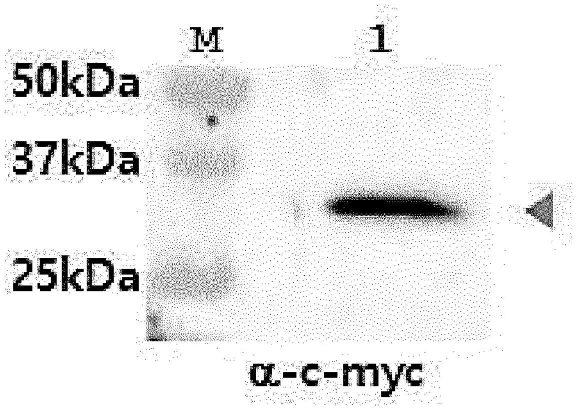
M
1
50kDa
37kDa
25kDa
α-c-myc

FIG. 55

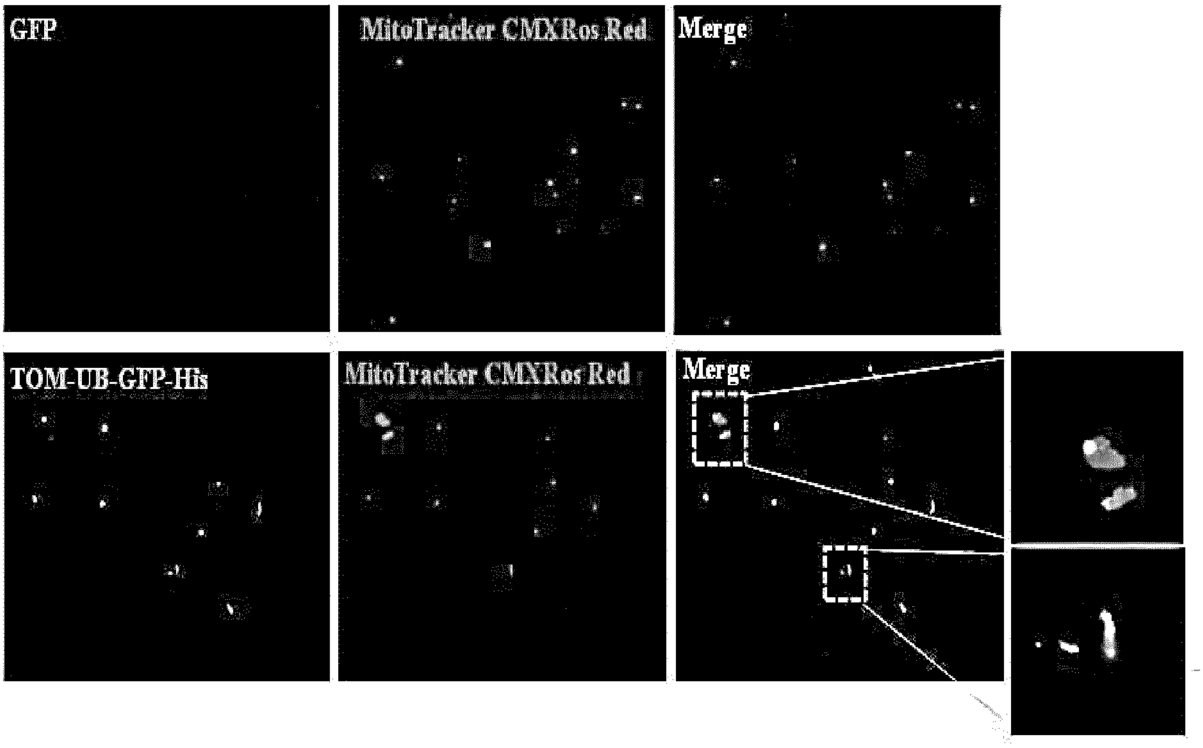
GFP
MitoTracker CMXRos Red
Merge
TOM-UB-GFP-His
MitoTracker CMXRos Red
Merge

FIG. 56

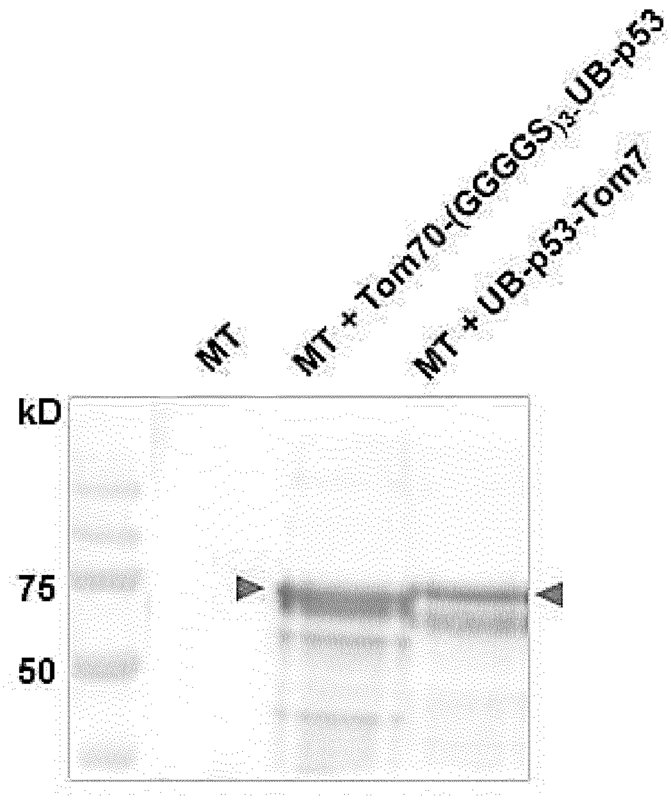
MT
MT + Tom70-(GGGGS)3-UB-p53
MT + UB-p53-Tom7
kD
75
50

FIG. 57

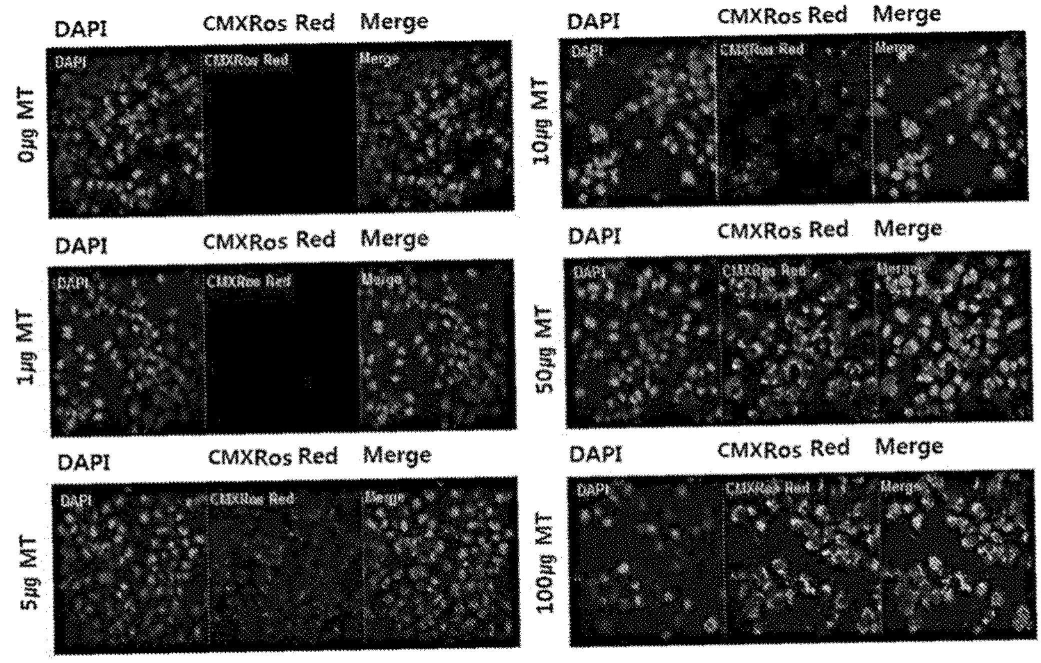
DAPI
CMXRos Red
Merge
0μg MT
1μg MT
5μg MT
10μg MT
50μg MT
100μg MT

FIG. 58

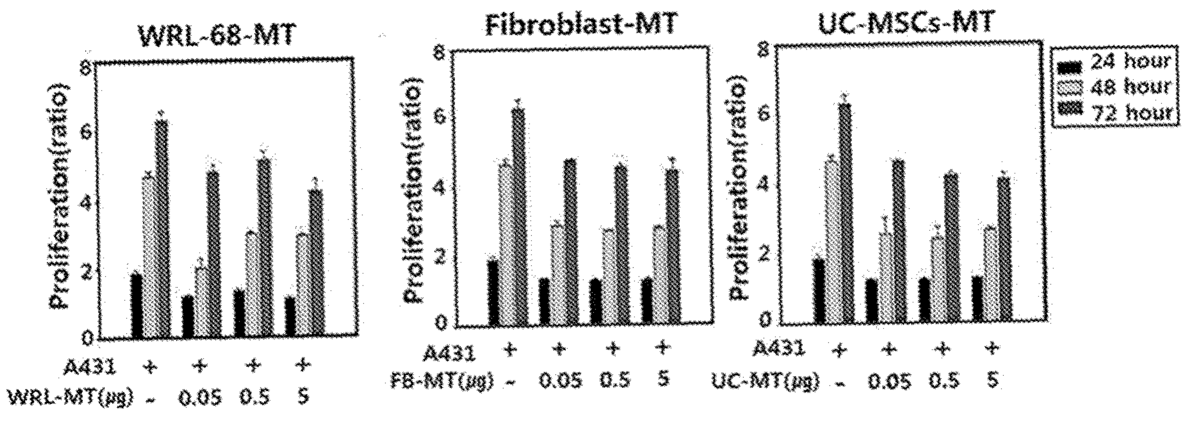
WRL-68-MT
Fibroblast-MT
UC-MSCs-MT
Proliferation(ratio)
24 hour
48 hour
72 hour
A431 + + + +
WRL-MT(μg) - 0.05 0.5 5
FB-MT(μg) - 0.05 0.5 5
UC-MT(μg) - 0.05 0.5 5

**FIG. 59**

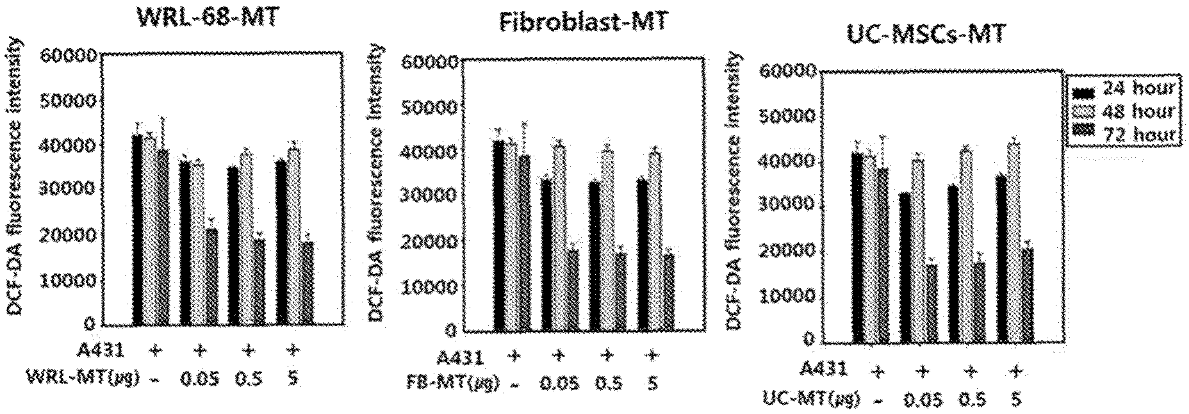
WRL-68-MT
Fibroblast-MT
UC-MSCs-MT
DCF-DA fluorescence intensity
24 hour
48 hour
72 hour
A431 + + + +
WRL-MT(μg) - 0.05 0.5 5
FB-MT(μg) - 0.05 0.5 5
UC-MT(μg) - 0.05 0.5 5

**FIG. 60**

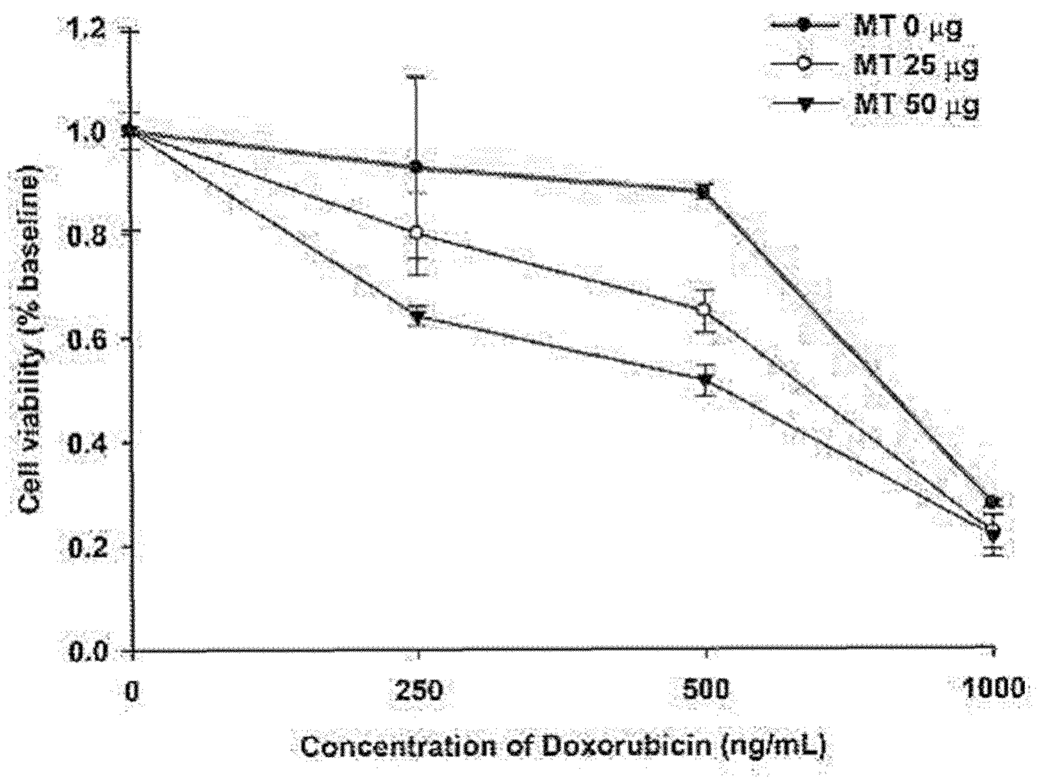
MT 0 μg
MT 25 μg
MT 50 μg
Cell viability (% baseline)
Concentration of Doxorubicin (ng/mL)

FIG. 61

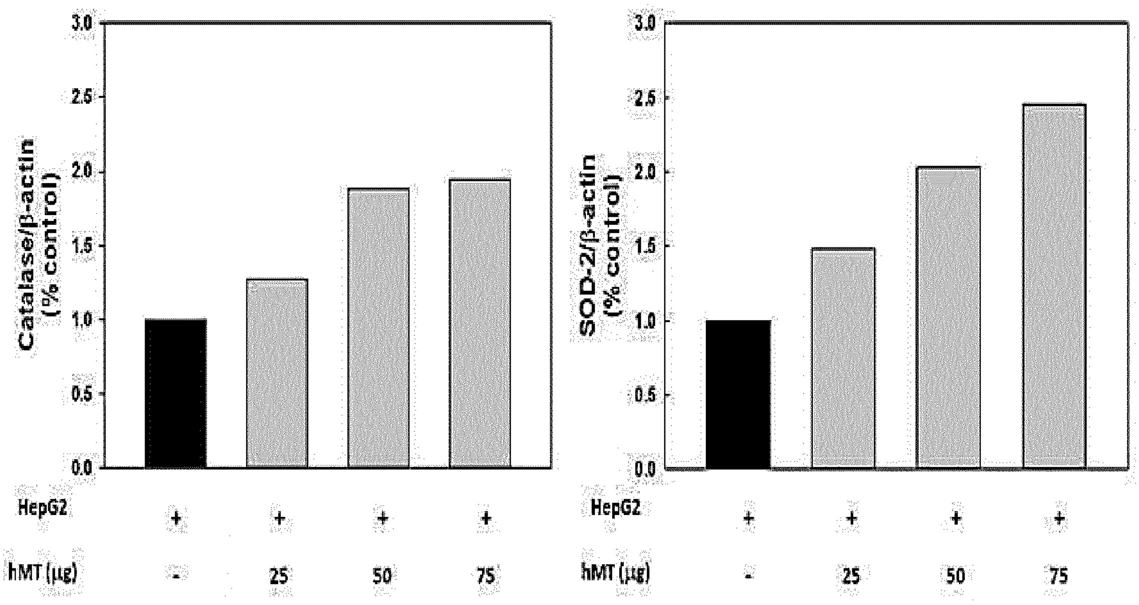
3.0
2.5
2.0
1.5
1.0
0.5
0.0
Catalase/β-actin
(% control)
SOD-2/β-actin
(% control)
HepG2
+
hMT (μg)
-
25
50
75

FIG. 62

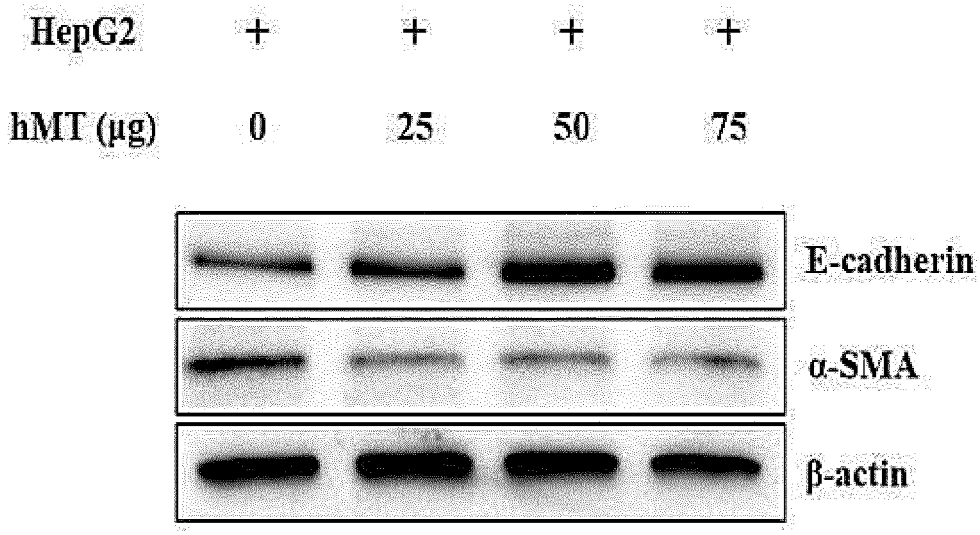
HepG2
+
+
+
+
hMT (μg)
0
25
50
75
E-cadherin
α-SMA
β-actin

# FIG. 63

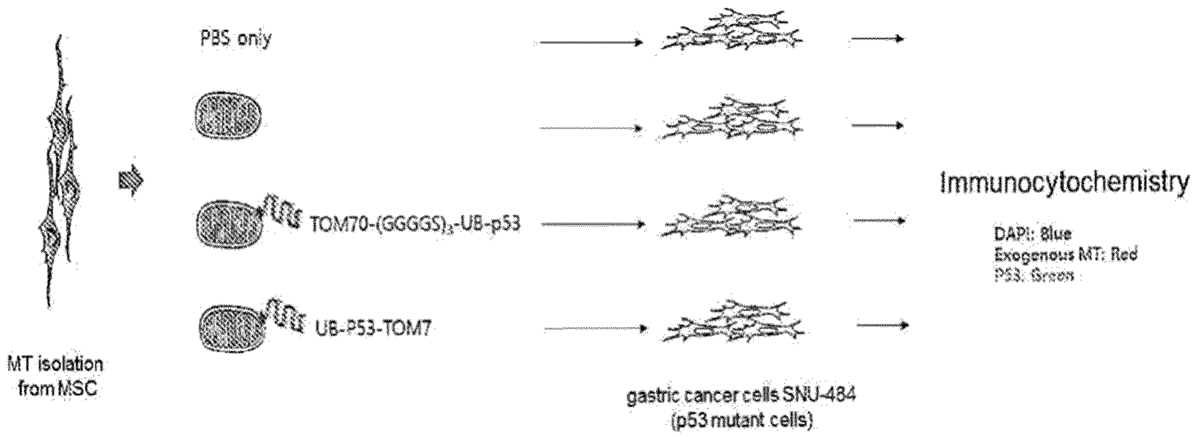
PBS only
TOM70-(GGGGS)3-UB-p53
UB-P53-TOM7
MT isolation from MSC
gastric cancer cells SNU-484 (p53 mutant cells)
Immunocytochemistry
DAPI: Blue
Exogenous MT: Red
P53: Green

# FIG. 64

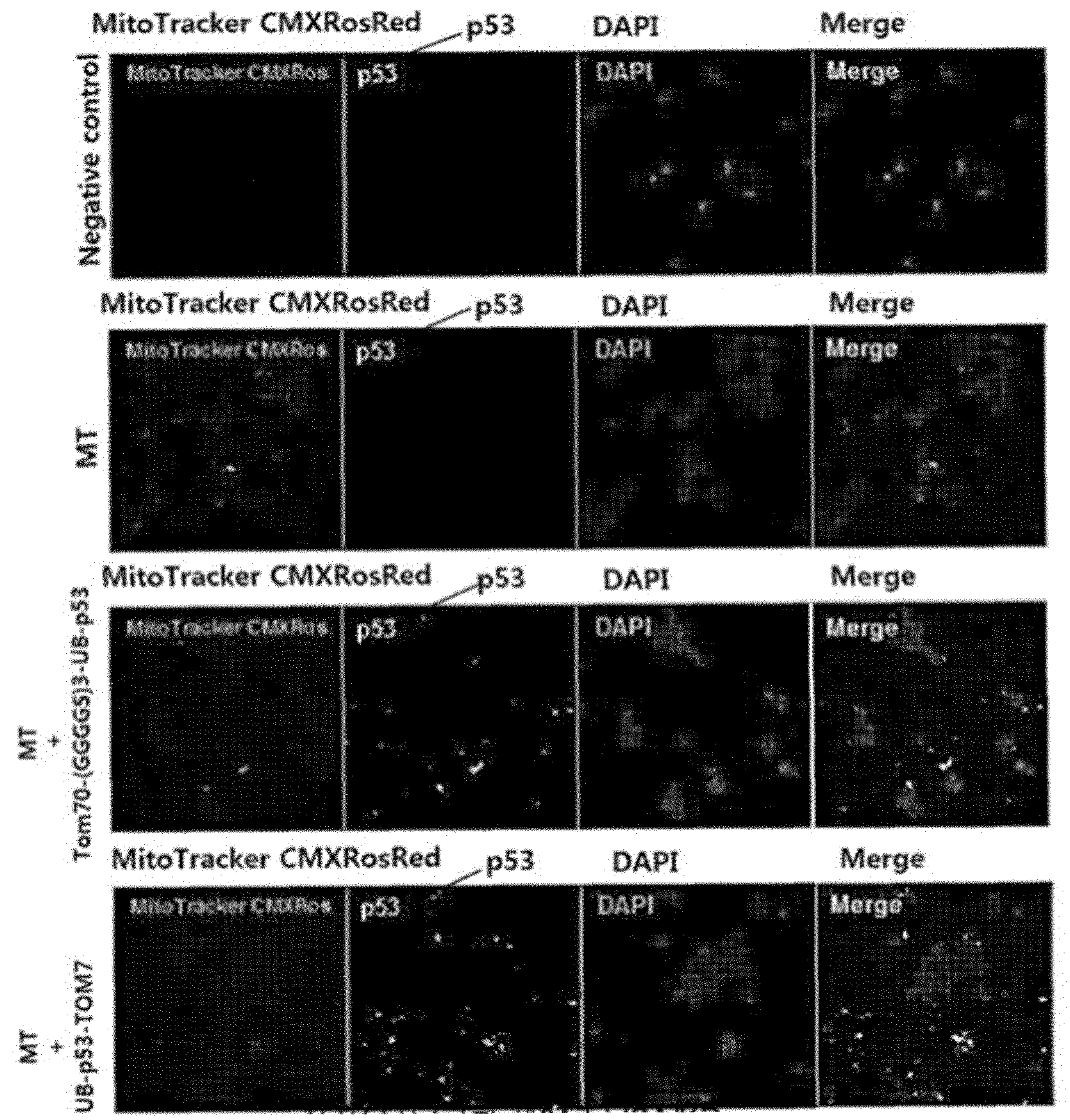
MitoTracker CMXRosRed
p53
DAPI
Merge
Negative control
MT
MT + Tom70-(GGGGS)3-UB-p53
MT + UB-p53-TOM7

FIG. 65

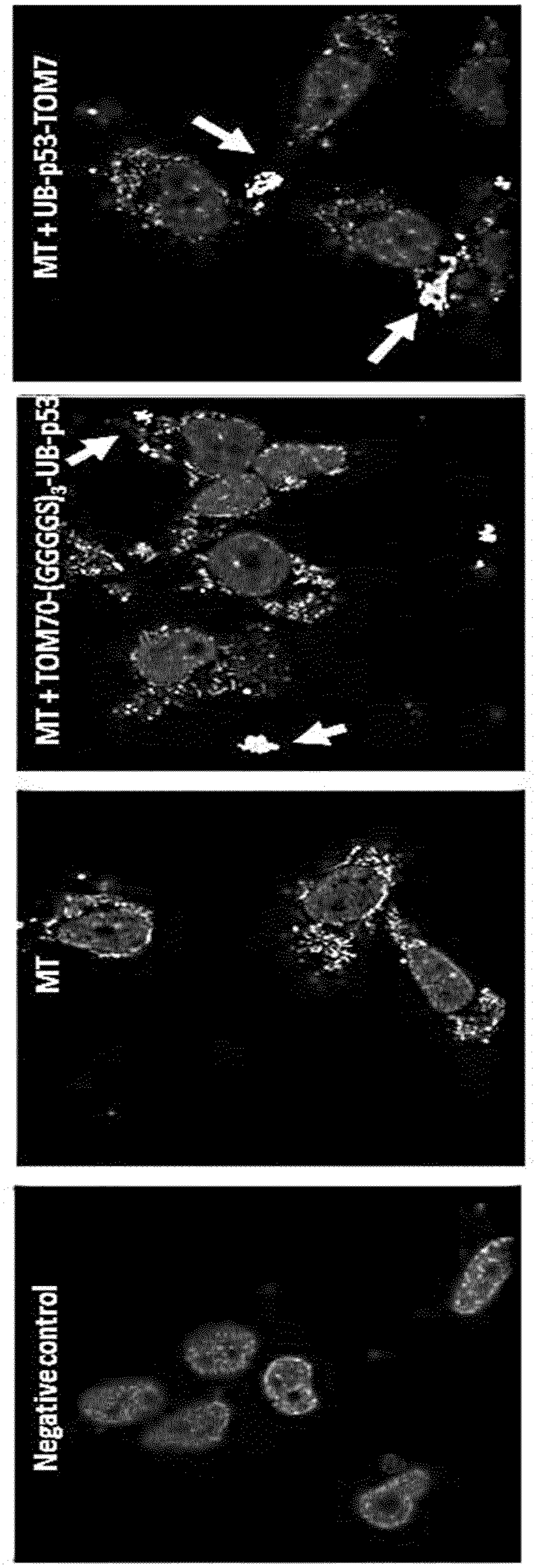

FIG. 66

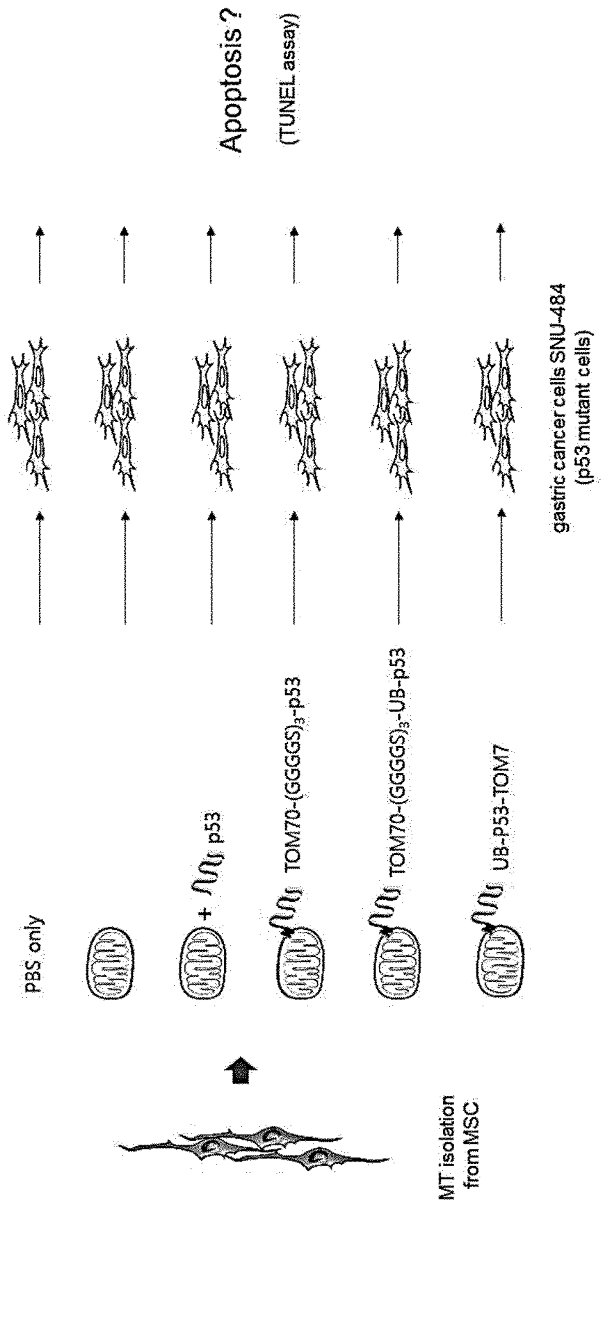
MT isolation
from MSC
PBS only
+ p53
TOM70-(GGGGS)$_3$-p53
TOM70-(GGGGS)$_3$-UB-p53
UB-P53-TOM7
gastric cancer cells SNU-484
(p53 mutant cells)
Apoptosis ?
(TUNEL assay)

FIG. 67a

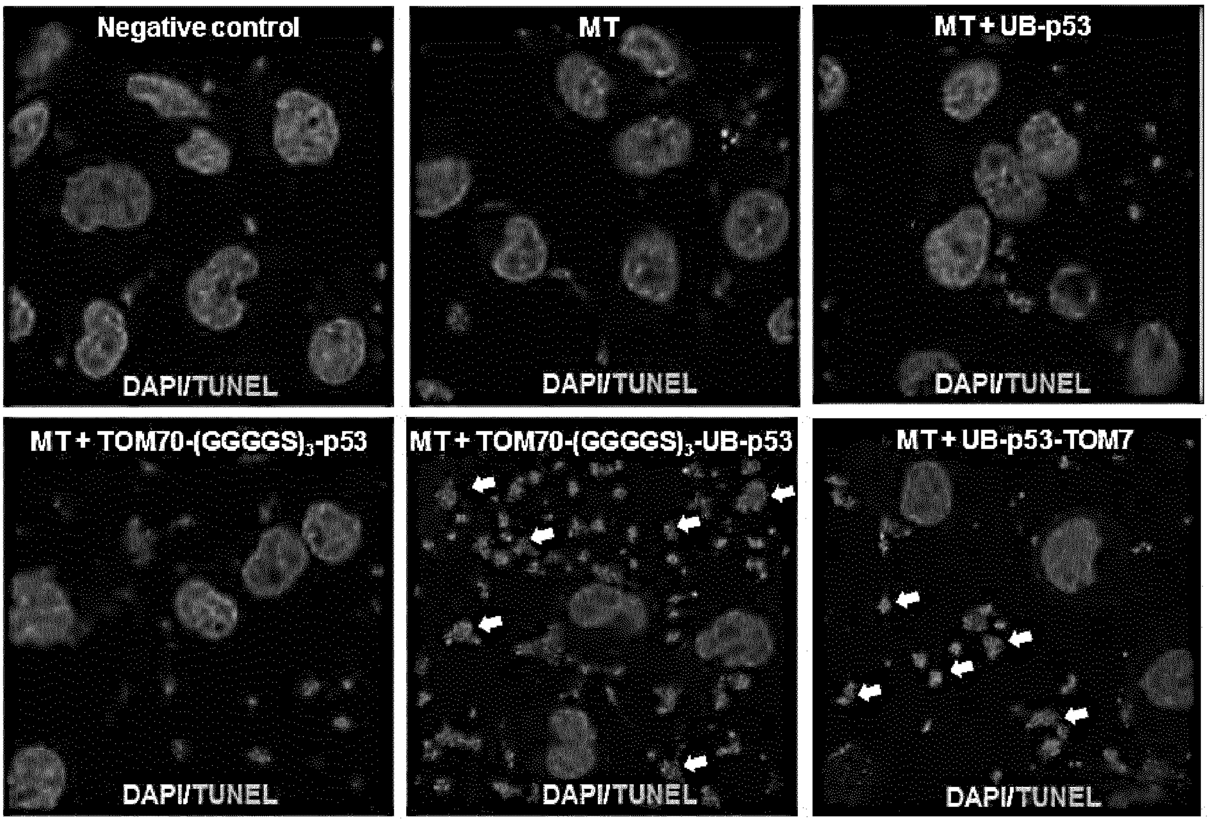


FIG. 67b

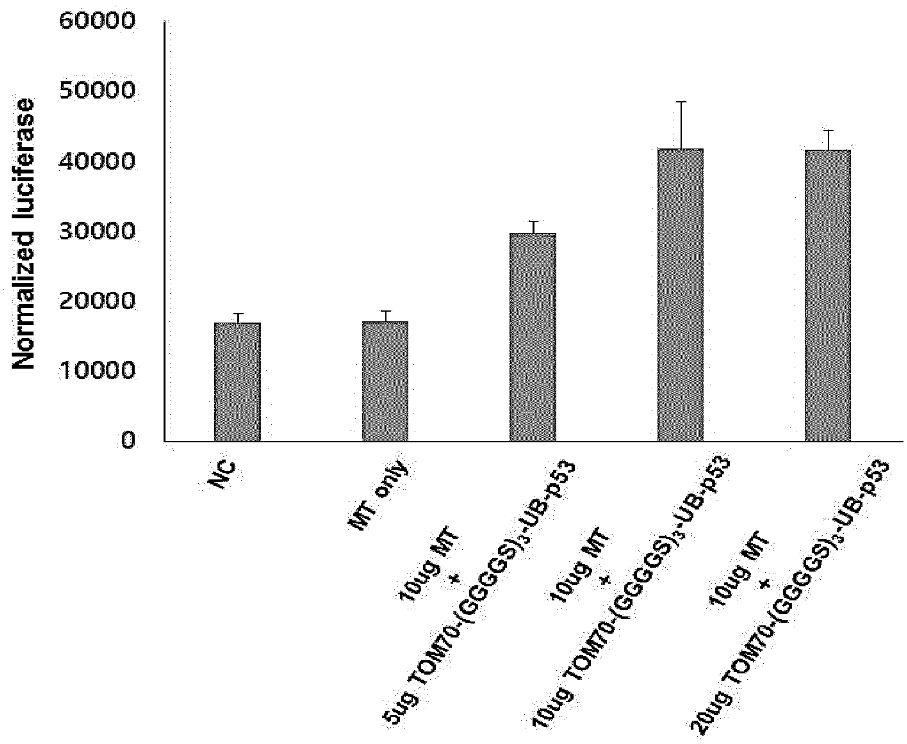

FIG. 68

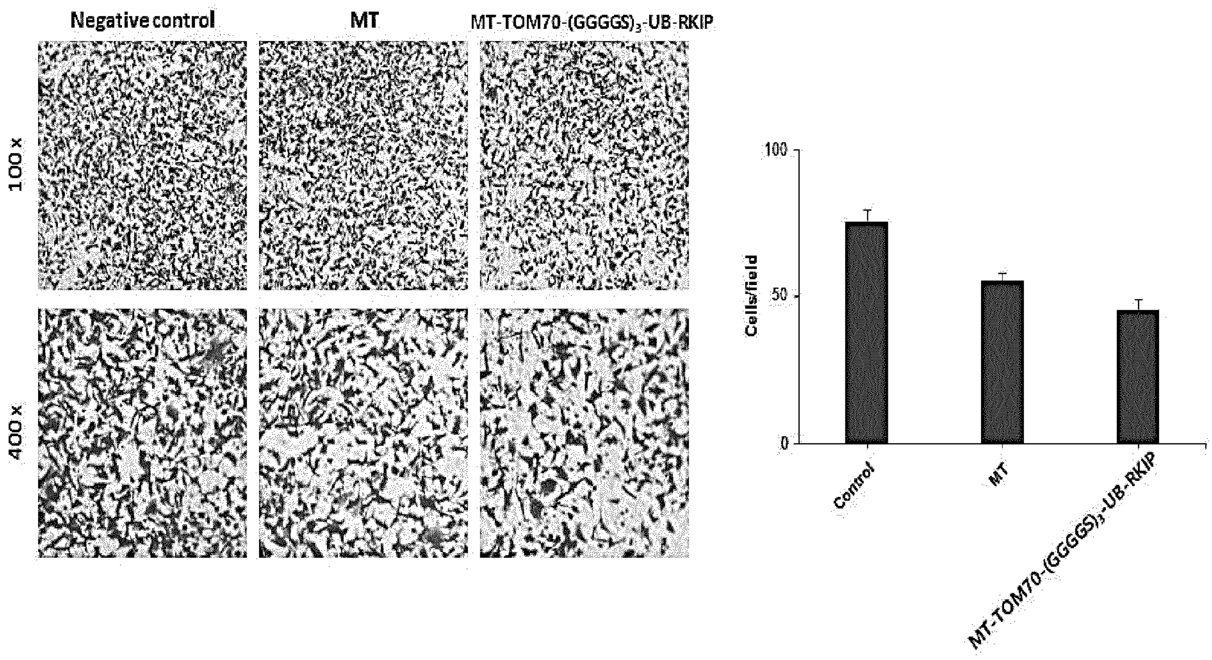
Negative control
MT
MT-TOM70-(GGGGS)3-UB-RKIP
100 x
400 x
100
50
0
Cells/field
Control
MT
MT-TOM70-(GGGGS)3-UB-RKIP

FIG. 69

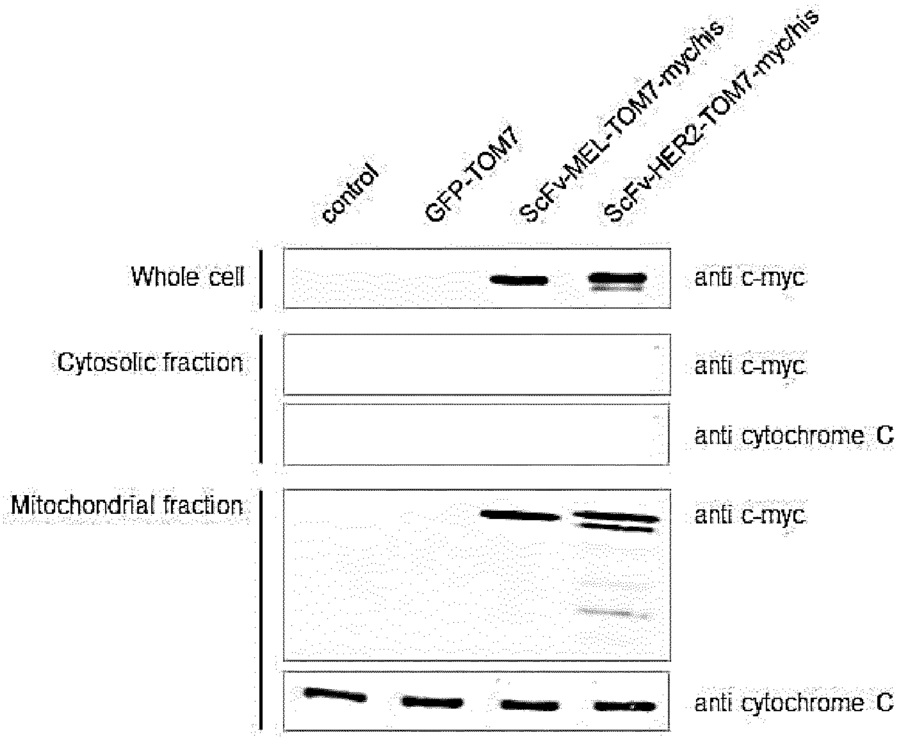
control
GFP-TOM7
ScFv-MEL-TOM7-myc/his
ScFv-HER2-TOM7-myc/his
Whole cell
anti c-myc
Cytosolic fraction
anti c-myc
anti cytochrome C
Mitochondrial fraction
anti c-myc
anti cytochrome C

FIG. 70

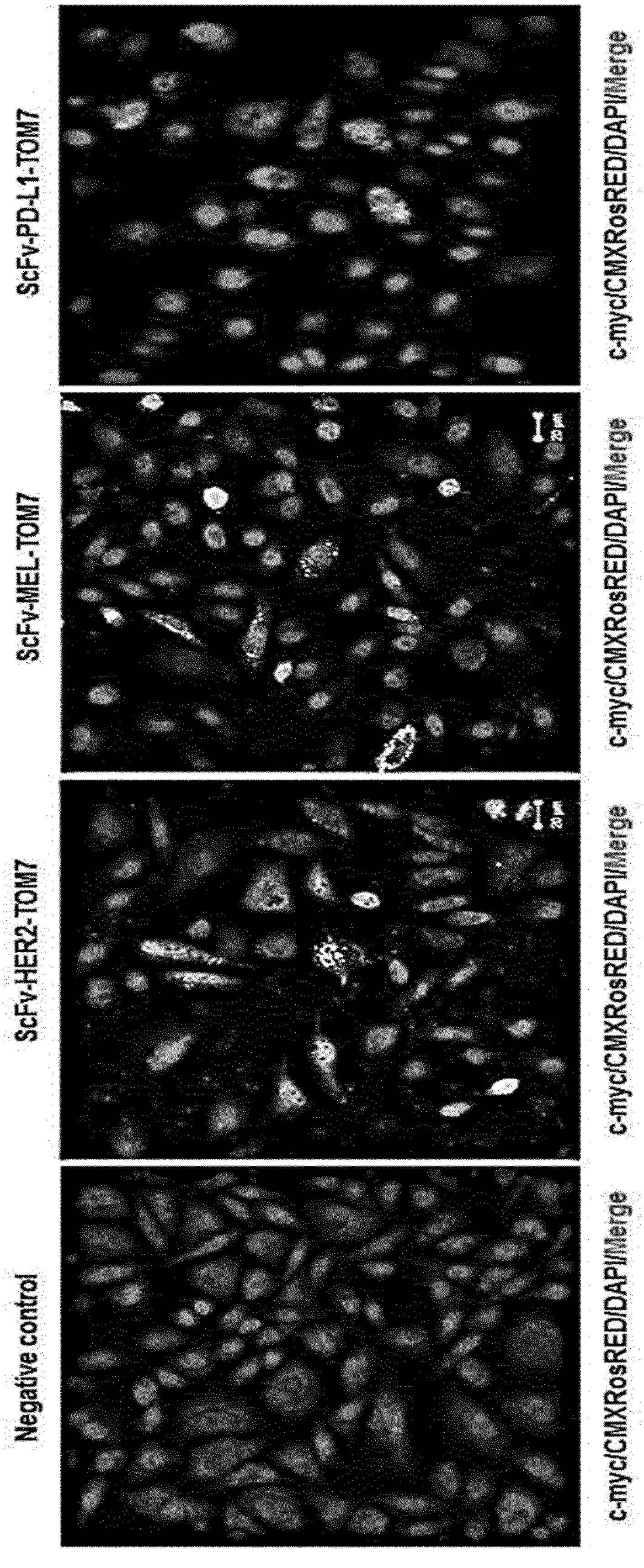
ScFv-PD-L1-TOM7
ScFv-MEL-TOM7
ScFv-HER2-TOM7
Negative control
c-myc/CMXRosRED/DAPI/Merge
c-myc/CMXRosRED/DAPI/Merge
c-myc/CMXRosRED/DAPI/Merge
c-myc/CMXRosRED/DAPI/Merge
20 μm
20 μm

FIG. 71

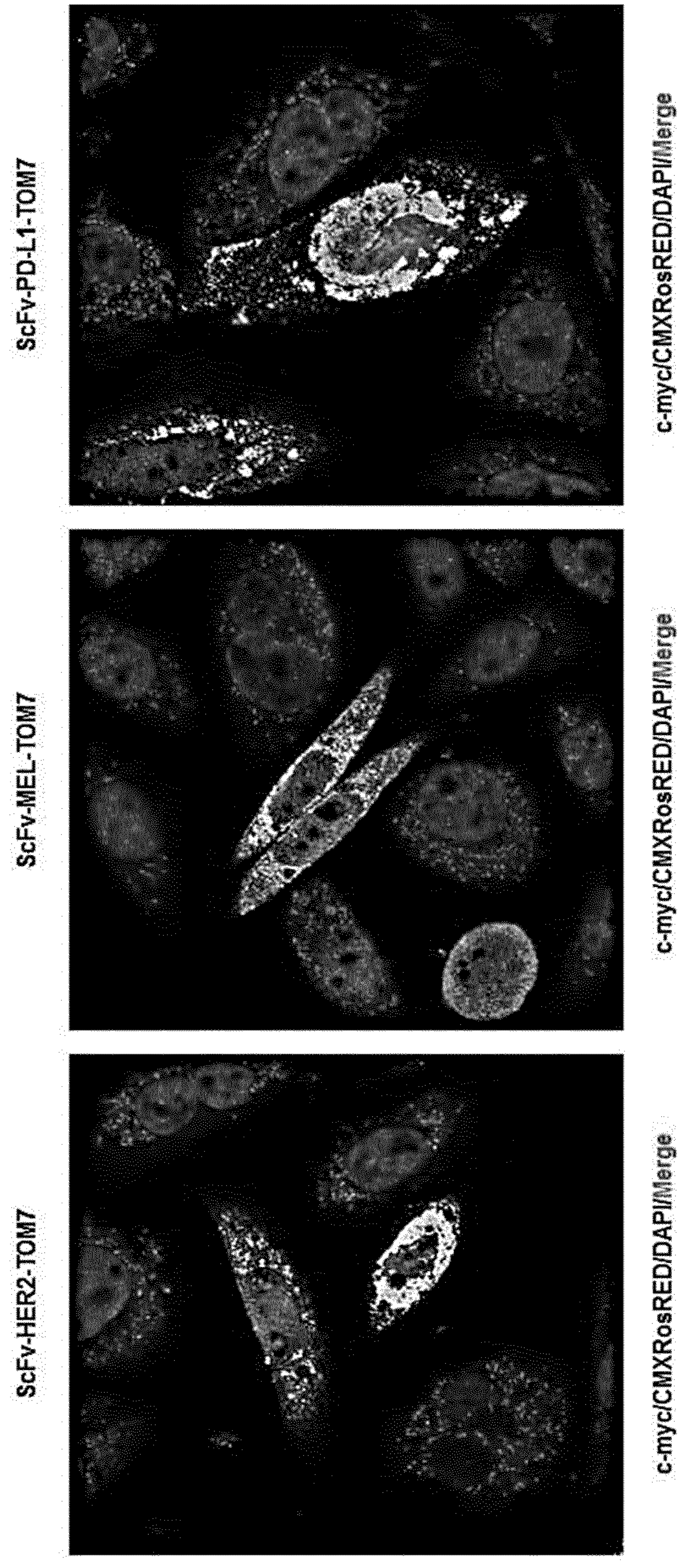
ScFv-PD-L1-TOM7
c-myc/CMXRosRED/DAPI/Merge
ScFv-MEL-TOM7
c-myc/CMXRosRED/DAPI/Merge
ScFv-HER2-TOM7
c-myc/CMXRosRED/DAPI/Merge

FIG. 72

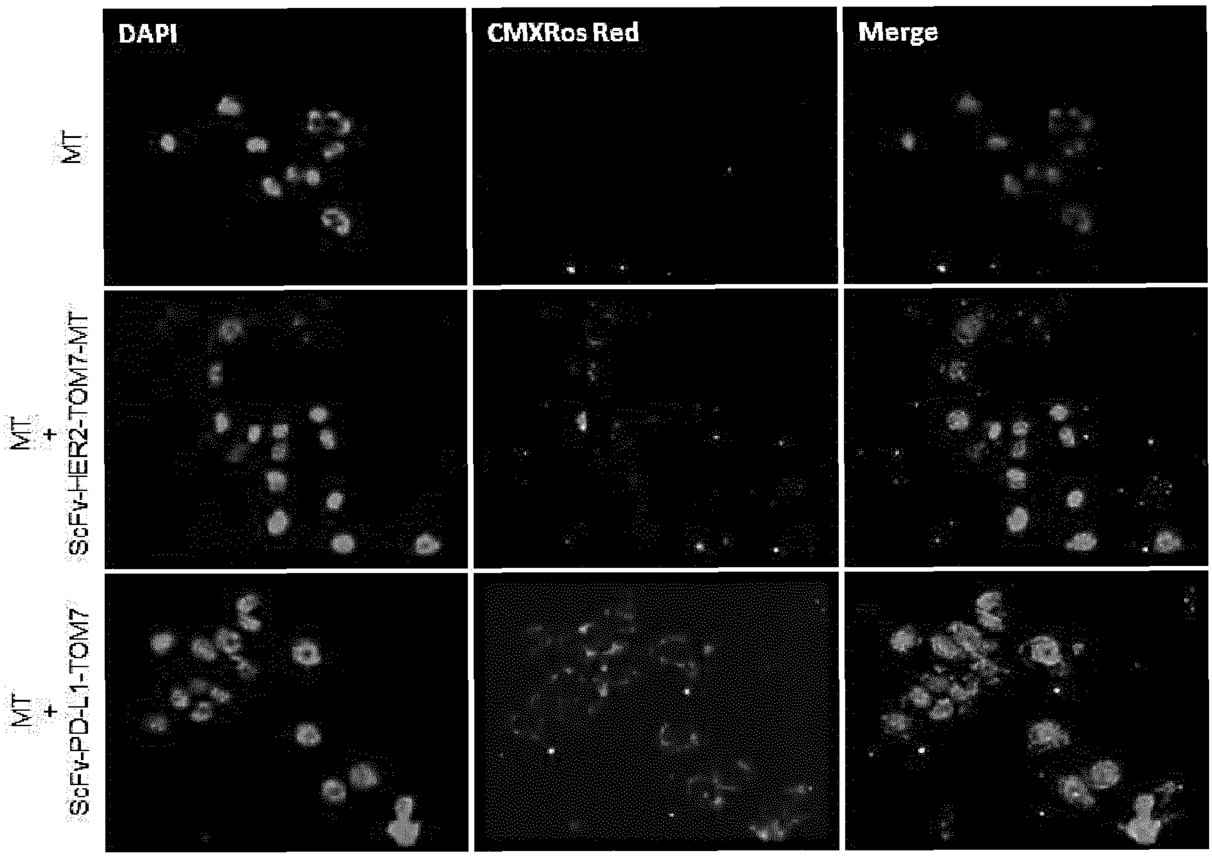
DAPI
CMXRos Red
Merge
MT
MT + ScFv-HER2-TOM7-MT
MT + ScFv-PD-L1-TOM7

FIG. 73

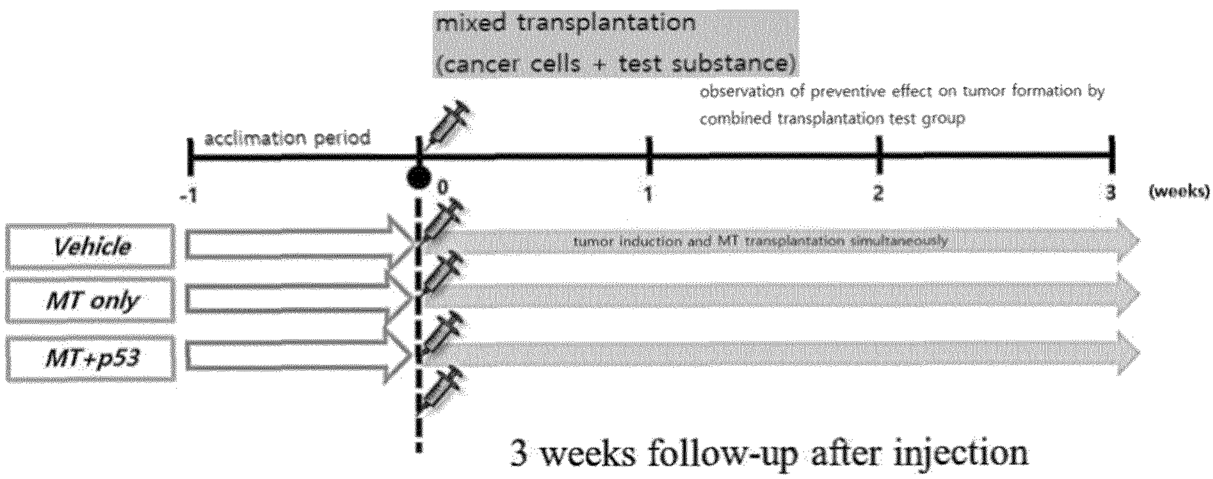
mixed transplantation
(cancer cells + test substance)
observation of preventive effect on tumor formation by combined transplantation test group
acclimation period
-1
0
1
2
3
(weeks)
Vehicle
MT only
MT+p53
tumor induction and MT transplantation simultaneously
3 weeks follow-up after injection

FIG. 74

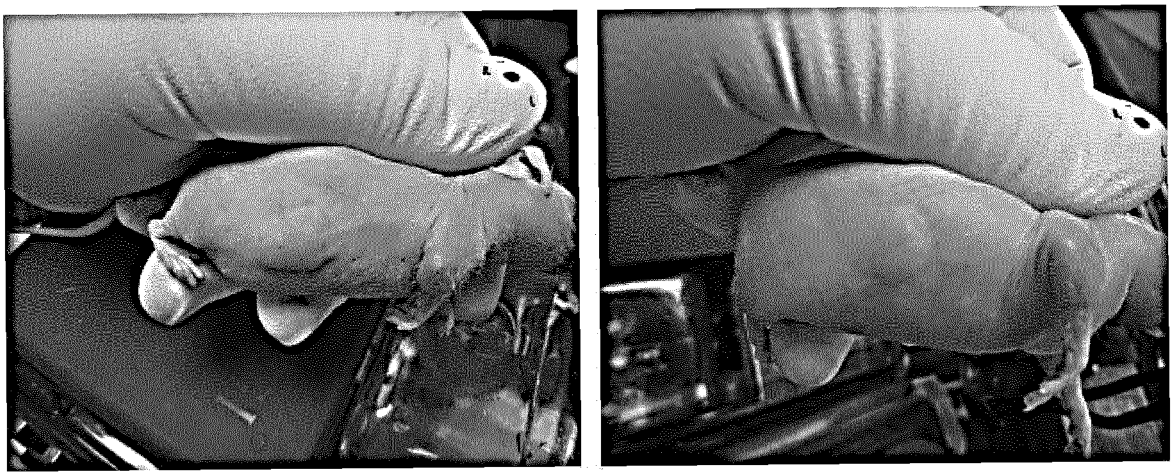

FIG. 75

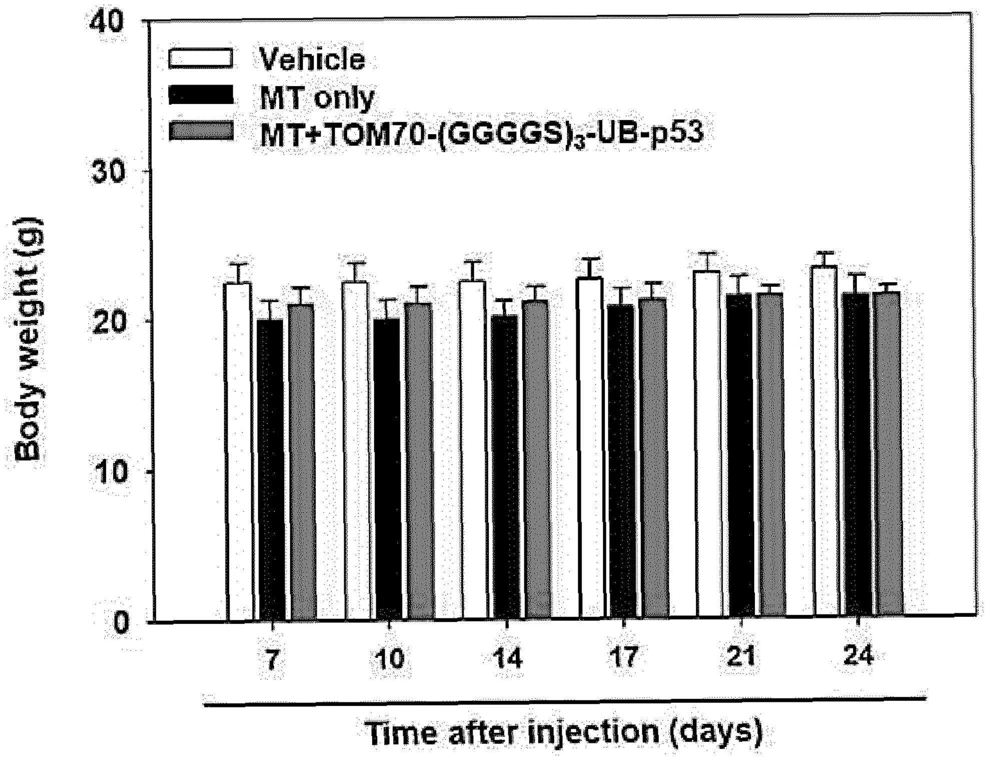
40
30
20
10
0
Body weight (g)
Vehicle
MT only
MT+TOM70-(GGGGS)$_3$-UB-p53
7
10
14
17
21
24
Time after injection (days)

FIG. 76

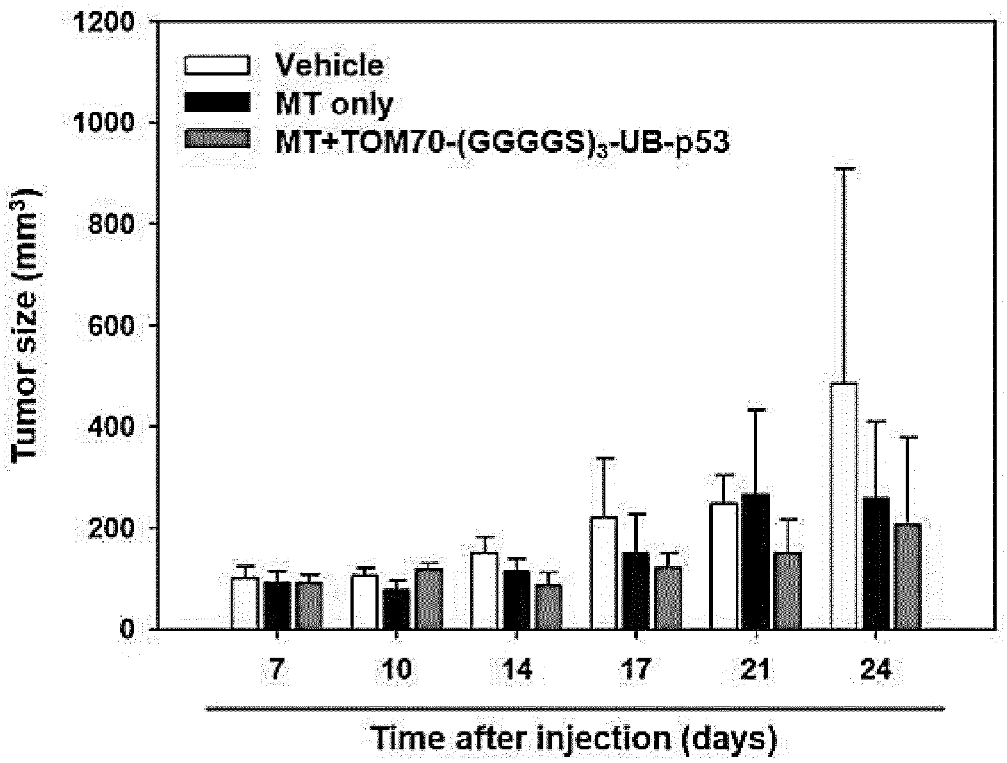
Vehicle
MT only
MT+TOM70-(GGGGS)3-UB-p53
Tumor size (mm3)
1200
1000
800
600
400
200
0
7
10
14
17
21
24
Time after injection (days)

FIG. 77

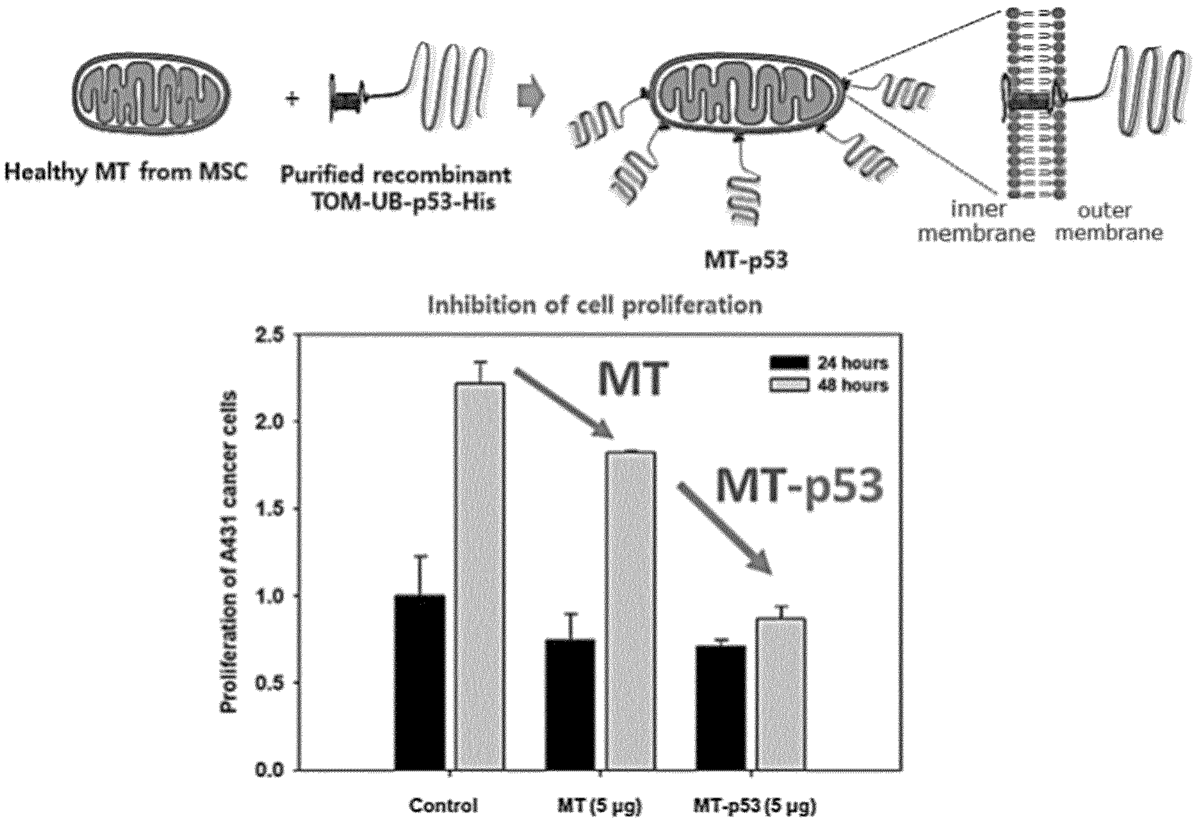
Healthy MT from MSC
+
Purified recombinant
TOM-UB-p53-His
MT-p53
inner membrane
outer membrane
Inhibition of cell proliferation
Proliferation of A431 cancer cells
2.5
2.0
1.5
1.0
0.5
0.0
24 hours
48 hours
MT
MT-p53
Control
MT (5 μg)
MT-p53 (5 μg)

FIG. 78

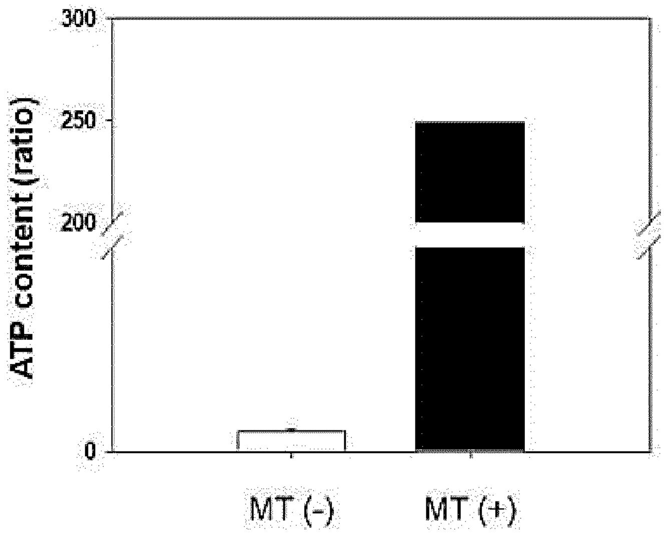
300
250
200
0
ATP content (ratio)
MT (-)
MT (+)

FIG. 79

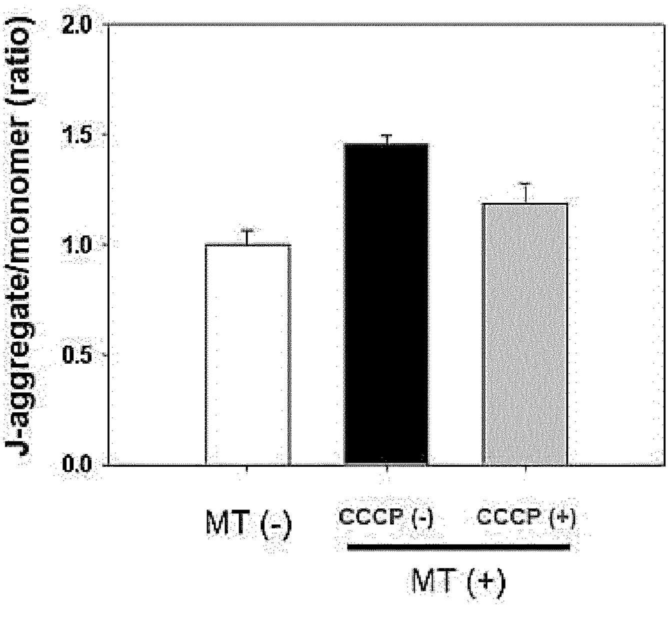
2.0
1.5
1.0
0.5
0.0
J-aggregate/monomer (ratio)
MT (-)
CCCP (-)
CCCP (+)
MT (+)

FIG. 80

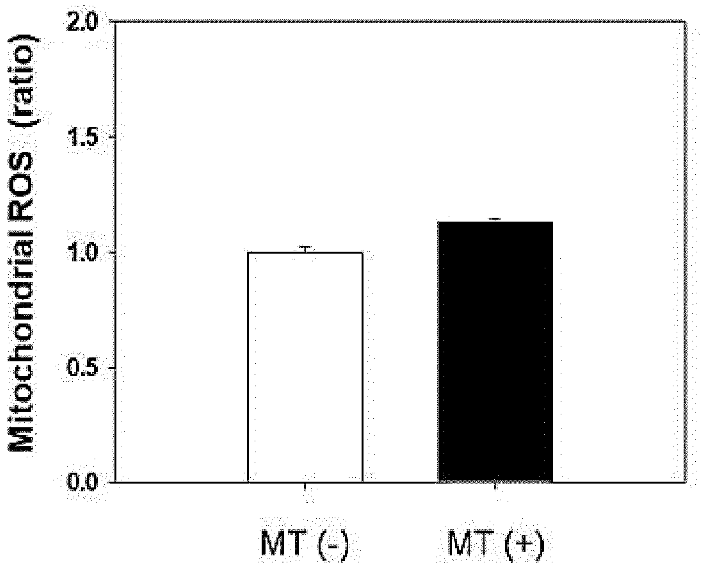


FIG. 81a

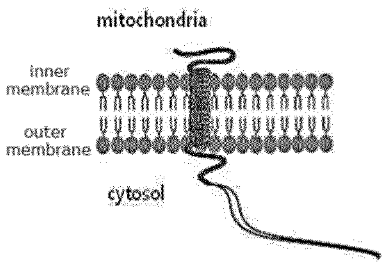


| | MT signal seq | MT outer membrane anchoring seq | |
|---|---|---|---|
| TOM70 | MKSFITRNK | TAILATVAATGTATGAYYYY | NQLQQQQQRGK...... |
| TOM20 | MSQSNPILR | GLAITTAIAALSATGYAIYF | DYQRRNSPQFR...... |
| OM45 | MSSR | IIVGSAALAAAITASIMV | REQKAKGQRREGNVSAYY...... |

FIG. 81b

| | | MT outer membrane anchoring seq | MT signal seq |
|---|---|---|---|
| TOM5 | ......REDVISS | IRNFLIYVALLRVTPFIL | KKLDSI |
| TOM7 | .........LFKGSQ | FAIRWGFIPLVIYLGF | KRGADPGMPEPTVLSLLWG |
| Fis1 | ......IQKETLK | GVVVAGGVLAGAVAVASFFL | RNKRR |
| VAMP1B | ............WKNCK | MIMLGAICAIIVVVIV | RRD |
| Cyt*b*5 | .........KNDTCK | SCWAYWILPIIGAVLLGFLY | RYYTSESKSS |
| BCL-2 | ......FSWLSLK | TLLSLALVGACITLGAYLG | HK |
| Bcl-x | ......GQERFNR | WFLTGMTVAGVVLLGSLFS | RK |

FIG. 82

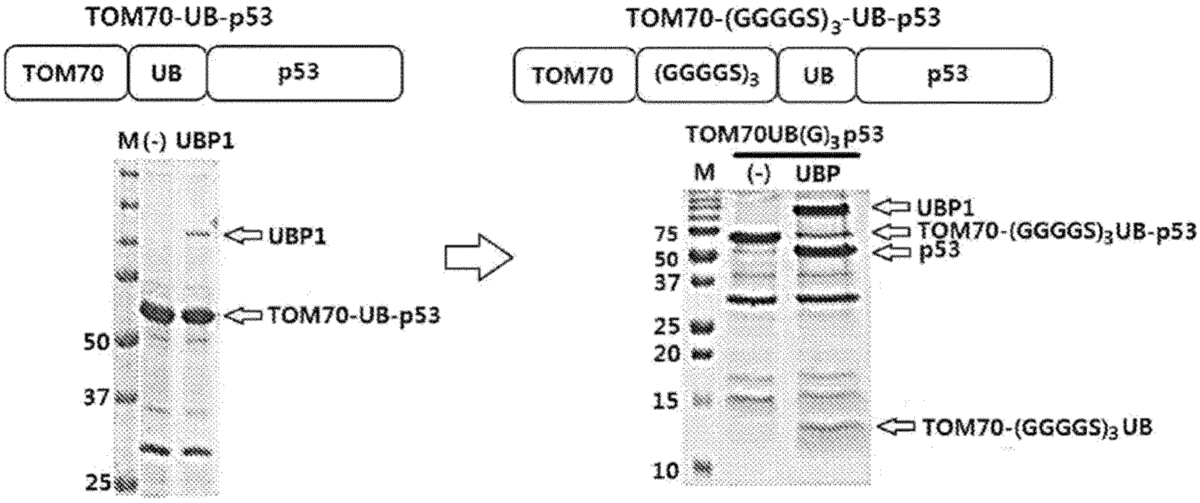
TOM70-UB-p53
TOM70
UB
p53
M (-) UBP1
UBP1
TOM70-UB-p53
50
37
25
TOM70-(GGGGS)$_3$-UB-p53
TOM70
(GGGGS)$_3$
UB
p53
TOM70UB(G)$_3$p53
M (-) UBP
75
50
37
25
20
15
10
UBP1
TOM70-(GGGGS)$_3$UB-p53
p53
TOM70-(GGGGS)$_3$ UB

FIG. 83

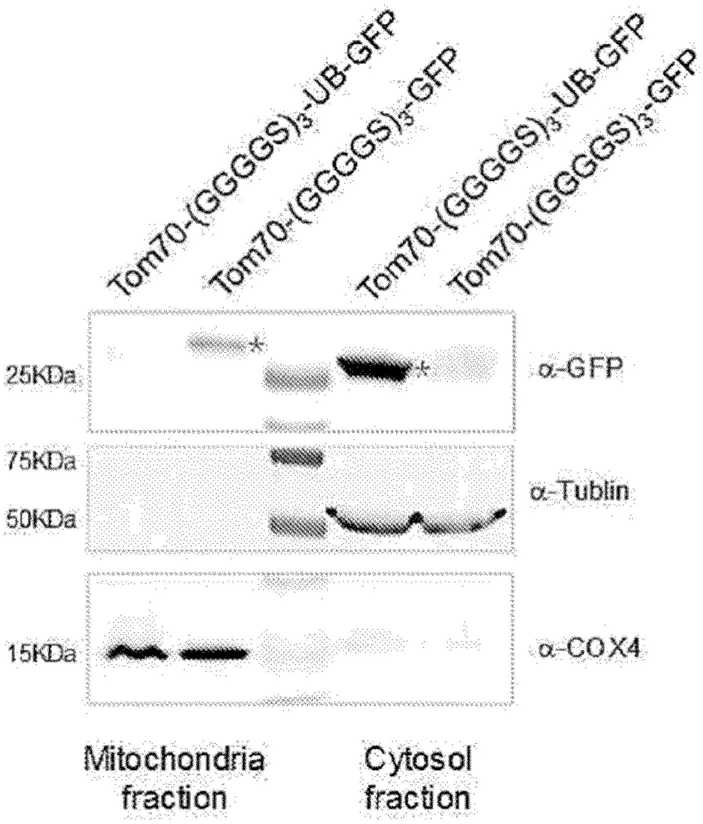
Tom70-(GGGGS)$_3$-UB-GFP
Tom70-(GGGGS)$_3$-GFP
Tom70-(GGGGS)$_3$-UB-GFP
Tom70-(GGGGS)$_3$-GFP
25KDa
75KDa
50KDa
15KDa
α-GFP
α-Tublin
α-COX4
Mitochondria fraction
Cytosol fraction

## INTERNATIONAL SEARCH REPORT

International application No. **PCT/KR2019/005020**

A. CLASSIFICATION OF SUBJECT MATTER

*C07K 14/705(2006.01)i, C07K 14/395(2006.01)i, C07K 14/47(2006.01)i, C07K 16/28(2006.01)i, C12N 5/00(2006.01)i, A61K 35/12(2006.01)i*

According to International Patent Classification (IPC) or to both national classification and IPC

B. FIELDS SEARCHED

Minimum documentation searched (classification system followed by classification symbols)

C07K 14/705; A61K 38/17; A61K 47/48; C12Q 1/68; G01N 33/567; C07K 14/395; C07K 14/47; C07K 16/28; C12N 5/00; A61K 35/12

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: mitochondria, outer membrane, anchoring peptide, target protein, fusion protein, ubiquitin, cancer

C. DOCUMENTS CONSIDERED TO BE RELEVANT

| Category* | Citation of document, with indication, where appropriate, of the relevant passages | Relevant to claim No. |
|---|---|---|
| X<br><br><br>Y | MOSSALAM, M. et al. Direct Induction of Apoptosis Using an Optimal Mitochondrially Targeted p53. Molecular Pharmaceutics. 2012, vol. 9, pages 1449-1458<br>See abstract; pages 1450, 1451, 1456; and table 1. | 1-16,23-27,31-41 ,44-51<br><br><br>17-22,28-30,42,43 |
| X | NGUYEN, Mai et al. Targeting of Bcl-2 to the Mitochondrial Outer Membrane by a COOH-terminal Signal Anchor Sequence. Journal of Biological Chemistry. 1993, vol. 268, no. 34, pages 25265-25268<br>See page 25267; and figure 2. | 1-9,11-13,25-27 ,36-38,41,44,45 |
| Y | US 2005-0084864 A1 (ROSSNER, Moritz et al.) 21 April 2005<br>See paragraphs [0023], [0178], [0179]. | 17-22,42,43 |
| Y | NCBI Reference Sequence: XP_004446139.1: polyubiquitin, variant [Coccidioides immitis RS]. (10 October 2017)<br>See the entire document. | 18 |
| Y | WO 2013-026015 A1 (DANA-FARBER CANCER INSTITUTE, INC. et al.) 21 February 2013<br>See figure 3; and claims 1, 12. | 20-22,28-30 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 AUGUST 2019 (08.08.2019) | **08 AUGUST 2019 (08.08.2019)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

INTERNATIONAL SEARCH REPORT

International application No.
PCT/KR2019/005020

C (Continuation). DOCUMENTS CONSIDERED TO BE RELEVANT

| Category* | Citation of document, with indication, where appropriate, of the relevant passages | Relevant to claim No. |
|---|---|---|
| A | PARK, Sungwoo et al. Targeting by AutophaGy proteins (TAG): targeting of IFNG-inducible GTPases to membranes by the LC3 conjugation system of autophagy. Autophagy. 2016, vol. 12, no. 7, pages 1153-1167 See page 1159; and figure 6A. | 1-51 |
| A | CN 104357470 B (UNIV SICHUAN WEST CHINA 2ND UNIV HOSPITAL) 15 February 2017 See abstract; and paragraph [0005]. | 1-51 |

Form PCT/ISA/210 (continuation of second sheet) (January 2015)

INTERNATIONAL SEARCH REPORT

International application No.
**PCT/KR2019/005020**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☒ Claims Nos.: **52, 53**
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
**PCT/KR2019/005020**

| Patent document cited in search report | Publication date | Patent family member | Publication date |
|---|---|---|---|
| US 2005-0084864 A1 | 21/04/2005 | AT 483971 T | 15/10/2010 |
| | | AU 2003-215660 A1 | 22/09/2003 |
| | | DE 10211063 A1 | 09/10/2003 |
| | | DK 1483576 T3 | 31/01/2011 |
| | | DK 2110666 T3 | 08/04/2013 |
| | | EP 1483576 A2 | 08/12/2004 |
| | | EP 1483576 B1 | 06/10/2010 |
| | | EP 2110666 A1 | 21/10/2009 |
| | | EP 2110666 B1 | 26/12/2012 |
| | | US 2009-0298089 A1 | 03/12/2009 |
| | | US 7585635 B2 | 08/09/2009 |
| | | US 8349619 B2 | 08/01/2013 |
| | | WO 03-076932 A2 | 18/09/2003 |
| WO 2013-026015 A1 | 21/02/2013 | US 2013-0101664 A1 | 25/04/2013 |
| CN 104357470 B | 15/02/2017 | CN 104357470 A | 18/02/2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020170151526

### Non-patent literature cited in the description


- **JAMES D. MCCULLY.** *J Vis Exp.,* 2014, vol. 91, 51682
- *CHEMICAL ABSTRACTS,* 555-60-2